(19) Europäisches Patentamt
European Patent Office
Office européen des brevets

(11) EP 1 115 695 B1

(12) EUROPEAN PATENT SPECIFICATION

(45) Date of publication and mention of the grant of the patent:
**01.06.2005 Bulletin 2005/22**

(21) Application number: **99948429.8**

(22) Date of filing: **23.09.1999**

(51) Int Cl.[7]: **C07C 217/90**, C07C 217/84, C07D 263/06, C07D 251/16, A61K 31/135, A61K 31/33, C07C 217/82, C07C 217/86, C07C 239/20

(86) International application number:
**PCT/US1999/022120**

(87) International publication number:
**WO 2000/018724 (06.04.2000 Gazette 2000/14)**

(54) **(R)-CHIRAL HALOGENATED 1-SUBSTITUTEDAMINO-(N+1)-ALKANOLS USEFUL FOR INHIBITING CHOLESTERYL ESTER TRANSFER PROTEIN ACTIVITY**

(R)-CHIRALE HALOGENIERTE 1-SUBSTITUIERTE AMINO-(N+1)-ALKANOLEN FÜR DIE HEMMUNG DER AKTIVITÄT DES CHOLESTERYL-ESTER-TRANSFER-PROTEINS

AMINO-(N+1)-ALCANOLS 1-SUBSTITUES HALOGENES R-CHIRAUX, UTILES COMME INHIBITEURS DE L'ACTIVITE DE LA PROTEINE DE TRANSFERT DE L'ESTER DE CHOLESTERYLE

(84) Designated Contracting States:
**AT BE CH CY DE DK ES FI FR GB GR IE IT LI LU MC NL PT SE**

(30) Priority: **25.09.1998 US 101663 P**

(43) Date of publication of application:
**18.07.2001 Bulletin 2001/29**

(73) Proprietor: **Monsanto Company**
**Chicago, IL 60680-5110 (US)**

(72) Inventors:
- **SIKORSKI, James, A.**
  **Des Peres, MO 63131 (US)**
- **DURLEY, Richard, C.**
  **Chesterfield, MO 63017 (US)**
- **MISCHKE, Deborah, A.**
  **Defiance, MO 63341 (US)**
- **REINHARD, Emily, J.**
  **Chesterfield, MO 62205 (US)**
- **FOBIAN, Yvette, M.**
  **Labadie, MO 63055 (US)**
- **TOLLEFSON, Michael, B.**
  **O'Fallon, MO 63366 (US)**
- **WANG, Lijuan**
  **Wildwood, MO 63021 (US)**
- **GRAPPERHAUS, Margaret, L.**
  **Troy, IL 62294 (US)**
- **HICKORY, Brian, S.**
  **Wildwood, MO 63011 (US)**
- **MASSA, Mark, A.**
  **Ballwin, MO 63021 (US)**
- **NORTON, Monica, B.**
  **St. Louis, MO 63117 (US)**
- **VERNIER, William, F.**
  **St. Louis, MO 63146 (US)**
- **PROMO, Michele, A.**
  **Chesterfield, MO 63017 (US)**
- **HAMME, Ashton, T.**
  **St. Louis, MO 63146 (US)**
- **SPANGLER, Dale, P.**
  **Deerfield, IL 60015 (US)**
- **RUEPPEL, Melvin, L.**
  **St. Louis, MO 63122 (US)**

(74) Representative: **Albrecht, Thomas, Dr. et al**
**Kraus & Weisert**
**Patent- und Rechtsanwälte**
**Thomas-Wimmer-Ring 15**
**80539 München (DE)**

(56) References cited:
**EP-A- 0 801 060**
**EP-A- 0 818 197**
**GB-A- 2 305 665**
**US-A- 2 700 686**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

(Cont. next page)

- DUNN C ET AL: "THE SYNTHESIS OF FLUORINE-CONTAINING PTERINS" TETRAHEDRON,NL,ELSEVIER SCIENCE PUBLISHERS, AMSTERDAM, vol. 52, no. 40, page 13017-13026 XP002063653 ISSN: 0040-4020
- KATAGIRI T ET AL: "Intramolecular SN2 reaction at alpha-carbon of trifluoromethyl group: preparation of optically active 2-trifluoromethylaziridine" TETRAHEDRON: ASYMMETRY,NL,ELSEVIER SCIENCE PUBLISHERS, AMSTERDAM, vol. 8, no. 17, page 2933-2937 XP004090383 ISSN: 0957-4166

## Description

## FIELD OF THE INVENTION

This invention is in the field of treating cardiovascular disease, and specifically relates to compounds and compositions for treating atherosclerosis and other coronary artery disease, and methods for making compounds of this invention. More particularly, the invention relates to (R)-chiral halogenated 1-substitutedamino-(n+1)-alkanol compounds that inhibit cholesteryl ester transfer protein (CETP), also known as plasma lipid transfer protein-I.

## BACKGROUND OF THE INVENTION

Numerous studies have demonstrated that a low plasma concentration of high density lipoprotein (HDL) cholesterol is a powerful risk factor for the development of atherosclerosis (Barter and Rye, *Atherosclerosis*, 121, 1-12 (1996)). HDL is one of the major classes of lipoproteins that function in the transport of lipids through the blood. The major lipids found associated with HDL include cholesterol, cholesteryl ester, triglycerides, phospholipids and fatty acids. The other classes of lipoproteins found in the blood are low density lipoprotein (LDL) and very low density lipoprotein (VLDL). Since low levels of HDL cholesterol increase the risk of atherosclerosis, methods for elevating plasma HDL cholesterol would be therapeutically beneficial for the treatment of atherosclerosis and other diseases associated with accumulation of lipid in the blood vessels. These diseases include, but are not limited to, coronary heart disease, peripheral vascular disease, and stroke.

Atherosclerosis underlies most coronary artery disease (CAD), a major cause of morbidity and mortality in modern society. High LDL cholesterol (above 180 mg/dl) and low HDL cholesterol (below 35 mg/dl) have been shown to be important contributors to the development of atherosclerosis. Other diseases, such as peripheral vascular disease, stroke, and hypercholesterolaemia are negatively affected by adverse HDL/LDL ratios. Inhibition of CETP by the subject compounds is shown to effectively modify plasma HDL/LDL ratios, and to check the progress and/or formation of these diseases.

CETP is a plasma protein that facilitates the movement of cholesteryl esters and triglycerides between the various lipoproteins in the blood (Tall, *J. Lipid Res.,* 34, 1255-74 (1993)). The movement of cholesteryl ester from HDL to LDL by CETP has the effect of lowering HDL cholesterol. It therefore follows that inhibition of CETP should lead to elevation of plasma HDL cholesterol and lowering of plasma LDL cholesterol, thereby providing a therapeutically beneficial plasma lipid profile (McCarthy, *Medicinal Res. Revs*., 13, 139-59 (1993); Sitori, *Pharmac. Ther.,* 67,443-47 (1995)). This exact phenomenon was first demonstrated by Swenson et al., (*J. Biol. Chem*., 264, 14318 (1989)) with the use of a monoclonal antibody that specifically inhibited CETP. In rabbits, the antibody caused an elevation of the plasma HDL cholesterol and a decrease in LDL cholesterol. Son et al. *(Biochim. Biophys. Acta* 795, 743-480 (1984)), Morton et al. *(J. Lipid Res.* 35, 836-847 (1994)) and Tollefson et al. *(Am. J. Physiol.,* 255, (Endocrinol. Metab. 18, E894-E902 (1988))) describe proteins from human plasma that inhibit CETP. U.S. Patent 5,519,001, issued to Kushwaha et al., describes a 36 amino acid peptide derived from baboon apo C-1 that inhibits CETP activity. Cho et al. *(Biochim. Biophys. Acta* 1391, 133-144 (1998)) describe a peptide from hog plasma that inhibits human CETP. Bonin et al. (*J. Peptide Res.,* 51, 216-225 (1998)) disclose a decapeptide inhibitor of CETP. A depsipeptide fungal metabolite is disclosed as a CETP inhibitor by Hedge et al. in *Bioorg. Med. Chem. Lett.,* 8, 1277-80 (1998).

There have been several reports of non-peptidic compounds that act as CETP inhibitors. Barrett et al. *(J. Am. Chem. Soc.,* 188, 7863-63 (1996)) and Kuo et al. *(J. Am. Chem. Soc.,* 117, 10629-34 (1995)) describe cyclopropane-containing CETP inhibitors. Pietzonka et al. *(Bioorg. Med. Chem. Lett*, 6, 1951-54 (1996)) describe phosphonate-containing analogs of cholesteryl ester as CETP inhibitors. Coval et al. (*Bioorg. Med. Chem. Lett*., 5, 605-610 (1995)) describe Wiedendiol-A and -B, and related sesquiterpene compounds as CETP inhibitors. Japanese Patent Application No. 10287662-A describes polycyclic, non-amine containing, polyhydroxylic natural compounds possessing CETP inhibition properties. Lee et al. (*J. Antibiotics*, 49, 693-96 (1996)) describe CETP inhibitors derived from an insect fungus. Busch et al. *(Lipids,* 25, 216-220, (1990)) describe cholesteryl acetyl bromide as a CETP inhibitor. Morton and Zilversmit (*J. Lipid Res.,* 35, 836-47 (1982)) describe that p-chloromercuriphenyl sulfonate, p-hydroxymercuribenzoate and ethyl mercurithiosalicylate inhibit CETP. Connolly et al. (*Biochem. Biophys. Res. Comm.* 223, 42-47 (1996)) describe other cysteine modification reagents as CETP inhibitors. Xia et al. describe 1,3,5-triazines as CETP inhibitors (Bioorg. Med. Chem. Lett., 6, 919-22 (1996)). Bisgaier et al. (*Lipids,* 29, 811-8 (1994)) describe 4-phenyl-5-tridecyl-4H-1,2,4-triazole-thiol as a CETP inhibitor. Oomura et al. disclose non-peptidic tetracyclic and hexacyclic phenols as CETP inhibitors in Japanese Patent Application No. 10287662.

Some substituted heteroalkylamine compounds are known. In European Patent Application No. 796846, Schmidt et al. describe 2-aryl-substituted pyridines as cholesteryl ester transfer protein inhibitors useful as cardiovascular agents. One substituted at C3 of the pyridine ring can be an hydroxyalkyl group. In European Patent Application No. 801060, Dow and Wright describe heterocyclic derivatives substituted with an aldehyde addition product of an

alkylamine to afford 1-hydroxy-1-amines. These are reported to be β3-adrenergic receptor agonists useful for treating diabetes and other disorders. In Great Britain Patent Application No. 2305665, Fisher et al. disclose 3-agonist secondary amino alcohol substituted pyridine derivatives useful for treating several disorders including cholesterol levels and artherosclerotic diseases. In European Patent Application No. 818448, Schmidt et al. describe tetrahydroquinoline derivatives as cholesteryl ester transfer protein inhibitors. European Patent Application No. 818197, Schmek et al. describe pyridines with fused heterocycles as cholesteryl ester transfer protein inhibitors. Brandes et al. in German Patent Application No. 19627430 describe bicyclic condensed pyridine derivatives as cholesteryl ester transfer protein inhibitors. In WO Patent Application No. 09839299, Muller-Gliemann et al. describe quinoline derivatives as cholesteryl ester transfer protein inhibitors. U.S. Patent 2,700,686, issued to Dickey and Towne, describes N-(2-haloalkyl-2-hydroxyethyl)amines in which the amine is further substituted with either 1 to 2 aliphatic groups or one aromatic group and one aliphatic group. U.S. Patent 2,700,686 further describes a process to prepare the N-(2-haloalkyl-2-hydroxyethyl)amines by reacting halogenated-1,2-epoxyalkanes with the corresponding aliphatic amines and N-alkylanilines and their use as dye intermediates.

## SUMMARY OF THE INVENTION

The present invention provides chiral compounds that can be used to inhibit cholesteryl ester transfer protein (CETP) activity and that have the general structure:

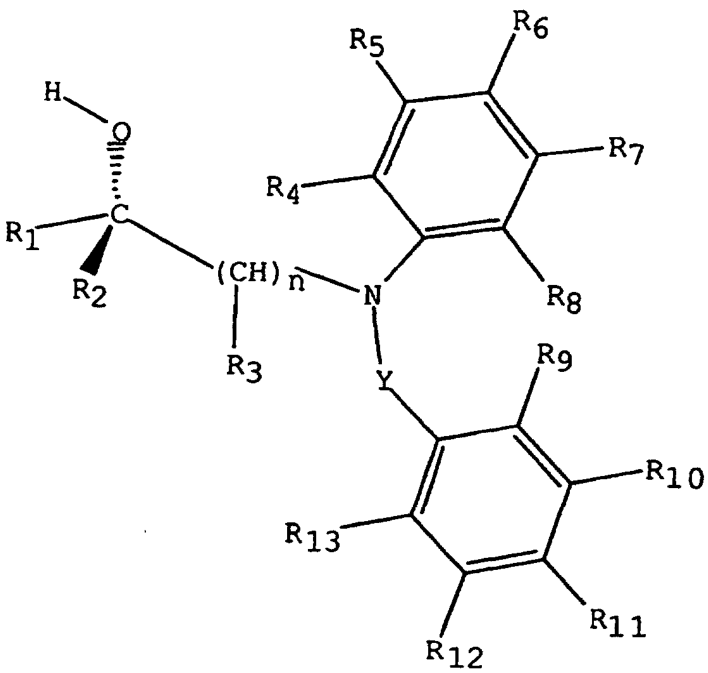

In another aspect, the present invention includes pharmaceutical compositions comprising a pharmaceutically effective amount of the chiral compounds of this invention and a pharmaceutically acceptable carrier.

In another aspect, this invention relates to, but does not claim as such, methods of using these chiral inhibitors as therapeutic agents in humans to inhibit cholesteryl ester transfer protein (CETP) activity, thereby decreasing the concentrations of low density lipoprotein (LDL) and raising the level of high density lipoprotein (HDL), resulting in a therapeutically beneficial plasma lipid profile. The compounds and methods of this invention can also be used to treat dyslipidemia (hypoalphalipoproteinemia), hyperlipoproteinaemia (chylomicronemia and hyperapobetalipoproteinemia), peripheral vascular disease, hypercholesterolaemia, atherosclerosis, coronary artery disease and other CETP-mediated disorders. The compounds can also be used in prophylactic treatment of subjects who are at risk of developing such disorders. The compounds can be used to lower the risk of atherosclerosis. The compounds of this invention would be also useful in prevention of cerebral vascular accident (CVA) or stroke. Besides being useful for human treatment, these compounds are also useful for veterinary treatment of companion animals, exotic animals and farm animals such as primates, rabbits, pigs, horses, and the like.

## DESCRIPTION OF THE INVENTION

The present invention relates to a class of compounds comprising (R)-chiral halogenated 1-substitutedamino-(n+1)-alkanols which are beneficial in the therapeutic and prophylactic treatment of coronary artery disease as given in the following Formula M:

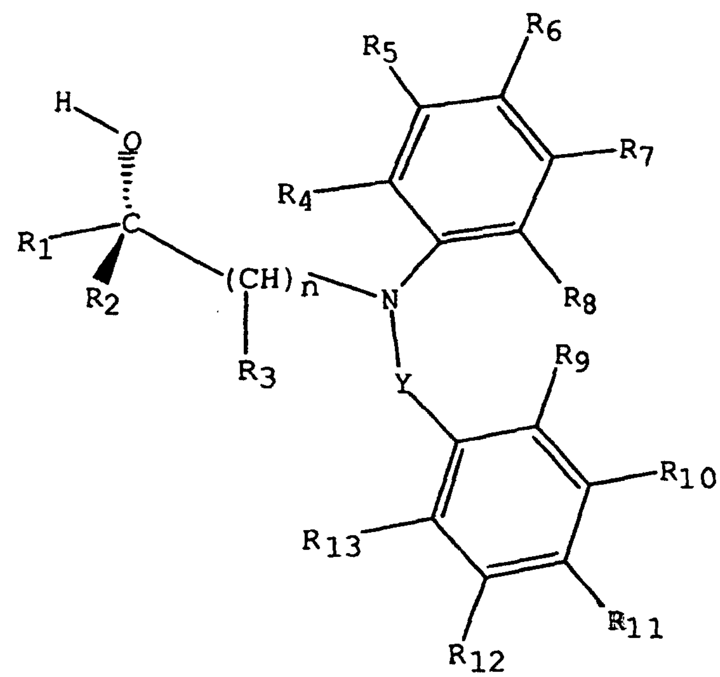

or a pharmaceutically acceptable salt thereof, wherein;

n is selected from the integers 1, 2, 3 and 4;

Y is $-(CH_2)_q-$ wherein q is 1 or 2;

$R_1$ is haloalkyl;

$R_2$ is hydrido;

$R_3$ is hydrido;

$R_4$, $R_8$, $R_9$, and $R_{13}$ are independently selected from the group consisting of hydride, halo, haloalkyl, and alkyl;

$R_5$, $R_6$, $R_7$, $R_{10}$, $R_{11}$, and $R_{12}$ are independently selected from the group consisting of hydrido, carboxy, heteroaralkylthio, heteroaralkoxy, cycloalkylamino, acylalkyl, acylalkoxy, aroylalkoxy, heterocyclyloxy, aralkylaryl, aralkyl, aralkenyl, aralkynyl, heterocyclyl, perhaloaralkyl, aralkylsulfonyl, aralkylsulfonylalkyl, aralkylsulfinyl, aralkylsulfinylalkyl, halocycloalkyl, halocycloalkenyl, cycloalkylsulfinyl, cycloalkylsulfinylalkyl, cycloalkylsulfonyl, cycloalkylsulfonylalkyl, heteroarylamino, N-heteroarylamino-N-alkylamino, heteroarylaminoalkyl, haloalkylthio, alkanoyloxy, alkoxy, alkoxyalkyl, heteroaralkoxy, cycloalkoxy, cycloalkenyloxy, cycloalkoxyalkyl, cycloalkylalkoxy, cycloalkenyloxyalkyl, halocycloalkoxy, halocycloalkoxyalkyl, halocycloalkenyloxy, halocycloalkenyloxyalkyl, hydroxy, amino, thio, nitro, C1-C6 alkylamino, alkylthio, alkylthioalkyl, arylamino, aralkylamino, arylthio, arylthioalkyl, heteroaralkoxyalkyl, alkylsulfinyl, alkylsulfinylalkyl, arylsulfinylalkyl, arylsulfonylalkyl, heteroarylsulfinylalkyl, heteroarylsulfonylalkyl, alkylsulfonyl, alkylsulfonylalkyl, haloalkylsulfinylalkyl, haloalkylsulfonylalkyl, alkylsulfonamido, alkylaminosulfonyl, amidosulfonyl, alkylamidosulfonyl, arylamidosulfonyl, arylsulfonamido, alkylarylamidosulfonyl, arylsulfinyl, arylsulfonyl, heteroarylthio, heteroarylsulfinyl, heteroarylsulfonyl, heterocyclylsulfonyl, heterocyclylthio, alkanoyl, alkenoyl, aroyl, heteroaroyl, aralkanoyl, heteroaralkanoyl, haloalkanoyl, alkyl, alkenyl, alkynyl, alkenyloxy, alkenyloxyalky, cycloalkyl, cycloalkylalkanoyl, cycloalkenyl, C3-C7 cycloalkylalkyl, C3-C7 cycloalkenylalkyl, halo, haloalkyl, haloalkenyl, haloalkoxy, hydroxyhaloalkyl, hydroxyaralkyl, hydroxyalkyl, hydoxyheteroaralkyl, haloalkoxyalkyl, aryl, heteroaralkynyl, aryloxy, aralkoxy, aryloxyalkyl, saturated heterocyclyl, partially saturated heterocyclyl, heteroaryl, heteroaryloxy, heteroaryloxyalkyl, heteroaralkyl, arylalkenyl, heteroarylalkenyl, carboxyalkyl, carboalkoxy, alkoxycarboxamido, alkylamidocarbonylamido, arylamidocarbonylamido, carboalkoxyalkyl, carboalkoxyalkenyl, carboaralkoxy, carboxamido, carboxamidoalkyl, cyano, carbohaloalkoxy, phosphono, phosphonoalkyl, diaralkoxyphosphono, and diaralkoxyphosphonoalkyl;

further, wherein when $R_7$ is aryloxy or aralkoxy, said aryloxy or aralkoxy may be substituted at one or more substitutable positions with one or more radicals selected from amino, halo, nitro, alkoxy, alkyl, cyano, cycloalkoxy,

cycloalkyl, cycloalkylalkoxy, haloalkoxy, C1-C6 alkylamino, haloalkyl, alkanoyl, haloalkylthio, perhaloaralkyl, aralkylsulfonyl, aralkylsulfonylalkyl, halocycloalkyl, halocycloalkenyl, haloalkylthio, alkanoyloxy, alkoxy, alkoxyalkyl, haloalkoxylalkyl, cycloalkoxy, cycloalkoxyalkyl, cycloalkylalkoxy, hydroxy, thio, alkylthio, alkylthioalkyl, arylamino, aralkylamino, arylthio, arylthioalkyl, alkylsulfonyl, alkylsulfonylalkyl, haloalkylsulfonylalkyl, alkylsulfonamido, alkylaminosulfonyl, amidosulfonyl, monoalkyl amidosulfonyl, dialkyl amidosulfonyl, arylsulfonyl, alkenoyl, aroyl, aralkanoyl, haloalkanoyl, alkenyl, alkynyl, alkenyloxy, alkenyloxyalky, cycloalkylalkanoyl, C3-C7 cycloalkylalkyl, haloalkenyl, hydroxyhaloalkyl, hydroxyaralkyl, hydroxyalkyl, haloalkoxyalkyl, aryl, aralkyl, aryloxy, aralkoxy, aryloxyalkyl, carboxyalkyl, carboalkoxy, alkoxycarbonyl, carboaralkoxy, carboxamido, carboxamidoalkyl, carbohaloalkoxy, phosphono, phosphonoalkyl, diaralkoxyphosphono, and diaralkoxyphosphonoalkyl,

with the proviso that at least one of $R_4$, $R_5$, $R_6$, $R_7$, and $R_8$ is not hydrido,

and with the further proviso that at least one of $R_9$, $R_{10}$, $R_{11}$, $R_{12}$, and $R_{13}$ is not hydrido.

In a preferred embodiment

n is an integer selected from 1 through 3;

Y is $-(CH_2)_q-$ wherein q is 1 or 2;

$R_1$ is haloalkyl;

$R_2$ is hydrido;

$R_3$ is hydrido;

$R_4$, $R_8$, $R_9$, and $R_{13}$ are independently selected from the group consisting of hydrido, halo, and haloalkyl;

$R_5$, $R_6$, $R_7$, $R_{10}$, $R_{11}$, and $R_{12}$ are independently selected from the group consisting of hydrido, heteroaralkoxy, acylalkyl, acylalkoxy, aroylalkoxy, heterocyclyloxy, aralkylaryl, aralkyl, perhaloaralkyl, aralkylsulfonyl, aralkylsulfonylalkyl, halocycloalkyl, cycloalkylsulfonyl, cycloalkylsulfonylalkyl, haloalkylthio, alkanoyloxy, alkoxy, alkoxyalkyl, heteroaralkoxy, cycloalkoxy, cycloalkoxyalkyl, cycloalkylalkoxy, halocycloalkoxy, halocycloalkoxyalkyl, hydroxy, amino, nitro, C1-C6 alkylamino, alkylthio, alkylthioalkyl, arylamino, aralkylamino, arylthio, arylsulfonylalkyl, alkylsulfonyl, alkylsulfonylalkyl, haloalkylsulfonylalkyl, alkylsulfonamido, alkylaminosulfonyl, amidosulfonyl, alkylamidosulfonyl, arylsulfonyl, alkanoyl, aroyl, aralkanoyl, heteroaralkanoyl, haloalkanoyl, alkyl, cycloalkyl, cycloalkylalkanoyl, C3-C7 cycloalkylalkyl, halo, haloalkyl, haloalkoxy, hydroxyhaloalkyl, hydroxyaralkyl, hydroxyalkyl, haloalkoxyalkyl, aryl, aryloxy, aralkoxy, aryloxyalkyl, heteroaryloxy, heteroaryloxyalkyl, carboxyalkyl, carboalkoxy, alkoxycarboxamido, carboalkoxyalkyl, carboaralkoxy, carboxamido, carboxamidoalkyl, cyano, carbohaloalkoxy, phosphono, phosphonoalkyl, diaralkoxyphosphono, and diaralkoxyphosphonoalkyl;

further, wherein when $R_7$ is aryloxy or aralkoxy, said aryloxy or aralkoxy may be substituted at one or more substitutable positions with one or more radicals selected from amino, halo, nitro, alkoxy, alkyl, cyano, cycloalkoxy, cycloalkyl, cycloalkylalkoxy, haloalkoxy, C1-C6 alkylamino, haloalkyl, alkanoyl and haloalkylthio,

with the proviso that at least one of $R_4$, $R_5$, $R_6$, $R_7$, and $R_8$ is not hydrido and with the further proviso that at least one of $R_9$, $R_{10}$, $R_{11}$, $R_{12}$, and $R_{13}$ is not hydrido.

In a further preferred embodiment,

n is an integer selected from 1 through 3;

Y is $-(CH_2)_q-$ wherein q is 1 or 2;

$R_1$ is haloalkyl;

$R_2$ is hydrido;

$R_3$ is hydrido;

$R_4$, $R_8$, $R_9$, and $R_{13}$ are independently selected from the group consisting of hydride, halo, and haloalkyl;

$R_5$, $R_6$, $R_7$, $R_{10}$, $R_{11}$, and $R_{12}$ are independently selected from the group consisting of hydride, heteroaralkoxy, acylalkyl, acylalkoxy, aroylalkoxy, heterocyclyloxy, aralkylaryl, aralkyl, perhaloaralkyl, haloalkylthio, alkanoyloxy, alkoxy, alkoxyalkyl, heteroaralkoxy, cycloalkoxy, cycloalkoxyalkyl, cycloalkylalkoxy, halocycloalkoxy, halocycloalkoxyalkyl, alkylthio, alkylthioalkyl, arylthio, alkylsulfonyl, alkylsulfonylalkyl, haloalkylsulfonylalkyl, alkylsulfonamido, alkylaminosulfonyl, amidosulfonyl, alkylamidosulfonyl, arylsulfonyl, alkanoyl, haloalkanoyl, alkyl, cycloalkyl, C3-C7 cycloalkylalkyl, halo, haloalkyl, haloalkoxy, haloalkoxyalkyl, aryl, aryloxy, aralkoxy, aryloxyalkyl, heteroaryloxy, heteroaryloxyalkyl, carboalkoxy, carboalkoxyalkyl, carboaralkoxy and carbohaloalkoxy;

further, wherein when $R_7$ is aryloxy or aralkoxy, said aryloxy or aralkoxy may be substituted at one or more substitutable positions with one or more radicals selected from amino, halo, nitro, alkoxy, alkyl, cycloalkoxy, cycloalkyl, cycloalkylalkoxy, haloalkoxy, C1-C6 alkylamino, haloalkyl, alkanoyl and haloalkylthio.

In a another preferred embodiment, the compounds of the invention are compounds of the following Formula D:

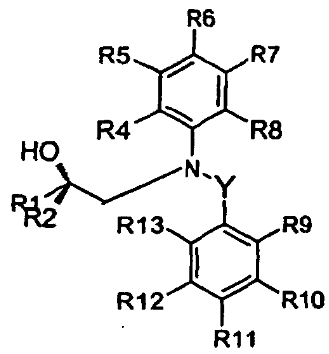

Formula "D"

wherein Y is $-CH_2-$;
wherein $R^1$ is haloalkyl;
wherein $R^2$ is hydrido;
wherein $R^4$ and $R^8$ are hydrido;
wherein $R^5$ and $R^6$ are selected from hydrido and alkoxy;
wherein $R^7$ is selected from aryloxy, arylalkoxy, 5,6,7,8-tetrahydronaphth-2-yloxy, alkoxy, cycloalkoxy, cycloalkylalkoxy and halo;
wherein said aryloxy and arylalkoxy groups in $R^7$ may be substituted at one or more substitutable positions with one or more radicals selected from halo, alkyl, alkoxy, haloalkoxy and haloalkyl;
wherein $R^9$ is selected from hydrido, halo and haloalkyl;
wherein $R^{10}$ is selected from haloalkoxy, haloalkyl and haloalkylthio;
wherein $R^{11}$ is selected from hydrido, halo and haloalkyl;
wherein $R^{12}$ is selected from hydrido and haloalkyl;
wherein $R^{13}$ is selected from hydrido, halo and haloalkyl.

In yet another preferred embodiment, the compounds of the invention are compounds of the following Formula C:

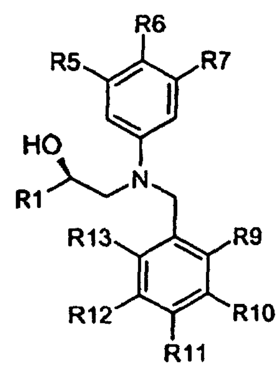

Formula "C"

wherein $R^1$ is selected from trifluoromethyl and chloromethyl;
wherein $R^5$ and $R^6$ are selected from hydrido and methoxy;
wherein $R^7$ is selected from phenyloxy, benzyloxy, 5,6,7,8-tetrahydronaphth-2-yloxy, isopropoxy, cyclopentoxy, bromo, cyclohexylmethoxy and methoxy;
wherein said phenyloxy and benzyloxy groups in $R^7$ may be substituted at one or more substitutable positions with one or more radicals selected from chloro, ethyl, trifluoromethoxy, bromo, fluoro, methyl, isopropyl, trifluoromethyl,

isopropoxy and tert-butyl;

wherein $R^9$ is selected from hydrido, fluoro and trifluoromethyl;

wherein $R^{10}$ is selected from 1,1,2,2-tetrafluoroethoxy, trifluoromethoxy, pentafluoroethyl, trifluoromethyl and trifluoromethylthio;

wherein $R^{11}$ is selected from hydrido, trifluoromethyl and fluoro;

wherein $R^{12}$ is selected from hydrido and trifluoromethyl;

wherein $R^{13}$ is selected from hydrido, fluoro and trifluoromethyl.

The compounds of Formula M are a sub-group of the compounds of the following Formula I-H (also referred to herein as generic polycyclic aryl and heteroaryl (R)-chiral halogenated 1-substitutedamino-(n + 1)-alkanols), which is described here (together with certain other sub-groups thereof, in particular, the compounds of Formulae I-C and I-CP) for purposes of reference, in particular, in connection with the discussion of synthetic procedures below. The references to compounds of Formula I-H are not intended to imply that the scope of the invention extends beyond that of the claims.

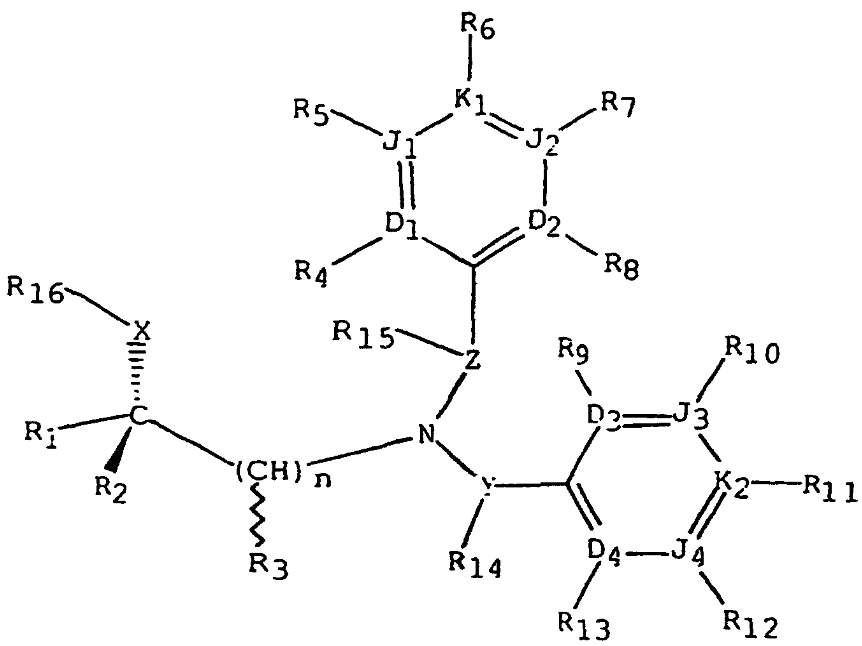

**(I-H)**

or a pharmaceutically-acceptable salt thereof, wherein;

n is an integer selected from 1 through 4:

X is oxy;

$R_1$ is selected from the group consisting of haloalkyl, haloalkenyl, haloalkoxymethyl, and haloalkenyloxymethyl with the proviso that $R_1$ has a higher Cahn-Ingold-Prelog stereochemical system ranking than both $R_2$ and $(CHR_3)_n$-N(A)Q wherein A is Formula (II) and Q is Formula (III);

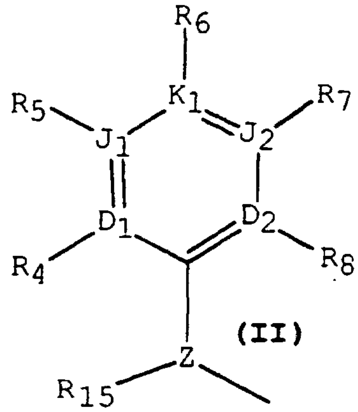

(II)

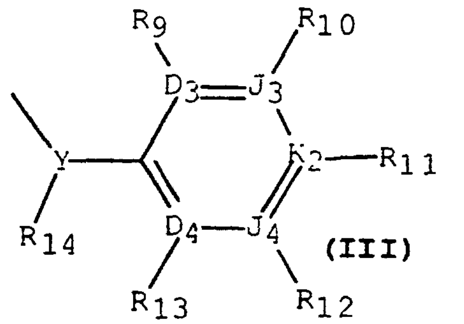

(III)

;

$R_{16}$ is selected from the group consisting of hydrido, alkyl, alkenyl, alkynyl, aryl, aralkyl, aryloxyalkyl, alkoxyalkyl, alkenyloxyalkyl, alkylthioalkyl, arylthioalkyl, aralkoxyalkyl, heteroaralkoxyalkyl, alkylsulfinylalkyl, alkylsulfonylalkyl, cycloalkyl, cycloalkylalkyl, cycloalkylalkenyl, cycloalkenyl, cycloalkenylalkyl, haloalkyl, haloalkenyl, halocycloalkyl, halocycloalkenyl, haloalkoxyalkyl, haloalkenyloxyalkyl, halocycloalkoxyalkyl, halocycloalkenyloxyalkyl, perhaloaryl, perhaloaralkyl, perhaloaryloxyalkyl, heteroaryl, heteroarylalkyl, monocarboalkoxyalkyl, monocarboalkoxy, dicarboalkoxyalkyl, monocarboxamido, monocyanoalkyl, dicyanoalkyl, carboalkoxycyanoalkyl, acyl, aroyl, heteroaroyl, heteroaryloxyalkyl, dialkoxyphosphonoalkyl, trialkylsilyl, and a spacer selected from the group consisting of a covalent single bond and a linear spacer moiety having a chain length of 1 to 4 atoms linked to the point of bonding of any aromatic substituent selected from the group consisting of $R_4$, $R_8$, $R_9$, $R_{13}$, $R_{14}$, and $R_{15}$ to form a heterocyclyl ring having from 5 through 10 contiguous members;

$D_1$, $D_2$, $J_1$, $J_2$ and $K_1$ are independently selected from the group consisting of C, N, O, S and covalent bond with the provisos that no more than one of $D_1$, $D_2$, $J_1$, $J_2$ and $K_1$ can be a covalent bond, no more than one of $D_1$, $D_2$, $J_1$, $J_2$ and $K_1$ can be O, no more than one of $D_1$, $D_2$, $J_1$, $J_2$ and $K_1$ can be S, one of $D_1$, $D_2$, $J_1$, $J_2$ and $K_1$ must be a covalent bond when two of $D_1$, $D_2$, $J_1$, $J_2$ and $K_1$ are O and S, and no more than four of $D_1$, $D_2$, $J_1$, $J_2$ and $K_1$ can be N;

$D_3$, $D_4$, $J_3$, $J_4$ and $K_2$ are independently selected from the group consisting of C, N, O, S and covalent bond with the provisos that no more than one can be a covalent bond, no more than one of $D_3$, $D_4$, $J_3$, $J_4$ and $K_2$ can be O, no more than one of $D_3$, $D_4$, $J_3$, $J_4$ and $K_2$ can be S, no more than two of $D_3$, $D_4$, $J_3$, $J_4$ and $K_2$ can be O and S, one of $D_3$, $D_4$, $J_3$, $J_4$ and $K_2$ must be a covalent bond when two of $D_3$, $D_4$, $J_3$, $J_4$ and $K_2$ are O and S, and no more than four of $D_3$, $D_4$, $J_3$, $J_4$ and $K_2$ can be N;

$R_2$ is hydrido;

$R_2$ can be selected from the group consisting of hydroxyalkyl, alkyl, alkenyl, alkynyl, aryl, aralkyl, aralkoxyalkyl, aryloxyalkyl, alkoxyalkyl, heteroaryloxyalkyl, alkenyloxyalkyl, cycloalkyl, cycloalkylalkyl, cycloalkylalkenyl, cycloalkenyl, cycloalkenylalkyl, haloalkyl, haloalkenyl, halocycloalkyl, haloalkoxy, haloalkoxyalkyl, haloalkenyloxyalkyl, halocycloalkoxy, halocycloalkoxyalkyl, halocycloalkenyloxyalkyl, heteroaryl, heteroarylalkyl, heteroarylthioalkyl, perhaloaryl, perhaloaralkyl, perhaloaralkyl, heteroaralkylthioalkyl, monocarboalkoxyalkyl, dicarboalkoxyalkyl, monocyanoalkyl, dicyanoalkyl, carboalkoxycyanoalkyl, alkylsulfinylalkyl, alkylsulfonylalkyl, arylsulfinylalkyl, arylsulfonylalkyl, cycloalkylsulfinylalkyl, cycloalkylsufonylalkyl, heteroarylsulfonylalkyl, heteroarylsulfinylalkyl, aralkylsulfinylalkyl, aralkylsulfonylalkyl, carboxy, carboxyalkyl, carboalkoxy, carboxamide, carboxamidoalkyl, carboaralkoxy, dicyanoalkyl, carboalkoxycyanoalkyl, dialkoxyphosphonoalkyl, and diaralkoxyphosphonoalkyl with the proviso that $R_2$ has a lower Cahn-Ingold-Prelog system ranking than both $R_1$ and $(CHR_3)_n$-N(A)Q;

$R_3$ is selected from the group consisting of hydride, hydroxy, halo, cyano, aryloxy, hydroxyalkyl, amino, alkylamino, dialkylamino, acyl, acylamido, alkoxy, alkyl, alkenyl, alkynyl, aryl, aralkyl, aryloxyalkyl, alkoxyalkyl, aralkoxyalkyl, alkylsulfinylalkyl, alkylsulfonylalkyl, aroyl, heteroaroyl, aralkylthioalkyl, heteroaralkylthioalkyl, heteroaryloxyalkyl, alkenyloxyalkyl, haloalkyl, haloalkenyl, haloalkoxy, haloalkoxyalkyl, haloalkenyloxyalkyl, alkylthioalkyl, arylthioalkyl, cycloalkyl, cycloalkylalkyl, cycloalkylalkenyl, cycloalkenyl, cycloalkenylalkyl, heteroaryl, heteroarylalkyl, heteroarylthioalkyl, monocarboalkoxyalkyl, dicarboalkoxyalkyl, monocyanoalkyl, dicyanoalkyl, carboalkoxycyanoalkyl, arylsulfinylalkyl, arylsulfonylalkyl, cycloalkylsulfinylalkyl, cycloalkylsufonylalkyl, heteroarylsulfonylalkyl, heteroarylsulfinylalkyl, aralkylsulfinylalkyl, aralkylsulfonylalkyl, carboxy, carboxyalkyl, carboalkoxy, carboxamide, carboxamidoalkyl, carboaralkoxy, dialkoxyphosphonoalkyl, and diaralkoxyphosphonoalkyl with the provisos that $(CHR_3)_n$-N(A)Q has a lower Cahn-Ingold-Prelog stereochemical system ranking than $R_1$ and a higher Cahn-Ingold-Prelog stereochemical system ranking than $R_2$;

Y is selected from a group consisting of a covalent single bond, $(C(R_{14})_2)_q$ wherein q is an integer selected from

1 through 2 and $(CH(R_{14}))_g$-W-$(CH(R_{14}))_p$ wherein g and p are integers independently selected from 0 through 1;

$R_{14}$ is independently selected from the group consisting of hydrido, hydroxy, halo, cyano, aryloxy, amino, alkylamino, dialkylamino, hydroxyalkyl, acyl, aroyl, heteroaroyl, heteroaryloxyalkyl, sulfhydryl, acylamido, alkoxy, alkylthio, arylthio, alkyl, alkenyl, alkynyl, aryl, aralkyl, aryloxyalkyl, aralkoxyalkylalkoxy, alkylsulfinylalkyl, alkylsutfonylalkyl, aralkylthioalkyl, heteroaralkoxythioalkyl, alkoxyalkyl, heteroaryloxyalkyl, alkenyloxyalkyl, alkylthioalkyl, arylthioalkyl, cycloalkyl, cycloalkylalkyl, cycloalkylalkenyl, cycloalkenyl, cycloalkenylalkyl, haloalkyl, haloalkenyl, halocycloalkyl, halocycloalkenyl, haloalkoxy, haloalkoxyalkyl, haloalkenyloxyalkyl, halocycloalkoxy, halocycloalkoxyalkyl, halocycloalkenyloxyalkyl, perhaloaryl, perhaloaralkyl, perhaloaryloxyalkyl, heteroaryl, heteroarylalkyl, heteroarylthioalkyl, heteroaralkylthioalkyl, monocarboalkoxyalkyl, dicarboalkoxyalkyl, monocyanoalkyl, dicyanoalkyl, carboalkoxycyanoalkyl, alkylsulfinyl, alkylsulfonyl, haloalkylsulfinyl, haloalkylsulfonyl, arylsulfinyl, arylsulfinylalkyl, arylsulfonyl, arylsulfonylalkyl, aralkylsulfinyl, aralkylsulfonyl, cycloalkylsulfinyl, cycloalkylsulfonyl, cycloalkylsulfinylalkyl, cycloalkylsufonylalkyl, heteroarylsulfonylalkyl, heteroarylsulfinyl, heteroarylsulfonyl, heteroarylsulfinylalkyl, aralkylsulfinylalkyl, aralkylsulfonylalkyl, carboxy, carboxyalkyl, carboalkoxy, carboxamide, carboxamidoalkyl, carboaralkoxy, dialkoxyphosphono, diaralkoxyphosphono, dialkoxyphosphonoalkyl, diaralkoxyphosphonoalkyl, a spacer selected from a moiety having a chain length of 3 to 6 atoms connected to the point of bonding selected from the group consisting of $R_9$ and $R_{13}$ to form a ring selected from the group consisting of a cycloalkenyl ring having from 5 through 8 contiguous members and a heterocyclyl ring having from 5 through 8 contiguous members, and a spacer selected from a moiety having a chain length of 2 to 5 atoms connected to the point of bonding selected from the group consisting of $R_4$ and $R_8$ to form a heterocyclyl having from 5 through 8 contiguous members with the proviso that, when Y is a covalent bond, an $R_{14}$ substituent is not attached to Y;

$R_{14}$ and $R_{15}$ can be taken together to form a spacer selected from a moiety having a chain length of 2 to 5 atoms to form a heterocyclyl ring having from 5 through 8 contiguous members;

$R_{14}$ and $R_{14}$, when bonded to the different atoms, can be taken together to form a group selected from the group consisting of a covalent bond, alkylene, haloalkylene, and a spacer selected from a group consisting of a moiety having a chain length of 2 to 5 atoms connected to form a ring selected from the group of a saturated cycloalkyl having from 5 through 8 contiguous members, a cycloalkenyl having from 5 through 8 contiguous members, and a heterocyclyl having from 5 through 8 contiguous members;

$R_{14}$ and $R_{14}$, when bonded to the same atom can be taken together to form a group selected from the group consisting of oxo, thiono, alkylene, haloalkylene, and a spacer selected from the group consisting of a moiety having a chain length of 3 to 7 atoms connected to form a ring selected from the group consisting of a cycloalkyl having from 4 through 8 contiguous members, a cycloalkenyl having from 4 through 8 contiguous members, and a heterocyclyl having from 4 through 8 contiguous members:

W is selected from the group consisting of O, C(O), C(S), C(O)N($R_{14}$), C(S)N($R_{14}$), ($R_{14}$)NC(O), ($R_{14}$)NC(S), S. S(O), $S(O)_2$, $S(O)_2N(R_{14})$, $(R_{14})NS(O)_2$, and N($R_{14}$) with the proviso that $R_{14}$ is selected from other than halo and cyano;

Z is independently selected from a group consisting of a covalent single bond, $(C(R_{15})_2)_q$ wherein q is an integer selected from 1 through 2, $(CH(R_{15}))_j$-W-$(CH(R_{15}))_k$ wherein j and k are integers independently selected from 0 through 1 with the proviso that, when Z is a covalent single bond, an $R_{15}$ substituent is not attached to Z;

$R_{15}$ is independently selected, when Z is $(C(R_{15})_2)_q$ wherein q is an integer selected from 1 through 2, from the group consisting of hydrido, hydroxy, halo, cyano, aryloxy, amino, alkylamino, dialkylamino, hydroxyalkyl, acyl, aroyl, heteroaroyl, heteroaryloxyalkyl, sulfhydryl, acylamido, alkoxy, alkylthio, arylthio, alkyl, alkenyl, alkynyl, aryl, aralkyl, aryloxyalkyl, aralkoxyalkyl, alkylsulfinylalkyl, alkylsulfonylalkyl, aralkylthioalkyl, heteroaralkylthioalkyl, alkoxyalkyl, heteroaryloxyalkyl, alkenyloxyalkyl, alkylthioalkyl, arylthioalkyl, cycloalkyl, cycloalkylalkyl, cycloalkylalkenyl, cycloalkenyl, cycloalkenylalkyl, haloalkyl, haloalkenyl, halocycloalkyl, halocycloalkenyl, haloalkoxy, haloalkoxyalkyl, haloalkenyloxyalkyl, halocycloalkoxy, halocycloalkoxyalkyl, halocycloalkenyloxyalkyl, perhaloaryl, perhaloaralkyl, perhaloaryloxyalkyl, heteroaryl, heteroarylalkyl, heteroarylthioalkyl, heteroaralkylthioalkyl, monocarboalkoxyalkyl, dicarboalkoxyalkyl, monocyanoalkyl, dicyanoalkyl, carboalkoxycyanoalkyl, alkylsulfinyl, alkylsulfonyl, haloalkylsulfinyl, haloalkylsulfonyl, arylsulfinyl, arylsulfinylalkyl, arylsulfonyl, arylsulfonylalkyl, aralkylsulfinyl, aralkylsulfonyl, cycloalkylsulfinyl, cycloalkylsulfonyl, cycloalkylsulfinylalkyl, cycloalkylsufonylalkyl, heteroarylsulfonylalkyl, heteroarylsulfinyl, heteroarylsulfonyl, heteroarylsulfinylalkyl, aralkylsulfinylalkyl, aralkylsulfonylalkyl, carboxy, carboxyalkyl, carboalkoxy, carboxamide, carboxamidoalkyl, carboaralkoxy, dialkoxyphosphono, diaralkoxyphosphono, dialkoxyphosphonoalkyl, diaralkoxyphosphonoalkyl, a spacer selected from a moiety having a chain length of 3 to 6 atoms connected to the point of bonding selected from the group consisting of $R_4$ and $R_8$ to form a ring selected from the group consisting of a cycloalkenyl ring having from 5 through 8 contiguous members and a heterocyclyl ring having from 5 through 8 contiguous members, and a spacer selected from a moiety having a chain length of 2 to 5 atoms connected to the point of bonding selected from the group consisting of $R_9$ and $R_{13}$ to form a heterocyclyl having from 5 through 8 contiguous members;

$R_{15}$ and $R_{15}$, when bonded to the different atoms, can be taken together to form a group selected from the group consisting of a covalent bond, alkylene, haloalkylene, and a spacer selected from a group consisting of a moiety having a chain length of 2 to 5 atoms connected to form a ring selected from the group of a saturated cycloalkyl having from

5 through 8 contiguous members, a cycloalkenyl having from 5 through 8 contiguous members, and a heterocyclyl having from 5 through 8 contiguous members;

$R_{15}$ and $R_{15}$, when bonded to the same atom, can be taken together to form a group selected from the group consisting of oxo, thiono, alkylene, haloalkylene, and a spacer selected from the group consisting of a moiety having a chain length of 3 to 7 atoms connected to form a ring selected from the group consisting of a cycloalkyl having from 4 through 8 contiguous members, a cycloalkenyl having from 4 through 8 contiguous members, and a heterocyclyl having from 4 through 8 contiguous members;

$R_{15}$ is independently selected, when Z is $(CH(R_{15}))_j$-W-$(CH(R_{15}))_k$ wherein j and k are integers independently selected from 0 through 1, from the group consisting of hydrido, halo, cyano, aryloxy, carboxyl, acyl, aroyl, heteroaroyl, hydroxyalkyl, heteroaryloxyalkyl, acylamido, alkoxy, alkylthio, arylthio, alkyl, alkenyl, alkynyl, aryl, aralkyl, aryloxyalkyl, alkoxyalkyl, heteroaryloxyalkyl, aralkoxyalkyl, heteroaralkoxyalkyl, alkylsulfonylalkyl, alkylsulfinylalkyl, alkenyloxyalkyl, alkylthioalkyl, arylthioalkyl, cycloalkyl, cycloalkylalkyl, cycloalkylalkenyl, cycloalkenyl, cycloalkenylalkyl, haloalkyl, haloalkenyl, halocycloalkyl, halocycloalkenyl, haloalkoxy, haloalkoxyalkyl, haloalkenyloxyalkyl, halocycloalkoxy, halocycloalkoxyalkyl, halocycloalkenyloxyalkyl, perhaloaryl, perhaloaralkyl, perhaloaryloxyalkyl, heteroaryl, heteroaralkyl, heteroarylthioalkyl, heteroaralkylthioalkyl, monocarboalkoxyalkyl, dicarboalkoxyalkyl, monocyanoalkyl, dicyanoalkyl, carboalkoxycyanoalkyl, alkylsulfinyl, alkylsulfonyl, haloalkylsulfinyl, haloalkylsulfonyl, arylsulfinyl, arylsulfinylalkyl, arylsulfonyl, arylsulfonylalkyl, aralkylsulfinyl, aralkylsulfonyl, cycloalkylsulfinyl, cycloalkylsulfonyl, cycloalkylsulfinylalkyl, cycloalkylsufonylalkyl, heteroarylsulfonylalkyl, heteroarylsulfinyl, heteroarylsulfonyl, heteroarylsulfinylalkyl, aralkylsulfinylalkyl, aralkylsulfonylalkyl, carboxyalkyl, carboalkoxy, carboxamide, carboxamidoalkyl, carboaralkoxy, dialkoxyphosphonoalkyl, diaralkoxyphosphonoalkyl, a spacer selected from a linear moiety having a chain length of 3 to 6 atoms connected to the point of bonding selected from the group consisting of $R_4$ and $R_8$ to form a ring selected from the group consisting of a cycloalkenyl ring having from 5 through 8 contiguous members and a heterocyclyl ring having from 5 through 8 contiguous members, and a spacer selected from a linear moiety having a chain length of 2 to 5 atoms connected to the point of bonding selected from the group consisting of $R_9$ and $R_{13}$ to form a heterocyclyl ring having from 5 through 8 contiguous members;

$R_4$, $R_5$, $R_6$, $R_7$, $R_8$, $R_9$, $R_{10}$, $R_{11}$, $R_{12}$, and $R_{13}$ are independently selected from the group consisting of hydrido, carboxy, heteroaralkylthio, heteroaralkoxy, cycloalkylamino, acylalkyl, acylalkoxy, aroylalkoxy, heterocyclyloxy, aralkylaryl, aralkyl, aralkenyl, aralkynyl, heterocyclyl, perhaloaralkyl, aralkylsulfonyl, aralkylsulfonylalkyl, aralkylsulfinyl, aralkylsulfinylalkyl, halocycloalkyl, halocycloalkenyl, cycloalkylsulfinyl, cycloalkylsulfinylalkyl, cycloalkylsulfonyl, cycloalkylsulfonylalkyl, heteroarylamino, N-heteroarylamino-N-alkylamino, heteroaralkyl, heteroarylaminoalkyl, haloalkylthio, alkanoyloxy, alkoxy, alkoxyalkyl, haloalkoxylalkyl, heteroaralkoxy, cycloalkoxy, cycloalkenyloxy, cycloalkoxyalkyl, cycloalkylalkoxy, cycloalkenyloxyalkyl, cycloalkylenedioxy, halocycloalkoxy, halocycloalkoxyalkyl, halocycloalkenyloxy, halocycloalkenyloxyalkyl, hydroxy, amino, thio, nitro, lower alkylamino, alkylthio, alkylthioalkyl, arylamino, aralkylamino, arylthio, arylthioalkyl, heteroaralkoxyalkyl, alkylsulfinyl, alkylsulfinylalkyl, arylsulfinylalkyl, arylsulfonylalkyl, heteroarylsulfinylalkyl, heteroarylsulfonylalkyl, alkylsulfonyl, alkylsulfonylalkyl, haloalkylsulfinylalkyl, haloalkylsulfonylalkyl, alkylsulfonamido, alkylaminosulfonyl, amidosulfonyl, monoalkyl amidosulfonyl, dialkyl amidosulfonyl, monoarylamidosulfonyl, arylsulfonamido, diarylamidosulfonyl, monoalkyl monoaryl amidosulfonyl, arylsulfinyl, arylsulfonyl, heteroarylthio, heteroarylsulfinyl, heteroarylsulfonyl, heterocyclylsulfonyl, heterocyclylthio, alkanoyl, alkenoyl, aroyl, heteroaroyl, aralkanoyl, heteroaralkanoyl, haloalkanoyl, alkyl, alkenyl, alkynyl, alkenyloxy, alkenyloxyalky, alkylenedioxy, haloalkylenedioxy, cycloalkyl, cycloalkylalkanoyl, cycloalkenyl, lower cycloalkylalkyl, lower cycloalkenylalkyl, halo, haloalkyl, haloalkenyl, haloalkoxy, hydroxyhaloalkyl, hydroxyaralkyl, hydroxyalkyl, hydoxyheteroaralkyl, haloalkoxyalkyl, aryl, heteroaralkynyl, aryloxy, aralkoxy, aryloxyalkyl, saturated heterocyclyl, partially saturated heterocyclyl, heteroaryl, heteroaryloxy, heteroaryloxyalkyl, arylalkenyl, heteroarylalkenyl, carboxyalkyl, carboalkoxy, alkoxycarboxamido, alkylamidocarbonylamido, arylamidocarbonylamido, carboalkoxyalkyl, carboalkoxyalkenyl, carboaralkoxy, carboxamido, carboxamidoalkyl, cyano, carbohaloalkoxy, phosphono, phosphonoalkyl, diaralkoxyphosphono, and diaralkoxyphosphonoalkyl with the proviso that there are one to five non-hydrido ring substituents $R_4$, $R_5$, $R_6$, $R_7$, and $R_8$ present, that there are one to five non-hydride ring substituents $R_9$, $R_{10}$, $R_{11}$, $R_{12}$, and $R_{13}$ present, and $R_4$, $R_5$, $R_6$, $R_7$, $R_8$, $R_9$, $R_{10}$, $R_{11}$, $R_{12}$, and $R_{13}$ are each independently selected to maintain the tetravalent nature of carbon, trivalent nature of nitrogen, the divalent nature of sulfur, and the divalent nature of oxygen;

$R_4$ and $R_5$, $R_5$ and $R_6$, $R_6$ and $R_7$, $R_7$ and $R_8$, $R_9$ and $R_{10}$, $R_{10}$ and $R_{11}$, $R_{11}$ and $R_{12}$, and $R_{12}$ and $R_{13}$ can be independently selected to form spacer pairs wherein a spacer pair is taken together to form a linear moiety having from 3 through 6 contiguous atoms connecting the points of bonding of said spacer pair members to form a ring selected from the group consisting of a cycloalkenyl ring having 5 through 8 contiguous members, a partially saturated heterocyclyl ring having 5 through 8 contiguous members, a heteroaryl ring having 5 through 6 contiguous members, and an aryl with the provisos that no more than one of the group consisting of spacer pairs $R_4$ and $R_5$, $R_5$ and $R_6$, $R_6$ and $R_7$, and $R_7$ and $R_8$, can be used at the same time and that no more than one of the group consisting of spacer pairs $R_9$ and $R_{10}$, $R_{10}$ and $R_{11}$, $R_{11}$ and $R_{12}$, and $R_{12}$ and $R_{13}$ can be used at the same time;

$R_4$ and $R_9$, $R_4$ and $R_{13}$, $R_8$ and $R_9$, and $R_8$ and $R_{13}$ can be independently selected to form a spacer pair wherein

said spacer pair is taken together to form a linear moiety wherein said linear moiety forms a ring selected from the group consisting of a partially saturated heterocyclyl ring having from 5 through 8 contiguous members and a heteroaryl ring having from 5 through 6 contiguous members with the proviso that no more than one of the group consisting of spacer pairs $R_4$ and $R_9$, $R_4$ and $R_{13}$, $R_8$ and $R_9$, and $R_8$ and $R_{13}$ can be used at the same time;

$R_5$ and $R_{10}$, $R_5$ and $R_{12}$, $R_7$ and $R_{10}$, and $R_7$ and $R_{12}$ can be independently selected to form a spacer pair wherein said spacer pair is taken together to form a linear moiety wherein said linear moiety forms a C8 to C13 heterocyclyl ring having from 8 through 13 contiguous members with the proviso that no more than one of the group consisting of spacer pairs $R_5$ and $R_{10}$, $R_5$ and $R_{12}$, $R_7$ and $R_{10}$, and $R_7$ and $R_{12}$ can be used at the same time, and $K_2$ can be S, one of $D_3$, $D_4$, $J_3$, $J_4$ and $K_2$ must be a covalent bond when two of $D_3$, $D_4$, $J_3$, $J_4$ and $K_2$ are O and S, and no more than four of $D_3$, $D_4$, $J_3$, $J_4$ and $K_2$ can be N;

$D_1$, $D_2$, $J_1$, $J_2$ and $K_1$ can be selected from the group consisting of C, O, S, N and covalent bond with the provisos that $D_3$, $D_4$, $J_3$, $J_4$ and $K_2$ are each carbon and at least one of $D_1$, $D_2$, $J_1$, $J_2$ and $K_1$ is selected from the group consisting of O, S, and N wherein, when $D_1$, $D_2$, $J_1$, $J_2$ and $K_1$ are selected from the group consisting of C, O, S, covalent bond, and N, no more than one of $D_1$, $D_2$, $J_1$, $J_2$ and $K_1$ can be a covalent bond, no more than one of $D_1$, $D_2$, $J_1$, $J_2$ and $K_1$ can be O, no more than one of $D_1$, $D_2$, $J_1$, $J_2$ and $K_1$ can be S, one of $D_1$, $D_2$, $J_1$, $J_2$ and $K_1$ must be a covalent bond when two of $D_1$, $D_2$, $J_1$, $J_2$ and $K_1$ are O and S, and no more than four of $D_1$, $D_2$, $J_1$, $J_2$ and $K_1$ can be N;

n is an integer selected from 1 through 4;

X is oxy;

$R_{16}$ is selected from the group consisting of hydrido, acyl, aroyl, and trialkylsilyl;

$R_1$ is selected from the group consisting of haloalkyl, haloalkenyl, haloalkoxymethyl, and haloalkenyloxymethyl with the proviso that $R_1$ has a higher Cahn-Ingold-Prelog stereochemical system ranking than both $R_2$ and $(CHR_3)_n$-N(A)Q wherein A is Formula (II) and Q is Formula (III);

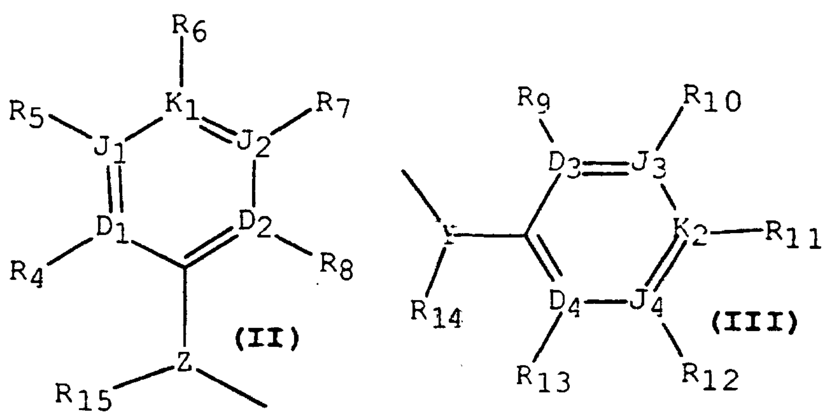

(II) (III) ;

$R_2$ is hydrido;

$R_2$ can be selected from the group consisting of aryl, aralkyl, alkyl, alkenyl, alkenyloxyalkyl, haloalkyl, haloalkenyl, halocycloalkyl, haloalkoxy, haloalkoxyalkyl, haloalkenyloxyalkyl, halocycloalkoxy, halocycloalkoxyalkyl, perhaloaryl, perhaloaralkyl, perhaloaryloxyalkyl, heteroaryl, dicyanoalkyl, and carboalkoxycyanoalkyl with the proviso that $R_2$ has a lower Cahn-Ingold-Prelog system ranking than both $R_1$ and $(CHR_3)_n$-N(A)Q;

$R_3$ is selected from the group consisting of hydrido, hydroxy, cyano, aryl, aralkyl, acyl, alkoxy, alkyl, alkenyl, alkoxyalkyl, heteroaryl, alkenyloxyalkyl, haloalkyl, haloalkenyl, haloalkoxy, haloalkoxyalkyl, haloalkenyloxyalkyl, monocyanoalkyl, dicyanoalkyl, carboxamide, and carboxamidoalkyl with the provisos that $(CHR_3)_n$-N(A)Q has a lower Cahn-Ingold-Prelog stereochemical system ranking than $R_1$ and a higher Cahn-Ingold-Prelog stereochemical system ranking than $R_2$;

Y is selected from the group consisting of covalent single bond and $(C(R_{14})_2)_q$ wherein q is an integer selected from 1 through 2;

$R_{14}$ is selected from the group consisting of hydrido, cyano, hydroxyalkyl, acyl, alkoxy, alkyl, alkenyl, alkoxyalkyl, haloalkyl, haloalkenyl, haloalkoxy, haloalkoxyalkyl, haloalkenyloxyalkyl, monocyanoalkyl, dicyanoalkyl, carboxamide, and carboxamidoalkyl;

Z is selected from the group consisting of covalent single bond, $(C(R_{15})_2)_q$ wherein q is an integer selected from 1 through 2. and $(CH(R_{15}))_j$-W-$(CH(R_{15}))_k$ wherein j and k are integers independently selected from 0 through 1;

W is oxy;

$R_{15}$ is selected from the group consisting of hydride, cyano, hydroxyalkyl, acyl, alkoxy, alkyl, alkenyl, alkoxyalkyl,

haloalkyl, haloalkenyl, haloalkoxy, haloalkoxyalkyl, haloalkenyloxyalkyl, monocyanoalkyl, dicyanoalkyl, carboxamide, and carboxamidoalkyl;

$R_4$, $R_8$, $R_9$, and $R_{13}$ are independently selected from the group consisting of hydrido, halo, haloalkyl, and alkyl ;

$R_5$, $R_6$, $R_7$, $R_{10}$, $R_{11}$, and $R_{12}$ are independently selected from the group consisting of hydrido, carboxy, heteroaralkylthio, heteroarylsulfonyl, heteroaralkoxy, cycloalkylamino, acylalkyl, acylalkoxy, aroylalkoxy, heterocyclyloxy, aralkylaryl, aralkyl, aralkenyl, aralkynyl, heterocyclyl, haloalkylthio, alkanoyloxy, alkoxy, alkoxyalkyl, cycloalkoxy, cycloalkylalkoxy, hydroxy, amino, thio, nitro, lower alkylamino, alkylthio, arylamino, aralkylamino, arylthio, arylthioalkyl, alkylsulfonyl, alkylsulfonamido, monoarylamidosulfonyl, arylsulfonyl, heteroarylthio, heterocyclylsulfonyl, heterocyclylthio, alkanoyl, alkenoyl, aroyl, alkyl, alkenyl, alkynyl, alkenyloxy, alkylenedioxy, haloalkylenedioxy, cycloalkyl, cycloalkylalkanoyl, halo, haloalkyl, haloalkoxy, hydroxyhaloalkyl, hydroxyalkyl, aryl, aryloxy, aralkoxy, saturated heterocyclyl, heteroaryl, heteroaryloxy, heteroaryloxyalkyl, heteroaralkyl, carboalkoxy, alkoxycarboxamido, alkylamidocarbonylamido, arylamidocarbonylamido, carboalkoxyalkyl, carboalkoxyalkenyl, carboxamido, carboxamidoalkyl, and cyano;

$R_4$ and $R_5$, $R_5$ and $R_6$, $R_6$ and $R_7$, $R_7$ and $R_8$, $R_9$ and $R_{10}$, $R_{10}$ and $R_{11}$, $R_{11}$ and $R_{12}$, and $R_{12}$ and $R_{13}$ spacer pairs can be independently selected from the group consisting of alkylene, alkenylene, alkylenedioxy, aralkylene, diacyl, haloalkylene, and aryloxylene with the provisos that no more than one of the group consisting of spacer pairs $R_4$ and $R_5$, $R_5$ and $R_6$, $R_6$ and $R_7$, and $R_7$ and $R_8$ can be used at the same time and that no more than one of the group consisting of spacer pairs $R_9$ and $R_{10}$, $R_{10}$ and $R_{11}$, $R_{11}$ and $R_{12}$, and $R_{12}$ and $R_{13}$ can be used at the same time.

In an even more specific embodiment of compounds Formula I-H,

$D_1$, $D_2$, $J_1$, $J_2$ and $K_1$ are each carbon;

$D_3$, $D_4$, $J_3$, $J_4$ and $K_2$ are independently selected from the group consisting of C, N, O, S and covalent bond with the provisos that at least one of $D_3$, $D_4$, $J_3$, $J_4$ and $K_2$ is selected from the group consisting of O, S, and N, wherein no more than one of $D_3$, $D_4$, $J_3$, $J_4$ and $K_2$ can be a covalent bond, no more than one of $D_3$, $D_4$, $J_3$, $J_4$ and $K_2$ can be O, no more than one of $D_3$, $D_4$, $J_3$, $J_4$ and $K_2$ can be S, one of $D_3$, $D_4$, $J_3$, $J_4$ and $K_2$ must be a covalent bond when two of $D_3$, $D_4$, $J_3$, $J_4$ and $K_2$ are O and S, and no more than four of $D_3$, $D_4$, $J_3$, $J_4$ and $K_2$ can be N;

n is an integer selected from 1 to 3;

X is oxy;

$R_1$ is selected from the group consisting of trifluoromethyl, 1,1,2,2-tetrafluoroethoxymethyl, trifluoromethoxymethyl, chloromethyl, fluoromethyl, difluoromethyl, chlorodifluoromethyl, pentafluoroethyl, 2,2,3,3,3-pentafluoropropyl, and heptafluoropropyl;

$R_{16}$ is selected from the group consisting of acetyl, benzoyl, dimethyl *tert* -butylsilyl, hydrido, and trimethylsilyl;

$R_2$ is hydrido;

$R_2$ can be selected from the group consisting of hydrido, methyl, ethyl, propyl, butyl, isopropyl, isobutyl, vinyl, phenyl, 4-trifluoromethylphenyl, 1,1,2,2-tetrafluoroethoxymethyl, chloromethyl, trifluoromethoxymethyl, fluoromethyl, difluoromethyl, 2,2,3,3,3-pentafluoropropyl, and pentafluorophenoxymethyl with the proviso that $R_2$ has a lower Cahn-Ingold-Prelog system ranking than both $R_1$ and $(CHR_3)_n$-N(A)Q:

$R_3$ is selected from the group consisting of hydride, hydroxy, cyano, acetyl, methoxy, ethoxy, methyl, ethyl, propyl, vinyl, phenyl, methoxymethyl, 4-trifluoromethylphenyl, trifluoromethyl, 1,1,2,2-tetrafluoroethoxymethyl, trifluoromethoxymethyl, chloromethyl, fluoromethyl, difluoromethyl, chlorodifluoromethyl, pentafluoroethyl, 2,2,3,3,3-pentafluoropropyl, heptafluoropropyl, pentafluorophenyl, and pentafluorophenoxymethyl with the provisos that $(CHR_3)_n$-N(A)Q has a lower Cahn-Ingold-Prelog stereochemical system ranking than $R_1$ and a higher Cahn-Ingold-Prelog stereochemical system ranking than $R_2$.

In another even more specific embodiment of compounds Formula I-H,

$D_3$, $D_4$, $J_3$, $J_4$ and $K_2$ are each carbon;

$D_1$, $D_2$, $J_1$, $J_2$ and $K_1$ are independently selected from the group consisting of C, N, O, S and covalent bond with the provisos that at least one of $D_1$, $D_2$, $J_1$, $J_2$ and $K_1$ is selected from the group consisting of O, S, and N, wherein no more than one of $D_1$, $D_2$, $J_1$, $J_2$ and $K_1$ can be a covalent bond, no more than one of $D_1$, $D_2$, $J_1$, $J_2$ and $K_1$ can be O, no more than one of $D_1$, $D_2$, $J_1$, $J_2$ and $K_1$ can be S, one of $D_1$, $D_2$, $J_1$, $J_2$ and $K_1$ must be a covalent bond when two of $D_1$, $D_2$, $J_1$, $J_2$ and $K_1$ are O and S, and no more than four of $D_1$, $D_2$, $J_1$, $J_2$ and $K_1$ can be N;

n is an integer selected from 1 to 3;

X is oxy;

In a specific sub-group of compounds of Formula I-H, the compounds correspond to the Formula I-C (also referred to herein as phenyl (R)-chiral halogenated 1-substitutedamino-(n+1)-alkanols):

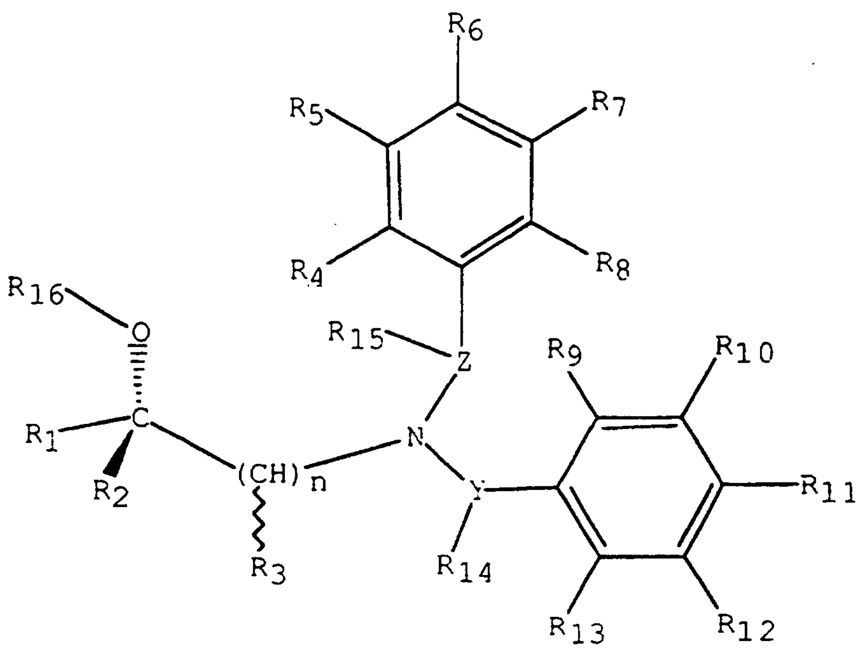

(I-C)

or a pharmacuetically acceptable salt thereof, wherein:

n is an integer selected from 1 through 4;

$R_{16}$ is selected from the group consisting of hydrido, alkyl, acyl, aroyl, heteroaroyl, trialkylsilyl, and a spacer selected from the group consisting of a covalent single bond and a linear spacer moiety having a chain length of 1 to 4 atoms linked to the point of bonding of any aromatic substituent selected from the group consisting of $R_4$, $R_8$, $R_9$, and $R_{13}$ to form a heterocyclyl ring having from 5 through 10 contiguous members with the proviso that said linear spacer moiety is other than covalent single bond when $R_2$ is alkyl;

$R_1$ is selected from the group consisting of haloalkyl, haloalkenyl, haloalkoxymethyl, and haloalkenyloxymethyl with the proviso that $R_1$ has a higher Cahn-Ingold-Prelog stereochemical system ranking than both $R_2$ and $(CHR_3)_n$-N(Ap)Qp wherein Ap is Formula (II-P) and Qp is Formula (III-P);

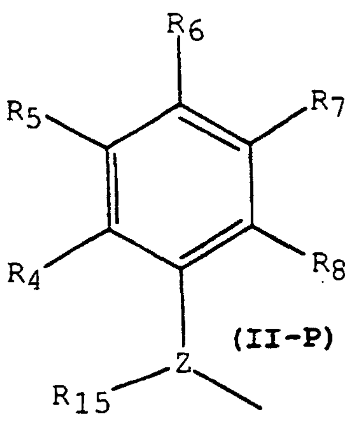

(II-P)

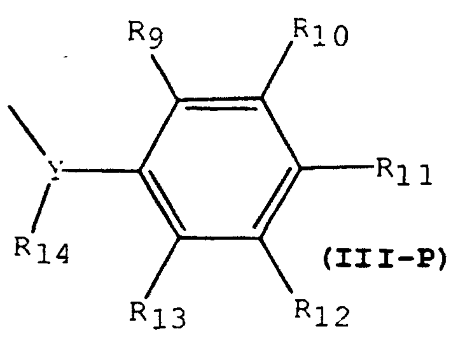

(III-P)

;

$R_2$ is hydrido:

$R_2$ can be selected from the group consisting of aryl, aralkyl, alkyl, alkenyl, alkenyloxyalkyl, haloalkyl, haloalkenyl, halocycloalkyl, haloalkoxy, haloalkoxyalkyl, haloalkenyloxyalkyl, halocycloalkoxy, halocycloalkoxyalkyl, perhaloaryl, perhaloaralkyl, perhaloaryloxyalkyl, heteroaryl, dicyanoalkyl, and carboalkoxycyanoalkyl with the proviso that $R_2$ has a lower Cahn-Ingold-Prelog system ranking than both $R_1$ and $(CHR_3)_n$-N(Ap)Qp:

$R_3$ is selected from the group consisting of hydrido, hydroxy, cyano, aryl, aralkyl, acyl, alkoxy, alkyl, alkenyl, alkoxyalkyl, heteroaryl, alkenyloxyalkyl, haloalkyl, haloalkenyl, haloalkoxy, haloalkoxyalkyl, haloalkenyloxyalkyl, monocyanoalkyl, dicyanoalkyl, carboxamide, and carboxamidoalkyl with the provisos that $(CHR_3)_n$-N(Ap)Qp has a lower Cahn-Ingold-Prelog stereochemical system ranking than $R_1$ and a higher Cahn-Ingold-Prelog stereochemical system ranking than $R_2$;

Y is selected from the group consisting of covalent single bond and $(C(R_{14})_2)_q$ wherein q is an integer selected from 1 through 2;

$R_{14}$ is selected from the group consisting of hydrido, hydroxy, cyano, hydroxyalkyl, acyl, alkoxy, alkyl, alkenyl, alkynyl, alkoxyalkyl, haloalkyl, haloalkenyl, haloalkoxy, haloalkoxyalkyl, haloalkenyloxyalkyl, monocarboalkoxyalkyl, monocyanoalkyl, dicyanoalkyl, carboalkoxycyanoalkyl, carboalkoxy, carboxamide, carboxamidoalkyl;

Z is selected from the group consisting of covalent single bond, $(C(R_{15})_2)_q$ wherein q is an integer selected from 1 through 2, and $(CH(R_{15}))_j$-W-$(CH(R_{15}))_k$ wherein j and k are integers independently selected from 0 through 1;

W is selected from the group consisting of O, C(O), C(S), C(O)N($R_{14}$), C(S)N($R_{14}$), ($R_{14}$)NC(O), ($R_{14}$)NC(S), S, S(O), $S(O)_2$, $S(O)_2N(R_{14})$, $(R_{14})NS(O)_2$, and N($R_{14}$) with the proviso that $R_{14}$ is other than cyano:

$R_{15}$ is selected from the group consisting of hydrido, cyano, hydroxyalkyl, acyl, alkoxy, alkyl, alkenyl, alkynyl, alkoxyalkyl, haloalkyl, haloalkenyl, haloalkoxy, haloalkoxyalkyl, haloalkenyloxyalkyl, monocarboalkoxyalkyl, monocyanoalkyl, dicyanoalkyl, carboalkoxycyanoalkyl, carboalkoxy, carboxamide, and carboxamidoalkyl;

$R_4$, $R_8$, $R_9$, and $R_{13}$ are independently selected from the group consisting of hydrido, halo, haloalkyl, and alkyl;

$R_5$, $R_6$, $R_7$, $R_{10}$, $R_{11}$, and $R_{12}$ are independently selected from the group consisting of hydrido, carboxy, heteroaralkylthio, heteroaralkoxy, cycloalkylamino, acylalkyl, acylalkoxy, aroylalkoxy, heterocyclyloxy, aralkylaryl, aralkyl, aralkenyl, aralkynyl, heterocyclyl, perhaloaralkyl, aralkylsulfonyl, aralkylsulfonylalkyl, aralkylsulfinyl, aralkylsulfinylalkyl, halocycloalkyl, halocycloalkenyl, cycloalkylsulfinyl, cycloalkylsulfinylalkyl, cycloalkylsulfonyl, cycloalkylsulfonylalkyl, heteroarylamino, N-heteroarylamino-N-alkylamino, heteroarylaminoalkyl,haloalkylthio, alkanoyloxy, alkoxy, alkoxyalkyl, haloalkoxylalkyl, heteroaralkoxy, cycloalkoxy, cycloalkenyloxy, cycloalkoxyalkyl, cyctoalkylalkoxy, cycloalkenyloxyalkyl, cycloalkylenedioxy, halocycloalkoxy, halocycloalkoxyalkyl, halocycloalkenyloxy, halocycloalkenyloxyalkyl, hydroxy, amino, thio, nitro, lower alkylamino, alkylthio, alkylthioalkyl, arylamino, aralkylamino, arylthio, arylthioalkyl, heteroaralkoxyalkyl, alkylsulfinyl, alkylsulfinylalkyl, arylsulfinylalkyl, arylsulfonylalkyl, heteroarylsulfinylalkyl, heteroarylsulfonylalkyl, alkylsulfonyl, alkylsulfonylalkyl, haloalkylsulfinylalkyl, haloalkylsulfonylalkyl, alkylsulfonamido, alkylaminosulfonyl, amidosulfonyl, monoalkyl amidosulfonyl, dialkyl amidosulfonyl, monoarylamidosulfonyl, arylsulfonamido, diarylamidosulfonyl, monoalkyl monoaryl amidosulfonyl, arylsulfinyl, arylsulfonyl, heteroarylthio, heteroarylsulfinyl, heteroarylsulfonyl, heterocyclylsulfonyl, heterocyclylthio, alkanoyl, alkenoyl, aroyl, heteroaroyl, aralkanoyl, heteroaralkanoyl, haloalkanoyl, alkyl, alkenyl, alkynyl, alkenyloxy, alkenyloxyalky, alkylenedioxy, haloalkylenedioxy, cycloalkyl, cycloalkylalkanoyl, cycloalkenyl, lower cycloalkylalkyl, lower cycloalkenylalkyl, halo, haloalkyl, haloalkenyl, haloalkoxy, hydroxyhaloalkyl, hydroxyaralkyl, hydroxyalkyl, hydoxyheteroaralkyl, haloalkoxyalkyl, aryl, heteroaralkynyl, aryloxy, aralkoxy, aryloxyalkyl, saturated heterocyclyl, partially saturated heterocyclyl, heteroaryl, heteroaryloxy, heteroaryloxyalkyl, heteroaralkyl, arylalkenyl, heteroarylalkenyl, carboxyalkyl, carboalkoxy, alkoxycarboxamido, alkylamidocarbonylamido, arylamidocarbonylamido, carboalkoxyalkyl, carboalkoxyalkenyl, carboaralkoxy, carboxamido, carboxamidoalkyl, cyano, carbohaloalkoxy, phosphono, phosphonoalkyl, diaralkoxyphosphono, and diaralkoxyphosphonoalkyl;

$R_4$ and $R_5$, $R_5$ and $R_6$, $R_6$ and $R_7$, $R_7$ and $R_8$, $R_9$ and $R_{10}$, $R_{10}$ and $R_{11}$, $R_{11}$ and $R_{12}$, and $R_{12}$ and $R_{13}$ can be independently selected to form spacer pairs wherein a spacer pair is taken together to form a linear moiety having from 3 through 6 contiguous atoms connecting the points of bonding of said spacer pair members to form a ring selected from the group consisting of a cycloalkenyl ring having 5 through 8 contiguous members, a partially saturated heterocyclyl ring having 5 through 8 contiguous members, a heteroaryl ring having 5 through 6 contiguous members, and an aryl with the provisos that no more than one of the group consisting of spacer pairs $R_4$ and $R_5$, $R_5$ and $R_6$, $R_6$ and $R_7$, and $R_7$ and $R_8$, can be used at the same time and that no more than one of the group consisting of spacer pairs $R_9$ and $R_{10}$, $R_{10}$ and $R_{11}$, $R_{11}$ and $R_{12}$, and $R_{12}$ and $R_{13}$ can be used at the same time.

$R_{16}$ is selected from the group consisting of hydride, acyl, aroyl, and trialkylsilyl;

$R_1$ is selected from the group consisting of haloalkyl, haloalkenyl, haloalkoxymethyl, and haloalkenyloxymethyl with the proviso that $R_1$ has a higher Cahn-Ingold-Prelog stereochemical system ranking than both $R_2$ and $(CHR_3)_n$-N(Ap)Qp wherein Ap is Formula (II-P) and Qp is Formula (III-P);

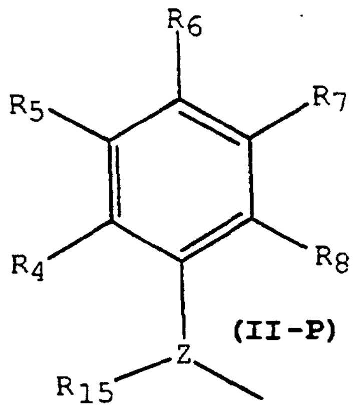
(II-P)

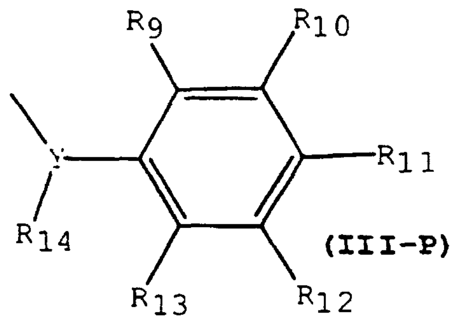
(III-P)

;

$R_2$ is hydrido;

$R_2$ can be selected from the group consisting of aryl, aralkyl, alkyl, alkenyl, alkenyloxyalkyl, haloalkyl, haloalkenyl, halocycloalkyl, haloalkoxy, haloalkoxyalkyl, haloalkenyloxyalkyl, halocycloalkoxy, halocycloalkoxyalkyl, perhaloaryl, perhaloaralkyl, perhaloaryloxyalkyl, heteroaryl, dicyanoalkyl, and carboalkoxycyanoalkyl with the proviso that $R_2$ has a lower Cahn-Ingold-Prelog system ranking than both $R_1$ and $(CHR_3)_n$-N(Ap)Qp;

$R_3$ is selected from the group consisting of hydrido, hydroxy, cyano, aryl, aralkyl, acyl, alkoxy, alkyl, alkenyl, alkoxyalkyl, heteroaryl, alkenyloxyalkyl, haloalkyl, haloalkenyl, haloalkoxy, haloalkoxyalkyl, haloalkenyloxyalkyl, monocyanoalkyl, dicyanoalkyl, carboxamide, and carboxamidoalkyl with the provisos that $(CHR_3)_n$-N(Ap)Qp has a lower Cahn-Ingold-Prelog stereochemical system ranking than $R_1$ and a higher Cahn-Ingold-Prelog stereochemical system ranking than $R_2$;

Y is selected from the group consisting of covalent single bond and $(C(R_{14})_2)_q$ wherein q is an integer selected from 1 through 2;

$R_{14}$ is selected from the group consisting of hydrido, cyano, hydroxyalkyl, acyl, alkoxy, alkyl, alkenyl, alkoxyalkyl, haloalkyl, haloalkenyl, haloalkoxy, haloalkoxyalkyl, haloalkenyloxyalkyl, monocyanoalkyl, dicyanoalkyl, carboxamide, and carboxamidoalkyl;

Z is selected from the group consisting of covalent single bond, $(C(R_{15})_2)_q$ wherein q is an integer selected from 1 through 2, and $(CH(R_{15}))_j$-W-$(CH(R_{15}))_k$ wherein j and k are integers independently selected from 0 through 1;

W is oxy;

$R_{15}$ is selected from the group consisting of hydride, cyano, hydroxyalkyl, acyl, alkoxy, alkyl, alkenyl, alkoxyalkyl, haloalkyl, haloalkenyl, haloalkoxy, haloalkoxyalkyl, haloalkenyloxyalkyl, monocyanoalkyl, dicyanoalkyl, carboxamide, and carboxamidoalkyl;

$R_4$, $R_8$, $R_9$, and $R_{13}$ are independently selected from the group consisting of hydrido, halo, haloalkyl, and alkyl ;

$R_5$, $R_6$, $R_7$, $R_{10}$, $R_{11}$, and $R_{12}$ are independently selected from the group consisting of hydrido, carboxy, heteroaralkylthio, heteroarylsulfonyl, heteroaralkoxy, cycloalkylamino, acylalkyl, acylalkoxy, aroylalkoxy, heterocyclyloxy, aralkylaryl, aralkyl, aralkenyl, aralkynyl, heterocyclyl, haloalkylthio, alkanoyloxy, alkoxy, alkoxyalkyl, cycloalkoxy, cycloalkylalkoxy, hydroxy, amino, thio, nitro, lower alkylamino, alkylthio, arylamino, aralkylamino, arylthio, arylthioalkyl, alkylsulfonyl, alkylsulfonamido, monoarylamidosulfonyl, arylsulfonyl, heteroarylthio, heterocyclylsulfonyl, heterocyclylthio, alkanoyl, alkenoyl, aroyl, alkyl, alkenyl, alkynyl, alkenyloxy, alkylenedioxy, haloalkylenedioxy, cycloalkyl, cycloalkylalkanoyl, halo, haloalkyl, haloalkoxy, hydroxyhaloalkyl, hydroxyalkyl, aryl, aralkyl, aryloxy, aralkoxy, saturated heterocyclyl, heteroaryl, heteroaryloxy, heteroaryloxyalkyl, arylalkyl, heteroarylalkyl, arylalkenyl, carboalkoxy, alkoxycarboxamido, alkylamidocarbonylamido, arylamidocarbonylamido, carboalkoxyalkyl, carboalkoxyalkenyl, carboxamido, carboxamidoalkyl, and cyano;

$R_4$ and $R_5$, $R_5$ and $R_6$, $R_6$ and $R_7$, $R_7$ and $R_8$, $R_9$ and $R_{10}$, $R_{10}$ and $R_{11}$, $R_{11}$ and $R_{12}$, and $R_{12}$ and $R_{13}$ spacer pairs can be independently selected from the group consisting of alkylene, alkenylene, alkylenedioxy, aralkylene, diacyl, haloalkylene, and aryloxylene with the provisos that no more than one of the group consisting of spacer pairs $R_4$ and $R_5$, $R_5$ and $R_6$, $R_6$ and $R_7$, and $R_7$ and $R_8$ can be used at the same time and that no more than one of the group consisting of spacer pairs $R_9$ and $R_{10}$, $R_{10}$ and $R_{11}$, $R_{11}$ and $R_{12}$, and $R_{12}$ and $R_{13}$ can be used at the same time.

In a more preferred embodiment of compounds of Formula 1-C,

n is an integer selected from 1 through 2;

$R_1$ is selected from the group consisting of haloalkyl and haloalkoxymethyl with the proviso that $R_1$ has a higher Cahn-Ingold-Prelog stereochemical system ranking than both $R_2$ and $(CHR_3)_n$-N(Ap)Qp wherein Ap is Formula (II-P) and Qp is Formula (III-P);

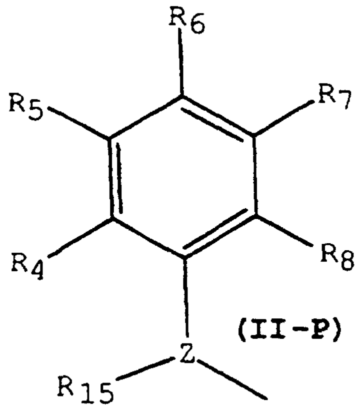
(II-P)
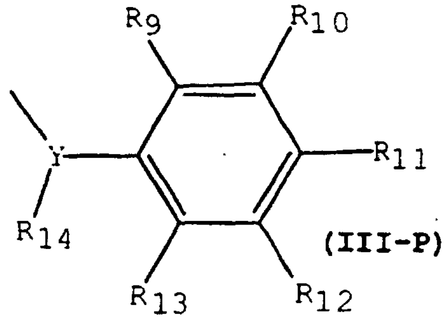
(III-P)
;

$R_{16}$ is hydrido;

$R_2$ is selected from the group consisting of hydrido, aryl, alkyl, alkenyl, haloalkyl, haloalkoxy, haloalkoxyalkyl, perhaloaryl, perhaloaralkyl, perhaloaryloxyalkyl, and heteroaryl with the proviso that $R_2$ has a lower Cahn-Ingold-Prelog system ranking than both $R_1$ and $(CHR_3)_n$-N(Ap)Qp;

$R_3$ is selected from the group consisting of hydrido, aryl, alkyl, alkenyl, haloalkyl, and haloalkoxyalkyl with the provisos that $(CHR_3)_n$-N(Ap)Qp has a lower Cahn-Ingold-Prelog stereochemical system ranking than $R_1$ and a higher Cahn-Ingold-Prelog stereochemical system ranking than $R_2$;

Y is selected from the group consisting of a covalent single bond and alkylene;

Z is selected from the group consisting of a covalent single bond and alkylene;

$R_{14}$ is selected from the group consisting of hydrido, alkyl, and haloalkyl;

$R_{15}$ is selected from the group consisting of hydrido, alkyl, and haloalkyl;

$R_4$, $R_8$, $R_9$, and $R_{13}$ are independently selected from the group consisting of hydride and halo;

$R_5$, $R_6$, $R_7$, $R_{10}$, $R_{11}$, and $R_{12}$ are independently selected from the group consisting of hydrido, alkyl, halo, haloalkyl, haloalkoxy, aryl, alkylthio, arylamino, arylthio, aroyl, arylsulfonyl, aryloxy, aralkoxy, heteroaryloxy, alkoxy, aralkyl, cycloalkoxy, cycloalkylalkoxy, cycloalkylalkanoyl, heteroaryl, cycloalkyl, haloalkylthio, hydroxyhaloalkyl, heteroaralkoxy, heterocyclyloxy, aralkylaryl, heteroaryloxyalkyl, heteroarylthio, and heteroarylsulfonyl.

In an even more preferred embodiment of compounds of Formula I-C,

n is the integer 1;

$R_{16}$ is hydrido;

$R_1$ is haloalkyl with the proviso that $R_1$ has a higher Cahn-Ingold-Prelog stereochemical system ranking than both $R_2$ and $(CHR_3)_n$-N(Ap)Qp wherein Ap is Formula (II-P) and Qp is Formula (III-P);

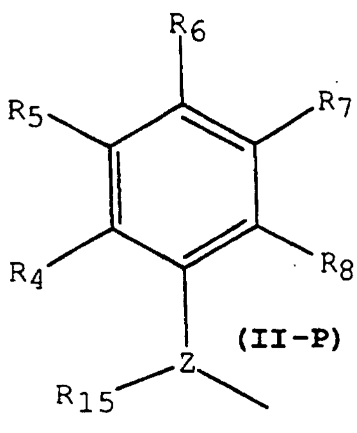
(II-P)
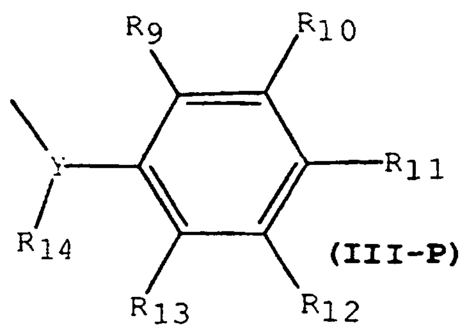
(III-P)
;

$R_2$ is hydride;

$R_2$ can be selected from the group consisting of alkyl, haloalkyl, aryl, and haloalkoxy with the proviso that $R_2$ has a lower Cahn-Ingold-Prelog system ranking than both $R_1$ and $(CHR_3)_n$-N(Ap)Qp;

$R_3$ is selected from the group consisting of hydrido, alkyl, and haloalkyl with the provisos that $(CHR_3)_n$-N(Ap)Qp has a lower Cahn-Ingold-Prelog stereochemical system ranking than $R_1$ and a higher Cahn-Ingold-Prelog stereochemical system ranking than $R_2$;

Y is alkylene;

Z is covalent single bond;

$R_{14}$ is hydrido;

$R_4$, $R_8$, $R_9$, and $R_{13}$ are independently selected from the group consisting of hydride and halo;

$R_5$, $R_6$, $R_7$, $R_{10}$, $R_{11}$, and $R_{12}$ are independently selected from the group consisting of hydrido, alkyl, halo, haloalkyl, haloalkoxy, aryl, alkylthio, arylamino, arylthio, aroyl, arylsulfonyl, aryloxy, aralkoxy, heteroaryloxy, alkoxy, aralkyl, cycloalkoxy, cycloalkylalkoxy, cycloalkylalkanoyl, heteroaryl, cycloalkyl, haloalkylthio, hydroxyhaloalkyl, heteroaralkoxy, and heteroaryloxyalkyl.

In an embodiment of compounds of Formula I-C,

n is an integer selected from 1 to 3;

$R_1$ is selected from the group consisting of trifluoromethyl,

1,1,2,2-tetrafluoroethoxymethyl, trifluoromethoxymethyl, chloromethyl, fluoromethyl, difluoromethyl, chlorodifluoromethyl, pentafluoroethyl, 2,2,3,3,3-pentafluoropropyl, and heptafluoropropyl with the proviso that $R_1$ has a higher Cahn-Ingold-Prelog stereochemical system ranking than both $R_2$ and $(CHR_3)_n$-N(Ap)Qp wherein Ap is Formula (II-P) and Qp is Formula (III-P);

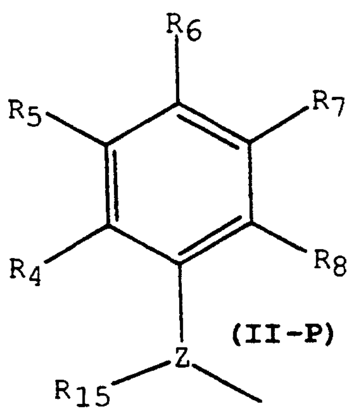

(II-P)

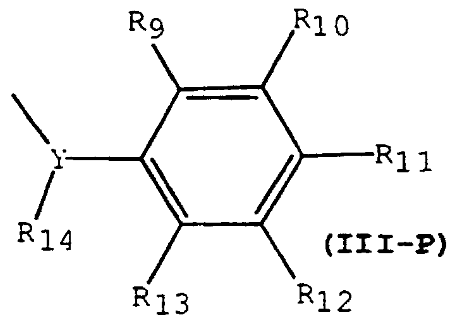

(III-P)

:

$R_{16}$ is selected from the group consisting of acetyl, benzoyl, dimethyl *tert* -butylsilyl, hydrido, and trimethylsilyl;

$R_2$ is hydrido;

$R_2$ can be selected from the group consisting of methyl, ethyl, propyl, butyl, isopropyl, isobutyl, vinyl, phenyl, 4-trifluoromethylphenyl, 1,1,2,2-tetrafluoroethoxymethyl, chloromethyl, trifluoromethoxymethyl, fluoromethyl, difluoromethyl, 2,2,3,3,3-pentafluoropropyl, and and pentafluorophenoxymethyl with the proviso that $R_2$ has a lower Cahn-Ingold-Prelog system ranking than both $R_1$ and $(CHR_3)_n$-N(Ap)Qp;

In a specific sub-group of compounds of Formula I-C, compounds have the Formula I-CP:

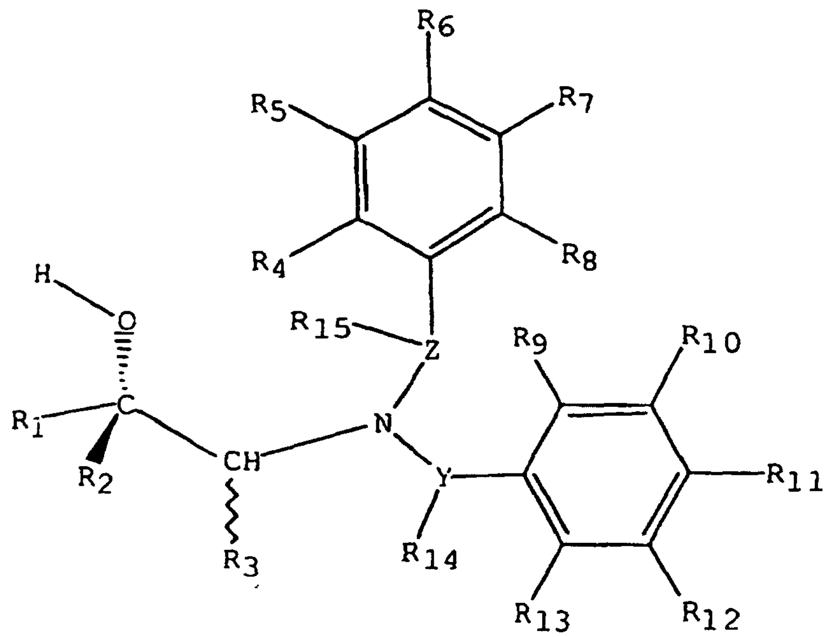

(I-CP)

or a pharmaceutically acceptable salt thereof, wherein;

$R_1$ is selected from the group consisting of trifluoromethyl, 1,1,2,2-tetrafluoroethoxymethyl, trifluoromethoxymethyl, difluoromethyl, chlorodifluoromethyl, and pentafluoroethyl with the proviso that $R_1$ has a higher Cahn-Ingold-Prelog stereochemical system ranking than both $R_2$ and $(CHR_3)_n$-N(Ap)Qp wherein Ap is Formula (II-P) and Qp is Formula (III-P);

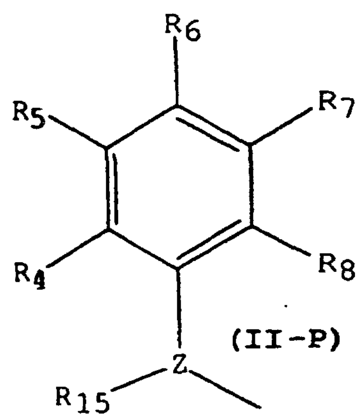

(II-P)

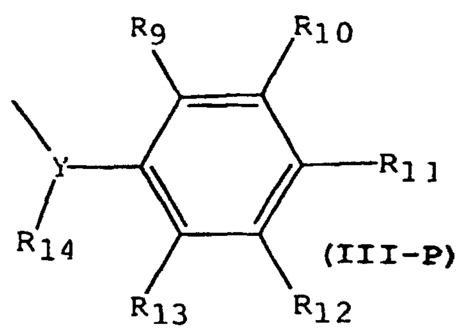

(III-P)

;

$R_2$ is hydrido;

$R_2$ can be selected from the group consisting of methyl, ethyl, propyl, butyl, vinyl, phenyl, 4-trifluoromethylphenyl, 1,1,2,2-tetrafluoroethoxymethyl, trifluoromethoxymethyl, difluoromethyl, and 2,2,3,3,3-pentafluoropropyl with the proviso that $R_2$ has a lower Cahn-Ingold-Prelog system ranking than both $R_1$ and $(CHR_3)_n$-N(Ap)Qp;

$R_3$ is selected from the group consisting of hydrido, phenyl, 4-trifluoromethylphenyl, methyl, ethyl, vinyl, methoxymethyl, trifluoromethyl, trifluoromethoxymethyl, difluoromethyl, chlorodifluoromethyl, and pentafluoroethyl with the provisos that $(CHR_3)_n$-N(Ap)Qp has a lower Cahn-Ingold-Prelog stereochemical system ranking than $R_1$ and a higher Cahn-Ingold-Prelog stereochemical system ranking than $R_2$.

In a specific sub-group of compounds of Formula I-CP,

$R_1$ is selected from the group consisting of trifluoromethyl, 1,1,2,2-tetrafluoroethoxymethyl, trifluoromethoxymethyl, difluoromethyl, chlorodifluoromethyl, and pentafluoroethyl with the proviso that $R_1$ has a higher Cahn-Ingold-Prelog stereochemical system ranking than both $R_2$ and $(CHR_3)_n$-N(Ap)Qp wherein Ap is Formula (II-P) and Qp is Formula (III-P):

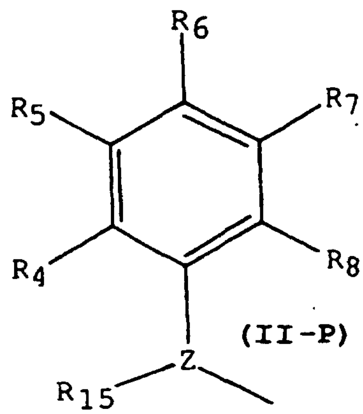 (II-P) 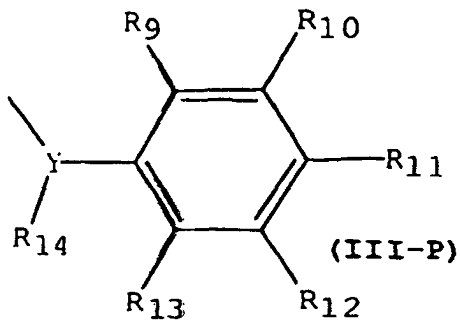 (III-P) ;

$R_2$ is hydrido:

$R_2$ can be selected from the group consisting of methyl, ethyl, phenyl, 4-trifluoromethylphenyl, trifluoromethoxymethyl, 1,1,2,2-tetrafluoroethoxymethyl, difluoromethyl, and 2,2,3,3,3-pentafluoropropyl with the proviso that $R_2$ has a lower Cahn-Ingold-Prelog system ranking than both $R_1$ and $(CHR_3)_n$-N(Ap)Qp;

$R_3$ is selected from the group consisting of hydrido, phenyl, 4-trifluoromethylphenyl, methyl, trifluoromethyl, difluoromethyl, and chlorodifluoromethyl with the provisos that $(CHR_3)_n$-N(Ap)Qp has a lower Cahn-Ingold-Prelog stereochemical system ranking than $R_1$ and a higher Cahn-Ingold-Prelog stereochemical system ranking than $R_2$.

In a further specific sub-group of compounds of Formula I-CP,

$R_1$ is selected from the group consisting of trifluoromethyl, chlorodifluoromethyl, and pentafluoroethyl with the proviso that $R_1$ has a higher Cahn-Ingold-Prelog stereochemical system ranking than both $R_2$ and $(CHR_3)_n$-N(Ap)Qp wherein Ap is Formula (II-P) and Qp is Formula (III-P);

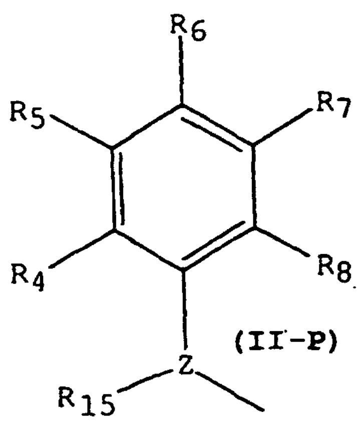 (II-P) 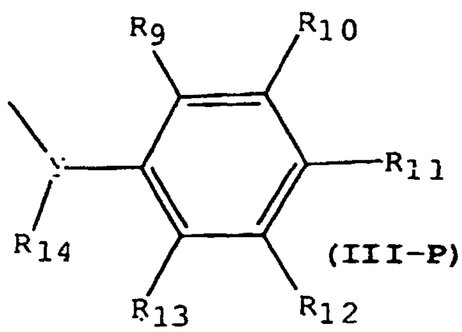 (III-P) ;

$R_2$ is hydrido;

$R_2$ can be phenyl with the proviso that $R_2$ has a lower Cahn-Ingold-Prelog system ranking than both $R_1$ and $(CHR_3)_n$-N(Ap)Qp;

$R_3$ is selected from the group consisting of hydrido, methyl, trifluoromethyl, and difluoromethyl with the provisos that $(CHR_3)_n$-N(Ap)Qp has a lower Cahn-Ingold-Prelog stereochemical system ranking than $R_1$ and a higher Cahn-Ingold-Prelog stereochemical system ranking than $R_2$.

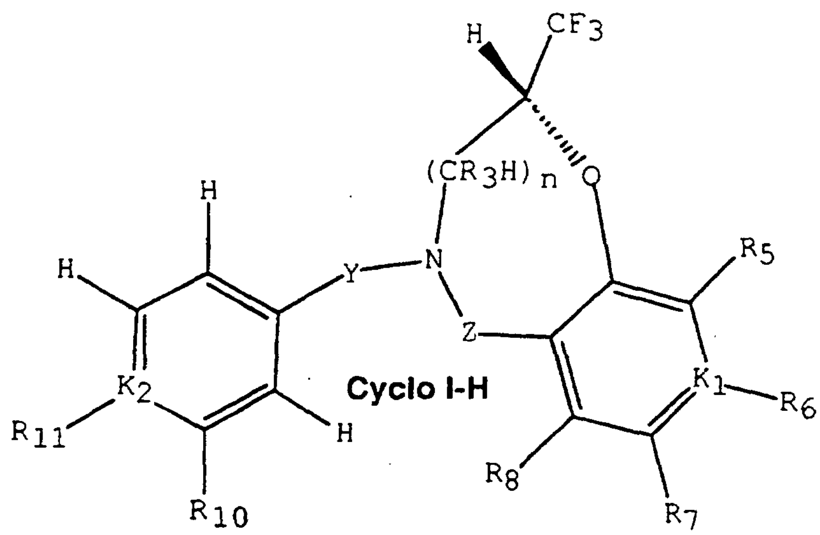

Cyclo I-H

wherein:

$K_1$ and $K_2$ are independently selected from the group consisting of C and N;

n is an integer selected from 1 through 3:

$R_1$ is selected from the group consisting of haloalkyl, haloalkenyl, haloalkoxymethyl, and haloalkenyloxymethyl with the proviso that $R_1$ has a higher Cahn-Ingold-Prelog stereochemical system ranking than both $R_2$ and $(CHR_3)_n$-N(Apch)Qph wherein Apch is Formula (II-PCH) and Qph is Formula (III-PH);

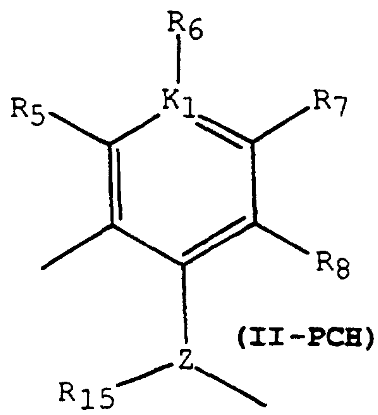

(II-PCH)

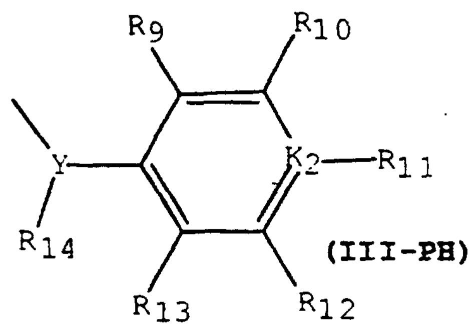

(III-PH)

;

$R_2$ is hydrido;

$R_2$ is selected from the group consisting of aryl, aralkyl, alkyl, alkenyl, alkoxyalkyl, alkenyloxyalkyl, haloalkyl, haloalkenyl, halocycloalkyl, haloalkoxy, haloalkoxyalkyl, haloalkenyloxyalkyl, halocycloalkoxy, perhaloaryl, perhaloaralkyl, perhaloaryloxyalkyl, heteroaryl, heteroarylalkyl, dicyanoalkyl, and carboalkoxycyanoalkyl with the proviso that $R_2$ has a lower Cahn-Ingold-Prelog system ranking than both $R_1$ and $(CHR_3)_n$-N(Apch)Qph;

$R_3$ is selected from the group consisting of hydride, hydroxy, halo, cyano, aryl. aralkyl, acyl, alkoxy, alkyl, alkenyl, alkoxyalkyl, heteroaryl, alkenyloxyalkyl, haloalkyl, haloalkenyl, haloalkoxy, haloalkoxyalkyl, haloalkenyloxyalkyl, monocyanoalkyl, dicyanoalkyl, carboalkoxycyanoalkyl, carboxamide, and carboxamidoalkyl with the provisos that $(CHR_3)_n$-N(Apch)Qph has a lower Cahn-Ingold-Prelog stereochemical system ranking than $R_1$ and a higher Cahn-Ingold-Prelog stereochemical system ranking than $R_2$;

Y is selected from the group consisting of a covalent single bond and $(C(R_{14})_2)_q$ wherein q is an integer selected from 1 through 2;

$R_{14}$ is selected from the group consisting of hydride, hydroxy, cyano, hydroxyalkyl, acyl, alkoxy, alkyl, alkenyl, alkynyl, alkoxyalkyl, haloalkyl, haloalkenyl, haloalkoxy, haloalkoxyalkyl, haloalkenyloxyalkyl, monocarboalkoxyalkyl,

monocyanoalkyl, dicyanoalkyl, carboalkoxycyanoalkyl, carboalkoxy, carboxamide, and carboxamidoalkyl;

Z is selected from the group consisting of covalent single bond, $(C(R_{15})_2)_q)$ wherein q is an integer selected from 1 through 2, and $(CH(R_{15}))_j$-W-$(CH(R_{15}))_k$ wherein j and k are integers independently selected from 0 through 1;

W is selected from the group consisting of O, C(O), S, S(O), and $S(O)_2$;

$R_{15}$ is selected from the group consisting of hydrido, cyano, hydroxyalkyl, acyl, alkoxy, alkyl, alkenyl, alkynyl, alkoxyalkyl, haloalkyl, haloalkenyl, haloalkoxy, haloalkoxyalkyl, haloalkenyloxyalkyl, monocarboalkoxyalkyl, monocyanoalkyl, dicyanoalkyl, carboalkoxycyanoalkyl, carboalkoxy, carboxamide, and carboxamidoalkyl;

$R_8$, $R_9$, and $R_{13}$ are independently selected from the group consisting of hydrido, halo, haloalkyl, and alkyl ;

$R_5$, $R_6$, $R_7$, $R_{10}$, $R_{11}$, and $R_{12}$ are independently selected from the group consisting of hydrido, carboxy, heteroaralkylthio, heteroaralkoxy, cycloalkylamino, acylalkyl, acylalkoxy, aroylalkoxy, heterocyclyloxy, aralkylaryl, aralkyl, aralkenyl, aralkynyl, heterocyclyl, perhaloaralkyl, aralkylsulfonyl, aralkylsulfonylalkyl, aralkylsulfinyl, aralkylsulfinylalkyl, halocycloalkyl, halocycloalkenyl, cycloalkylsulfinyl, cycloalkylsulfinylalkyl, cycloalkylsulfonyl, cycloalkylsulfonylalkyl, heteroarylamino, N-heteroarylamino-N-alkylamino, heteroarylaminoalkyl,haloalkylthio, alkanoyloxy, alkoxy, alkoxyalkyl, haloalkoxylalkyl, heteroaralkoxy, cycloalkoxy, cycloalkenyloxy, cycloalkoxyalkyl, cycloalkylalkoxy, cycloalkenyloxyalkyl, cycloalkylenedioxy, halocycloalkoxy, halocycloalkoxyalkyl, halocycloalkenyloxy, halocycloalkenyloxyalkyl, hydroxy, amino, thio, nitro, lower alkylamino, alkylthio, alkylthioalkyl, arylamino, aralkylamino, arylthio, arylthioalkyl, heteroaralkoxyalkyl, alkylsulfinyl, alkylsulfinylalkyl, arylsulfinylalkyl, arylsulfonylalkyl, heteroarylsulfinylalkyl, heteroarylsulfonylalkyl, alkylsulfonyl, alkylsulfonylalkyl, haloalkylsulfinylalkyl, haloalkylsulfonylalkyl, alkylsulfonamido, alkylaminosulfonyl, amidosulfonyl, monoalkyl amidosulfonyl, dialkyl amidosulfonyl, monoarylamidosulfonyl, arylsulfonamido, diarylamidosulfonyl, monoalkyl monoaryl amidosulfonyl, arylsulfinyl, arylsulfonyl, heteroarylthio, heteroarylsulfinyl, heteroarylsulfonyl, heterocyclylsulfonyl, heterocyclylthio, alkanoyl, alkenoyl, aroyl, heteroaroyl, aralkanoyl, heteroaralkanoyl, haloalkanoyl, alkyl, alkenyl, alkynyl, alkenyloxy, alkenyloxyalky, alkylenedioxy, haloalkylenedioxy, cycloalkyl, cycloalkylalkanoyl, cycloalkenyl, lower cycloalkylalkyl, lower cycloalkenylalkyl, halo, haloalkyl, haloalkenyl, haloalkoxy, hydroxyhaloalkyl, hydroxyaralkyl, hydroxyalkyl, hydoxyheteroaralkyl, haloalkoxyalkyl, aryl, heteroaralkynyl, aryloxy, aralkoxy, aryloxyalkyl, saturated heterocyclyl, partially saturated heterocyclyl, heteroaryl, heteroaryloxy, heteroaryloxyalkyl, heteroaralkyl, arylalkenyl, heteroarylalkenyl, carboxyalkyl, carboalkoxy, alkoxycarboxamido, alkylamidocarbonylamido, arylamidocarbonylamido, carboalkoxyalkyl, carboalkoxyalkenyl, carboaralkoxy, carboxamido, carboxamidoalkyl, cyano, carbohaloalkoxy, phosphono, phosphonoalkyl, diaralkoxyphosphono, and diaralkoxyphosphonoalkyl;

$R_5$ and $R_6$, $R_6$ and $R_7$, $R_7$ and $R_8$, $R_9$ and $R_{10}$, $R_{10}$ and $R_{11}$, $R_{11}$ and $R_{12}$, and $R_{12}$ and $R_{13}$ can be independently selected to form spacer pairs wherein a spacer pair is taken together to form a linear moiety having from 3 through 6 contiguous atoms connecting the points of bonding of said spacer pair members to form a ring selected from the group consisting of a cycloalkenyl ring having 5 through 8 contiguous members, a partially saturated heterocyclyl ring having 5 through 8 contiguous members, a heteroaryl ring having 5 through 6 contiguous members, and an aryl with the provisos that no more than one of the group consisting of spacer pairs $R_5$ and $R_6$, $R_6$ and $R_7$, and $R_7$ and $R_8$, can be used at the same time and that no more than one of the group consisting of spacer pairs $R_9$ and $R_{10}$, $R_{10}$ and $R_{11}$, $R_{11}$ and $R_{12}$, and $R_{12}$ and $R_{13}$ can be used at the same time.

In an embodiment of compounds of Formula Cyclo I-H,

n is the integer 1;

$R_1$ is selected from the group consisting of trifluoromethyl, 1,1,2,2-tetrafluoroethoxymethyl, trifluoromethoxymethyl, difluoromethyl, chlorodifluoromethyl, and pentafluoroethyl with the proviso that $R_1$ has a higher Cahn-Ingold-Prelog stereochemical system ranking than both $R_2$ and $(CHR_3)_n$-N(Apch)Qph wherein Apch is Formula (II-PCH) and Qph is Formula (III-PH);

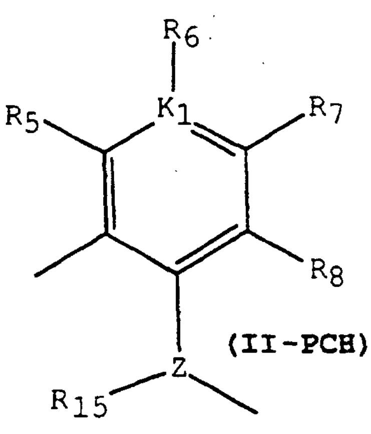

(II-PCH)

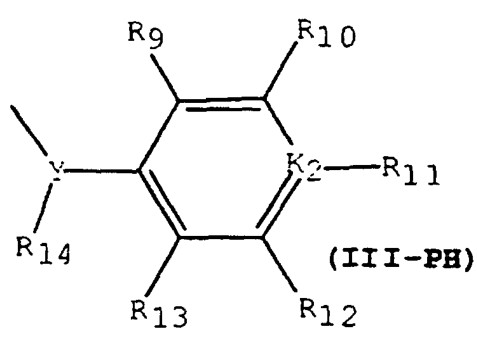

(III-PH)

:

$R_2$ is hydrido;

$R_2$ is selected from the group consisting of phenyl, 4-trifluoromethylphenyl, vinyl, 1,1,2,2-tetrafluoroethoxymethyl, trifluoromethoxymethyl, difluoromethyl, and 2,2,3,3,3-pentafluoropropyl with the proviso that $R_2$ has a lower Cahn-Ingold-Prelog system ranking than both $R_1$ and $(CHR_3)_n$-N(Apch)Qph;

$R_3$ is selected from the group consisting of hydrido, methyl, ethyl, vinyl, phenyl, 4-trifluoromethylphenyl, methoxymethyl, trifluoromethyl, trifluoromethoxymethyl, difluoromethyl, chlorodifluoromethyl, and pentafluoroethyl with the provisos that $(CHR_3)_n$-N(Apch)Qph has a lower Cahn-Ingold-Prelog stereochemical system ranking than $R_1$ and a higher Cahn-Ingold-Prelog stereochemical system ranking than $R_2$.

In another embodiment of compounds of Formula Cyclo I-H,

n is the integer 1;

$R_1$ is selected from the group consisting of trifluoromethyl, 1,1,2,2-tetrafluoroethoxymethyl, trifluoromethoxymethyl, difluoromethyl, chlorodifluoromethyl, and pentafluoroethyl;

$R_2$ is hydrido;

In a preferred embodiment of compounds of the invention,

Y is selected from the group consisting of methylene, ethylene, and ethylidene;

Z is covalent single bond;

$R_4$, $R_8$, $R_9$, and $R_{13}$ are independently selected from the group consisting of hydride and fluoro with the proviso that there is no $R_4$, $R_8$, $R_9$, or $R_{13}$ when the embodiment is a compound of Formula Cyclo I-H;

$R_5$ and $R_{10}$ are independently selected from the group consisting of 4-aminophenoxy, benzoyl, benzyl, benzyloxy, 5-bromo-2-fluorophenoxy, 4-bromo-3-fluorophenoxy, 4-bromo-2-nitrophenoxy, 3-bromobenzyloxy, 4-bromobenzytoxy, 4-bromophenoxy, 5-bromopyrid-2-yloxy, 4-butoxyphenoxy, chloro, 3-chlorobenzyl, 2-chlorophenoxy, 4-chlorophenoxy, 4-chloro-3-ethylphenoxy, 3-chloro-4-fluorobenzyl, 3-chloro-4-fluorophenyl, 3-chloro-2-fluorobenzyloxy, 3-chlorobenzyloxy, 4-chlorobenzyloxy, 4-chloro-3-methylphenoxy, 2-chloro-4-fluorophenoxy, 4-chloro-2-fluorophenoxy, 4-chlorophenoxy, 3-chloro-4-ethylphenoxy, 3-chloro-4-methylphenoxy, 3-chloro-4-fluorophenoxy, 4-chloro-3-fluorophenoxy, 4-chlorophenylamino, 5-chloropyrid-3-yloxy, 2-cyanopyrid-3-yloxy, 4-cyanophenoxy, cyclobutoxy, cyclobutyl, cyclohexoxy, cyclohexylmethoxy, cyclopentoxy, cyclopentyl, cyclopentylcarbonyl, cyclopropyl, cyclopropylmethoxy, cyclopropoxy, 2,3-dichlorophenoxy, 2,4-dichlorophenoxy, 2,4-dichlorophenyl, 3,5-dichlorophenyl, 3,5-dichlorobenzyl, 3,4-dichlorophenoxy, 3,4-difluorophenoxy, 2,3-difluorobenzyloxy, 2,4-difluorobenzyloxy, 3,4-difluorobenzyloxy, 2,5-difluorobenzyloxy, 3,5-difluorophenoxy, 3,4-difluorophenyl, 3,5-difluorobenzyloxy, 4-difluoromethoxybenzyloxy, 2,3-difluorophenoxy, 2,4-difluorophenoxy, 2,5-difluorophenoxy, 3,5-dimethoxyphenoxy, 3-dimethylaminophenoxy, 3,5-dimethylphenoxy, 3,4-dimethylphenoxy, 3,4-dimethylbenzyl, 3,4-dimethylbenzyloxy, 3,5-dimethylbenzyloxy, 2,2-dimethylpropoxy, 1,3-dioxan-2-yl, 1,4-dioxan-2-yl, 1,3-dioxolan-2-yl, ethoxy, 4-ethoxyphenoxy, 4-ethylbenzyloxy, 3-ethylphenoxy, 4-ethylaminophenoxy, 3-ethyl-5-methylphenoxy, fluoro, 4-fluoro-3-methylbenzyl, 4-fluoro-3-methylphenyl, 4-fluoro-3-methylbenzoyl, 4-fluorobenzyloxy, 2-fluoro-3-methylphenoxy, 3-fluoro-4-methylphenoxy, 3-fluorophenoxy, 3-fluoro-2-nitrophenoxy, 2-fluoro-3-trifluoromethylbenzyloxy, 3-fluoro-5-trifluoromethylbenzyloxy, 4-fluoro-2-trifluoromethylbenzyloxy, 4-fluoro-3-trifluoromethylbenzyloxy, 2-fluorophenoxy, 4-fluorophenoxy, 2-fluoro-3-trifluoromethylphenoxy, 2-fluorobenzyloxy, 4-fluorophenylamino, 2-fluoro-4-trifluoromethylphenoxy, 4-fluoropyrid-2-yloxy, 2-furyl, 3-furyl, heptafluoropropyl, 1,1,1,3,3,3-hexafluoropropyl, 2-hydroxy-3,3,3-trifluoropropoxy, 3-iodobenzyloxy, isobutyl, isobutylamino, isobutoxy, 3-isoxazolyl, 4-isoxazolyl, 5-isoxazolyl, isopropoxy, isopropyl, 4-isopropylbenzyloxy, 3-isopropylphenoxy, 4-isopropylphenoxy, isopropylthio, 4-isopropyl-3-methylphenoxy, 3-isothiazolyl, 4-isothiazolyl, 5-isothiazolyl, 3-methoxybenzyl, 4-methoxycarbonylbutoxy, 3-methoxycarbonylprop-2-enyloxy, 4-methoxyphenyl, 3-methoxyphenylamino, 4-methoxyphenylamino, 3-methylbenzyloxy, 4-methylbenzyloxy, 3-methylphenoxy, 3-methyl-4-methylthiophenoxy, 4-methylphenoxy, 1-methylpropoxy, 2-methylpyrid-5-yloxy, 4-methylthiophenoxy, 2-naphthyloxy, 2-nitrophenoxy, 4-nitrophenoxy, 3-nitrophenyl, 4-nitrophenylthio, 2-oxazolyl, 4-oxazolyl, 5-oxazolyl, pentafluoroethyl, pentafluoroethylthio, 2,2,3,3,3-pentafluoropropyl, 1,1,3,3,3-pentafluoropropyl, 1,1,2,2,3-pentafluoropropyl, phenoxy, phenylamino, 1-phenylethoxy, phenylsulfonyl, 4-propanoylphenoxy, propoxy, 4-propylphenoxy, 4-propoxyphenoxy, thiophen-3-yl, *sec*-butyl, 4-*sec*-butylphenoxy,*tert* -butoxy, 3-*tert* -butylphenoxy, 4-*tert* -butylphenoxy, 1,1,2,2-tetrafluoroethoxy, tetrahydrofuran-2-yl, 2-(5,6,7,8-tetrahydronaphthyloxy), thiazol-2-yl, thiazol-4-yl, thiazol-5-yl, thiophen-2-yl, 2,3,5-trifluorobenzyloxy, 2,2,2-trifluoroethoxy, 2,2,2-trifluoroethyl, 3,3,3-trifluoro-2-hydroxypropyl, trifluoromethoxy, 3-trifluoromethoxybenzyloxy, 4-trifluoromethoxybenzyloxy, 3-trifluoromethoxyphenoxy, 4-trifluoromethoxyphenoxy, trifluoromethyl, 3-trifluoromethylbenzyloxy, 4-trifluoromethylbenzyloxy, 2,4-bis-trifluoromethylbenzyloxy, 1,1-bis-trifluoromethyl-1-hydroxymethyl, 3-trifluoromethylbenzyl, 3,5-bis-trifluoromethylbenzyloxy, 4-trifluoromethylphenoxy, 3-trifluoromethylphenoxy, 3-trifluoromethylphenyl, 3-trifluoromethylthiobenzyloxy, 4-trifluoromethylthiobenzyloxy, 2,3,4-trifluorophenoxy, 2,3,4-trifluorophenyl, 2,3,5-trifluorophenoxy, 3,4,5-trimethylphenoxy, 3-difluoromethoxyphenoxy, 3-pentafluoroethylphenoxy, 3-(1,1,2,2-tetrafluoroethoxy)phenoxy, 3-trifluoromethylthiophenoxy, and trifluoromethylthio;

$R_6$ and $R_{11}$ are independently selected from the group consisting of chloro, fluoro, hydrido, pentafluoroethyl, 1,1,2,2-tetrafluoroethoxy, trifluoromethyl, and trifluoromethoxy;

$R_7$ and $R_{12}$ are independently selected from the group consisting of hydrido, fluoro, and trifluoromethyl.

In an even more preferred embodiment of compounds of the invention,

Y is methylene;

Z is covalent single bond;

$R_4$, $R_8$, $R_9$, and $R_{13}$ are independently selected from the group consisting of hydrido and fluoro with the proviso that there is no $R_4$, $R_8$, $R_9$, or $R_{13}$ when the embodiment is a compound of Formula Cyclo I-H;

$R_5$ and $R_{10}$ are independently selected from the group consisting of benzyloxy, 5-bromo-2-fluorophenoxy, 4-bromo-3-fluorophenoxy, 3-bromobenzyloxy, 4-bromophenoxy,4-butoxyphenoxy, 3-chlorobenzyloxy, 2-chlorophenoxy, 4-chloro-3-ethylphenoxy, 4-chloro-3-methylphenoxy, 2-chloro-4-fluorophenoxy, 4-chloro-2-fluorophenoxy, 4-chlorophenoxy, 3-chloro-4-ethylphenoxy, 3-chloro-4-methylphenoxy,3-chloro-4-fluorophenoxy, 4-chloro-3-fluorophenoxy, 4-chlorophenylamino, 5-chloropyrid-3-yloxy, cyclobutoxy, cyclobutyl, cyclohexylmethoxy, cyclopentoxy, cyclopentyl, cyclopentylcarbonyl, cyclopropylmethoxy, 2,3-dichlorophenoxy, 2,4-dichlorophenoxy, 2,4-dichlorophenyl, 3.5-dichlorophenyl, 3,5-dichlorobenzyl, 3,4-dichlorophenoxy, 3,4-difluorophenoxy, 2,3-difluorobenzyloxy, 3,5-difluorobenzyloxy, difluoromethoxy, 3,5-difluorophenoxy, 3,4-difluorophenyl, 2,3-difluorophenoxy, 2,4-difluorophenoxy, 2,5-difluorophenoxy, 3,5-dimethoxyphenoxy, 3-dimethylaminophenoxy, 3,4-dimethylbenzyloxy, 3,5-dimethylbenzyloxy, 3,5-dimethylphenoxy, 3,4-dimethylphenoxy, 1,3-dioxolan-2-yl, 3-ethylbenzyloxy, 3-ethylphenoxy, 4-ethylaminophenoxy, 3-ethyl-5-methylphenoxy, 4-fluoro-3-methylbenzyl, 4-fluorobenzyloxy, 2-fluoro-3-methylphenoxy, 3-fluoro-4-methylphenoxy, 3-fluorophenoxy, 3-fluoro-2-nitrophenoxy, 2-fluoro-3-trifluoromethylbenzyloxy, 3-fluoro-5-trifluoromethylbenzyloxy, 2-fluorophenoxy,4-fluorophenoxy, 2-fluoro-3-trifluoromethylphenoxy, 2-fluorobenzyloxy, 4-fluorophenylamino, 2-fluoro-4-trifluoromethylphenoxy, 2-furyl, 3-furyl, heptafluoropropyl, 1,1,1,3,3,3-hexafluoropropyl, 2-hydroxy-3,3,3-trifluoropropoxy, isobutoxy, isobutyl, 3-isoxazolyl, 4-isoxazolyl, 5-isoxazolyl, isopropoxy, 3-isopropylbenzyloxy, 3-isopropylphenoxy, isopropylthio, 4-isopropyl-3-methylphenoxy, 3-isothiazolyl, 4-isothiazolyl, 5-isothiazolyl, 3-methoxybenzyl, 4-methoxyphenylamino, 3-methylbenzyloxy, 4-methylbenxyloxy, 3-methylphenoxy, 3-methyl-4-methylthiophenoxy, 4-methylphenoxy, 1-methylpropoxy, 2-methylpyrid-5-yloxy, 4-methylthiophenoxy, 2-naphthyloxy, 2-nitrophenoxy, 4-nitrophenoxy, 3-nitrophenyl, 2-oxazolyl, 4-oxazolyl, 5-oxazolyl, pentafluoroethyl, pentafluoroethylthio, 2,2,3,3,3-pentafluoropropyl, 1,1,3,3,3-pentafluoropropyl, 1,1,2,2,3-pentafluoropropyl, phenoxy, phenylamino, 1-phenylethoxy, 4-propylphenoxy, 4-propoxyphenoxy, thiophen-3-yl.tert -butoxy, 3-tert -butylphenoxy, 4-tert -butylphenoxy, 1,1,2,2-tetrafluoroethoxy, tetrahydrofuran-2-yl, 2-(5,6,7,8-tetrahydronaphthyloxy), thiazol-2-yl, thiazol-4-yl, thiazol-5-yl, thiophen-2-yl, 2,2,2-trifluoroethoxy, 2,2,2-trifluoroethyl, 3,3,3-trifluoro-2-hydroxypropyl, trifluoromethoxy, 3-trifluoromethoxybenzyloxy, 4-trifluoromethoxybenzyloxy, 4-trifluoromethoxyphenoxy, 3-trifluoromethoxyphenoxy, trifluoromethyl, 3-trifluoromethylbenzyloxy, 1,1-bis-trifluoromethyl-1-hydroxymethyl, 3-trifluoromethylbenzyl, 3,5-bis-trifluoromethylbenzyloxy, 4-trifluoromethylphenoxy, 3-trifluoromethylphenoxy, 3-trifluoromethylphenyl, 2,3,4-trifluorophenoxy, 2,3,5-trifluorophenoxy, 3,4,5-trimethylphenoxy, 3-difluoromethoxyphenoxy, 3-pentafluoroethylphenoxy, 3-(1,1,2,2-tetrafluoroethoxy) phenoxy, 3-trifluoromethylthiophenoxy, 3-trifluoromethylthiobenzyloxy, and trifluoromethylthio;

$R_6$ and $R_{11}$ are independently selected from the group consisting of chloro, fluoro, hydrido, pentafluoroethyl, 1,1,2,2-tetrafluoroethoxy, and trifluoromethyl;

$R_7$ and $R_{12}$ are independently selected from the group consisting of hydrido, fluoro, and trifluoromethyl.

In a most preferred embodiment of compounds of the invention

Y is methylene;

Z is covalent single bond;

$R_4$, $R_8$, $R_9$, and $R_{13}$ are independently selected from the group consisting of hydrido and fluoro with the proviso that there is no $R_4$, $R_8$, $R_9$, or $R_{13}$ when the embodiment is a compound of Formula Cyclo I-H;

$R_5$ is selected from the group consisting of 5-bromo-2-fluorophenoxy, 4-chloro-3-ethylphenoxy, 2,3-dichlorophenoxy, 3,4-dichlorophenoxy, 3-difluoromethoxyphenoxy, 3,5-dimethylphenoxy, 3,4-dimethylphenoxy, 3-ethylphenoxy, 3-ethyl-5-methylphenoxy, 4-fluoro-3-methylphenoxy, 4-fluorophenoxy, 3-isopropylphenoxy, 3-methylphenoxy, 3-pentafluoroethylphenoxy, 3-tert -butylphenoxy, 3-(1,1,2,2-tetrafluoroethoxy)phenoxy, 2-(5,6,7,8-tetrahydronaphthyloxy), 3-trifluoromethoxybenzyloxy,3-trifluoromethoxyphenoxy, 3-trifluoromethylbenzyloxy, and 3-trifluoromethylthiophenoxy:

$R_{10}$ is selected from the group consisting of cyclopentyl, 1,1,2,2-tetrafluoroethoxy, 2-furyl, 1,1-bis-trifluoromethyl-1-hydroxymethyl, isobutyl, isopropoxy, pentafluoroethyl, trifluoromethoxy, trifluoromethyl, and trifluoromethylthio;

$R_6$ and $R_{11}$ are independently selected from the group consisting of fluoro and hydrido;

$R_7$ and $R_{12}$ are independently selected from the group consisting of hydrido and fluoro.

## DEFINITIONS

The use of generic terms in the description of the compounds are herein defined for clarity.

Standard single letter elemental symbols are used to represent specific types of atoms unless otherwise defined. The symbol "C" represents a carbon atom. The symbol "O" represents an oxygen atom. The symbol "N"

represents a nitrogen atom. The symbol "P" represents a phosphorus atom. The symbol "S" represents a sulfur atom. The symbol "H" represents a hydrogen atom. Double letter elemental symbols are used as defined for the elements of the periodical table (i.e., Cl represents chlorine, Se represents selenium, etc.).

As utilized herein, the term "alkyl", either alone or within other terms such as "haloalkyl" and "alkylthio", means an acyclic alkyl radical containing from 1 to about 10, preferably from 1 to about 8 carbon atoms and more preferably 1 to about 6 carbon atoms. Said alkyl radicals may be optionally substituted with groups as defined below. Examples of such radicals include methyl, ethyl, chloroethyl, hydroxyethyl, n-propyl, oxopropyl, isopropyl, n-butyl, cyanobutyl, isobutyl, sec-butyl, tert-butyl, pentyl, aminopentyl, iso-amyl, hexyl, octyl and the like.

The term "alkenyl" refers to an unsaturated, acyclic hydrocarbon radical in so much as it contains at least one double bond. Such alkenyl radicals contain from about 2 to about 10 carbon atoms, preferably from about 2 to about 8 carbon atoms and more preferably 2 to about 6 carbon atoms. Said alkenyl radicals may be optionally substituted with groups as defined below. Examples of suitable alkenyl radicals include propenyl, 2-chloropropenyl, buten-1-yl, isobutenyl, penten-1-yl, 2-2-methylbuten-1-yl, 3-methylbuten-1-yl, hexen-1-yl, 3-hydroxyhexen-1-yl, hepten-1-yl, and octen-1-yl, and the like.

The term "alkynyl" refers to an unsaturated, acyclic hydrocarbon radical in so much as it contains one or more triple bonds, such radicals containing about 2 to about 10 carbon atoms, preferably having from about 2 to about 8 carbon atoms and more preferably having 2 to about 6 carbon atoms. Said alkynyl radicals may be optionally substituted with groups as defined below. Examples of suitable alkynyl radicals include ethynyl, propynyl, hydroxypropynyl, butyn-1-yl, butyn-2-yl, pentyn-1-yl, pentyn-2-yl, 4-methoxypentyn-2-yl, 3-methylbutyn-1-yl, hexyn-1-yl, hexyn-2-yl, hexyn-3-yl, 3,3-dimethylbutyn-1-yl radicals and the like.

The term "hydrido" denotes a single hydrogen atom (H). This hydrido radical may be attached, for example, to an oxygen atom to form a "hydroxyl" radical, one hydrido radical may be attached to a carbon atom to form a "methine" radical (=CH-), or two hydrido radicals may be attached to a carbon atom to form a "methylene" ($-CH_2-$) radical.

The term "carbon" radical denotes a carbon atom without any covalent bonds and capable of forming four covalent bonds.

The term "cyano" radical denotes a carbon radical having three of four covalent bonds shared by a nitrogen atom.

The term "hydroxyalkyl" embraces radicals wherein any one or more of the alkyl carbon atoms is substituted with a hydroxyl as defined above. Specifically embraced are monohydroxyalkyl, dihydroxyalkyl and polyhydroxyalkyl radicals.

The term "alkanoyl" embraces radicals wherein one or more of the terminal alkyl carbon atoms are substituted with one or more carbonyl radicals as defined below. Specifically embraced are monocarbonylalkyl and dicarbonylalkyl radicals. Examples of monocarbonylalkyl radicals include formyl, acetyl, and pentanoyl. Examples of dicarbonylalkyl radicals include oxalyl, malonyl, and succinyl.

The term "alkylene" radical denotes linear or branched radicals having from 1 to about 10 carbon atoms and having attachment points for two or more covalent bonds. Examples of such radicals are methylene, ethylene, ethylidene, methylethylene, and isopropylidene.

The term "alkenylene" radical denotes linear or branched radicals having from 2 to about 10 carbon atoms, at least one double bond, and having attachment points for two or more covalent bonds. Examples of such radicals are 1,1-vinylidene ($CH_2=C$), 1,2-vinylidene (-CH=CH-), and 1,4-butadienyl (-CH=CH-CH=CH-).

The term "halo" means halogens such as fluorine, chlorine, bromine or iodine atoms.

The term "haloalkyl" embraces radicals wherein any one or more of the alkyl carbon atoms is substituted with halo as defined above. Specifically embraced are monohaloalkyl, dihaloalkyl and polyhaloalkyl radicals. A monohaloalkyl radical, for one example, may have either a bromo, chloro or a fluoro atom within the radical. Dihalo radicals may have two or more of the same halo atoms or a combination of different halo radicals and polyhaloalkyl radicals may have more than two of the same halo atoms or a combination of different halo radicals. More preferred haloalkyl radicals are "lower haloalkyl" radicals having one to about six carbon atoms. Examples of such haloalkyl radicals include fluoromethyl, difluoromethyl, trifluoromethyl, chloromethyl, dichloromethyl, trichloromethyl, trifluoroethyl, pentafluoroethyl, heptafluoropropyt, difluorochtoromethyl, dichlorofluoromethyl, difluoroethyl, difluoropropyl, dichloroethyl and dichloropropyl.

The term "hydroxyhaloalkyl" embraces radicals wherein any one or more of the haloalkyl carbon atoms is substituted with hydroxy as defined above. Examples of "hydroxyhaloalkyl" radicals include hexafluorohydoxypropyl.

The term "haloalkylene radical" denotes alkylene radicals wherein any one or more of the alkylene carbon atoms is substituted with halo as defined above. Dihalo alkylene radicals may have two or more of the same halo atoms or a combination of different halo radicals and polyhaloalkylene radicals may have more than two of the same halo atoms or a combination of different halo radicals. More preferred haloalkylene radicals are "lower haloalkylene" radicals having one to about six carbon atoms. Examples of "haloalkylene" radicals include difluoromethylene, tetrafluoroethylene, tetrachloroethylene, alkyl substituted monofluoromethylene, and aryl substituted trifluoromethylene.

The term "haloalkenyl" denotes linear or branched radicals having from 1 to about 10 carbon atoms and having one or more double bonds wherein any one or more of the alkenyl carbon atoms is substituted with halo as defined above. Dihaloalkenyl radicals may have two or more of the same halo atoms or a combination of different halo radicals and polyhaloalkenyl radicals may have more than two of the same halo atoms or a combination of different halo radicals.

The terms "alkoxy" and "alkoxyalkyl" embrace linear or branched oxy-containing radicals each having alkyl portions of one to about ten carbon atoms, such as methoxy radical. The term "alkoxyalkyl" also embraces alkyl radicals having one or more alkoxy radicals attached to the alkyl radical, that is, to form monoalkoxyalkyl and dialkoxyalkyl radicals. More preferred alkoxy radicals are "lower alkoxy" radicals having one to six carbon atoms. Examples of such radicals include methoxy, ethoxy, propoxy, butoxy, isopropoxy and *tert*-butoxy alkyls. The "alkoxy" radicals may be further substituted with one or more halo atoms, such as fluoro, chloro or bromo, to provide "haloalkoxy" and "haloalkoxyalkyl" radicals. Examples of such haloalkoxy radicals include fluoromethoxy, chloromethoxy, trifluoromethoxy, difluoromethoxy, trifluoroethoxy, fluoroethoxy, tetrafluoroethoxy, pentafluoroethoxy, and fluoropropoxy. Examples of such haloalkoxyalkyl radicals include fluoromethoxymethyl, chloromethoxyethyl, trifluoromethoxymethyl, difluoromethoxyethyl, and trifluoroethoxymethyl.

The terms "alkenyloxy" and "alkenyloxyalkyl" embrace linear or branched oxy-containing radicals each having alkenyl portions of two to about ten carbon atoms, such as ethenyloxy or propenyloxy radical. The term "alkenyloxyalkyl" also embraces alkenyl radicals having one or more alkenyloxy radicals attached to the alkyl radical, that is, to form monoalkenyloxyalkyl and dialkenyloxyalkyl radicals. More preferred alkenyloxy radicals are "lower alkenyloxy" radicals having two to six carbon atoms. Examples of such radicals include ethenyloxy, propenyloxy, butenyloxy, and isopropenyloxy alkyls. The "alkenyloxy" radicals may be further substituted with one or more halo atoms, such as fluoro, chloro or bromo, to provide "haloalkenyloxy" radicals. Examples of such radicals include trifluoroethenyloxy, fluoroethenyloxy, difluoroethenyhloxy, and fluoropropenyloxy.

The term "haloalkoxyalkyl" also embraces alkyl radicals having one or more haloalkoxy radicals attached to the alkyl radical, that is, to form monohaloalkoxyalkyl and dihaloalkexyalkyl radicals. The term "haloalkenyloxy" also embraces oxygen radicals having one or more haloalkenyloxy radicals attached to the oxygen radical, that is, to form monohaloalkenyloxy and dihaloalkenyloxy radicals. The term "haloalkenyloxyalkyl" also embraces alkyl radicals having one or more haloalkenyloxy radicals attached to the alkyl radical, that is, to form monohaloalkenyloxyalkyl and dihaloalkenyloxyalkyl radicals.

The term "alkylenedioxy" radicals denotes alkylene radicals having at least two oxygens bonded to a single alkylene group. Examples of "alkylenedioxy" radicals include methylenedioxy, ethylenedioxy, alkylsubstituted methylenedioxy, and arylsubstituted methylenedioxy. The term "haloalkylenedioxy" radicals denotes haloalkylene radicals having at least two oxy groups bonded to a single haloalkyl group. Examples of "haloalkylenedioxy" radicals include difluoromethylenedioxy, tetrafluoroethylenedioxy, tetrachloroethylenedioxy, alkylsubstituted monofluoromethylenedioxy, and arylsubstituted monofluoromethylenedioxy.

The term "aryl", alone or in combination, means a carbocyclic aromatic system containing one, two or three rings wherein such rings may be attached together in a pendant manner or may be fused. The term "fused" means that a second ring is present (ie, attached or formed) by having two adjacent atoms in common (ie, shared) with the first ring. The term "fused" is equivalent to the term "condensed". The term "aryl" embraces aromatic radicals such as phenyl, naphthyl, tetrahydronaphthyl, indane and biphenyl.

The term "perhaloaryl" embraces aromatic radicals such as phenyl, naphthyl, tetrahydronaphthyl, indane and biphenyl wherein the aryl radical is substituted with 3 or more halo radicals as defined below.

The term "heterocyclyl" embraces saturated, partially saturated and unsaturated heteroatom-containing ring-shaped radicals having from 5 through 15 ring members selected from carbon, nitrogen, sulfur and oxygen, wherein at least one ring atom is a heteroatom. Heterocyclyl radicals may contain one, two or three rings wherein such rings may be attached in a pendant manner or may be fused. Examples of saturated heterocyclic radicals include saturated 3 to 6-membered heteromonocylic group containing 1 to 4 nitrogen atoms[e.g. pyrrolidinyl, imidazolidinyl, piperidino, piperazinyl, etc.]; saturated 3 to 6-membered heteromonocyclic group containing 1 to 2 oxygen atoms and 1 to 3 nitrogen atoms [e.g. morpholinyl, etc.]; saturated 3 to 6-membered heteromonocyclic group containing 1 to 2 sulfur atoms and 1 to 3 nitrogen atoms [e.g., thiazolidinyl, etc.]. Examples of partially saturated heterocyclyl radicals include dihydrothiophene, dihydropyran, dihydrofuran and dihydrothiazole. Examples of unsaturated heterocyclic radicals, also termed "heteroaryl" radicals, include unsaturated 5 to 6 membered heteromonocyclyl group containing 1 to 4 nitrogen atoms, for example, pyrrolyl, pyrrolinyl, imidazolyl, pyrazolyl, 2-pyridyl, 3-pyridyl, 4-pyridyl, pyrimidyl, pyrazinyl, pyridazinyl, triazolyl [e.g., 4H-1,2,4-triazolyl, 1H-1,2,3-triazolyl, 2H-1,2,3-triazolyl, etc.] tetrazolyl [e.g. 1H-tetrazolyl, 2H-tetrazolyl, etc.], etc.: unsaturated condensed heterocyclic group containing 1 to 5 nitrogen atoms, for example, indolyl, isoindolyl, indolizinyl, benzimidazolyl, quinolyl, isoquinolyl, indazolyl, benzotriazolyl, tetrazolopyridazinyl [e.g., tetrazolo [1,5-b]pyridazinyl, etc.], etc.; unsaturated 3 to 6-membered heteromonocyclic group containing an oxygen atom, for example, pyranyl, 2-furyl, 3-furyl, etc.; unsaturated 5 to 6-membered heteromonocyclic group containing a sulfur atom,

for example, 2-thienyl, 3-thienyl, etc.; unsaturated 5- to 6-membered heteromonocyclic group containing 1 to 2 oxygen atoms and 1 to 3 nitrogen atoms, for example, oxazolyl, isoxazolyl, oxadiazolyl [e.g., 1,2,4-oxadiazolyl, 1,3,4-oxadiazolyl. 1,2,5-oxadiazolyl, etc.] etc.; unsaturated condensed heterocyclic group containing 1 to 2 oxygen atoms and 1 to 3 nitrogen atoms [e.g. benzoxazolyl, benzoxadiazolyl, etc.]; unsaturated 5 to 6-membered heteromonocyclic group containing 1 to 2 sulfur atoms and 1 to 3 nitrogen atoms, for example, thiazolyl, thiadiazolyl [e.g., 1.2.4- thiadiazolyl, 1,3,4-thiadiazolyl, 1.2.5-thiadiazolyl, etc.] etc.; unsaturated condensed heterocyclic group containing 1 to 2 sulfur atoms and 1 to 3 nitrogen atoms [e.g., benzothiazolyl, benzothiadiazolyl, etc.] and the like. The term also embraces radicals where heterocyclic radicals are fused with aryl radicals. Examples of such fused bicyclic radicals include benzofuran, benzothiophene, and the like. Said "heterocyclyl" group may have 1 to 3 substituents as defined below. Preferred heterocyclic radicals include five to twelve membered fused or unfused radicals. Non-limiting examples of heterocyclic radicals include pyrrolyl, pyridinyl, pyridyloxy, pyrazolyl, triazolyl, pyrimidinyl, pyridazinyl, oxazolyl, thiazolyl, imidazolyl, indolyl, thiophenyl, furanyl, tetrazolyl, 2-pyrrolinyl, 3-pyrrolinyl, pyrrolindinyl, 1,3-dioxolanyl, 2-imidazolinyl, imidazolidinyl, 2-pyrazolinyl, pyrazolidinyl, isoxazolyl, isothiazolyl, 1,2,3-oxadiazolyl, 1,2,3-triazolyl, 1,3,4-thiadiazolyl, 2H-pyranyl, 4H-pyranyl, piperidinyl, 1,4-dioxanyl, morpholinyl, 1,4-dithianyl, thiomorpholinyl, pyrazinyl, piperazinyl, 1.3.5-triazinyl, 1,3,5-trithianyl, benzo(b)thiophenyl, benzimidazoyl, quinolinyl, tetraazolyl, and the like.

The term "sulfonyl", whether used alone or linked to other terms such as alkylsulfonyl, denotes respectively divalent radicals $-SO_2-$. "Alkylsulfonyl", embraces alkyl radicals attached to a sulfonyl radical, where alkyl is defined as above. "Alkylsulfonylalkyl", embraces alkylsulfonyl radicals attached to an alkyl radical, where alkyl is defined as above. "Haloalkylsulfonyl", embraces haloalkyl radicals attached to a sulfonyl radical, where haloalkyl is defined as above. "Haloalkylsulfonylalkyl", embraces haloalkylsulfonyl radicals attached to an alkyl radical, where alkyl is defined as above. The term "aminosulfonyl" denotes an amino radical attached to a sulfonyl radical.

The term "sulfinyl", whether used alone or linked to other terms such as alkylsulfinyl, denotes respectively divalent radicals -S(O)-. "Alkylsulfinyl", embraces alkyl radicals attached to a sulfinyl radical, where alkyl is defined as above. "Alkylsulfinylalkyl", embraces alkylsulfinyl radicals attached to an alkyl radical, where alkyl is defined as above. "Haloalkylsulfinyl", embraces haloalkyl radicals attached to a sulfinyl radical, where haloalkyl is defined as above. "Haloalkylsulfinylalkyl", embraces haloalkylsulfinyl radicals attached to an alkyl radical, where alkyl is defined as above.

The term "aralkyl" embraces aryl-substituted alkyl radicals. Preferable aralkyl radicals are "lower aralkyl" radicals having aryl radicals attached to alkyl radicals having one to six carbon atoms. Examples of such radicals include benzyl, diphenylmethyl, triphenylmethyl, phenylethyl and diphenylethyl. The terms benzyl and phenylmethyl are interchangeable.

The term "heteroaralkyl" embraces heteroaryl-substituted alkyl radicals wherein the heteroaralkyl radical may be additionally substituted with three or more substituents as defined above for aralkyl radicals. The term "perhaloaralkyl" embraces aryl-substituted alkyl radicals wherein the aralkyl radical is substituted with three or more halo radicals as defined above.

The term "aralkylsulfinyl", embraces aralkyl radicals attached to a sulfinyl radical, where aralkyl is defined as above. "Aralkylsulfinylalkyl", embraces aralkylsulfinyl radicals attached to an alkyl radical, where alkyl is defined as above.

The term "aralkylsulfonyl", embraces aralkyl radicals attached to a sulfonyl radical, where aralkyl is defined as above. "Aralkylsulfonylalkyl", embraces aralkylsulfonyl radicals attached to an alkyl radical, where alkyl is defined as above.

The term "cycloalkyl" embraces radicals having three to ten carbon atoms. More preferred cycloalkyl radicals are "lower cycloalkyl" radicals having three to seven carbon atoms. Examples include radicals such as cyclopropyl, cyclobutyl, cyclopentyl, cyclohexyl and cycloheptyl. The term "cycloalkylalkyl" embraces cycloalkyl-substituted alkyl radicals. Preferable cycloalkylalkyl radicals are "lower cycloalkylalkyl" radicals having cycloalkyl radicals attached to alkyl radicals having one to six carbon atoms. Examples of such radicals include cyclohexylhexyl. The term "cycloalkenyl" embraces radicals having three to ten carbon atoms and one or more carbon-carbon double bonds. Preferred cycloalkenyl radicals are "lower cycloalkenyl" radicals having three to seven carbon atoms. Examples include radicals such as cyclobutenyl, cyclopentenyl, cyclohexenyl and cycloheptenyl. The term "halocycloalkyl" embraces radicals wherein any one or more of the cycloalkyl carbon atoms is substituted with halo as defined above. Specifically embraced are monohalocycloalkyl, dihalocycloalkyl and polyhalocycloalkyl radicals. A monohalocycloalkyl radical, for one example, may have either a bromo. chloro or a fluoro atom within the radical. Dihalo radicals may have two or more of the same halo atoms or a combination of different halo radicals and polyhalocycloalkyl radicals may have more than two of the same halo atoms or a combination of different halo radicals. More preferred halocycloalkyl radicals are "lower halocycloalkyl" radicals having three to about eight carbon atoms. Examples of such halocycloalkyl radicals include fluorocyclopropyl, difluorocyclobutyl, trifluorocyclopentyl, tetrafluorocyclohexyl, and dichlorocyclopropyl. The term "halocycloalkenyl" embraces radicals wherein any one or more of the cycloalkenyl carbon atoms is substituted with halo as defined above. Specifically embraced are monohalocycloalkenyl, dihalocycloalkenyl and polyhalocycloalkenyl radicals.

The term "cycloalkoxy" embraces cycloalkyl radicals attached to an oxy radical. Examples of such radicals includes cyclohexoxy and cyclopentoxy. The term "cycloalkoxyalkyl" also embraces alkyl radicals having one or more cycloalkoxy radicals attached to the alkyl radical, that is, to form monocycloalkoxyalkyl and dicycloalkoxyalkyl radicals. Examples of such radicals include cyclohexoxyethyl. The "cycloalkoxy" radicals may be further substituted with one or more halo atoms, such as fluoro, chloro or bromo, to provide "halocycloalkoxy" and "halocycloalkoxyalkyl" radicals.

The term "cycloalkylalkoxy" embraces cycloalkyl radicals attached to an alkoxy radical. Examples of such radicals includes cyclohexylmethoxy and cyclopentylmethoxy.

The term "cycloalkenyloxy" embraces cycloalkenyl radicals attached to an oxy radical. Examples of such radicals includes cyclohexenyloxy and cyclopentenyloxy. The term "cycloalkenyloxyalkyl" also embraces alkyl radicals having one or more cycloalkenyloxy radicals attached to the alkyl radical, that is, to form monocycloalkenyloxyalkyl and dicycloalkenyloxyalkyl radicals. Examples of such radicals include cyclohexenyloxyethyl. The "cycloalkenyloxy" radicals may be further substituted with one or more halo atoms, such as fluoro, chloro or bromo, to provide "halocycloalkenyloxy" and "halocycloalkenyloxyalkyl" radicals.

The term "cycloalkylenedioxy" radicals denotes cycloalkylene radicals having at least two oxygens bonded to a single cycloalkylene group. Examples of "alkylenedioxy" radicals include 1,2-dioxycyclohexylene.

The term "cycloalkylsulfinyl ", embraces cycloalkyl radicals attached to a sulfinyl radical, where cycloalkyl is defined as above. "Cycloalkylsulfinylalkyl", embraces cycloalkylsulfinyl radicals attached to an alkyl radical, where alkyl is defined as above. The term "Cycloalkylsulfonyl", embraces cycloalkyl radicals attached to a sulfonyl radical, where cycloalkyl is defined as above. "Cycloalkylsulfonylalkyl", embraces cycloalkylsulfonyl radicals attached to an alkyl radical, where alkyl is defined as above.

The term "cycloalkylalkanoyl" embraces radicals wherein one or more of the cycloalkyl carbon atoms are substituted with one or more carbonyl radicals as defined below. Specifically embraced are monocarbonylcycloalkyl and dicarbonylcycloalkyl radicals. Examples of monocarbonylcycloalkyl radicals include cyclohexylcarbonyl, cyclohexylacetyl, and cyclopentylcarbonyl. Examples of dicarbonylcycloalkyl radicals include 1,2-dicarbonylcyclohexane.

The term "alkylthio" embraces radicals containing a linear or branched alkyl radical, of one to ten carbon atoms, attached to a divalent sulfur atom. More preferred alkylthio radicals are "lower alkylthio" radicals having one to six carbon atoms. An example of "lower alkylthio" is methylthio ($CH_3$-S-). The "alkylthio" radicals may be further substituted with one or more halo atoms, such as fluoro, chloro or bromo, to provide "haloalkylthio" radicals. Examples of such radicals include fluoromethylthio, chloromethylthio, trifluoromethylthio, difluoromethylthio, trifluoroethylthio, fluoroethylthio, tetrafluoroethylthio, pentafluoroethylthio, and fluoropropylthio.

The term "alkyl aryl amino" embraces radicals containing a linear or branched alkyl radical, of one to ten carbon atoms, and one aryl radical both attached to an amino radical. Examples include N-methyl-4-methoxyaniline. N-ethyl-4-methoxyaniline, and N-methyl-4-trifluoromethoxyaniline.

The terms alkylamino denotes "monoalkylamino" and "dialkylamino" containing one or two alkyl radicals, respectively, attached to an amino radical.

The terms arylamino denotes "monoarylamino" and "diarylamino" containing one or two aryl radicals, respectively, attached to an amino radical. Examples of such radicals include N-phenylamino and N-naphthylamino.

The term "aralkylamino", embraces aralkyl radicals attached to an amino radical, where aralkyl is defined as above. The term aralkylamino denotes "monoaralkylamino" and "diaralkylamino" containing one or two aralkyl radicals, respectively, attached to an amino radical. The term aralkylamino further denotes "monoaralkyl monoalkylamino" containing one aralkyl radical and one alkyl radical attached to an amino radical.

The term "arylsulfinyl" embraces radicals containing an aryl radical, as defined above, attached to a divalent S(=O) atom. The term "arylsulfinylalkyl" denotes arylsulfinyl radicals attached to a linear or branched alkyl radical, of one to ten carbon atoms.

The term "arylsulfonyl", embraces aryl radicals attached to a sulfonyl radical, where aryl is defined as above, "arylsulfonylalkyl", embraces arylsulfonyl radicals attached to an alkyl radical, where alkyl is defined as above. The term "heteroarylsulfinyl" embraces radicals containing an heteroaryl radical, as defined above, attached to a divalent S(=O) atom. The term "heteroarylsulfinylalkyl" denotes heteroarylsulfinyl radicals attached to a linear or branched alkyl radical, of one to ten carbon atoms. The term "Heteroarylsulfonyl", embraces heteroaryl radicals attached to a sulfonyl radical, where heteroaryl is defined as above. "Heteroarylsulfonylalkyl", embraces heteroarylsulfonyl radicals attached to an alkyl radical, where alkyl is defined as above.

The term "aryloxy" embraces aryl radicals, as defined above, attached to an oxygen atom. Examples of such radicals include phenoxy, 4-chloro-3-ethylphenoxy, 4-chloro-3-methylphenoxy, 3-chloro-4-ethylphenoxy, 3,4-dichlorophenoxy, 4-methylphenoxy, 3-trifluoromethoxyphenoxy, 3-trifluoromethylphenoxy, 4-fluorophenoxy, 3,4-dimethylphenoxy, 5-bromo-2-fluorophenoxy, 4-bromo-3-fluorophenoxy, 4-fluoro-3-methylphenoxy, 5,6,7,8-tetrahydronaphthyloxy, 3-isopropylphenoxy, 3-cyclopropylphenoxy, 3-ethylphenoxy, 4-*tert*-butylphenoxy, 3-pentafluoroethylphenoxy, and 3-(1,1,2,2-tetrafluoroethoxy)phenoxy.

The term "aroyl" embraces aryl radicals, as defined above, attached to an carbonyl radical as defined above.

Examples of such radicals include benzoyl and toluoyl.

The term "aralkanoyl" embraces aralkyl radicals, as defined herein, attached to an carbonyl radical as defined above. Examples of such radicals include, for example, phenylacetyl.

The term "aralkoxy" embraces oxy-containing aralkyl radicals attached through an oxygen atom to other radicals. More preferred aralkoxy radicals are "lower aralkoxy" radicals having phenyl radicals attached to lower alkoxy radical as described above. Examples of such radicals include benzyloxy, 1-phenylethoxy, 3-trifluoromethoxybenzyloxy, 3-trifluoromethylbenzyloxy, 3,5-difluorobenyloxy, 3-bromobenzyloxy, 4-propylbenzyloxy, 2-fluoro-3-trifluoromethylbenzyloxy, and 2-phenylethoxy.

The term "aryloxyalkyl" embraces aryloxy radicals, as defined above, attached to an alkyl group. Examples of such radicals include phenoxymethyl.

The term "haloaryloxyalkyl" embraces aryloxyalkyl radicals, as defined above, wherein one to five halo radicals are attached to an aryloxy group.

The term "heteroaroyl" embraces heteroaryl radicals, as defined above, attached to an carbonyl radical as defined above. Examples of such radicals include furoyl and nicotinyl.

The term "heteroaralkanoyl" embraces heteroaralkyl radicals, as defined herein, attached to an carbonyl radical as defined above. Examples of such radicals include, for example, pyridylacetyl and furylbutyryl.

The term "heteroaralkoxy" embraces oxy-containing heteroaralkyl radicals attached through an oxygen atom to other radicals. More preferred heteroaralkoxy radicals are "lower heteroaralkoxy" radicals having heteroaryl radicals attached to lower alkoxy radical as described above.

The term "haloheteroaryloxyalkyl" embraces heteroaryloxyalkyl radicals, as defined above, wherein one to four halo radicals are attached to an heteroaryloxy group.

The term "heteroarylamino" embraces heterocyclyl radicals, as defined above, attached to an amino group. Examples of such radicals include pyridylamino.

The term "heteroarylaminoalkyl" embraces heteroarylamino radicals, as defined above, attached to an alkyl group. Examples of such radicals include pyridylmethylamino.

The term "heteroaryloxy" embraces heterocyclyl radicals, as defined above, attached to an oxy group. Examples of such radicals include 2-thiophenyloxy, 2-pyrimidyloxy, 2-pyridyloxy, 3-pyridyloxy, and 4-pyridyloxy.

The term "heteroaryloxyalkyl" embraces heteroaryloxy radicals, as defined above, attached to an alkyl group. Examples of such radicals include 2-pyridyloxymethyl, 3-pyridyloxyethyl, and 4-pyridyloxymethyl.

The term "arylthio" embraces aryl radicals, as defined above, attached to an sulfur atom. Examples of such radicals include phenylthio.

The term "arylthioalkyl" embraces arylthio radicals, as defined above, attached to an alkyl group. Examples of such radicals include phenylthiomethyl.

The term "alkylthioalkyl" embraces alkylthio radicals, as defined above, attached to an alkyl group. Examples of such radicals include methylthiomethyl. The term "alkoxyalkyl" embraces alkoxy radicals, as defined above, attached to an alkyl group. Examples of such radicals include methoxymethyl.

The term "carbonyl" denotes a carbon radical having two of the four covalent bonds shared with an oxygen atom. The term "carboxy" embraces a hydroxyl radical, as defined above, attached to one of two unshared bonds in a carbonyl group. The term "carboxamide" embraces amino, monoalkylamino, dialkylamino, monocycloalkylamino, alkylcycloalkylamino, and dicycloalkylamino radicals, attached to one of two unshared bonds in a carbonyl group. The term "carboxamidoalkyl" embraces carboxamide radicals, as defined above, attached to an alkyl group. The term "carboxyalkyl" embraces a carboxy radical, as defined above, attached to an alkyl group. The term "carboalkoxy" embraces alkoxy radicals, as defined above, attached to one of two unshared bonds in a carbonyl group. The term "carboaralkoxy" embraces aralkoxy radicals, as defined above, attached to one of two unshared bonds in a carbonyl group. The term "monocarboalkoxyalkyl" embraces one carboalkoxy radical, as defined above, attached to an alkyl group. The term "dicarboalkoxyalkyl" embraces two carboalkoxy radicals, as defined above, attached to an alkylene group. The term "monocyanoalkyl" embraces one cyano radical, as defined above, attached to an alkyl group. The term "dicyanoalkylene" embraces two cyano radicals, as defined above, attached to an alkyl group. The term "carboalkoxycyanoalkyl" embraces one cyano radical, as defined above, attached to an carboalkoxyalkyl group.

The term "acyl", alone or in combination, means a carbonyl or thionocarbonyl group bonded to a radical selected from, for example, hydrido, alkyl, alkenyl, alkynyl, haloalkyl, alkoxy, alkoxyalkyl, haloalkoxy, aryl, heterocyclyl, heteroaryl, alkylsulfinylalkyl, alkylsulfonylalkyl, aralkyl, cycloalkyl, cycloalkylalkyl, cycloalkenyl, alkylthio, arylthio, amino, alkylamino, dialkylamino, aralkoxy, arylthio, and alkylthioalkyl. Examples of "acyl" are formyl, acetyl, benzoyl, trifluoroacetyl, phthaloyl, malonyl, nicotinyl, and the like. The term "haloalkanoyl" embraces one or more halo radicals, as defined herein, attached to an alkanoyl radical as defined above. Examples of such radicals include, for example, chloroacetyl, trifluoroacetyl, bromopropanoyl, and heptafluorobutanoyl. The term "diacyl", alone or in combination, means having two or more carbonyl or thionocarbonyl groups bonded to a radical selected from, for example. alkylene, alkenylene, alkynylene, haloalkylene, alkoxyalkylene, aryl, heterocyclyl, heteroaryl, aralkyl, cycloalkyl, cycloalkylalkyl,

and cycloalkenyl. Examples of "diacyl" are phthaloyl, malonyl, succinyl, adipoyl, and the like.

The term "benzylidenyl" radical denotes substituted and unsubstituted benzyl groups having attachment points for two covalent bonds. One attachment point is through the methylene of the benzyl group with the other attachment point through an ortho carbon of the phenyl ring. The methylene group is designated for attached to the lowest numbered position. Examples include the base compound benzylidene of structure:

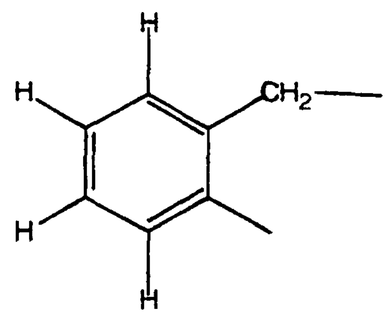

The term "phenoxylidenyl" radical denotes substituted and unsubstituted phenoxy groups having attachment points for two covalent bonds. One attachment point is through the oxy of the phenoxy group with the other attachment point through an ortho carbon of the phenyl ring. The oxy group is designated for attached to the lowest numbered position. Examples include the base compound phenoxylidene of structure:

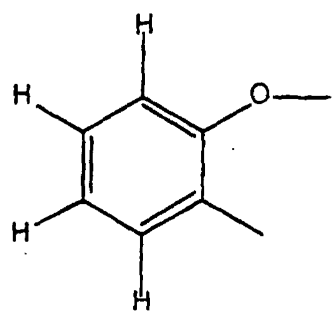

The term "phosphono" embraces a pentavalent phosphorus attached with two covalent bonds to an oxygen radical. The term "dialkoxyphosphono" denotes two alkoxy radicals, as defined above, attached to a phosphono radical with two covalent bonds. The term "diaralkoxyphosphono" denotes two aralkoxy radicals, as defined above, attached to a phosphono radical with two covalent bonds. The term "dialkoxyphosphonoalkyl" denotes dialkoxyphosphono radicals, as defined above, attached to an alkyl radical. The term "diaralkoxyphosphonoalkyl" denotes diaralkoxyphosphono radicals, as defined above, attached to an alkyl radical.

Said "alkyl", "alkenyl", "alkynyl", "alkanoyl", "alkylene", "alkenylene", "benzylidenyl", "phenoxylidenyl", "hydroxyalkyl", "haloalkyl", "haloalkylene", "haloalkenyl", "alkoxy", "alkenyloxy", "alkenyloxyalkyl", "alkoxyalkyl", "aryl", "perhaloaryl", "haloalkoxy", "haloalkoxyalkyl", "haloalkenyloxy", "haloalkenyloxyalkyl", "alkylenedioxy", "haloalkylenedioxy", "heterocyclyl", "heteroaryl", "hydroxyhaloalkyl", "alkylsulfonyl", "haloalkylsulfonyl", "alkylsulfonylalkyl", "haloalkylsulfonylalkyl", "alkylsulfinyl", "alkylsulfinylalkyl", "haloalkylsulfinylalkyl", "aralkyl", "heteroaralkyl", "perhaloaralkyl", "aralkylsulfonyl", "aralkylsulfonylalkyl", "aralkylsulfinyl", "aralkylsulfinylalkyl", "cycloalkyl", "cycloalkylalkanoyl", "cycloalkylalkyl", "cycloalkenyl", "halocycloalkyl", "halocycloalkenyl", "cycloalkylsulfinyl", "cycloalkylsulfinylalkyl", "cycloalkylsulfonyl", "cycloalkylsulfonylalkyl", "cycloalkoxy", "cycloalkoxyalkyl", "cycloalkylalkoxy", "cycloalkenyloxy", "cycloalkenyloxyalkyl", "cycloalkylenedioxy", "halocycloalkoxy", "halocycloalkoxyalkyl", "halocycloalkenyloxy", "halocycloalkenyloxyalkyl", "alkylthio", "haloalkylthio", "alkylsulfinyl", "amino", "oxy", "thio", "alkylamino", "arylamino", "aralkylamino", "arylsulfinyl", "arylsulfinylalkyl", "arylsulfonyl", "arylsulfonylalkyl", "heteroarylsulfinyl", "heteroarylsulfinylalkyl", "heteroarylsulfonyl", "heteroarylsulfonylalkyl", "heteroarylamino", "heteroarylaminoalkyl", "heteroaryloxy", "heteroaryloxylalkyl", "aryloxy", "aroyl", "aralkanoyl", "aralkoxy", "aryloxyalkyl", "haloaryloxyalkyl", "heteroaroyl", "heteroaralkanoyl", "heteroaralkoxy", "heteroaralkoxyalkyl", "arylthio", "arylthioalkyl", "alkoxyalkyl", "acyl" and "diacyl" groups defined above may optionally have 1 to 5 non-hydrido substituents such as perhaloaralkyl, aralkylsulfonyl, aralkylsulfonylalkyl, aralkylsulfinyl, aralkylsulfinylalkyl, halocycloalkyl, halocycloalkenyl, cycloalkylsulfinyl, cycloalkylsulfinylalkyl, cycloalkylsulfonyl, cycloalkylsulfonylalkyl, heteroarylamino, N-heteroarylamino-N-alkylamino, heteroarylaminoalkyl, heteroaryloxy, heteroaryloxylalkyl, haloalkylthio, alkanoyloxy, alkoxy, alkoxyalkyl, haloalkoxylalkyl, heteroaralkoxy, cycloalkoxy, cycloalkenyloxy, cycloalkoxyalkyl, cycloalkylalkoxy, cycloalkenyloxyalkyl, cycloalkylenedioxy, halocycloalkoxy, halocycloalkoxyalkyl, halocycloalkenyloxy, halocycloalkenyloxyalkyl, hydroxy, amino, thio, nitro, lower alkylamino, alkylthio, alkylthioalkyl, arylamino, aralkylamino, arylthio, arylthioalkyl, heteroaralkoxyalkyl, alkylsulfinyl, alkylsulfinylalkyl, arylsulfinylalkyl, arylsulfonylalkyl, heteroarylsulfinylalkyl, heteroarylsulfonylalkyl, alkylsulfonyl, alkylsulfonylalkyl, haloalkylsulfinylalkyl, haloalkylsulfonylalkyl, alkylsulfonamido, alkylaminosulfonyl, amidosulfonyl, monoalkyl amidosulfonyl, dialkyl amidosulfonyl, monoarylamidosulfonyl, arylsulfonamido, diarylamidosulfonyl, monoalkyl monoaryl amidosulfonyl, arylsulfinyl, arylsulfonyl, heteroarylthio, heteroarylsulfinyl, heteroarylsulfonyl, alkanoyl, alkenoyl, aroyl, heteroaroyl, aralkanoyl, heteroaralkanoyl, haloalkanoyl, alkyl, alkenyl, alkynyl, alkenyloxy, alkenyloxyalky, alkylenedioxy, haloalkylenedioxy, cycloalkyl, cycloalkylalkanoyl, cycloalkenyl, lower cycloalkylalkyl, lower cycloalkenylalkyl, halo, haloalkyl, haloalkenyl, haloalkoxy, hydroxyhaloalkyl, hydroxyaralkyl, hydroxyalkyl, hydoxyheteroaralkyl, haloalkoxyalkyl, aryl, aralkyl, aryloxy, aralkoxy, aryloxyalkyl, saturated heterocyclyl, partially saturated heterocyclyl, heteroaryl, heteroaryloxy, heteroaryloxyalkyl, arylalkyl, heteroarylalkyl, arylalkenyl, heteroarylalkenyl, carboxyalkyl, carboalkoxy, alkoxycarbonyl, carboaralkoxy, carboxamido, carboxamidoalkyl, cyano, carbohaloalkoxy, phosphono, phosphonoalkyl, diaralkoxyphosphono, and diaralkoxyphosphonoalkyl,

The term "spacer" can include a covalent bond and a linear moiety having a backbone of 1 to 7 continous atoms. The spacer may have 1 to 7 atoms of a univalent or multi-valent chain. Univalent chains may be constituted by a radical selected from =C(H)-, =C($R_{17}$)-, -O-, -S-, -S(O)-, -S(O)$_2$-, -NH-, -N($R_{17}$)-, -N=, -CH(OH)-, =C(OH)-, -CH(O$R_{17}$)-, =C(O$R_{17}$)-, and -C(O)- wherein $R_{17}$ is selected from alkyl, alkenyl, alkynyl, aryl, heteroaryl, aralkyl, aryloxyalkyl, alkoxyalkyl, alkylthioalkyl, arylthioalkyl, cycloalkyl, cycloalkylalkyl, haloalkyl, haloalkenyl, haloalkoxyalkyl, perhaloaralkyl, heteroarylalkyl, heteroaryloxyalkyl, heteroarylthioalkyl, and heteroarylalkenyl. Multi-valent chains may consist of a straight chain of 1 or 2 or 3 or 4 or 5 or 6 or 7 atoms or a straight chain of 1 or 2 or 3 or 4 or 5 or 6 atoms with a side chain. The chain may be constituted of one or more radicals selected from: lower alkylene, lower alkenyl, -O-, -O-$CH_2$-, -S-$CH_2$-. -$CH_2CH_2$-, ethenyl, -CH=CH(OH)-, -$OCH_2O$-, -O$(CH_2)_2$O-, -NH$CH_2$-, -OCH($R_{17}$)O-, -O($CH_2$CH$R_{17}$)O-, -O$CF_2$O-, -O$(CF_2)_2$O-, -S-, -S(O)-, -S(O)$_2$-, -N(H)-, -N(H)O-, -N($R_{17}$)O-, -N($R_{17}$)-, -C(O)-, -C(O)NH-, -C(O)N$R_{17}$-, -N=, -$OCH_2$-, -$SCH_2$-, S(O)$CH_2$-, -$CH_2$C(O)-, -CH(OH)-, =C(OH)-, -CH(O$R_{17}$)-, =C(O$R_{17}$)-, S(O)$_2CH_2$-, and -N$R_{17}CH_2$- and many other radicals defined above or generally known or ascertained by one of skill-in-the art. Side chains may include substituents such as 1 to 5 non-hydrido substituents such as perhaloaralkyl, aralkylsulfonyl, aralkylsulfonylalkyl, aralkylsulfinyl, aralkylsulfinylalkyl, halocycloalkyl, halocycloalkenyl, cycloalkylsulfinyl, cycloalkylsulfinylalkyl, cycloalkylsulfonyl, cycloalkylsulfonylalkyl, heteroarylamino, N-heteroarylamino-N-alkylamino, heteroarylaminoalkyl, heteroaryloxy, heteroaryloxylalkyl, haloalkylthio, alkanoyloxy, alkoxy, alkoxyalkyl, haloalkoxylalkyl, heteroaralkoxy, cycloalkoxy, cycloalkenyloxy, cycloalkoxyalkyl, cycloalkylalkoxy, cycloalkenyloxyalkyl, cycloalkylenedioxy, halocycloalkoxy, halocycloalkoxyalkyl, halocycloalkenyloxy, halocycloalkenyloxyalkyl, hydroxy, amino, thio, nitro, lower alkylamino, alkylthio, alkylthioalkyl, arylamino, aralkylamino, arylthio, arylthioalkyl, heteroaralkoxyalkyl, alkylsulfinyl, alkylsulfinylalkyl, arylsulfinylalkyl, arylsulfonylalkyl, heteroarylsulfinylalkyl, heteroarylsulfonylalkyl, alkylsulfonyl, alkylsulfonylalkyl, haloalkylsulfinylalkyl, haloalkylsulfonylalkyl, alkylsulfonamido, alkylaminosulfonyl, amidosulfonyl, monoalkyl amidosulfonyl, dialkyl amidosulfonyl, monoarylamidosulfonyl, arylsulfonamido, diarylamidosulfonyl, monoalkyl monoaryl amidosulfonyl, arylsulfinyl, arylsulfonyl, heteroarylthio, heteroarylsulfinyl, heteroarylsulfonyl, alkanoyl, alkenoyl, aroyl, heteroaroyl, aralkanoyl, heteroaralkanoyl, haloalkanoyl, alkyl, alkenyl, alkynyl, alkenyloxy, alkenyloxyalky, alkylenedioxy, haloalkylenedioxy, cycloalkyl, cycloalkenyl, lower cycloalkylalkyl, lower cycloalkenylalkyl, halo, haloalkyl, haloalkenyl, haloalkoxy, hydroxyhaloalkyl, hydroxyaralkyl, hydroxyalkyl, hydoxyheteroaralkyl, haloalkoxyalkyl, aryl, aralkyl, aryloxy, aralkoxy, aryloxyalkyl, saturated heterocyclyl, partially saturated heterocyclyl, heteroaryl, heteroaryloxy, heteroaryloxyalkyl, arylalkyl, heteroarylalkyl, arylalkenyl, heteroarylalkenyl, carboxyalkyl, carboalkoxy, carboaralkoxy, carboxamido, carboxamidoalkyl, cyano, carbohaloalkoxy, phosphono, phosphonoalkyl, diaralkoxyphosphono, and diaralkoxyphosphonoalkyl.

Chiral compounds of the present invention have a hydroxyl group substitutent on a chiral carbon of the alkanol and propanol compounds of the present invention specifically in the R-stereoisomeric configuration based on the Cahn-Ingold-Prelog convention for stereoisomeric carbon atoms. The R-stereoisomeric configuration compounds of the present invention may optionally have one or more additional chiral carbons present in each compound. The R-stereoisomeric configuration compounds of the present invention can exist in tautomeric, geometric, and other stereoisomeric forms. The present invention having a hydroxyl group substitutent on a chiral carbon of the alkanol and propanol compounds in the R-stereoisomeric configuration contemplates all such forms of said invented compounds, including cis- and trans-geometric isomers, E- and Z-geometric isomers, diastereomers, and other mixtures thereof, as falling within the scope of the invention. Pharmaceutically acceptable sales of such tautomeric, geometric or stereoisomeric forms are also included within the invention. The standard definitions for the Cahn-Ingold-Prelog convention and stereochemical system can be found in Pure Applied Chemistry, 1976, Vol. 45, pages 15-30 and Cahn et al., Angewandte

Chemie International Edition English, 1966, Vol. 5, pages 385-415.

The terms "cis" and "trans" denote a form of geometric isomerism in which two carbon atoms connected by a double bond will each have a hydrogen atom on the same side of the double bond ("cis") or on opposite sides of the double bond ("trans").

Some of the compounds described contain alkenyl groups, and are meant to include both cis and trans or "E" and "Z" geometric forms.

Some of the compounds described contain one or more stereocenters in addition to said hydroxyl group substitutent on a chiral carbon of the alkanol and propanol compounds in the R-stereoisomeric configuration and are meant to include R, S, and mixtures of R and S forms for each additional stereocenter present.

Some of the compounds described herein may contain one or more ketonic or aldehydic carbonyl groups or combinations thereof alone or as part of a heterocyclic ring system. Such carbonyl groups may exist in part or principally in the "keto" form and in part or principally as one or more "enol" forms of each aldehyde and ketone group present. Compounds of the present invention having aldehydic or ketonic carbonyl groups are meant to include both "keto" and "enol" tautomeric forms.

Some of the compounds described herein may contain one or more amide carbonyl groups or combinations thereof alone or as part of a heterocyclic ring system. Such carbonyl groups may exist in part or principally in the "keto" form and in part or principally as one or more "enol" forms of each amide group present. Compounds of the present invention having amidic carbonyl groups are meant to include both "keto" and "enol" tautomeric forms. Said amide carbonyl groups may be both oxo (C=O) and thiono (C=S) in type.

Some of the compounds described herein may contain one or more imine or enamine groups or combinations thereof. Such groups may exist in part or principally in the "imine" form and in part or principally as one or more "enamine" forms of each group present. Compounds of the present invention having said imine or enamine groups are meant to include both "imine" and "enamine" tautomeric forms.

The following general synthetic sequences are useful in making the present invention. Abbreviations used in the schemes are as follows: "AA" represents amino acids, "BINAP" represents 2,2'-bis(diphenylphosphino)-1,1'-binaphthyl, "Boc" represents tert-butyloxycarbonyl, "BOP" represents benzotriazol-1-yl-oxy-tris-(dimethylamino), "bu" represents butyl, "dba" represents dibenzylideneacetone, "DCC" represents 1,3-dicyclohexylcarbodiimide, "DIBAH" represents diisobutylaluminum hydride, "DIPEA" represents diisopropylethylamine, "DMF" represents dimethylformamide, "DMSO" represents dimethylsulfoxide. "Fmoc" represents 9-fluorenylmethoxycarbonyl, "LDA" represents lithium diisopropylamide, "PHTH" represents a phthaloyl group, "pnZ" represents 4-nitrobenzyloxycarbonyl, "PTC" represents a phase transfer catalyst, "p-TsOH" represents paratoluenesulfonic acid, "TBAF" represents tetrabutylammonium fluoride, "TBTU" represents 2-(1H-benzotriozole-1-yl)-1,1,3,3-tetramethyl uronium tetrafluoroborate. "TEA" represents triethylamine, "TFA" represents trifluoroacetic acid, "THF" represents tetrahydrofuran, "TMS" represents trimethylsilyl, and "Z" represents benzyloxycarbonyl.

The present invention comprises a pharmaceutical composition comprising a therapeutically-effective amount of a compound of Formula M together with a pharmaceutically-acceptable carrier.

The present invention also comprises the use of a compound of Formula M or a pharmaceutically acceptable salt thereof for the manufacture of a medicament for the treatment or prophylaxis of coronary artery disease and other CETP-mediated disorders in a subject.

Compounds of the invention are capable of inhibiting activity of cholesteryl ester transfer protein (CETP), and thus could be used in the manufacture of a medicament, a method for the prophylactic or therapeutic treatment of diseases mediated by CETP, such as peripheral vascular disease, hyperlipidaemia, hypercholesterolemia, and other diseases attributable to either high LDL and low HDL or a combination of both, or a procedure to study the mechanism of action of the cholesteryl ester transfer protein (CETP) to enable the design of better inhibitors. The compounds of the invention would be also useful in prevention of cerebral vascular accident (CV A) or stroke.

Also included in the family of compounds of the invention are the pharmaceutically-acceptable salts thereof. The term "pharmaceutically-acceptable salts" embraces salts commonly used to form alkali metal salts and to form addition salts of free acids or free bases. The nature of the salt is not critical, provided that it is pharmaceutically acceptable. Suitable pharmaceutically-acceptable acid addition salts of compounds of the invention may be prepared from inorganic acid or from an organic acid. Examples of such inorganic acids are hydrochloric, hydrobromic, hydroiodic, nitric, carbonic, sulfuric and phosphoric acid. Appropriate organic acids may be selected from aliphatic, cycloaliphatic, aromatic, araliphatic, heterocyclic, carboxylic and sulfonic classes of organic acids, examples of which are formic, acetic, propionic, succinic, glycolic, gluconic, lactic, malic, tartaric, citric, ascorbic, glucoronic, maleic, fumaric, pyruvic, aspartic, glutamic, benzoic, anthranilic, mesylic, salicylic, p-hydroxybenzoic, phenylacetic, mandelic, embonic (pamoic), methanesulfonic, ethylsulfonic, benzenesulfonic, sulfanilic, stearic, cyclohexylaminosulfonic, algenic, galacturonic acid. Suitable pharmaceutically-acceptable base addition salts of compounds of the invention include metallic salts made from aluminum, calcium, lithium, magnesium, potassium, sodium and zinc or organic salts made from N,N'-dibenzylethyleneldiamine, choline, chloroprocaine, diethanolamine, ethylenediamine, meglumine (N-methylglucamine) and procain. All of these salts may be prepared by conventional means from the corresponding compound of the invention by reacting, for example, the appropriate acid or base with the compound of the invention.

Also embraced within this invention is a class of pharmaceutical composition comprising the active compounds of the invention in association with one or more non-toxic, pharmaceutically-acceptabie carriers and/or diluents and/or adjuvants (collectively referred to herein as "carrier" materials) and, if desired, other active ingredients. The active compounds of the present invention may be administered by any suitable route, preferably in the form of a pharmaceutical composition adapted to such a route, and in a dose effective for the treatment intended. The active compounds and composition may, for example, be administered orally, intravascularly, intraperitoneally, subcutaneously, intramuscularly or topically.

For oral administration, the pharmaceutical composition may be in the form of, for example, a tablet, capsule, suspension or liquid. The pharmaceutical composition is preferably made in the form of a dosage unit containing a particular amount of the active ingredient. Examples of such dosage units are tablets or capsules. The active ingredient may also be administered by injection as a composition wherein, for example, saline, dextrose or water may be used as a suitable carrier.

The amount of therapeutically active compounds which are administered and the dosage regimen for treating a disease condition with the compounds and/or compositions of this invention depends on a variety of factors, including the age, weight, sex and medical condition of the subject, the severity of the disease, the route and frequency of administration, and the particular compound employed, and thus may vary widely.

The pharmaceutical compositions may contain active ingredients in the range of about 0.1 to 2000 mg, and preferably in the range of about 0.5 to 500 mg. A daily dose of about 0.01 to 100 mg/kg body weight, and preferably between about 0.5 and about 20 mg/kg body weight, may be appropriate. The daily dose can be administered in one to four doses per day.

The compounds may be formulated in topical ointment or cream, or as a suppository, containing the active ingredients in a total amount of, for example, 0.075 to 30% w/w, preferably 0.2 to 20% w/w and most preferably 0.4 to 15% w/w. When formulated in an ointment. the active ingredients may be employed with either paraffinic or a water-miscible ointment base. Alternatively, the active ingredients may be formulated in a cream with an oil-in-water cream base. If desired, the aqueous phase of the cream base may include, for example at least 30% w/w of a polyhydric alcohol such as propylene glycol, butane-1,3-diol, mannitol, sorbitol, glycerol, polyethylene glycol and mixtures thereof. The topical formulation may desirably include a compound which enhances absorption or penetration of the active ingredient through the skin or other affected areas. Examples of such dermal penetration enhancers include dimethylsulfoxide and related analogs. The compounds of this invention can also be administered by a transdermal device. Preferably topical administration will be accomplished using a patch either of the reservoir and porous membrane type or of a solid matrix variety. In either case, the active agent is delivered continuously from the reservoir or microcapsules through a membrane into the active agent permeable adhesive, which is in contact with the skin or mucosa of the recipient. If the active agent is absorbed through the skin, a controlled and predetermined flow of the active agent is administered to the recipient. In the case of microcapsules, the encapsulating agent may also function as the membrane.

The oily phase of the emulsions of this invention may be constituted from known ingredients in a known manner. While the phase may comprise merely an emulsifier, it may comprise a mixture of at least one emulsifier with a fat or an oil or with both a fat and an oil. Preferably, a hydrophilic emulsifier is included together with a lipophilic emulsifier which acts as a stabilizer. It is also preferred to include both an oil and a fat. Together, the emulsifier(s) with or without stabilizer(s) make-up the so-called emulsifying wax, and the wax together with the oil and fat make up the so-called emulsifying ointment base which forms the oily dispersed phase of the cream formulations. Emulsifiers and emulsion stabilizers suitable for use in the formulation of the present invention include Tween 60, Span 80, cetostearyl alcohol, myristyl alcohol, glyceryl monostearate, and sodium lauryl sulfate, among others.

The choice of suitable oils or fats for the formulation is based on achieving the desired cosmetic properties, since the solubility of the active compound in most oils likely to be used in pharmaceutical emulsion formulations is very low. Thus, the cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters may be used. These may be used alone or in combination depending on the properties required. Alternatively, high melting point lipids such as white soft paraffin and/or liquid paraffin or other mineral oils can be used.

For therapeutic purposes, the active compounds of this combination invention are ordinarily combined with one or more adjuvants appropriate to the indicated route of administration. If administered *per os*, the compounds may be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient

administration. Such capsules or tablets may contain a controlled-release formulation as may be provided in a dispersion of active compound in hydroxypropylmethyl cellulose. Formulations for parenteral administration may be in the form of aqueous or non-aqueous isotonic sterile injection solutions or suspensions. These solutions and suspensions may be prepared from sterile powders or granules having one or more of the carriers or diluents mentioned for use in the formulations for oral administration. The compounds may be dissolved in water, polyethylene glycol, propylene glycol, ethanol, corn oil, cottonseed oil, peanut oil, sesame oil, benzyl alcohol, sodium chloride, and/or various buffers. Other adjuvants and modes of administration are well and widely known in the pharmaceutical art.

The present invention further provides a process for the preparation of (R)-chiral compounds of the invention

by reacting suitable secondary amines with (R)-chiral forms of alcohols, epoxides, and cyclic sulfate esters.

The present invention also provides a process for the preparation of (R)-chiral compounds of the invention

by reacting a suitable secondary amine with a substantially stoichiometric amount of a (R)-chiral epoxide in the presence of a transition metal-based salt.

The present invention also provides a process for the preparation of (R)-chiral precursor compounds useful in the preparation of compounds of the invention

by reacting a suitable primary amine with a substantially stoichiometric amount of a (R)-chiral epoxide with or without the presence of an added transition metal-based compound.

All mentioned references are incorporated by reference as if here written.

Although this invention has been described with respect to specific embodiments, the details of these embodiments are not to be construed as limitations.

## GENERAL SYNTHETIC PROCEDURES

The compounds of the present invention can be synthesized, for example, according to the following procedures of Schemes through 15 below, wherein the substituents are as defined for Formulas I-H, I-C and I-CP above except where further noted.

It will be understood that the following description of synthetic procedures is applicable to the synthesis of the compounds of Formula M as well as to the broader group of compounds of Formula I-H. However, this is not intended to imply that the scope of the invention extends beyond that of the claims.

Synthetic Scheme 1 shows the preparation of compounds of formula XIIIA-H ("Secondary Heteroaryl Amines") which are intermediates in the preparation of the compounds corresponding to Formula I-H ("Generic Polycyclic Aryl and Heteroaryl (R)-Chiral Halogenated 1-Substitutedamino-(n+1)-alkanols"). Formula I-HP ("Generic Polycyclic Aryl and Heteroaryl (R)-Chiral Halogenated 1-Substitutedamino-2-propanols"). Formula I-HPC ("Polycyclic Aryl-Heteroaryl (R)-Chiral Halogenated 1-Substitutedamino-2-Propanols"), Formula I-C ("Polycyclic Phenyl (R)-Chiral Halogenated 1-Substitutedamino-(n+1)-Alkanols"), and Formula I-CP ("Polycyclic Phenyl (R)-Chiral Halogenated 1-Substitutedamino-2-Propanols") wherein A and Q are independently aryl and heteroaryl. Schemes 1 through 3, taken together, prepare 1-substitutedamino-2-alkanols by addition of a halogenated, oxygen containing precursor to a secondary amine to introduce an oxy containing alkyl group wherein the two groups making up the secondary amine both are made up of aromatic groups or both groups contain aromatic rings wherein said aromatic rings maybe 0 to 2 aryl rings and 0 to 2 heteroaryl rings.

The "Generic Imine" corresponding to Formula XII can be prepared through dehydration techniques generally known in or adaptable from the art by reacting "Generic Amine-I" of Formula X with the "Generic Carbonyl Compound" of Formula XI in Scheme 1 and subsequent specific examples. For example, when Z is a covalent bond, methylene, methine substituted with another subsitutent, ethylene, or another subsituent as defined in Formula I-H, the two reactants (X and XI) react by refluxing them in an aprotic solvent, such as hexane, toluene, cyclohexane, benzene, and the like, using a Dean-Stark type trap to remove water. After about 2-8 hours or until the removal of water is complete, the aprotic solvent is removed *in vacuo* to yield the "Generic Imine" of Formula XII. Alternately, when Z is an oxygen, the "Generic Imine" is an oxime derivative. Alternately, when Z is a nitrogen, the "Generic Imine" is a hydrazone derivative. Hydrazone type "Generic Imine" compounds are readily prepared from the corresponding hydrazine and the appropriate aldehyde or ketone type "Generic Carbonyl Compound". Suitable procedures for forming oxime and hydrazone imines are also described by Shriner, Fuson, and Curtin in The Systematic Indentification of Organic Compounds, 5th Edition, John Wiley & Sons, and by Fieser and Fieser in Reagents for Organic Synthesis, Volume 1, John Wiley & Sons, which are incorporated herein by reference.

The "Generic Secondary Amines" of Formula XIII can be prepared from the corresponding "Generic Imine" of Formula XII in several ways. For example, in one synthetic scheme (Reduction Method-1), which is preferred when Z is a nitrogen, the "Generic Imine" hydrazone of Formula XII is partially or completely dissolved in lower alkanols containing sufficient organic acid or mineral acid as described in WO Patent Application No.9738973, Swiss Patent CH 441366 and U. S. Patent Nos. 3359316 and 3334017, which are incorporated herein by reference, and then hydrogenated at 0-100°C, more preferrably 20-50°C, and most preferrably between 20-30°C and pressures of 10-200

psi hydrogen or more preferrably between 50-70 psi hydrogen in the presence of a noble metal catalyst such as $PtO_2$.

In another synthetic scheme (Reduction Method-2), which is preferrred when Z is a single bond or carbon, the "Generic Imine" of Formula XII is slurried in a lower alcohol such as ethanol, methanol or like solvent at 0-10°C and solid sodium borohydride is added in batches over 5-10 minutes at 0-10°C with stirring. The reaction mixture is stirred below 10°C for 30-90 minutes and then is warmed gradually to 15-30°C. After about 1-10 hours, the mixture is cooled and acid is added until the aqueous layer was just acidic (pH 5-7).

In yet another synthetic scheme (Reduction Method-3), which is preferrred when Z is an oxygen, the "Generic Imine" oxime of Formula XII is slurried in a lower alcohol solvent such methanol or like solvent at 0-10°C and acidified to a pH less than 4. Solid sodium cyanoborohydride is added in batches over 30-90 minutes at 0-20°C with stirring and addition of a suitable organic or mineral acid to keep the pH at or below 4. The reaction mixture is stirred and warmed gradually to about 20-25°C. After about 1-10 hours. the mixture is cooled and base added until the mixture was just slightly alkaline.

The "Generic Secondary Amines" of Formula XIII can also be prepared, according to Scheme 1 by an alkylation procedures based on the nucleophilic substitution of bromides by amines. In one synthetic alkylation scheme (Alkylation Method-1), a "Generic Amine-1" of Formula X is reacted with a "Generic Bromide-2" of Formula XXIII as described in Vogel's Textbook of Practical Organic Chemistry, Fifth Edition, 1989, pages 902 to 905 and references cited therein all of which are incorporated herein by reference. In an alternate synthetic alkylation scheme (Alkylation Method-2), a "Generic Amine-2" of Formula XXII is reacted with a "Generic Bromide-2" of Formula XXIII in a method employing palliadium catalyzed carbon-nitrogen bond formation. Suitable procedures for this conversion are described in Wagaw and Buchwald, J. Org. Chem.(1996), 61, 7240-7241. Wolfe, Wagaw and Buchwald, J. Am. Chem. Soc. (1996), 118, 7215-7216, and Wolfe and Buchwald, Tetrahedron Letters (1997), 38(36), 6359-6362 and references cited therein all of which are incorporated herein by reference. The preferred "Generic Bromide-2" of Formula XXIII are generally aryl bromides, aryl Inflates, and heteroaryl bromides.

The "Generic Amine-1" and "Generic Amine-2" amines, hydroxylamines, and hydrazines, the "Generic Carbonyl Compound" aldehydes, ketones, hydrazones, and oximes, and "Generic Bromide-1" and "Generic Bromide-2" halides, tosylates, mesylates, triflates, and precursor alcohols required to prepare the "Generic Secondary Amine" compounds are available from commercial sources or can be prepared by one skilled in the art from published procedures. Commercial sources include but are not limited to Aldrich Chemical, TCI-America, Lancaster-Synthesis, Oakwood Products, Acros Organics, and Maybridge Chemical. Disclosed procedures for "Generic Amine" amines, hydroxylamines, and hydrazines include Sheradsky and Nov. J. Chem. Soc., Perkin Trans. 1 (1980), (12), 2781-6: Marcoux. Dove. and Buchwald, J. Am. Chem. Soc. (1997), 119, 1053-9; Sternbach and Jamison, Tetrahedron Lett. (1981), 22 (35), 3331-4; U. S. Patent No. 5306718; EP No. 314435; WO No. 9001874; WO No. 9002113: JP No. 05320117: WO No. 9738973; Swiss Patent No. CH 441366; U. S. Patents Nos. 3359316 and 3334017; and references cited therein which are incorporated herein by reference.

Synthetic Scheme 2 shows the preparation of the class of compounds corresponding to Formula I-H ("Generic Polycyclic Aryl and Heteroaryl (R)-Chiral Halogenated 1-Substitutedamino-(n+1)-alkanols"), Formula I-HP ("Generic Polycyclic Aryl and Heteroaryl (R)-Chiral Halogenated 1-Substitutedamino-2-propanols"). Formula 1-HPC("Polycyclic Aryl-Heteroaryl (R)-Chiral Halogenated 1-Substitutedamino-2-Propanols"), Formula I-C ("Polycyclic Phenyl (R)-Chiral Halogenated 1-Substitutedamino-(n+1)-Alkanols"), and Formula I-CP ("Polycyclic Phenyl (R)-Chiral Halogenated 1-Substitutedamino-2-Propanols") wherein A and Q are independently aryl and heteroaryl.

Derivatives of "Generic Polycyclic Aryl and Heteroaryl (R)-Chiral Halogenated 1-Substitutedamino-(n+1)-alkanols", "Generic Polycyclic Aryl and Heteroaryl (R)-Chiral Halogenated 1-Substitutedamino-2-propanols", "Polycyclic Aryl-Heteroaryl (R)-Chiral Halogenated 1-Substitutedamino-2-Propanols". "Polycyclic Phenyl (R)-Chiral Halogenated 1-Substitutedamino-(n+1)-Alkanols", and "Polycyclic Phenyl (R)-Chiral Halogenated 1-Substitutedamino-2-Propanols", in which the hetero atom (-O-) is attached to an alkyl group removed from the amine by two or more carbons are readily prepared by anion chemistry using the method of Scheme 2. The anion of "Generic Secondary Amine" amines, hydroxylamines, and hydrazines of Formula XIII is readily formed by dissolving the specific amine, hydroxylamine, or hydrazine in an aprotic solvent, such as tetrahydrofuran, toluene, ether, dimethylformamide, and dimethylformamide, under anhydrous conditions. The solution is cooled to a temperature between -78 and 0°C, preferrably between -78 and -60°C, and the anion formed by the addition of at least one equivalent of a strong, aprotic, nonnucleophillic base, such as NaH or n-butyllithium under an inert atmosphere, for each acidic group present. Maintaining the temperature between -78 and 0°C, preferrably between -78 and -60°C, with suitable cooling, an appropriate alkyl halide, alkyl benzenesulfonate such as a alkyl tosylate, alkyl mesylate, alkyl triflate or similar alkylating reagent of the general structure:

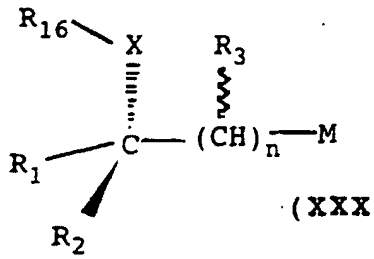

(XXX)

where M is a readily displaceable group such as chloride, bromide, iodide, tosylate, triflate, and mesylate and X is oxy. After allowing the reaction mixture to warm to room temperature, the reaction product is added to water, neutralized if necessary, and extracted with a water-immiscible solvent such as diethyl ether or methylene chloride. The combined aprotic solvent extract is washed with saturated brine, dried over drying agent such as anhydrous MgSO4 and concentrated *in vacuo* to yield crude Formula I-H ("Generic Polycyclic Aryl and Heteroaryl (R)-Chiral Halogenated 1-Substitutedamino-(n+1)-alkanols"), Formula I-HP ("Generic Polycyclic Aryl and Heteroaryl (R)-Chiral Halogenated 1-Substitutedamino-2-propanols"), Formula I-HPC ("Polycyclic Aryl-Heteroaryl (R)-Chiral Halogenated 1-Substitutedamino-2-Propanols"), Formula I-C ("Polycyclic Phenyl (R)-Chiral Halogenated 1-Substitutedamino-(n+1)-Alkanols"), and Formula I-CP ("Polycyclic Phenyl (R)-Chiral Halogenated 1-Substitutedamino-2-Propanols"). This material is purified, for example, by eluting through silica gel with a medium polar solvent such as ethyl acetate in a non-polar solvent such as hexanes to yield Formula I-H ("Generic Polycyclic Aryl and Heteroaryl (R)-Chiral Halogenated 1-Substitutedamino-(n+1)-alkanols"), Formula I-HP ("Generic Polycyclic Aryl and Heteroaryl (R)-Chiral Halogenated 1-Substitutedamino-2-propanols"), Formula I-HPC ("Polycyclic Aryl-Heteroaryl (R)-Chiral Halogenated 1-Substitutedamino-2-Propanols"), Formula I-C ("Polycyclic Phenyl (R)-Chiral Halogenated 1-Substitutedamino-(n+1)-Alkanols"), and Formula I-CP ("Polycyclic Phenyl (R)-Chiral Halogenated 1-Substitutedamino-2-Propanols"). Products are structurally confirmed by low and high resolution mass spectrometry and NMR.

Compounds of Formula (XXX), which can be used to prepare the "Polycyclic Phenyl (R)-Chiral Halogenated 1-Substitutedamino-(n+1)-Alkanol" compounds of Tables 3 and 4, are given in Table 2. Reagents 1a and 2a in Table 2 are prepared from the corresponding alcohols. (R)-Chiral alcohol precursors to 1a, 2a, and similiar alcohols that can be envisioned by one of inventive skill can be obtained from the corresponding racemic mixture of the R-enatiomer and S-enantiomer by separation procedures using preparative gas chromatography and high pressure liquid chromatography using chiral chromatographic columns. The tosylates of chiral alcohols and racemic mixtures are readily obtained by reacting the corresponding alcohol with tosyl chloride using procedures found in House's Modern Synthetic Reactions, Chapter 7, W. A. Benjamin, Inc., Shriner, Fuson, and Curtin in The Systematic Indentification of Organic Compounds, 5th Edition, John Wiley & Sons. and Fieser and Fieser in Reagents for Organic Synthesis, Volume 1, John Wiley & Sons, which are incorporated herein by reference.

Formula I-H ("Generic Polycyclic Aryl and Heteroaryl (R)-Chiral Halogenated 1-Substitutedamino-(n+1)-alkanols"). Formula I-HP ("Generic Polycyclic Aryl and Heteroaryl (R)-Chiral Halogenated 1-Substitutedamino-2-propanols"), Formula I-HPC ("Polycyclic Aryl-Heteroaryl (R)-Chiral Halogenated 1-Substitutedamino-2-Propanols"), Formula I-C ("Polycyclic Phenyl (R)-Chiral Halogenated 1-Substitutedamino-(n+1)-Alkanols"), and Formula I-CP ("Polycyclic Phenyl (R)-Chiral Halogenated 1-Substitutedamino-2-Propanols") compounds can also be prepared using the method of Scheme 2 through the use of racemic (XXX) as described followed by preparative separation of the R-enantiomer from the S-enatiomer using chiral chromatographic procedures such as preparative gas chromatography and high pressure liquid chromatography using readily available chiral chromatographic columns and procedures.

A preferred procedure for Formula I-HP ("Generic Polycyclic Aryl and Heteroaryl (R)-Chiral Halogenated 1-Substitutedamino-2-propanols"), Formula I-HPC ("Polycyclic Aryl-Heteroaryl (R)-Chiral Halogenated 1-Substitutedamino-2-Propanols"), and Formula I-CP ("Polycyclic Phenyl (R)-Chiral Halogenated 1-Substitutedamino-2-Propanols") compounds is the novel inventive Method A of Scheme 3. (R)-Chiral oxirane reagents useful in Method A are exemplified, but not limited to those in Table 1. Formula I-HP ("Generic Polycyclic Aryl and Heteroaryl (R)-Chiral Halogenated 1-Substitutedamino-2-propanols"), Formula I-HPC ("Polycyclic Aryl-Heteroaryl (R)-Chiral Halogenated 1-Substitutedamino-2-Propanols"), and Formula I-CP ("Polycyclic Phenyl (R)-Chiral Halogenated 1-Substitutedamino-2-Propanols") compounds are prepared by reacting "Generic Secondary Amine" amines, hydroxylamines, and hydrazines of Formula XIII with (R)-chiral oxiranes of the type listed in Table 1 and represented by the general structure:

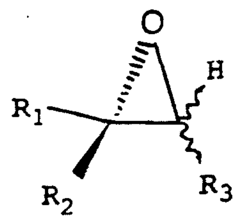
(XX)

Oxiranes having a specific stereochemical arrangement of $R_1$, $R_2$ and $R_3$ can be prepared using chiral procedures such as those published in 1995 by Ramachandran, Gong, and Brown in the Journal of Organic Chemistry, Vol. 60. pages 41 to 46; cited references also detail alternate procedures to prepare chiral and achiral epoxides, which are incorporated herein by reference. For example, the specific preparation of *R*-(+)-1,1,1-trifluoro-2,3-epoxypropane.

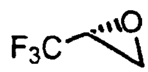,

using a procedure adopted from H.C.Brown et al. (*J. Org. Chem.* **60**, 41-46, (1995)), is accomplished as described in **Example 4**. Many of the epoxides summarized in Table 1 can be prepared in the (R)-configuration using procedures analogous to that given above for *R*-(+)-1,1,1-trifluoro-2,3-epoxypropane.

In some cases, achiral oxiranes of (XX) can be prepared from the corresponding alkenes by reaction of epoxidation reagents such as meta-chloroperbenzoic acid (MCPBA) and similar type reagents readily selectable by a person of skill-in-the-art with alkenes. Fieser and Fieser in Reagents for Organic Synthesis, John Wiley & Sons provides, along with cited references, numerous suitable epoxidation reagents and reaction conditions, which are incorporated herein by reference. These achiral oxiranes can be reacted in an identical manner to that described for (R)-chiral oxiranes with "Generic Secondary Amine" amines, hydroxylamines, and hydrazines of Formula XIII to afford racemic compounds structurally identical to those of Formula I-HP, Formula I-HPC, and Formula I-C but with the corresponding (S) chiral configuration present in an equivalent amount. Formula I-HP ("Generic Polycyclic Aryl and Heteroaryl (R)-Chiral Halogenated 1-Substitutedamino-2-propanols"), Formula I-HPC ("Polycyclic Aryl-Heteroaryl (R)-Chiral Halogenated 1-Substitutedamino-2-Propanols"), and Formula I-CP ("Polycyclic Phenyl (R)-Chiral Halogenated 1-Substitutedamino-2-Propanols") compounds can be obtained by preparative chiral chromatography of said racemic mixtures to obtain the (R)-chiral configuration of Formula I-HP, Formula I-HPC, and Formula I-CP substantially free of the (S)-chiral configuration enantiomer. Alternatively, achiral oxiranes may be separated by chiral preparative chromatography into their respective (R)-Chiral and (S)-Chiral enantiomers and the (R)-Chiral enantiomer reacted to afford Formula I-HP ("Generic Polycyclic Aryl and Heteroaryl (R)-Chiral Halogenated 1-Substitutedamino-2-propanols"), Formula I-HPC ("Polycyclic Aryl-Heteroaryl (R)-Chiral Halogenated 1-Substitutedamino-2-Propanols"), and Formula I-CP ("Polycyclic Phenyl (R)-Chiral Halogenated 1-Substitutedamino-2-Propanols") compounds.

Table 1. Structure of (R)-Chiral Oxirane Reagents.

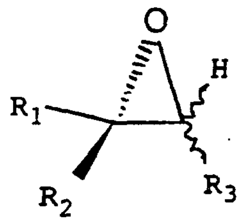

(XX)

| Reagent Number | $R_1$ | $R_2$ | $R_3$ |
|---|---|---|---|
| 1 | $CF_3$ | H | H |
| 2 | $CCl_3$ | H | H |
| 3 | $CF_3$ | $CH_3$ | H |
| 4 | $CF_3CF_2$ | H | H |
| 5 | $CF_3CF_2CF_2$ | H | H |
| 6 | $CF_3OCF_2CF_2$ | H | H |
| 7 | $CF_3CH_2$ | H | H |
| 9 | $CF_3$ | H | $CF_3$ |
| 11 | $CF_3$ | $C_6H_5$ | H |
| 12 | $CCl_3$ | $C_6H_5$ | H |
| 13 | $CCl_3$ | Cyclopropyl | H |
| 14 | $CCl_3$ | $CH_3$ | H |
| 15 | $CCl_3$ | $(CH_3)_2CH$ | H |
| 16 | $CHCl_2$ | H | H |
| 18 | $CF_3$ | H | $CH_3$ |
| 27 | $CCl_3CH_2$ | H | H |
| 28 | $CBr_3CH_2$ | H | H |
| 29 | $CHBr_2CH_2$ | H | H |
| 30 | $CBrCl_2$ | H | H |
| 31 | $CClF_2$ | H | H |
| 32 | $CCl_2F$ | H | H |

Table 1. (continued) Structure of (R)-Chiral Oxirane Reagents.

| Reagent Number | $R_1$ | $R_2$ | $R_3$ |
|---|---|---|---|
| 33 | $CCl_3CCl_2$ | H | H |
| 43 | $FCH_2$ | H | H |
| 56 | $CBrF_2CClFCH_2$ | H | H |
| 57 | $HCF_2CF_2OCH_2$ | H | H |

Table 2. Structure and Source of Alcohol and Glycol Reagents.

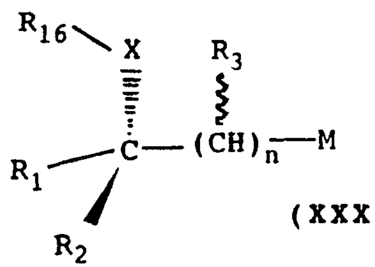

(XXX)

| Reagent Number | $R_1$ | n | M | $R_2$ | $R_3$ | $X-R_{16}$ | Source of Reagent |
|---|---|---|---|---|---|---|---|
| | | | | | | | |
| 1A | $CF_3$ | 3 | OTs | H | H | OH | Chiral separation and then tosylation of alcohol from Justus Liebigs Ann. Chem. (1969), 720, 81-97. |
| 2A | $CF_3CH_2CH_2$ | 3 | OTs | H | H | OH | Chiral separation and then tosylation of alcohol from Z. Naturforsch., B: Chem. Sci. (1997), 52 (3), 413-418 |

Table 3. Structure of Phenyl (R)-Chiral Halogenated 1-Substitutedamino-(n+1)-Alkanols

(Y is CH; $R_8$, $R_9$, $R_{12}$, $R_{13}$, and $R_{14}$ are each H; Z is covalent bond and $R_{15}$ is absent).

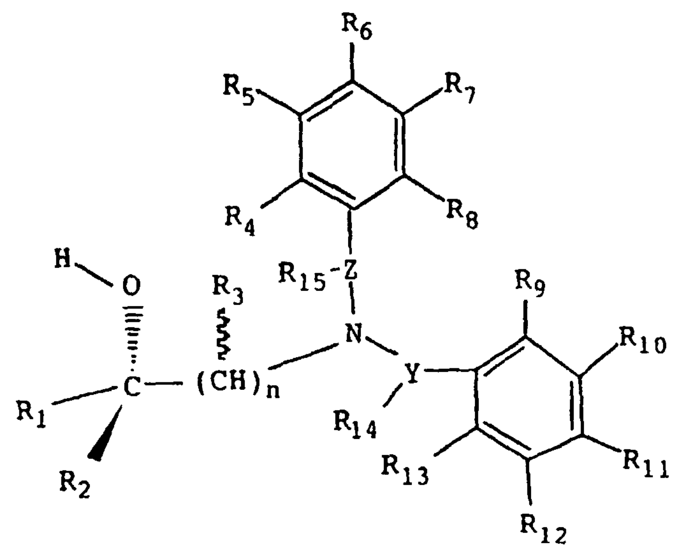

| Inhibitor Number Column1+Column 2 | | $R_1$ | n | $R_2$ | $R_3$ | $R_4$ | $R_5$ | $R_6$ | $R_7$ | $R_{10}$ | $R_{11}$ |
|---|---|---|---|---|---|---|---|---|---|---|---|
| Reagent | Reagent | | | | | | | | | | |
| 1A | 1N | $CF_3$ | 3 | H | H | H | $C_6H_5O$ | H | H | $OCF_2CF_2H$ | H |
| 1A | 2N | $CF_3$ | 3 | H | H | H | $OCF_3$ | H | H | $OCF_2CF_2H$ | H |

Table 3. (continued) Structure of Phenyl (R)-Chiral Halogenated 1-Substitutedamino-(n+1)-Alkanols

(Y is CH; $R_8$, $R_9$, $R_{12}$, $R_{13}$, and $R_{14}$ are each H; Z is covalent bond and $R_{15}$ is absent).

| Inhibitor Number Column1+Column 2 | | $R_1$ | n | $R_2$ | $R_3$ | $R_4$ | $R_5$ | $R_6$ | $R_7$ | $R_{10}$ | $R_{11}$ |
|---|---|---|---|---|---|---|---|---|---|---|---|
| Reagent | Reagent | | | | | | | | | | |
| 1A | 3N | $CF_3$ | 3 | H | H | F | H | H | F | $OCF_2CF_2H$ | H |
| 1A | 4N | $CF_3$ | 3 | H | H | H | F | H | H | $OCF_2CF_2H$ | H |
| 1A | 5N | $CF_3$ | 3 | H | H | H | $C_6H_5O$ | H | H | $OCF_3$ | H |
| 1A | 6N | $CF_3$ | 3 | H | H | H | $OCF_3$ | H | H | $OCF_3$ | H |
| 1A | 7N | $CF_3$ | 3 | H | H | H | H | phenyl | H | $OCF_3$ | H |
| 1A | 8N | $CF_3$ | 3 | H | H | H | phenyl | H | H | $OCF_3$ | H |
| 1A | 9N | $CF_3$ | 3 | H | H | H | H | H | H | $OCF_3$ | H |
| 1A | 10N | $CF_3$ | 3 | H | H | H | Br | H | H | $OCF_3$ | H |
| 1A | 11N | $CF_3$ | 3 | H | H | H | $CF_3$ | F | H | $CF_3$ | H |
| 1A | 12N | $CF_3$ | 3 | H | H | H | $CH_3$ | H | H | $CF_3$ | H |
| 1A | 13N | $CF_3$ | 3 | H | H | H | $CF_3$ | H | H | $CF_3$ | H |
| 1A | 14N | $CF_3$ | 3 | H | H | H | $CH_3$ | H | H | $OCF_3$ | H |

Table 3. (continued) Structure of Phenyl (R)-Chiral Halogenated 1-Substitutedamino-(n+1)-Alkanols

(Y is CH; $R_8$, $R_9$, $R_{12}$, $R_{13}$, and $R_{14}$ are each H; Z is covalent bond and $R_{15}$ is absent).

| Inhibitor Number Column1+Column 2 | | $R_1$ | n | $R_2$ | $R_3$ | $R_4$ | $R_5$ | $R_6$ | $R_7$ | $R_{10}$ | $R_{11}$ |
|---|---|---|---|---|---|---|---|---|---|---|---|
| Reagent | Reagent | | | | | | | | | | |
| 1A | 15N | $CF_3$ | 3 | H | H | H | F | F | H | $OCF_3$ | H |
| 1A | 16N | $CF_3$ | 3 | H | H | H | Br | H | H | $CF_3$ | H |
| 1A | 17N | $CF_3$ | 3 | H | H | H | $CF_3$ | F | H | $OCF_3$ | H |
| 1A | 18N | $CF_3$ | 3 | H | H | H | F | H | H | $OCF_3$ | H |
| 1A | 19N | $CF_3$ | 3 | H | H | H | Cl | H | H | $OCF_3$ | H |
| 1A | 20N | $CF_3$ | 3 | H | H | H | F | H | H | $CF_3$ | H |
| 1A | 21N | $CF_3$ | 3 | H | H | H | F | F | H | $CF_3$ | H |
| 1A | 22N | $CF_3$ | 3 | H | H | H | Cl | H | H | $CF_3$ | H |
| 1A | 23N | $CF_3$ | 3 | H | H | H | F | H | H | phenoxy | H |
| 1A | 24N | $CF_3$ | 3 | H | H | H | $CF_3$ | Cl | H | $CH_3$ | H |
| 1A | 25N | $CF_3$ | 3 | H | H | H | $CF_3$ | F | H | $CH_3$ | H |
| 1A | 26N | $CF_3$ | 3 | H | H | H | H | H | H | $CF_3$ | H |

Table 3. (continued) Structure of Phenyl (R)-Chiral Halogenated 1-Substitutedamino-(n+1)-Alkanols

(Y is CH; $R_8$, $R_9$, $R_{12}$, $R_{13}$, and $R_{14}$ are each H; Z is covalent bond and $R_{15}$ is absent).

| Inhibitor Number Column1+Column 2 | | $R_1$ | n | $R_2$ | $R_3$ | $R_4$ | $R_5$ | $R_6$ | $R_7$ | $R_{10}$ | $R_{11}$ |
|---|---|---|---|---|---|---|---|---|---|---|---|
| Reagent | Reagent | | | | | | | | | | |
| IA | 27N | $CF_3$ | 3 | H | H | F | F | H | H | $CF_3$ | H |
| IA | 28N | $CF_3$ | 3 | H | H | H | H | $OCH_3$ | H | $CF_3$ | H |
| IA | 29N | $CF_3$ | 3 | H | H | H | F | F | H | $CH_3$ | H |
| IA | 30N | $CF_3$ | 3 | H | H | H | $OCH_3$ | H | H | $CH_3$ | H |
| IA | 31N | $CF_3$ | 3 | H | H | H | H | $CH_3$ | H | H | H |
| IA | 32N | $CF_3$ | 3 | H | H | H | Cl | H | H | H | H |
| IA | 33N | $CF_3$ | 3 | H | H | H | F | H | H | F | H |
| IA | 34N | $CF_3$ | 3 | H | H | H | H | $OCH_3$ | H | $CH_3$ | H |
| IA | 35N | $CF_3$ | 3 | H | H | H | H | H | H | H | H |
| IA | 36N | $CF_3$ | 3 | H | H | H | H | $CH_3$ | H | $CH_3$ | H |
| IA | 37N | $CF_3$ | 3 | H | H | H | H | Cl | H | H | H |
| IA | 38N | $CF_3$ | 3 | H | H | H | F | H | H | 3-$CF_3$- | H |

Table 3. (continued) Structure of Phenyl (R)-Chiral Halogenated 1-Substitutedamino-(n+1)-Alkanols

(Y is CH; $R_8$, $R_9$, $R_{12}$, $R_{13}$, and $R_{14}$ are each H; Z is covalent bond and $R_{15}$ is absent).

| Inhibitor Number Column1+Column 2 | | $R_1$ | n | $R_2$ | $R_3$ | $R_4$ | $R_5$ | $R_6$ | $R_7$ | $R_{10}$ | $R_{11}$ |
|---|---|---|---|---|---|---|---|---|---|---|---|
| Reagent | Reagent | | | | | | | | | | |
| | | | | | | | | | | phenoxy | |
| IA | 39N | $CF_3$ | 3 | H | H | H | F | H | H | 4-$CH_3O$-phenoxy | H |
| IA | 40N | $CF_3$ | 3 | H | H | H | F | H | H | 4-Cl-phenoxy | H |
| IA | 41N | $CF_3$ | 3 | H | H | H | F | H | H | H | H |
| IA | 42N | $CF_3$ | 3 | H | H | H | F | H | H | $CH_3$ | H |
| IA | 43N | $CF_3$ | 3 | H | H | H | F | H | F | $CH_3$ | H |
| IA | 44N | $CF_3$ | 3 | H | H | F | F | H | H | $CH_3$ | H |
| IA | 45N | $CF_3$ | 3 | H | H | H | Cl | H | H | $CH_3$ | H |
| IA | 46N | $CF_3$ | 3 | H | H | H | $CH_3$ | H | H | $CH_3$ | H |
| IA | 48N | $CF_3$ | 3 | H | H | H | H | $CH_3$ | H | $CF_3$ | H |
| IA | 51N | $CF_3$ | 3 | H | H | H | H | $CH_3$ | H | F | H |

Table 3. (continued) Structure of Phenyl (R)-Chiral Halogenated 1-Substitutedamino-(n+1)-Alkanols

(Y is CH; $R_8$, $R_9$, $R_{12}$, $R_{13}$, and $R_{14}$ are each H; Z is covalent bond and $R_{15}$ is absent).

| Inhibitor Number Column1+Column 2 | | $R_1$ | n | $R_2$ | $R_3$ | $R_4$ | $R_5$ | $R_6$ | $R_7$ | $R_{10}$ | $R_{11}$ |
|---|---|---|---|---|---|---|---|---|---|---|---|
| Reagent | Reagent | | | | | | | | | | |
| 1A | 52N | $CF_3$ | 3 | H | H | H | $CF_3$ | H | H | F | H |
| 1A | 53N | $CF_3$ | 3 | H | H | H | $CF_3$ | H | H | $CH_3$ | H |
| 1A | 54N | $CF_3$ | 3 | H | H | H | $OCH_3$ | H | H | $CF_3$ | H |
| 1A | 56N | $CF_3$ | 3 | H | H | H | H | $CH_3$ | H | $CF_3$ | H |
| 1A | 57N | $CF_3$ | 3 | H | H | H | $C_6H_5O$ | H | H | H | $OCF_3$ |
| 1A | 58N | $CF_3$ | 3 | H | H | H | H | H | H | H | $OCF_3$ |
| 1A | 59N | $CF_3$ | 3 | H | H | H | $OCF_3$ | H | H | H | $OCF_3$ |
| 1A | 60N | $CF_3$ | 3 | H | H | H | $CF_3$ | F | H | H | $CF_3$ |
| 1A | 61N | $CF_3$ | 3 | H | H | H | H | $OCH_3$ | H | H | $CF_3$ |
| 1A | 62N | $CF_3$ | 3 | H | H | H | $CH_3$ | H | H | H | $CF_3$ |
| 1A | 63N | $CF_3$ | 3 | H | H | H | Cl | H | H | H | $CF_3$ |
| 1A | 64N | $CF_3$ | 3 | H | H | H | $CF_3$ | H | H | H | $OCF_3$ |

Table 3. (continued) Structure of Phenyl (R)-Chiral Halogenated 1-Substitutedamino-(n+1)-Alkanols

(Y is CH; $R_8$, $R_9$, $R_{12}$, $R_{13}$, and $R_{14}$ are each H; Z is covalent bond and $R_{15}$ is absent).

| Inhibitor Number Column1+Column 2 | | $R_1$ | n | $R_2$ | $R_3$ | $R_4$ | $R_5$ | $R_6$ | $R_7$ | $R_{10}$ | $R_{11}$ |
|---|---|---|---|---|---|---|---|---|---|---|---|
| Reagent | Reagent | | | | | | | | | | |
| 1A | 65N | $CF_3$ | 3 | H | H | H | F | H | H | H | $OCF_3$ |
| 1A | 66N | $CF_3$ | 3 | H | H | H | F | H | F | H | $OCF_3$ |
| 1A | 67N | $CF_3$ | 3 | H | H | H | Br | H | H | H | $OCF_3$ |
| 1A | 68N | $CF_3$ | 3 | H | H | H | Cl | H | H | H | $OCF_3$ |
| 1A | 69N | $CF_3$ | 3 | H | H | H | F | F | H | H | $OCF_3$ |
| 1A | 70N | $CF_3$ | 3 | H | H | H | F | H | H | H | phenyl |
| 1A | 71N | $CF_3$ | 3 | H | H | H | $CH_3$ | H | H | H | $OCF_3$ |
| 1A | 72N | $CF_3$ | 3 | H | H | H | F | F | H | H | $CF_3$ |
| 1A | 73N | $CF_3$ | 3 | H | H | H | Cl | H | H | H | $CH_3$ |
| 1A | 74N | $CF_3$ | 3 | H | H | H | $OCH_3$ | H | H | H | $CH_3$ |
| 1A | 75N | $CF_3$ | 3 | H | H | H | F | H | H | H | $CH_3$ |
| 1A | 76N | $CF_3$ | 3 | H | H | F | F | H | H | H | $OCF_3$ |

Table 3. (continued) Structure of Phenyl (R)-Chiral Halogenated 1-Substitutedamino-(n+1)-Alkanols

(Y is CH; $R_8$, $R_9$, $R_{12}$, $R_{13}$, and $R_{14}$ are each H; Z is covalent bond and $R_{15}$ is absent).

| Inhibitor Number Column1+Column 2 | | $R_1$ | n | $R_2$ | $R_3$ | $R_4$ | $R_5$ | $R_6$ | $R_7$ | $R_{10}$ | $R_{11}$ |
|---|---|---|---|---|---|---|---|---|---|---|---|
| Reagent | Reagent | | | | | | | | | | |
| IA | 78N | $CF_3$ | 3 | H | H | H | H | $OCH_3$ | H | H | $CH_3$ |
| IA | 79N | $CF_3$ | 3 | H | H | H | H | $CH_3$ | H | H | $CH_3$ |
| IA | 80N | $CF_3$ | 3 | H | H | H | $CH_3$ | H | H | H | $CH_3$ |
| IA | 82N | $CF_3$ | 3 | H | H | H | F | F | H | H | $CH_3$ |
| IA | 83N | $CF_3$ | 3 | H | H | H | F | H | F | H | $CH_3$ |
| IA | 84N | $CF_3$ | 3 | H | H | F | F | H | H | H | $CH_3$ |
| IA | 85N | $CF_3$ | 3 | H | H | F | $CF_3$ | H | H | H | $CH_3$ |
| IA | 86N | $CF_3$ | 3 | H | H | H | H | $CH_3$ | H | H | $CF_3$ |
| IA | 88N | $CF_3$ | 3 | H | H | H | $CF_3$ | H | H | H | $CH_3$ |
| IA | 90N | $CF_3$ | 3 | H | H | H | H | $CF_3$ | H | H | $CH_3$ |
| IA | 92N | $CF_3$ | 3 | H | H | H | $CF_3$ | F | H | H | $CH_3$ |

Table 4. Structure of Phenyl (R)-Chiral Halogenated 1-Substitutedamino-(n+1)-Alkanols

(Y and Z are each CH: $R_8$, $R_9$, $R_{12}$, $R_{13}$, $R_{14}$ and $R_{15}$ are each H).

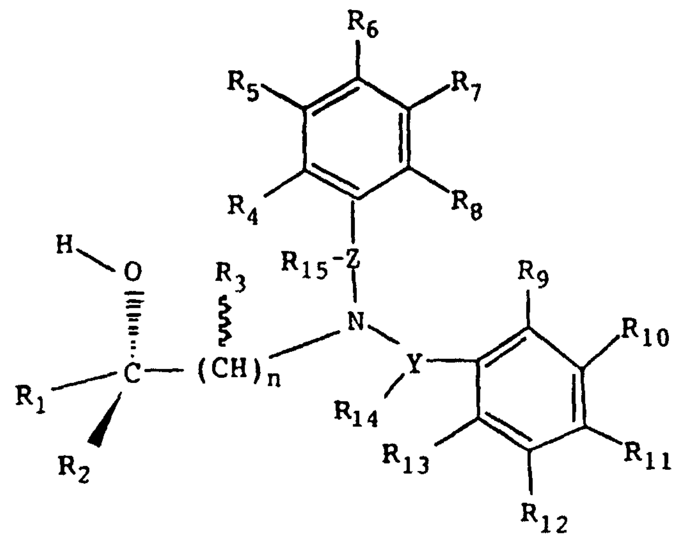

| Inhibitor Number Column 1+Column 2 | | $R_1$ | n | $R_2$ | $R_3$ | $R_4$ | $R_5$ | $R_6$ | $R_9$ | $R_{10}$ | $R_{11}$ |
|---|---|---|---|---|---|---|---|---|---|---|---|
| Reagent | Reagent | | | | | | | | | | |
| IA | IDB | $CF_3$ | 3 | H | H | H | $OCF_3$ | H | H | $OCF_3$ | H |

Table 4. (continued) Structure of Phenyl (R)-Chiral Halogenated 1-Substitutedamino-(n+1)-Alkanols

(Y and Z are each CH; $R_8$, $R_9$, $R_{12}$, $R_{13}$, $R_{14}$ and $R_{15}$ are each H).

| Inhibitor Number Column 1+Column 2 | | $R_1$ | n | $R_2$ | $R_3$ | $R_4$ | $R_5$ | $R_6$ | $R_9$ | $R_{10}$ | $R_{11}$ |
|---|---|---|---|---|---|---|---|---|---|---|---|
| Reagent | Reagent | | | | | | | | | | |
| 1A | 2DB | $CF_3$ | 3 | H | H | H | Cl | H | H | H | $CF_3$ |
| 1A | 3DB | $CF_3$ | 3 | H | H | H | Br | H | H | $OCF_3$ | H |
| 1A | 4DB | $CF_3$ | 3 | H | H | H | Cl | H | H | $OCF_3$ | H |
| 1A | 5DB | $CF_3$ | 3 | H | H | H | Cl | H | H | $CF_3$ | H |
| 1A | 6DB | $CF_3$ | 3 | H | H | H | H | Cl | H | $CF_3$ | H |
| 1A | 7DB | $CF_3$ | 3 | H | H | H | F | H | H | $OCF_3$ | H |
| 1A | 8DB | $CF_3$ | 3 | H | H | H | H | Cl | H | H | $CF_3$ |
| 1A | 9DB | $CF_3$ | 3 | H | H | H | F | H | H | H | $CF_3$ |
| 1A | 10DB | $CF_3$ | 3 | H | H | H | H | F | H | H | $CF_3$ |
| 1A | 11DB | $CF_3$ | 3 | H | H | F | H | H | H | H | $CF_3$ |
| 1A | 12DB | $CF_3$ | 3 | H | H | H | Cl | H | $CF_3$ | H | H |

Table 4. (continued) Structure of Phenyl (R)-Chiral Halogenated 1-Substitutedamino-(n+1)-Alkanols

(Y and Z are each CH; $R_8$, $R_9$, $R_{12}$, $R_{13}$, $R_{14}$ and $R_{15}$ are each H).

| Inhibitor Number Column 1+Column 2 | | $R_1$ | n | $R_2$ | $R_3$ | $R_4$ | $R_5$ | $R_6$ | $R_9$ | $R_{10}$ | $R_{11}$ |
|---|---|---|---|---|---|---|---|---|---|---|---|
| Reagent | Reagent | | | | | | | | | | |
| 1A | 13DB | $CF_3$ | 3 | H | H | H | H | Cl | $CF_3$ | H | H |
| 1A | 14DB | $CF_3$ | 3 | H | H | Cl | H | H | $CF_3$ | H | H |
| 1A | 15DB | $CF_3$ | 3 | H | H | H | F | H | $CH_3$ | H | H |
| 1A | 16DB | $CF_3$ | 3 | H | H | H | H | F | H | H | $CH_3$ |
| 1A | 17DB | $CF_3$ | 3 | H | H | H | F | H | H | $CH_3$ | H |
| 1A | 18DB | $CF_3$ | 3 | H | H | F | H | H | $CH_3$ | H | H |
| 1A | 19DB | $CF_3$ | 3 | H | H | H | H | F | H | $CH_3$ | H |
| 1A | 20DB | $CF_3$ | 3 | H | H | F | H | H | H | H | $CH_3$ |
| 1A | 21DB | $CF_3$ | 3 | H | H | F | H | H | H | $CF_3$ | H |
| 1A | 22DB | $CF_3$ | 3 | H | H | Cl | H | H | H | $CF_3$ | H |
| 1A | 23DB | $CF_3$ | 3 | H | H | H | F | H | $CF_3$ | H | H |

Table 4. (continued) Structure of Phenyl (R)-Chiral Halogenated 1-Substitutedamino-(n+1)-Alkanols (Y and Z are each CH; $R_8$, $R_9$, $R_{12}$, $R_{13}$, $R_{14}$ and $R_{15}$ are each H).

| Inhibitor Number Column 1+Column 2 | | $R_1$ | n | $R_2$ | $R_3$ | $R_4$ | $R_5$ | $R_6$ | $R_9$ | $R_{10}$ | $R_{11}$ |
|---|---|---|---|---|---|---|---|---|---|---|---|
| Reagent | Reagent | | | | | | | | | | |
| 1A | 24DB | $CF_3$ | 3 | H | H | H | H | F | $CF_3$ | H | H |
| 1A | 25DB | $CF_3$ | 3 | H | H | H | F | H | H | $CF_3$ | H |
| 1A | 26DB | $CF_3$ | 3 | H | H | H | H | F | H | $CF_3$ | H |
| 1A | 27DB | $CF_3$ | 3 | H | H | H | $OCF_3$ | H | H | H | $OCF_3$ |

A mixture of a "Generic Secondary Amine" amine, hydroxylamine, or hydrazine of Formula XIII and an excess of a halogenated oxirane of (R)-chiral configuration of Formula XX are stirred and heated to 40-90°C for 5 to 48 hours in a tightly capped or contained reaction vessel. More preferrably, a Lewis acid such as a transition metal-based salts (for example, ytterbium triflate, hafnium triflate, scandium triflate, neodynium inflate, gadolium triflate, and zirconium triflate) in methylene chloride, tetrahydrofuran, or, more preferrably, acetonitrile is added to speed up the reaction to a total time of 4 to 18 hours, improve yields, to permit the reaction temperature to be reduced to 15-65°C, and to use a smaller excess of halogenated oxirane. When a Lewis acid is used. the reaction should be carried out under inert, anhydrous conditions using a blanket of dry nitrogen or argon gas. After cooling to room temperature and testing the

reaction mixture for complete reaction by thin layer chromatography or high pressure liquid chromatography (hplc), the reaction product is added to water and extracted with a water immiscible solvent such as diethyl ether or methylene chloride. (Note: If the above analysis indicates that reaction is incomplete, heating should be resumed until complete with the optional addition of more of the oxirane). The combined aprotic solvent extract is washed with saturated brine, dried over drying agent such as anhydrous $MgSO_4$ and concentrated *in vacuo* to yield crude Formula 1-HP ("Generic Polycyclic Aryl and Heteroaryl (R)-Chiral Halogenated 1-Substitutedamino-2-propanols"), Formula I-HPC ("Polycyclic Aryl-Heteroaryl (R)-Chiral Halogenated 1-Substitutedamino-2-Propanols"), and Formula I-C ("Polycyclic Phenyl (R) -Chiral Halogenated 1-Substitutedamino-2-Propanols") compounds. This material is purified by eluting through silica gel with 5-40% of a medium polar solvent such as ethyl acetate in a non-polar solvent such as hexanes to yield Formula I-HP ("Generic Polycyclic Aryl and Heteroaryl (R)-Chiral Halogenated 1-Substitutedamino-2-propanols"), Formula I-HPC ("Polycyclic Aryl-Heteroaryl (R)-Chiral Halogenated 1-Substitutedamino-2-Propanols"), and Formula I-C ("Polycyclic Phenyl (R)-Chiral Halogenated 1-Substitutedamino-2-Propanols") compounds. Products are tested for purity by HPLC. If necessary, the Formula I-HP ("Generic Polycyclic Aryl and Heteroaryl (R)-Chiral Halogenated 1-Substitutedamino-2-propanols"), Formula I-HPC ("Polycyclic Aryl-Heteroaryl (R)-Chiral Halogenated 1-Substitutedamino-2-Propanols"), and Formula I-C ("Polycyclic Phenyl (R)-Chiral Halogenated 1-Substitutedamino-2-Propanols") compounds are purified by additional chromatography or recrystallization. Products are structurally confirmed by low and high resolution mass spectrometry and NMR. Examples of specific Formula I-HP ("Generic Polycyclic Aryl and Heteroaryl (R)-Chiral Halogenated 1-Substitutedamino-2-propanols"). Formula 1-HPC ("Polycyclic Aryl-Heteroaryl (R)-Chiral Halogenated 1-Substitutedamino-2-Propanols"), and Formula I-C ("Polycyclic Phenyl (R)-Chiral Halogenated 1-Substitutedamino-2-Propanols") compounds prepared are summarized in the Examples 1 through 44, and Example Tables 1 through 12.

Formula I-HP ("Generic Polycyclic Aryl and Heteroaryl (R)-Chiral Halogenated 1-Substitutedamino-2-propanols"). Formula I-HPC ("Polycyclic Aryl-Heteroaryl (R)-Chiral Halogenated 1-Substitutedamino-2-Propanols"), and Formula I-C ("Polycyclic Phenyl (R)-Chiral Halogenated 1-Substitutedamino-2-Propanols") compounds can further be prepared in an alternate manner to procedures disclosed above and in Schemes 1, 2, and 3. Schemes 9 and 10 detail such procedures to prepare aminopropanol compounds by initial formation of an halogenated, oxygen containing primary alkylamine XVL ("Generic Substituted Alkylamine"). Said halogenated, oxygen containing primary alkylamine XVL, formed in Scheme 9, is itself converted to secondary amines, VLX-H ("Heteroaryl Alkyl Amine) and VLX ("Phenyl Alkyl Amine"), using procedures disclosed above. Primary alkylamine XVL is first reacted with an aldehydic or ketonic carbonyl compound, XI-AH ("Heteroaryl Carbonyl") with azeotropic distillation to form imine, VL-H ("Heteroaryl Imine"). Said imine VL-H is then reduced with or without prior isolation by Reduction Methods 1, 2 or 3 as disclosed above and in Scheme 1 to yield secondary amine. VLX-H ("Heteroaryl Alkyl Amine). Said secondary amine VLX-H can be converted according to Scheme 10 to give Formula I-HP ("Generic Polycyclic Aryl and Heteroaryl (R)-Chiral Halogenated 1-Substitutedamino-2-propanols") and Formula I-HPC ("Polycyclic Aryl-Heteroaryl (R)-Chiral Halogenated 1-Substitutedamino-2-Propanols") and Formula I-C ("Polycyclic Phenyl (R)-Chiral Halogenated 1-Substitutedamino-2-Propanols") compounds. Using similar Schemes, VLX can be converted to Formula I-C ("Polycyclic Phenyl (R)-Chiral Halogenated 1-Substitutedamino-2-Propanols") compounds. Compounds in which one aromatic substituent is aryl and the other aromatic substitutent is heteroaryl can be readily prepared by reacting VLX-H with an aralkyl bromide or aryl bromide instead of using an heteroaralkyl bromide or heteroaryl bromide. Similarly, compounds in which one aromatic substituent is aryl and the other aromatic substituted is heteroaryl can be readily prepared by reacting VLX with an heteroaryl bromide or heteroaralkyl bromide instead of using an aryl bromide or an aralkyl bromide.

Formula I-HP ("Generic Polycyclic Aryl and Heteroaryl (R)-Chiral Halogenated 1-Substitutedamino-2-propanols"). Formula I-HPC ("Polycyclic Aryl-Heteroaryl (R)-Chiral Halogenated 1-Substitutedamino-2-Propanols"). and Formula I-C ("Polycyclic Phenyl (R)-Chiral Halogenated 1-Substitutedamino-2-Propanols") compounds can further be prepared in an alternate manner to procedures disclosed above and in Schemes 1, 2, 3, 9, and 10. Schemes 13, 14, and 15 detail alternate procedures to prepare (R)-Chiral Halogenated 1-Substitutedamino-2-propanols" compounds by initial formation of an halogenated, oxygen containing secondary alkylamines VLX and VLXX ("Phenyl Alkylamines") and VLXX-O ("Phenyl Oxy Alkylamines"). Said secondary alkylamines VLX and VLXX ("Phenyl Alkylamines") and VLXX-O ("Phenyl Oxy Alkylamines") can be converted according to Schemes 13, 14, and 15 to Formula 1-HP ("Generic Polycyclic Aryl and Heteroaryl (R)-Chiral Halogenated 1-Substitutedamino-2-propanols"), Formula I-HPC ("Polycyclic Aryl-Heteroaryl (R)-Chiral Halogenated 1-Substitutedamino-2-Propanols"), and Formula I-C ("Polycyclic Phenyl (R) -Chiral Halogenated 1-Substitutedamino-2-Propanols") compounds by reaction with appropriate aromatic halides such as aryl bromides and heteroaryl bromides as desired.

Formula I-HP ("Generic Polycyclic Aryl and Heteroaryl (R)-Chiral Halogenated 1-Substitutedamino-2-propanols"). Formuta I-HPC ("Polycyclic Aryl-Heteroaryl (R)-Chiral Halogenated 1-Substitutedamino-2-Propanols"), and Formula I-C ("Polycyclic Phenyl (R)-Chiral Halogenated 1-Substitutedamino-2-Propanols") compounds can further be prepared in an alternate manner to procedures disclosed above and in Schemes 1, 2, 3, 9, 10, 13, 14, and 15. Another alternate procedure to prepare "(R)-Chiral Halogenated 1-Substitutedamino-2-propanols" compounds can be achieved

by reacting secondary amines of Formula XIIIA-H ("Secondary Heteroaryl Amines") and Formula XIII-A ("Secondary Phenyl Amines") with certain cyclic sulfates. Cyclic sulfates useful in the preparation of "(R)-Chiral Halogenated 1-Substitutedamino-2-propanols" compounds of Formulas I-HP, I-HPC, and I-CP have a halogenated or haloalkoxy carbon adjacent to the cyclic sulfate. Some cyclic sulfates useful for the preparation of "(R)-Chiral Halogenated 1-Substitutedamino-2-propanols" compounds of Formulas I-HP, I-HPC, and I-CP have been described by K. P. M. Vanhessche and K. B. Sharpless in Chem. Eur. J. 1997, Vol. 3, No. 4, pages 517-522 and references cited therein. (2R)-(+)-3,3,3-Trifluoro-1,2-propanediol can be prepared as described in the reference cited immediately above from 3,3,3-trifluoropropene followed by separation from the predominating (2S)-(-)-3,3,3-trifluoro-1,2-propanediol. Alternatively, (2R)-(+)-3,3,3-Trifluoro-1,2-propanediol can be prepared by hydrolysis of (2R)-(+)-3,3,3-Trifluoro-2,3-epxoypropane analogous to the procedure described by described by McBee and Burton in J. Am. Chem. Soc.. 1952, Vol. 74, page 3022. (2R)-(+)-3,3,3-Trifluoro-1,2-propanediol is converted by reaction with a slight excess of sulfuryl chloride in the presence of 2.5 molar equivalents of imidazole, methylene chloride solvent, and at a temperature of -20 °C to give the desired (4R)-(+)-4-trifluoromethyl-2,2-dioxo-1,3,2-dioxathiolane. Reaction of other (R)-Chiral haloalkyl or haloalkoxyalkyl substituted 1,2-ethanediols can afford the corresponding (4R)-substituted-2,2-dioxo-1,3,2-dioxathiolanes. Reaction of (4R)-(+)-4-trifluoromethyl-2,2-1,3,2-dioxathiolane or another (4R)-substituted-2,2-dioxo-1,3,2-dioxathiolane with a secondary amine of Formula XIIIA-H ("Secondary Heteroaryl Amines") and Formula XIII-A ("Secondary Phenyl Amines") in an anhydrous polar, non-protic solvent such as tetrahydrofuran or acetonitrile at 25-60 °C until the reaction is complete can afford the mono-sulfate ester of a compound of Formulas I-HP, I-HPC, and I-CP. Removal of the solvent followed by addition of diethyl ether and excess 20% aqueous sulfuric acid can lead to a precipitant of the crude mono-sulfate ester of a compound of Formulas I-HP, I-HPC, and I-CP. This precipitant can be filtered, the solid can be washed with ether, it can be resuspended in aqueous 20% sulfuric acid, and can be heated to 80-95 °C to give an aqueous solution of the sulfate salt of crude a compound of Formulas I-HP, I-HPC, and I-CP. Neutralization of the aqueous solution, extraction with a water immiscible solvent such as diethyl ether or methylene chloride, drying the organic solvent over anhydrous magnesium sulfate, and removal of solvent can afford a compound of Formulas I-HP, I-HPC, and I-CP. Compounds of Formulas I-HP, I-HPC, and I-CP can be purified as described previously. By using a wide variety of (R)-Chiral diols, secondary amines of Formula XIIIA-H ("Secondary Heteroaryl Amines") and Formula XIII-A ("Secondary Phenyl Amines"), and reaction conditions described herein, a large variety of compounds of Formulas I-HP, I-HPC, and I-CP may be preparable.

A particularly useful procedures to prepare Formula I-H ("Generic Polycyclic Aryl and Heteroaryl (R)-Chiral Halogenated 1-Substitutedamino-(n+1)-alkanols"), Formula I-HP ("Generic Polycyclic Aryl and Heteroaryl (R)-Chiral Halogenated 1-Substitutedamino-2-propanols"). Formula I-HPC ("Polycyclic Aryl-Heteroaryl (R)-Chiral Halogenated 1-Substitutedamino-2-Propanols"), Formula I-C ("Polycyclic Phenyl (R)-Chiral Halogenated 1-Substitutedamino-(n+1)-Alkanols"), and Formula 1-CP ("Polycyclic Phenyl (R)-Chiral Halogenated 1-Substitutedamino-2-Propanols") compounds in which the heteroaryl group is directly bonded is disclosed in Schemes 11 and 12. An halogenated, hydroxy containing primary alkylamine XVL ("Generic Substituted Alkylamine") formed according to Scheme 9 is itself converted by reaction with LXXI-AH ("Heteroaryl Halide") to afford secondary amine VLXX-H ("Heteroaryl Secondary Amine) using procedures disclosed in Scheme 11 and above. VLXX-H is converted to Formula I-HP ("Generic Polycyclic Aryl and Heteroaryl (R)-Chiral Halogenated 1-Substitutedamino-2-propanols"), Formula I-HPC ("Polycyclic Aryl-Heteroaryl (R)-Chiral Halogenated 1-Substitutedamino-2-Propanols"), and Formula I-CP ("Polycyclic Phenyl (R)-Chiral Halogenated 1-Substitutedamino-2-Propanols") compounds by alkylation chemistry with an aralkyl bromide or aralkyloxyalkyl bromide using either of two procedures disclosed in Scheme 12. Isolation and purification is effected as disclosed previously.

Formula I-H ("Generic Polycyclic Aryl and Heteroaryl (R)-Chiral Halogenated 1-Substitutedamino-(n+1)-alkanols"), Formula I-HP ("Generic Polycyclic Aryl and Heteroaryl (R)-Chiral Halogenated 1-Substitutedamino-2-propanols"), Formula I-HPC ("Polycyclic Aryl-Heteroaryl (R)-Chiral Halogenated 1-Substitutedamino-2-Propanols"), Formula I-C ("Polycyclic Phenyl (R)-Chiral Halogenated 1-Substitutedamino-(n+1)-Alkanols"), and Formula I-CP ("Polycyclic Phenyl (R)-Chiral Haloaenated 1-Substitutedamino-2-Propanols") compounds can themselves serve as intermediates for conversion to additional compounds of this invention. Compounds of Formulas I-H, I-HP, I-C, I-CP, I-HPC and others useful as intermediates include those in which the $R_7$ position substituent in Formulas I-H, I-HP, I-C, I-CP, and I-HPC is a bromo group, hydroxyl group, sulfhydryl group, bromomethyl or other bromoalkyl groups, nitro group, amino group, methoxycarbonyl or other alkoxy carbonyl groups, cyano group, or acyl group. Other preferred compounds of Formulas I-H, 1-HP, I-C, I-CP, I-HPC useful as intermediates include those in which the $R_{10}$ position substituent is a bromo group, hydroxyl group, sulfhydryl group, bromomethyl or other bromoalkyl groups, nitro group, amino group, methoxy carbonyl or other alkoxy carbonyl groups, cyano group, or acyl groups. Other compounds of Formulas I-H, I-HP, I-C, I-CP, I-HPC useful as intermediates include those in which one or more of $R_6$ , $R_7$ , $R_{11}$ , and $R_{12}$ substituents in Formula VII is a bromo group, hydroxyl group, sulfhydryl group, bromomethyl or other bromoalkyl groups, nitro group, amino group, methoxy carbonyl or other alkoxy carbonyl groups, cyano group, or acyl groups.

A 3-bromo substituent at the $R_7$ position in Formula I-CP ("Polycyclic 3-Bromophenyl (R)-Chiral Halogenated

1-Substitutedamino-2-Propanols") compounds can be reacted with a phenol to afford, as described in **Examples,** 3-phenoxy compounds of the present invention of Formula I-CP ("Polycyclic 3-Phenoxyphenyl (R)-Chiral Halogenated 1-Substitutedamino-2-Propanols").

A 3-bromo substituent at the $R_7$ position in Formula I-HP and I-HPC ("Polycyclic 3-Bromophenyl amd 3-Bromoheteroaryl/Aryl-Heteroaryl (R)-Chiral Halogenated 1-Substitutedamino-2-Propanols") can, as shown in Scheme 4, be reacted with a phenol to afford, as described in **Examples**, additional compounds of Formula I-HP and I-HPC ("Polycyclic 3-Aryloxyaryl, 3-Heteroaryloxyaryl, 3-Heteroaryloxyheteroaryl, 3-Aryloxyheteroaryl , 3-Arylthioaryl, 3-Heteroarylthioaryl, 3-Heteroarylthioheteroaryl, and 3-Arylthioheteroaryl Aryl amd Heteroaryl/Aryl-Heteroaryl (R)-Chiral Halogenated 1-Substitutedamino-2-Propanols").

A 3-bromo substituent at the $R_7$ position in Formula I-CP ("Polycyclic 3-Bromophenyl (R)-Chiral Halogenated 1-Substitutedamino-2-Propanols") can be reacted, as shown in Scheme 7, with an aryl borinate or an aryl tin to afford, as described in **Examples,** additional compounds of Formula I-CP ("Polycyclic 3-Arylphenyl (R)-Chiral Halogenated 1-Substitutedamino-2-Propanols").

Scheme 8 discloses the conversion of a 3-bromo substituent at the $R_7$ position in Formula I-CP ("Polycyclic 3-Bromophenyl (R)-Chiral Halogenated 1-Substitutedamino-2-Propanols") compounds by reaction with a primary or secondary amine to afford, as described in **Examples,** additional compounds of Formula I-CP ("Polycyclic 3-$R_{22}$aminophenyl (R)-Chiral Halogenated 1-Substitutedamino-2-Propanols").

Conversion of a 3-bromo substituent at the $R_{10}$ position in Formula I-CP ("Polycyclic 3-Bromophenyl (R)-Chiral Halogenated 1-Substitutedamino-2-Propanols") compounds by reaction with an aryl borinate can afford, as described in **Examples,** additional compounds of Formula I-CP ("Polycyclic 3-Arylphenyl (R)-Chiral Halogenated 1-Substitutedamino-2-Propanols").

Conversion of a 3-bromo substituent at the $R_{10}$ position in Formula 1-CP ("Polycyclic 3-Bromophenyl (R) -Chiral Halogenated 1-Substitutedamino-2-Propanols") compounds by reaction with a heteroaryl dibutyl tin compound can afford, as described in **Examples,** additional compounds of the present invention of Formula I-CP ("Polycyclic 3-Heteroarylphenyl (R)-Chiral Halogenated 1-Substitutedamino-2-Propanols").

Conversion of a 3-bromomethyl substituent at the $R_7$ position in Formula I-CP ("Polycyclic 3-Bromomethylphenyl (R)-Chiral Halogenated 1-Substitutedamino-2-Propanols") by reaction with an aryl borinate can afford, as described in **Examples**, additional compounds of Formula I-CP ("Polycyclic 3-Arylmethylphenyl (R)-Chiral Halogenated 1-Substitutedamino-2-Propanols").

Scheme 5 discloses the conversion of a 3-hydroxyl substituent at the $R_7$ position in Formula I-HP and I-HPC ("Polycyclic 3-Hydroxyphenyl amd 3-Hydroxyheteroaryl/Aryl-Heteroaryl (R)-Chiral Halogenated 1-Substitutedamino-2-Propanols") by reaction with an aryl bromide or heteroaryl bromide to afford, as described in **Examples,** additional compounds of Formula I-HP and I-HPC ("Polycyclic 3-Aryloxyaryl, 3-Heteroaryloxyaryl, 3-Heteroaryloxyheteroaryl, and 3-Aryloxyheteroaryl Aryl-Heteroaryl (R)-Chiral Halogenated 1-Substitutedamino-2-Propanols").

Conversion of a 3-hydroxyl substituent at the $R_7$ position in Formula I-CP ("Polycyclic 3-Hyroxyphenyl (R) -Chiral Halogenated 1-Substitutedamino-2-Propanols") compounds by reaction with an aryl bromide can afford, as described in **Examples,** additional compounds of Formula I-CP ("Polycyclic 3-Phenoxyphenyl (R)-Chiral Halogenated 1-Substitutedamino-2-Propanols").

Conversion of a 3-hydroxyl substituent at the $R_7$ position in Formula I-HP and I-HPC ("Polycyclic 3-Hydroxyphenyl amd 3-Hydroxyheteroaryl/Aryl-Heteroaryl (R)-Chiral Halogenated 1-Substitutedamino-2-Propanols") compounds by reaction with an aralkyl bromide or heteroaralkyl bromide can afford, as described above for Scheme 5 and in **Examples**, additional compounds of Formula I-HP and I-HPC ("Polycyclic 3-Aralkyloxyaryl, 3-Heteroaralkyloxyaryl, 3-Heteroaralkyloxyheteroaryl, and 3-Aralkyloxyheteroaryl Aryl-Heteroaryl (R)-Chiral Halogenated 1-Substitutedamino-2-Propanols").

Conversion of a 3-hydroxyl substituent at the $R_7$ position in Formula I-CP ("Polycyclic 3-Hyroxyphenyl (R) -Chiral Halogenated 1-Substitutedamino-2-Propanols") compounds by reaction with an aralkyl bromide can afford, as described in **Examples**, additional compounds of Formula I-CP ("Polycyclic 3-Aralkyloxyaryl (R)-Chiral Halogenated 1-Substitutedamino-2-Propanols").

Conversion of a 3-hydroxyl substituent at the $R_7$ position in Formula I-CP ("Polycyclic 3-Hyroxyphenyl (R) -Chiral Halogenated 1-Substitutedamino-2-Propanols") compounds by reaction with an $R_{17}$-bromide can afford, as described in **Examples**, additional compounds of Formula I-CP ("Polycyclic 3- $R_{17}$-oxyaryl (R)-Chiral Halogenated 1-Substitutedamino-2-Propanols").

Conversion of a 3-thio substituent at the $R_7$ position in Formula I-CP ("Polycyclic 3-thiophenyl (R)-Chiral Halogenated 1-Substitutedamino-2-Propanols") compounds by reaction with an $R_{17}$-bromide can afford, as described in **Examples**, additional compounds of Formula I-CP ("Polycyclic 3- $R_{17}$thiaaryl (R)-Chiral Halogenated 1-Substitutedamino-2-Propanols"). "Polycyclic 3- $R_{17}$thiaaryl (R)-Chiral Halogenated 1-Substitutedamino-2-Propanols" can be oxidized to sulfonyl compounds of Formula I-CP ("Polycyclic 3- R $_{17}$sulfonylphenyl (R)-Chiral Halogenated 1-Substitutedamino-2-Propanols").

Conversion of a 3-nitro substituent at the $R_7$ position in Formula I-CP ("Polycyclic 3-Nitrophenyl (R)-Chiral Halogenated 1-Substitutedamino-2-Propanols") compounds by hydrogenation can afford, as described in **Examples**. additional compounds of Formula I-CP ("Polycyclic 3-Aminophenyl (R)-Chiral Halogenated 1-Substitutedamino-2-Propanols"). "Polycyclic 3-Aminophenyl (R)-Chiral Halogenated 1-Substitutedamino-2-Propanols" can be acylated to acyl amide compounds of Formula I-CP ("Polycyclic 3-$R_{17}$-C(O)amidophenyl (R)-Chiral Halogenated 1-Substitutedamino-2-Propanols").

Conversion of a 3-amino substituent at the $R_7$ position in Formula I-CP ("Polycyclic 3-Aminophenyl (R)-Chiral Halogenated 1-Substitutedamino-2-Propanols") compounds by reaction with carbonyl compounds can afford, as described in **Examples**, additional compounds of Formula I-CP ("Polycyclic 3-(Saturated Nitrogen Heterocycl-1yl)aryl (R)-Chiral Halogenated I-Substitutedamino-2-Propanols" and ("Polycyclic 3-(Unsaturated Nitrogen Heterocycl-1yl)aryl (R)-Chiral Halogenated 1-Substitutedamino-2-Propanols").

Conversion of a 3-methoxycarbonyl substituent at the $R_7$ position in Formula I-CP ("Polycyclic 3-Carbomethoxyphenyl (R)-Chiral Halogenated 1-Substitutedamino-2-Propanols") compounds by reaction with amination reagents can afford, as described in **Examples**. additional compounds of Formula I-CP ("Polycyclic 3-Carboxamidophenyl (R) -Chiral Halogenated 1-Substitutedamino-2-Propanols").

Conversion of a 3-cyano substituent at the $R_7$ position in Formula I-CP ("Polycyclic 3-Cyanophenyl (R)-Chiral Halogenated 1-Substitutedamino-2-Propanols") compounds by reaction with organometallic reagents can afford, as described in **Examples**, additional compounds of Formula I-CP ("Polycyclic 3-Acylphenyl (R)-Chiral Halogenated 1-Substitutedamino-2-Propanols"). Said "Polycyclic 3-Acylphenyl (R)-Chiral Halogenated 1-Substitutedamino-2-Propanols" can be reduced to hydroxyl compounds of Formula I-CP ("Polycyclic 3-hydroxysubstitutedmethylphenyl (R) -Chiral Halogenated 1-Substitutedamino-2-Propanols").

Conversion of a 3-methoxycarbonyl substituent at the $R_{10}$ position in Formula I-CP ("Polycyclic 3-Carbomethoxyphenyl (R)-Chiral Halogenated 1-Substitutedamino-2-Propanols") compounds by reaction with amination reagents can afford, as described in **Examples**, additional compounds of Formula I-CP "Polycyclic 3-Carboxamdophenyl (R) -Chiral Halogenated 1-Substitutedamino-2-Propanols").

Conversion of a 3-methoxycarbonyl substituent at the $R_{10}$ position in Formula I-CP ("Polycyclic 3-Carbomethoxyphenyl (R)-Chiral Halogenated 1-Substitutedamino-2-Propanols") compounds by reaction with an organometallic reagent can afford, as described in **Examples**, additional compounds of Formula I-CP "Polycyclic 3-(bis- $R_{20}$-hydroxymethyl)aryl (R)-Chiral Halogenated 1-Substitutedamino-2-Propanols").

Conversion of a 3-methoxycarbonyl substituent at the $R_{10}$ position in Formula I-CP ("Polycyclic 3-Carbomethoxyphenyl (R)-Chiral Halogenated 1-Substitutedamino-2-Propanols") compounds by reaction with lithium aluminum hydride can afford, as described in **Examples**. additional compounds of Formula I-CP ("Polycyclic 3-Hydroxymethylphenyl (R)-Chiral Halogenated 1-Substitutedamino-2-Propanols").

Conversion of a 3-methoxycarbonyl substituent at the $R_{10}$ position in Formula I-CP ("Polycyclic 3-Carbomethoxyphenyl (R)-Chiral Halogenated 1-Substitutedamino-2-Propanols") compounds by reaction with an alkylation reagent can afford, as described in **Examples**, additional compounds of Formula I-CP ("Polycyclic 3-(bis- $R_{21}$-hydroxymethyl)phenyl (R)-Chiral Halogenated 1-Substitutedamino-2-Propanols").

Conversion of a 3-methoxycarbonyl substituent at the $R_{10}$ position in Formula I-CP ("Polycyclic 3-Carbomethoxyphenyl (R)-Chiral Halogenated 1-Substitutedamino-2-Propanols") compounds by reaction intially with an amidation reagent and then an $R_{20}$-organometallic reagent can afford, as described in **Examples**, additional compounds of Formula I-CP ("Polycyclic 3-( $R_{20}$-carbonyl)phenyl (R)-Chiral Halogenated 1-Substitutedamino-2-Propanols").

Formula I-H ("Generic Polycyclic Aryl and Heteroaryl (R)-Chiral Halogenated 1-Substitutedamino-(n+1)-alkanols"), Formula I-HP ("Generic Polycyclic Aryl and Heteroaryl (R)-Chiral Halogenated 1-Substitutedamino-2-propanols"), Formula I-HPC ("Polycyclic Aryl-Heteroaryl (R)-Chiral Halogenated 1-Substitutedamino-2-Propanols"). Formula I-C ("Polycyclic Phenyl (R)-Chiral Halogenated 1-Substitutedamino-(n+1)-Alkanols"), and Formula I-CP ("Polycyclic Phenyl (R)-Chiral Halogenated 1-Substitutedamino-2-Propanols") and other compounds posssessing hydroxyl, thiol, and amine functional groups can be converted to a wide variety derivatives. The hydroxyl group, wherein $R_{16}$ is a hydrogen and X is oxy, of compounds of Formulas I-H, I-HP, I-HPC, I-C, and I-CP can be readily converted to esters of carboxylic, sulfonic, carbamic, phosphonic, and phosphoric acids. Acylation to form a carboxylic acid ester is readily effected using a suitable acylating reagent such as an aliphatic acid anhydride or acid chloride. The corresponding aryl and heteroaryl acid anhydrides and acid chlorides can also be used. Such reactions are generally carried out using an amine catalyst such as pyridine in an inert solvent. In like manner, compounds of Formulas I-H, I-HP, I-C, I-CP, I-HPC, Cyclo I-H, Cyclo I-C, and Cyclo I-CP that have at least one hydroxyl group present in the form of an alcohol or phenol can be acylated to its corresponding esters. Similarly, carbamic acid esters (urethans) can be obtained by reacting any hydroxyl group with isocyanates and carbamoyl chlorides. Sulfonate, phosphonate, and phosphate esters can be prepared using the corresponding acid chloride and similar reagents- Compounds of Formulas I-H, I-HP, I-C, I-CP, I-HPC, Cyclo I-H, Cyclo I-C, and Cyclo I-CP that have at least one thiol group present can be converted to the corresponding thioesters derivatives analogous to those of alcohols and phenols using the same reagents and comparable reaction conditions. Compounds of Formulas I-H, I-HP, I-C, I-CP, I-HPC, Cyclo I-H, Cyclo I-C, and Cyclo I-CP that have at least one primary or secondary amine group present can be converted to the corresponding amide derivatives. Amides of carboxylic acids can be prepared using the appropriate acid chloride or anhydrides with reaction conditions analogous to those used with alcohols and phenols. Ureas of the corresponding primary or secondary amine can be prepared using isocyanates directly and carbamoyl chlorides in the presence of an acid scavenger such as triethylamine or pyridine. Sulfonamides can be prepared from the corresponding sulfonyl chloride in the presence of aqueous sodium hydroxide. Suitable procedures and methods for preparing these derivatives can be found in House's Modem Synthetic Reactions, W. A. Benjamin, Inc., Shriner, Fuson, and Curtin in The Systematic Indentification of Organic Compounds, 5th Edition, John Wiley & Sons, and Fieser and Fieser in Reagents for Organic Synthesis, Volume 1, John Wiley & Sons. Reagents of a wide variety that can be used to derivatize hydroxyl, thiol, and amines of compounds of Formulas I-H, I-HP, I-C, I-CP, I-HPC, Cyclo I-H, Cyclo I-C, and Cyclo I-CP are available from commerical sources or the references cited above, which are incorporated herein by reference.

Formula I-H ("Generic Polycyclic Aryl and Heteroaryl (R)-Chiral Halogenated 1-Substitutedamino-(n+1)-alkanols"), Formula I-HP ("Generic Polycyclic Aryl and Heteroaryl (R)-Chiral Halogenated 1-Substitutedamino-2-propanols"), Formula I-HPC ("Polycyclic Aryl-Heteroaryl (R)-Chiral Halogenated 1-Substitutedamino-2-Propanols"), Formula I-C ("Polycyclic Phenyl (R)-Chiral Halogenated 1-Substitutedamino-(n+1)-Alkanols"), and Formula I-CP ("Polycyclic Phenyl (R)-Chiral Halogenated 1-Substitutedamino-2-Propanols") and other compounds posssessing hydroxyl, thiol, and amine functional groups can be alkylated to a wide variety derivatives. The hydroxyl group, wherein $R_{16}$ is a hydrogen and X is oxy, of compounds of Formulas I-H, I-HP, I-C, I-CP, I-HPC, Cyclo I-H, Cyclo I-C, and Cyclo I-CP can be readily converted to ethers. Alkylation to form an ether is readily effected using a suitable alkylating reagent such as an alkyl bromide, alkyl iodide or alkyl sulfonate. The corresponding aralkyl, heteroaralkyl, alkoxyalkyl, aralkyloxyalkyl, and heteroaralkyloxyalkyl bromides, iodides, and sulfonates can also be used. Such reactions are generally carried out using an alkoxide forming reagent such as sodium hydride, potassium t-butoxide, sodium amide, lithium amide, and n-butyl lithium using an inert polar solvent such as DMF, DMSO, THF, and similar, comparable solvents. In like manner, compounds of Formulas I-H, I-HP, I-C, I-CP, I-HPC, Cyclo I-H, Cyclo I-C, and Cyclo I-CP that have at least one hydroxyl group present in the form of an alcohol or phenol can be alkylated to their corresponding ethers. Compounds of Formulas I-H, I-HP, I-C, I-CP, I-HPC, Cyclo I-H, Cyclo I-C, and Cyclo I-CP that have at least one thiol group present can be converted to the corresponding thioether derivatives analogous to those of alcohols and phenols using the same reagents and comparable reaction conditions. Compounds of Formulas I-H, I-HP, I-C, I-CP, I-HPC, Cyclo I-H, Cyclo I-C, and Cyclo I-CP that have at least one primary, secondary or tertiary amine group present can be converted to the corresponding quaternary ammonium derivatives. Quaternary ammonium derivatives can be prepared using the appropriate bromides, iodides, and sulfonates analogous to those used with alcohols and phenols. Conditions involve reaction of the amine by warming it with the alkylating reagent with a stoichiometric amount of the amine (i.e., one equivalent with a tertiary amine, two with a secondary, and three with a primary). With primary and secondary amines, two and one equivalents, respectively, of an acid scavenger are used concurrently. Tertiary amines can be preparedfrom the corresponding primary or secondary amine by reductive alkylation with aldehydes and ketones using reduction methods 1, 2, or 3 as shown in Scheme 1. Suitable procedures and methods for preparing these derivatives can be found in House's Modem Synthetic Reactions, W. A. Benjamin, Inc., Shriner. Fuson. and Curtin in The Systematic Indentification of Organic Compounds, 5th Edition, John Wiley & Sons, and Fieser and Fieser in Reagents for Organic Synthesis, Volume 1, John Wiley & Sons. Perfluoroalkyl derivatives can be prepared as described by DesMarteau in J. Chem. Soc. Chem. Commun. 2241 (1998). Reagents of a wide variety that can be used to derivatize hydroxyl, thiol, and amines of compounds of Formulas I-H, I-HP, I-C, I-CP, I-HPC, Cyclo I-H, Cyclo I-C, and Cyclo I-CP are available from commerical sources or the references cited above, which are incorporated herein by reference.

Formula I-H ("Generic Polycyclic Aryl and Heteroaryl (R)-Chiral Halogenated 1-Substitutedamino-(n+1)-alkanols"), Formula I-HP ("Generic Polycyclic Aryl and Heteroaryl (R)-Chiral Halogenated I-Substitutedamino-2-propanols"), Formula I-HPC ("Polycyclic Aryl-Heteroaryl (R)-Chiral Halogenated 1-Substitutedamino-2-Propanols"), Formula I-C ("Polycyclic Phenyl (R)-Chiral Halogenated 1-Substitutedamino-(n+1)-Alkanols"), and Formula I-CP ("Polycyclic Phenyl (R)-Chiral Halogenated 1-Substitutedamino-2-Propanols") and certain other compounds of this invention can be convened, according to Scheme 6, to the corresponding cyclic derivatives represented by "Tricyclic *tertiary*-oxyalkylamines" and exemplified by Formulas Cyclo I-H ("Polycyclic Aryl and Heteroaryl (R)-Chiral Halogenated (N+1) -Cycloazaalkoxy"), Cyclo I-C ("Polycyclic Aryl Phenyl (R)-Chiral Halogenated (N+1)-Cycloazaalkoxy") and Cyclo I-CP ("Polycyclic Phenyl Phenyl (R)-Chiral Halogenated Cycloazaalkoxy"). The hydroxyl group, wherein $R_{16}$ is a hydrogen and X is oxy, of compounds of Formulas I-H, I-HP, I-C, I-CP, and I-HPC can be cyclized to corresponding cyclic ethers. Compounds suitable for cyclization will normally have at least one leaving group within 5 to 10 continuous atoms of the hydroxyl group wherein $R_{16}$ is a hydrogen and X is oxy. Most preferrably the leaving group will be within 5 to 7 atoms of the hydroxyl group so as to form a 6 to 8 membered ring heteroatom containing ring. When the leaving group is part of an aromatic ring system, the leaving group will be preferrably in an ortho position. Suitable leaving groups generally include halides, sulfates, sulfonates, trisubsituted amino, disubstituted sulfonium, diazonium, and like, and.

in the case of aromatic systems, also includes nitro, alkoxy, aryloxy, heteroaryloxy, and alkylthio.

The cyclization reaction to form "Tricyclic *tertiary*-oxyalkylamines" of Formulas Cyclo I-H, Cyclo I-C and Cyclo I-CP can be accomplished by aromatic and aliphatic nucleophilic substitution reactions such as those disclosed in March's Advanced Organic Chemistry, 4th Edition, John Wiley & Sons, especially at pages 293-412 and 649-658 and the references cited therein, which are incorporated herein by reference. Hydroxyl containing suitably substituted compounds can be converted to a cyclic analog by heating a suitably substituted compound under anhydrous conditions in a suitable solvent, such as dimethylformamide, dimethylsulfoxide, N-methylpyrrolidone, tetraglyme, or hexamethylphosphoramide, in the presence of a suitable base such as potassium carbonate, cesium carbonate, sodium hydroxide, potassium *tertiary*-butoxide, or lithium diisopropylamide. Alternately, sodium amide in anhydrous ammonia solvent can be used. Temperatures in the range of -20 °C to 200 °C can be used for time periods of 30 minutes to more than 24 hours. The preferred temperature can be selected by standard synthetic chemical technique balancing maximum yield, maximum purity, cost, ease of isolation and operation, and time required. Isolation of the "Tricyclic *tertiary*-oxyalkylamines" can be effected as described above for other tertiary-oxyalkylamines. Representative "Tricyclic *tertiary*-oxyalkylamines" prepared using the methodology described above are included in Table 5.

The following examples are provided to illustrate the present invention and are not intended to limit the scope thereof. Those skilled in the art will readily understand that known variations of the conditions and processes of the following preparative procedures can be used to prepare these compounds.

Table 5. Structure of Substituted Tricyclic*tertiary*-2-oxyalkylamines.

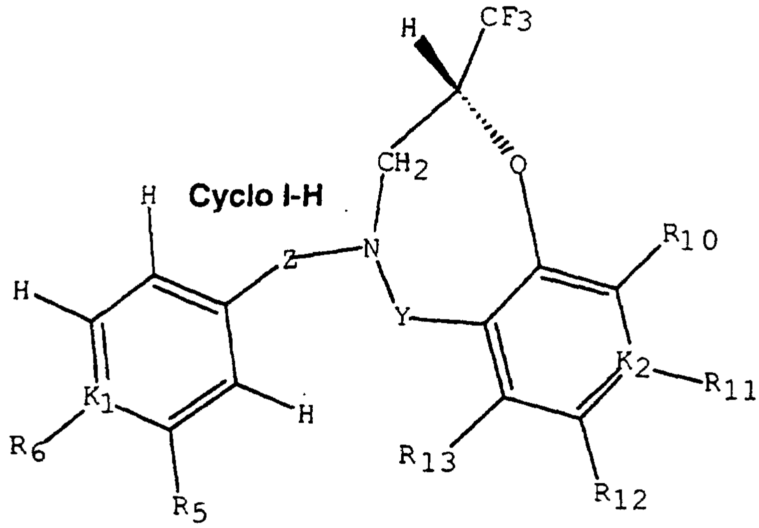

Cyclo I-H

| Y | Z | $R_5$ | $K_1$-$R_6$ | $R_{10}$ | $K_2$-$R_{11}$ | $R_{12}$ | $R_{13}$ |
|---|---|---|---|---|---|---|---|
| | | | | | | | |
| $CH_2$ | - | 4-chloro-3-ethylphenoxy | C-H | H | C- $CF_3$ | H | H |
| $CH_2$ | - | 4-chloro-3-ethylphenoxy | N | H | C- $CF_3$ | H | H |
| $CH_2$ | - | 4-chloro-3-ethylphenoxy | C-H | H | C- H | $CF_3$ | H |
| $CH_2$ | - | 4-chloro-3-ethylphenoxy | N | H | C- H | $CF_3$ | H |
| $CH_2$ | - | 4-chloro-3-ethylphenoxy | C-H | H | N | $CF_3$ | H |
| - | - | 4-chloro-3-ethylphenoxy | C-H | H | C- $CF_3$ | H | H |
| - | - | 4-chloro-3-ethylphenoxy | N | H | C- $CF_3$ | H | H |
| - | - | 4-chloro-3-ethylphenoxy | C-H | H | C- H | $CF_3$ | H |
| - | - | 4-chloro-3-ethylphenoxy | N | H | C- H | $CF_3$ | H |
| - | - | 4-chloro-3-ethylphenoxy | C-H | H | N | $CF_3$ | H |

Table 5. (cont.) Structure of Substituted Tricyclic *tertiary*-2-oxyalkylamines.

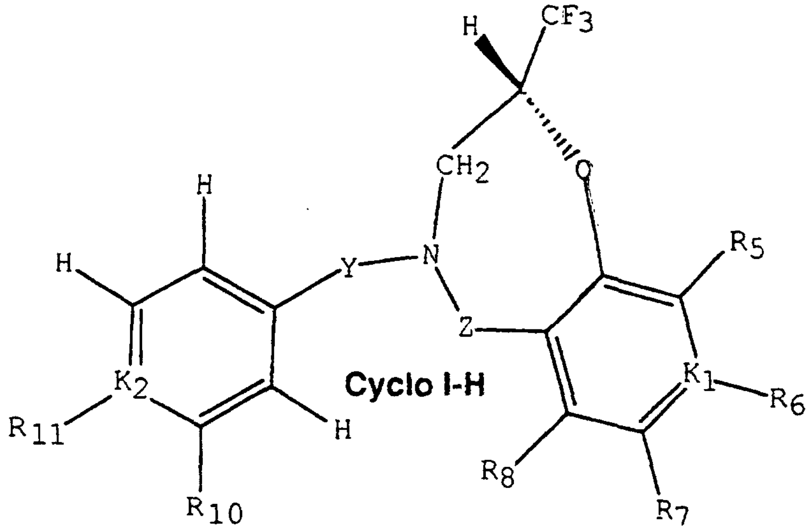

| Y | Z | $R_5$ | $K_1$-$R_6$ | $R_{10}$ | $K_2$-$R_{11}$ | $R_7$ | $R_8$ |
|---|---|---|---|---|---|---|---|
| | | | | | | | |
| $CH_2$ | - | 4-chloro-3-ethylphenoxy | C-H | $OCF_2CF_2H$ | C-H | H | H |
| $CH_2$ | - | 4-chloro-3-ethylphenoxy | N | $OCF_2CF_2H$ | C-H | H | H |
| $CH_2$ | - | 4-chloro-3-ethylphenoxy | C-H | $OCF_2CF_2H$ | N | H | H |
| $CH_2$ | - | phenoxy | C-H | $OCF_2CF_2H$ | C-H | H | H |
| $CH_2$ | - | phenoxy | N | $OCF_2CF_2H$ | C-H | H | H |
| $CH_2$ | - | phenoxy | C-H | $OCF_2CF_2H$ | N | H | H |
| $CH_2$ | - | 4-chloro-3-ethylphenoxy | C-H | $CF_2CF_3$ | C-H | H | H |
| $CH_2$ | - | 4-chloro-3-ethylphenoxy | N | $CF_2CF_3$ | C-H | H | H |
| $CH_2$ | - | 4-chloro-3-ethylphenoxy | C-H | $CF_2CF_3$ | N | H | H |
| $CH_2$ | - | phenoxy | C-H | $CF_2CF_3$ | C-H | H | H |
| $CH_2$ | - | phenoxy | N | $CF_2CF_3$ | C-H | H | H |
| $CH_2$ | - | phenoxy | C-H | $CF_2CF_3$ | N | H | H |

Table 5. (cont.) Structure of Substituted Tricyclic *tertiary*-2-oxyalkylamines.

| Y | Z | $R_5$ | $K_1$-$R_6$ | $R_{10}$ | $K_2$-$R_{11}$ | $R_7$ | $R_8$ |
|---|---|---|---|---|---|---|---|
| $CH_2$ | - | 4-chloro-3-ethylphenoxy | C-H | $CF_3$ | C- H | H | H |
| $CH_2$ | - | 4-chloro-3-ethylphenoxy | N | $CF_3$ | C- H | H | H |
| $CH_2$ | - | 4-chloro-3-ethylphenoxy | C-H | $CF_3$ | N | H | H |
| $CH_2$ | - | phenoxy | C-H | $CF_3$ | C- H | H | H |
| $CH_2$ | - | phenoxy | N | $CF_3$ | C- H | H | H |
| $CH_2$ | - | phenoxy | C-H | $CF_3$ | N | H | H |
| $CH_2$ | - | 4-chloro-3-ethylphenoxy | C-H | $OCF_2CF_2H$ | C- H | H | F |
| $CH_2$ | - | 4-chloro-3-ethylphenoxy | N | $OCF_2CF_2H$ | C- H | H | F |
| $CH_2$ | - | 4-chloro-3-ethylphenoxy | C-H | $OCF_2CF_2H$ | N | H | F |
| $CH_2$ | - | 4-chloro-3-ethylphenoxy | C-H | 2-furyl | C- H | H | H |
| $CH_2$ | - | 4-chloro-3-ethylphenoxy | N | 2-furyl | C- H | H | H |
| $CH_2$ | - | 4-chloro-3-ethylphenoxy | C-H | 2-furyl | N | H | H |
| $CH_2$ | - | 4-chloro-3-ethylphenoxy | C-H | $SCF_3$ | C- H | H | H |
| $CH_2$ | - | 4-chloro-3-ethylphenoxy | N | $SCF_3$ | C- H | H | H |
| $CH_2$ | - | 4-chloro-3-ethylphenoxy | C-H | $SCF_3$ | N | H | H |

# Scheme 1

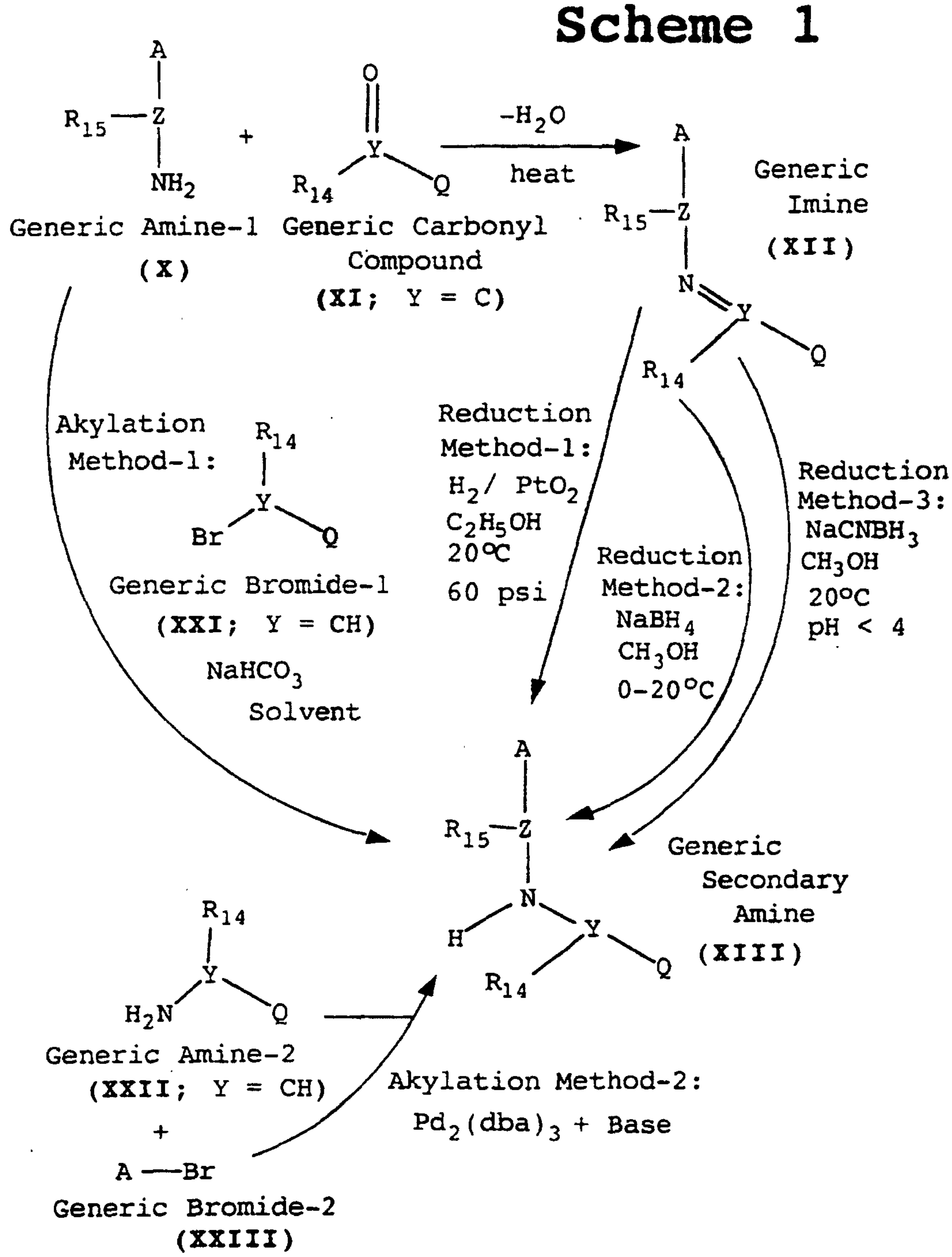
Generic Amine-1
(X)
Generic Carbonyl Compound
(XI; Y = C)
$-H_2O$
heat
Generic Imine
(XII)
Akylation Method-1:
Generic Bromide-1
(XXI; Y = CH)
$NaHCO_3$
Solvent
Reduction Method-1:
$H_2$/ $PtO_2$
$C_2H_5OH$
20°C
60 psi
Reduction Method-2:
$NaBH_4$
$CH_3OH$
0-20°C
Reduction Method-3:
$NaCNBH_3$
$CH_3OH$
20°C
pH < 4
Generic Secondary Amine
(XIII)
Generic Amine-2
(XXII; Y = CH)
Generic Bromide-2
(XXIII)
Akylation Method-2:
$Pd_2(dba)_3$ + Base

# Scheme 2

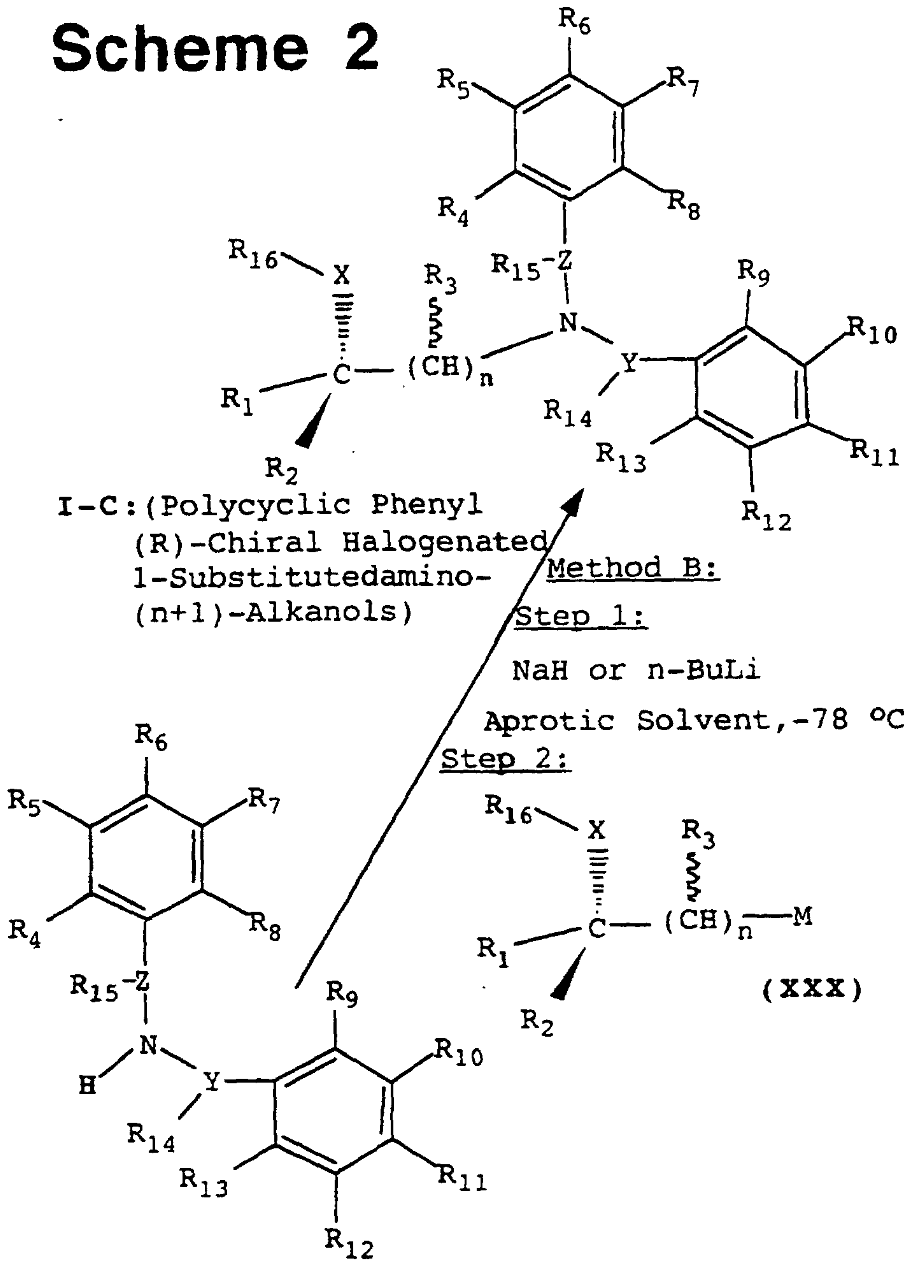


**I-C:** (Polycyclic Phenyl (R)-Chiral Halogenated 1-Substitutedamino-(n+1)-Alkanols)

**(XXX)**

Secondary Phenyl Amine

**(XIIIA)**

# Scheme 3

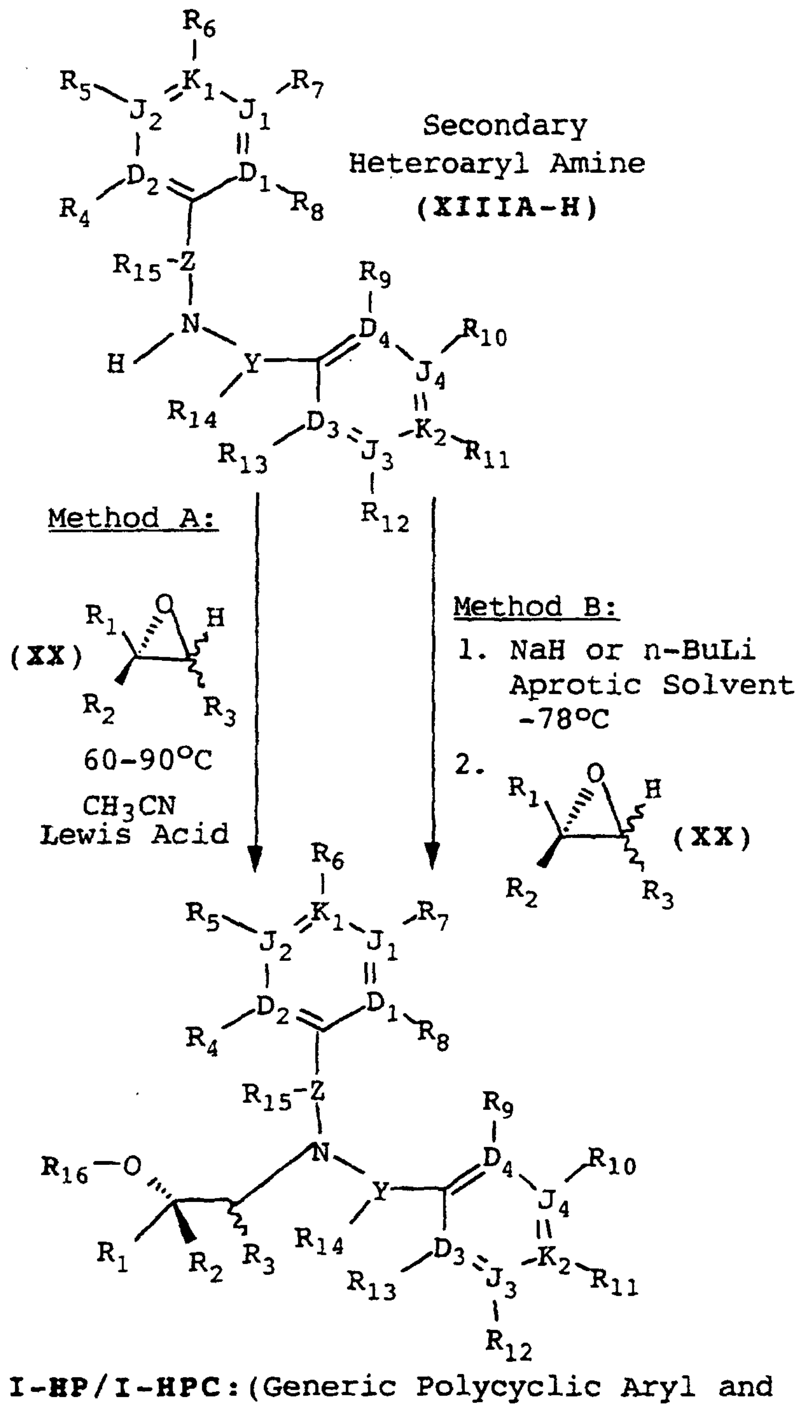


**I-HP/I-HPC:** (Generic Polycyclic Aryl and Heteroaryl/Aryl-Heteroaryl (R)-Chiral Halogenated 1-Substitutedamino-2-Propanols; $R_{16}$ = H)

**I-HP/I-HPC:** (Generic Polycyclic 3-Aryloxyaryl, 3-Heteroaryloxyaryl, 3-Heteroaryloxyheteroaryl, 3-Aryloxyheteroaryl, 3-Arylthioaryl, 3-Heteroarylthioaryl, 3-Heteroarylthioheteroaryl, 3-Arylthioheteroaryl Aryl and Heteroaryl/Aryl-Heteroaryl (R)-Chiral Halogenated 1-Substitutedamino-2-Propanols)

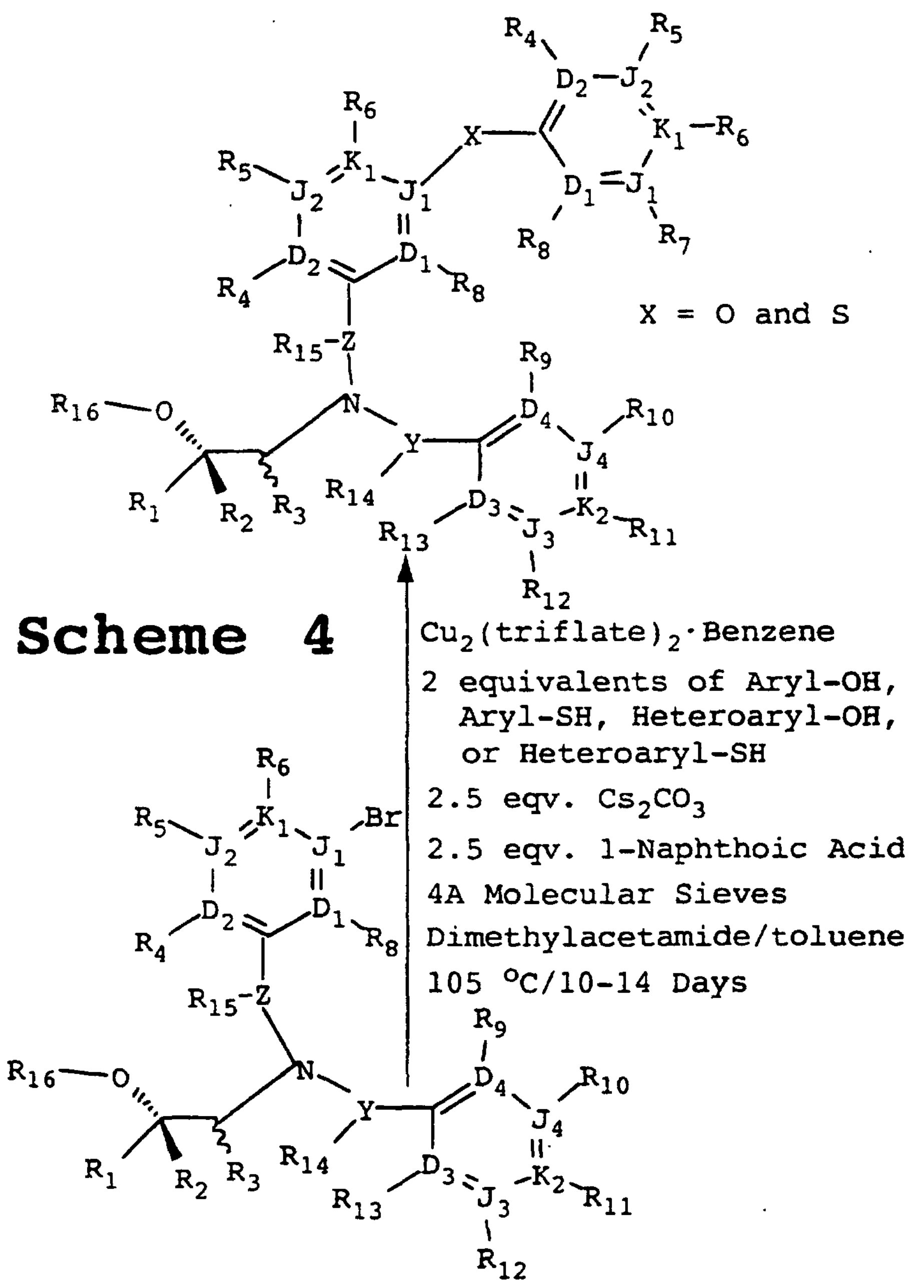


**Scheme 4**

**I-HP/I-HPC:** (Generic Polycyclic 3-Bromo Aryl and Heteroaryl/Aryl-Heteroaryl (R)-Chiral Halogenated 1-Substitutedamino-2-Propanols)

# Scheme 5

**I-HP/I-HPC:**(Generic Polycyclic 3-Aryloxyaryl, 3-Heteroaryloxyaryl3-Aryloxyheteroaryl or 3-Heteroaryloxyheteroaryl Aryl-Heteroaryl (R)-Chiral Halogenated 1-Substitutedamino-2-Propanols)

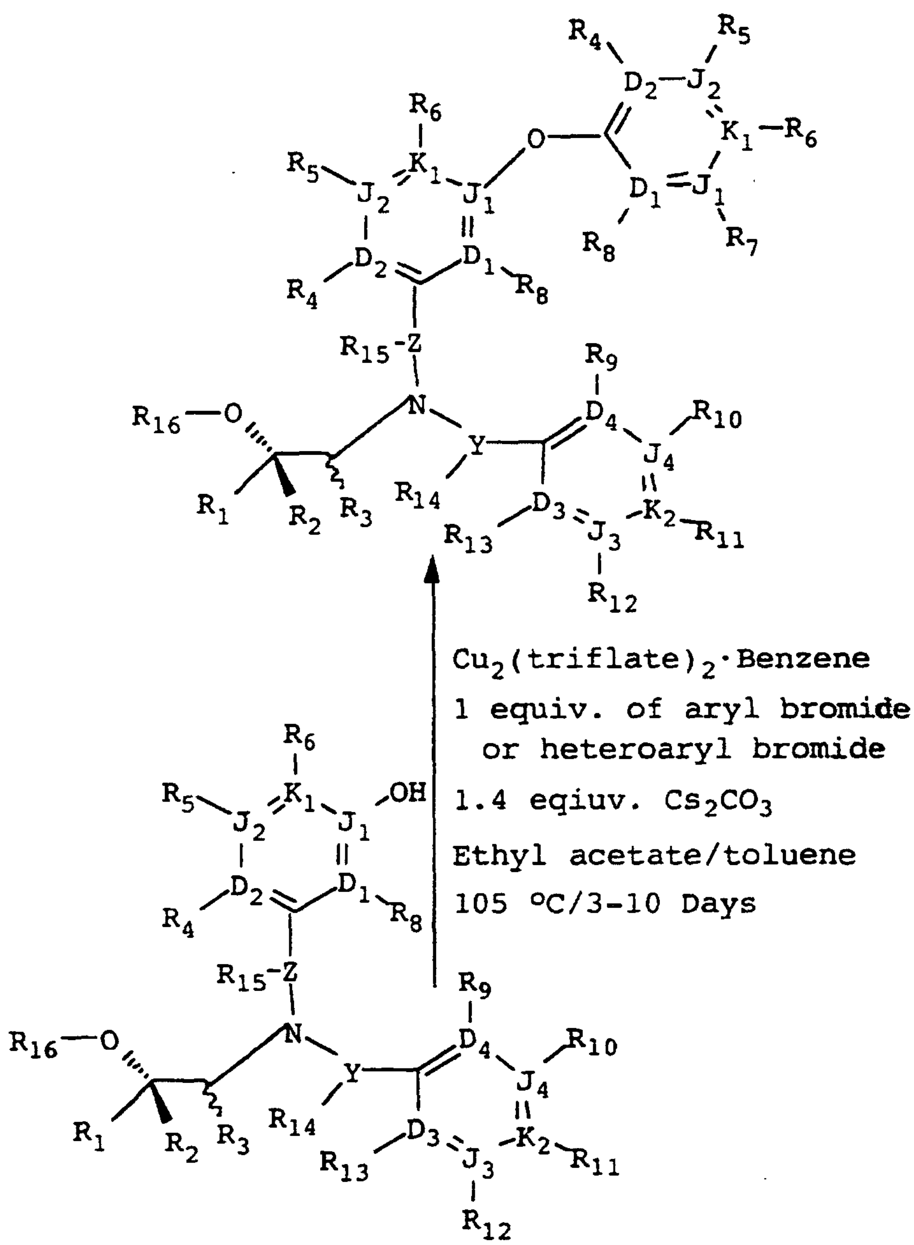


**I-HP/I-HPC:**(Generic Polycyclic 3-Hydroxy Aryl and Heteroaryl/Aryl-Heteroaryl (R)-Chiral Halogenated 1-Substitutedamino-2-Propanols)

# Scheme 6

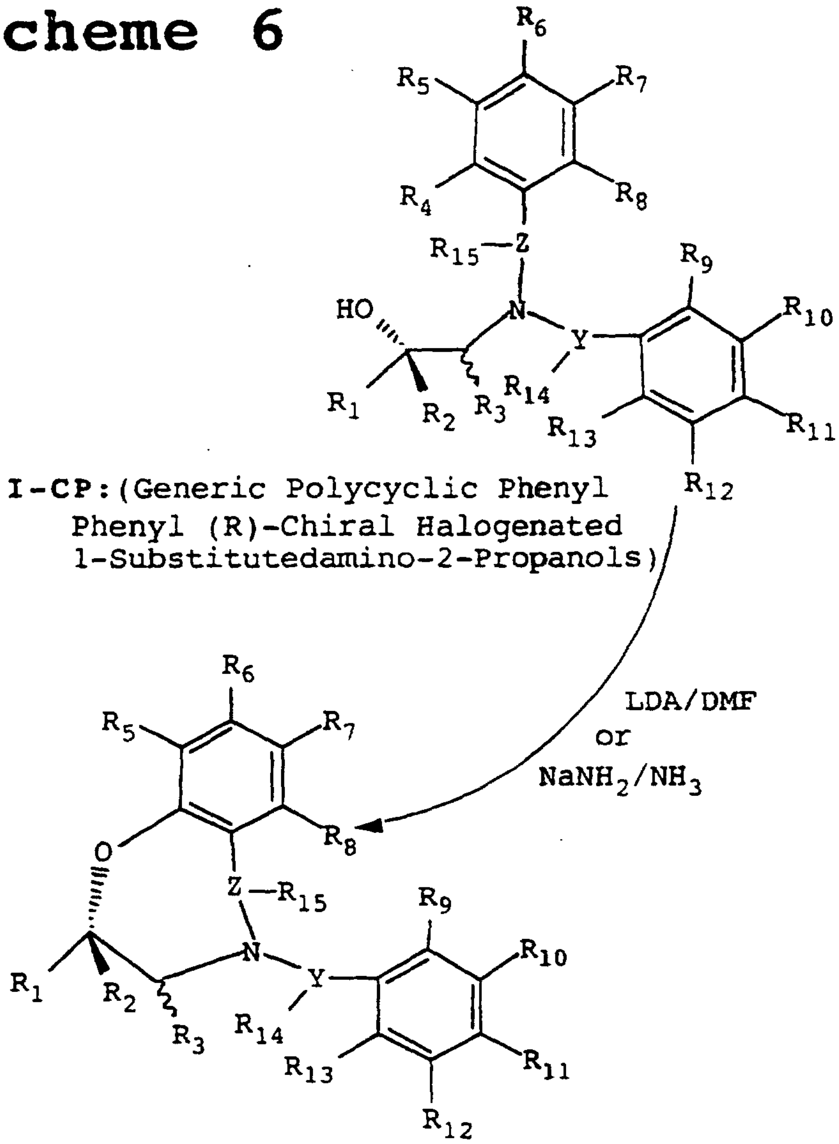


**I-CP:** (Generic Polycyclic Phenyl Phenyl (R)-Chiral Halogenated 1-Substitutedamino-2-Propanols)

**Cyclo I-CP:** (Polycyclic Phenyl Phenyl (R)-Chiral Halogenated Cycloazaalkoxy)

# Scheme 7

**I-CP**: (Polycyclic 3-Arylphenyl Phenyl (R)-Chiral Halogenated 1-Substitutedamino-2-Propanols)

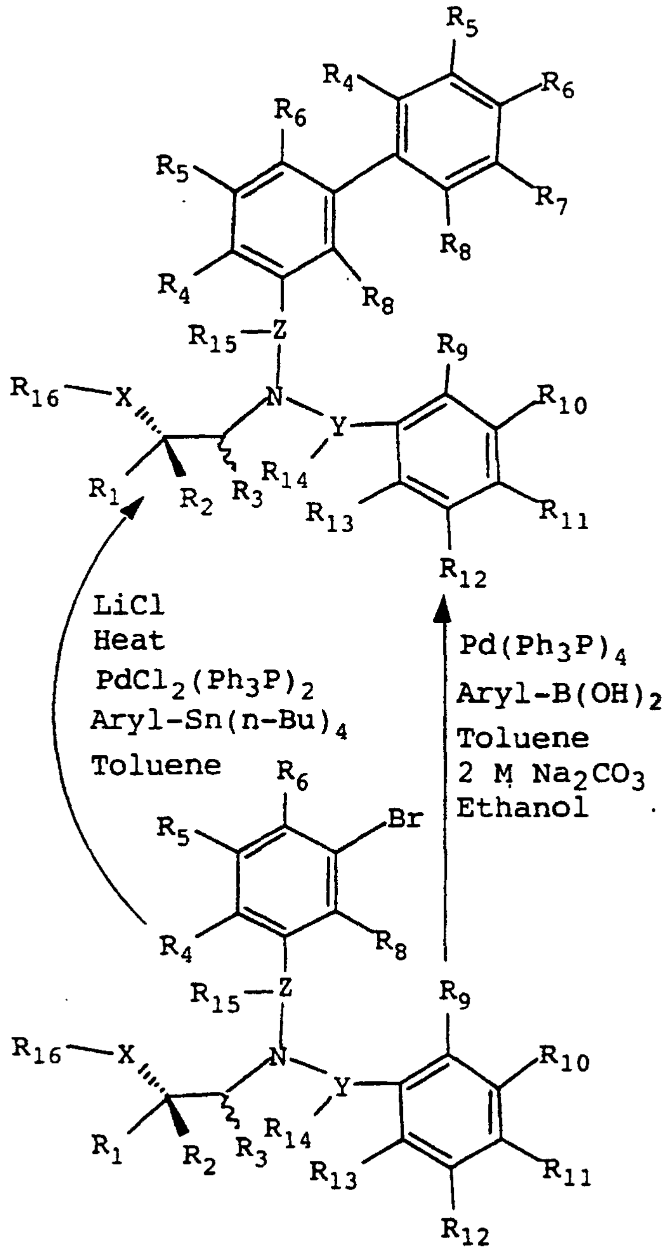


**I-CP**: (Polycyclic 3-Bromophenyl Phenyl (R)-Chiral Halogenated 1-Substitutedamino-2-Propanols)

<u>NOTE:</u> Use of Heteroaryl-B(OH)$_2$ will give the heteroarylmethyl analog of **I-CP**.

**I-CP:** (Polycyclic 3-$R_{22}$-aminophenyl Phenyl (R)-Chiral Halogenated 1-Substitutedamino-2-Propanols)

## Scheme 8

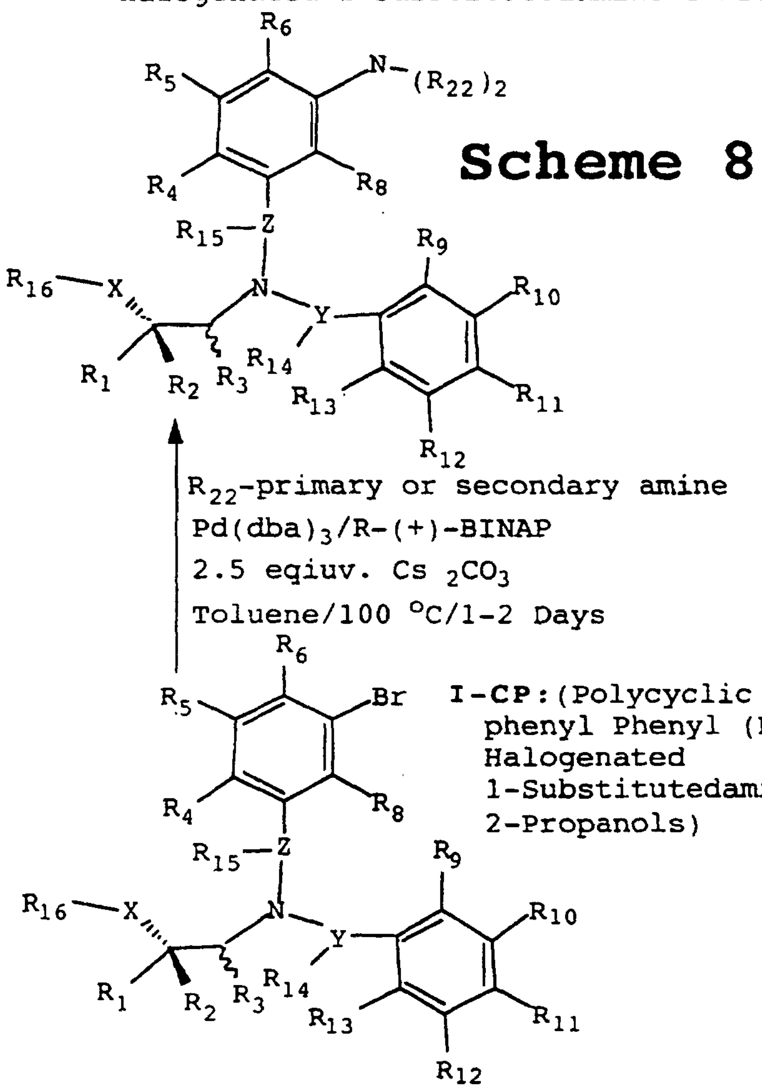


**I-CP:** (Polycyclic 3-Bromophenyl Phenyl (R)-Chiral Halogenated 1-Substitutedamino-2-Propanols)

$R_{22}$ is selected independently from any one or two of the following groups: hydrido, hydroxy, aryloxy, alkyl, alkenyl, alkynyl, aryl, aralkyl, alkoxyalkyl, aralkoxyalkyl, alkylsulfinylalkyl, haloalkyl, haloalkenyl, halocycloalkyl, halocycloalkoxy, halocycloalkoxyalkyl, arylsulfinylalkyl, arylsulfonylalkyl, alkylamino cycloalkylsulfinylalkyl, cycloalkylsufonylalkyl, heteroarylsulfonylalkyl, heteroarylsulfinylalkyl, aralkylsulfinylalkyl, aralkylsulfonylalkyl, hydroxyalkyl, amino, alkoxy, alkylsulfonylalkyl, aralkylthioalkyl, heteroaralkylthioalkyl, heteroaryloxyalkyl, alkenyloxyalkyl, haloalkoxy, haloalkoxyalkyl, haloalkenyloxyalkyl, heteroaryl, halocycloalkenyloxyalkyl, heteroarylalkyl, aryloxyalkyl, halocycloalkenyl, and heteroarylthioalkyl.

# Scheme 9

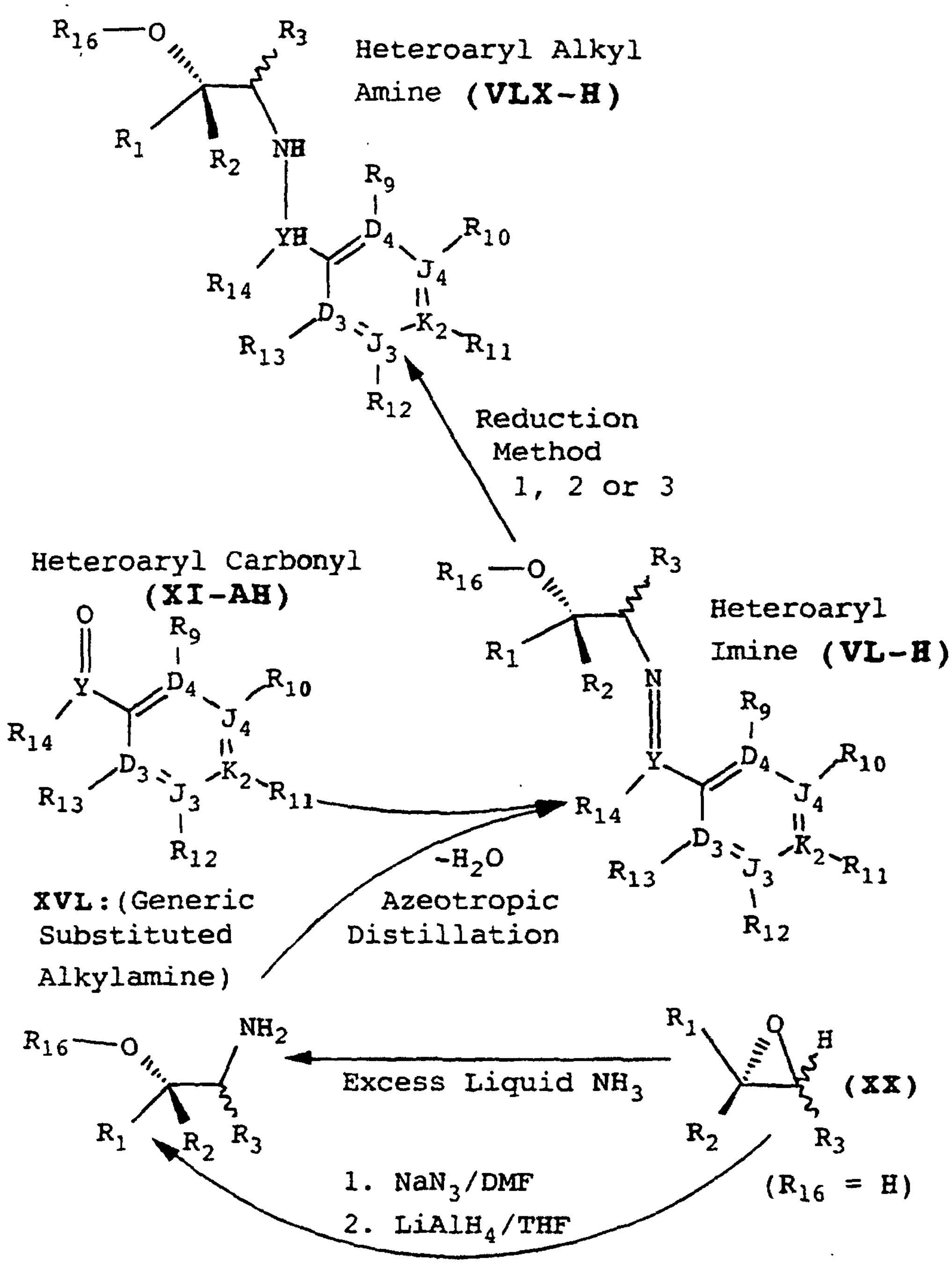
Heteroaryl Alkyl Amine (VLX-H)
Reduction Method 1, 2 or 3
Heteroaryl Carbonyl (XI-AH)
Heteroaryl Imine (VL-H)
-H2O
Azeotropic Distillation
XVL: (Generic Substituted Alkylamine)
Excess Liquid NH3
(XX)
(R16 = H)
1. NaN3/DMF
2. LiAlH4/THF

# Scheme 10

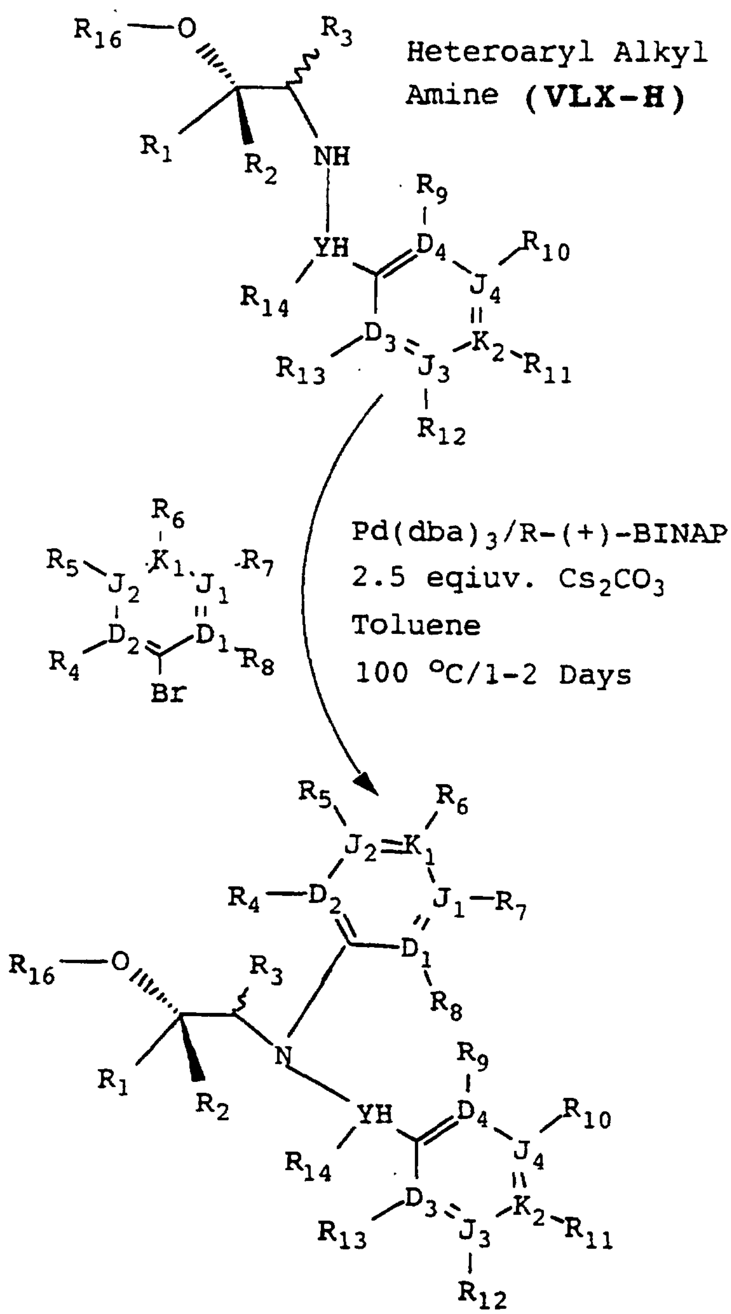


**I-HP/I-HPC:**(Generic Polycyclic Heteroaryl/Aryl-Heteroaryl (R)-Chiral Halogenated 1-Substitutedamino-2-Propanols)

# Scheme 11

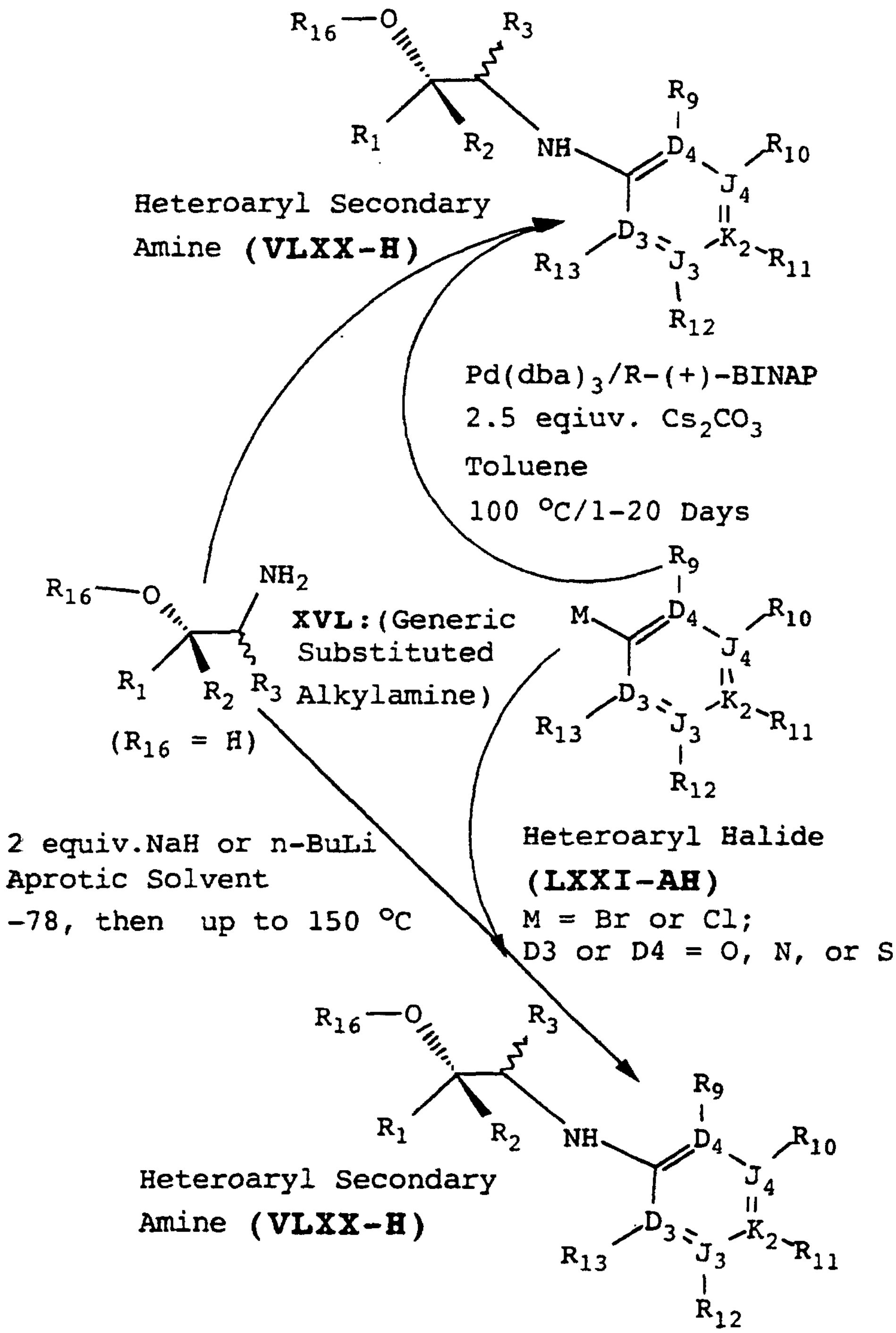
Heteroaryl Secondary
Amine (VLXX-H)
Pd(dba)3/R-(+)-BINAP
2.5 eqiuv. Cs2CO3
Toluene
100 °C/1-20 Days
XVL:(Generic
Substituted
Alkylamine)
(R16 = H)
2 equiv.NaH or n-BuLi
Aprotic Solvent
-78, then up to 150 °C
Heteroaryl Halide
(LXXI-AH)
M = Br or Cl;
D3 or D4 = O, N, or S
Heteroaryl Secondary
Amine (VLXX-H)

# Scheme 12

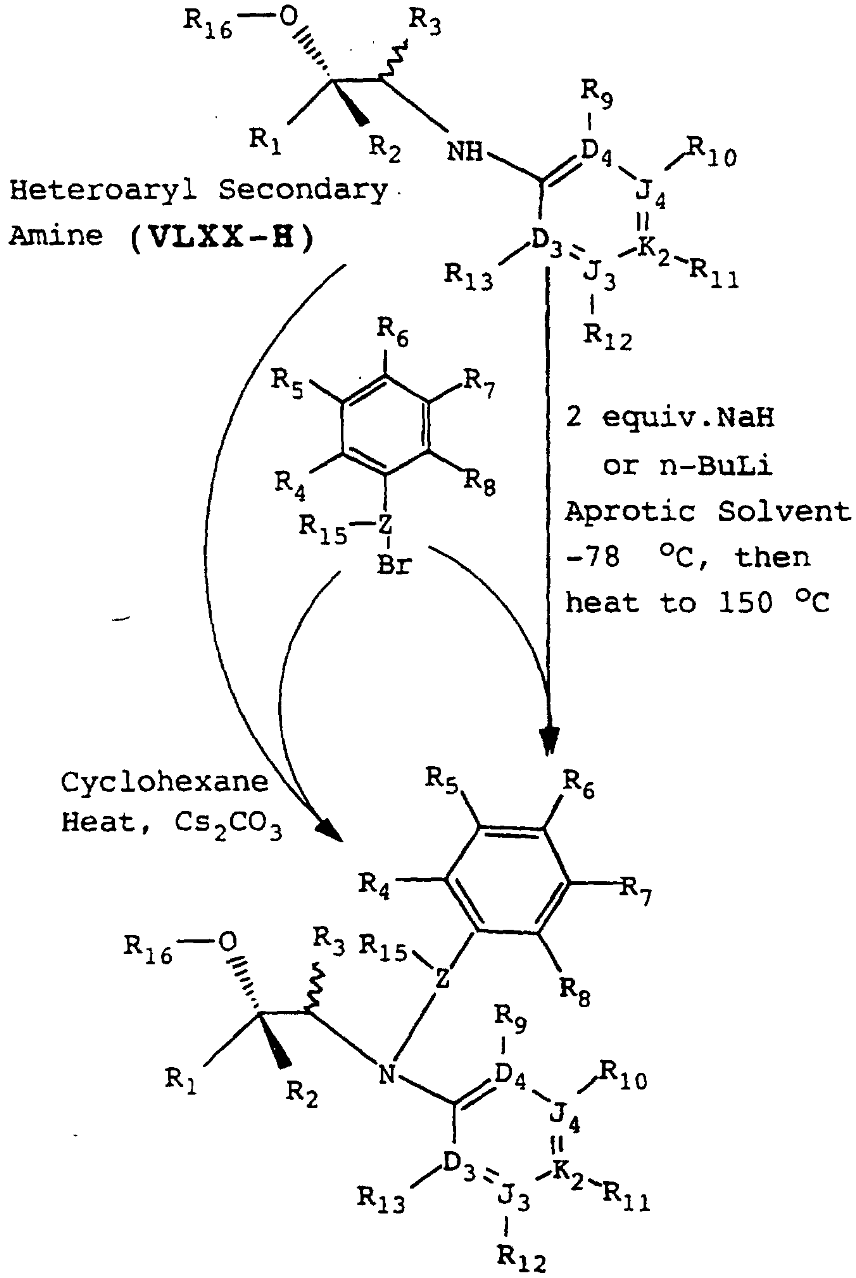


**I-HP/I-HPC/I-CP:**(Generic Polycyclic Aryl Aryl and Heteroaryl (R)-Chiral Halogenated 1-Substitutedamino-2-Propanols)

**I-CP:** (Polycyclic 3-Heteroarylaryl Phenyl (R)-Chiral Halogenated 1-Substitutedamino-2-Propanols; $R_{16}$ = H)

**Scheme 13**

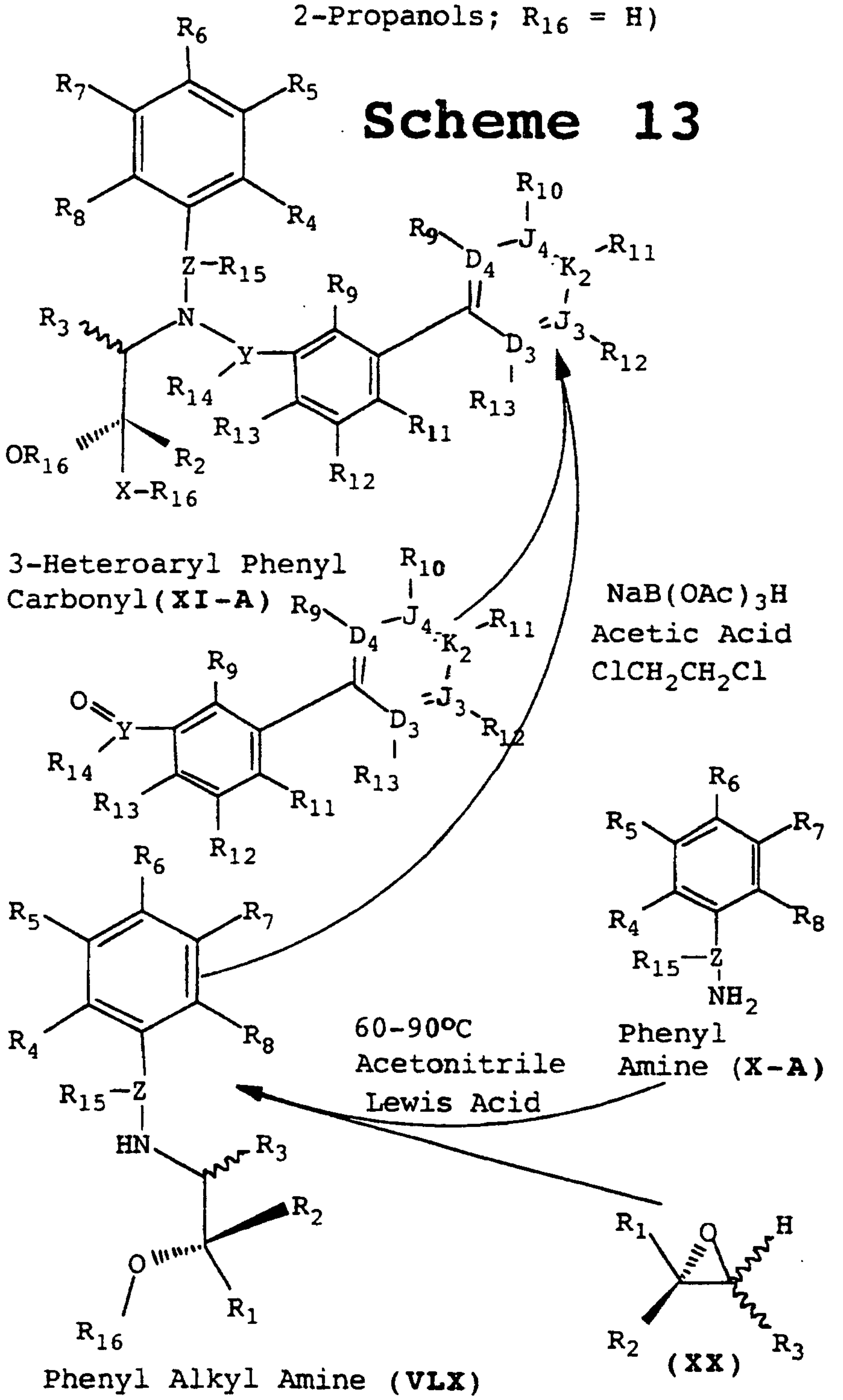


3-Heteroaryl Phenyl Carbonyl (**XI-A**)

Phenyl Amine (**X-A**)

(**XX**)

Phenyl Alkyl Amine (**VLX**)

NOTE: HeteroarylAnalogs Can Be Prepared Using Heteroaryl Analogs of **X-A**, **VLX**, and **XI-A**.

# Scheme 14

Method A:

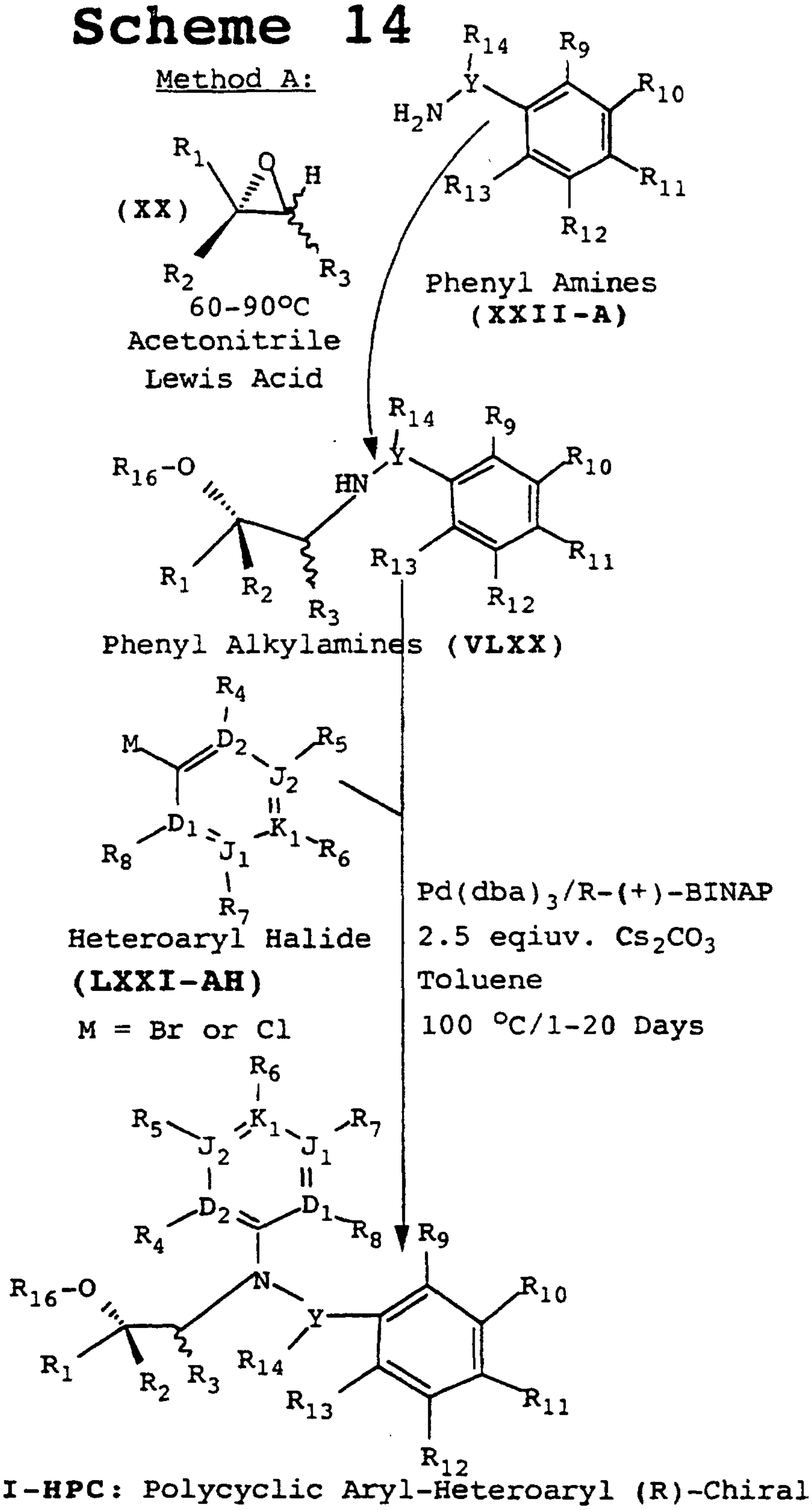


(XX)

Phenyl Amines (XXII-A)

Phenyl Alkylamines (VLXX)

Heteroaryl Halide (LXXI-AH)

M = Br or Cl

**I-HPC:** Polycyclic Aryl-Heteroaryl (R)-Chiral Halogenated 1-Substitutedamino-2-Propanols when $R_{16}$ equals H) NOTE: Aryl Analogs **(I-CP)** of **(I-HPC)** Can Be Prepared by Starting With Aryl Bromide Analogs of **(LXXI-AH)**.

## Scheme 15

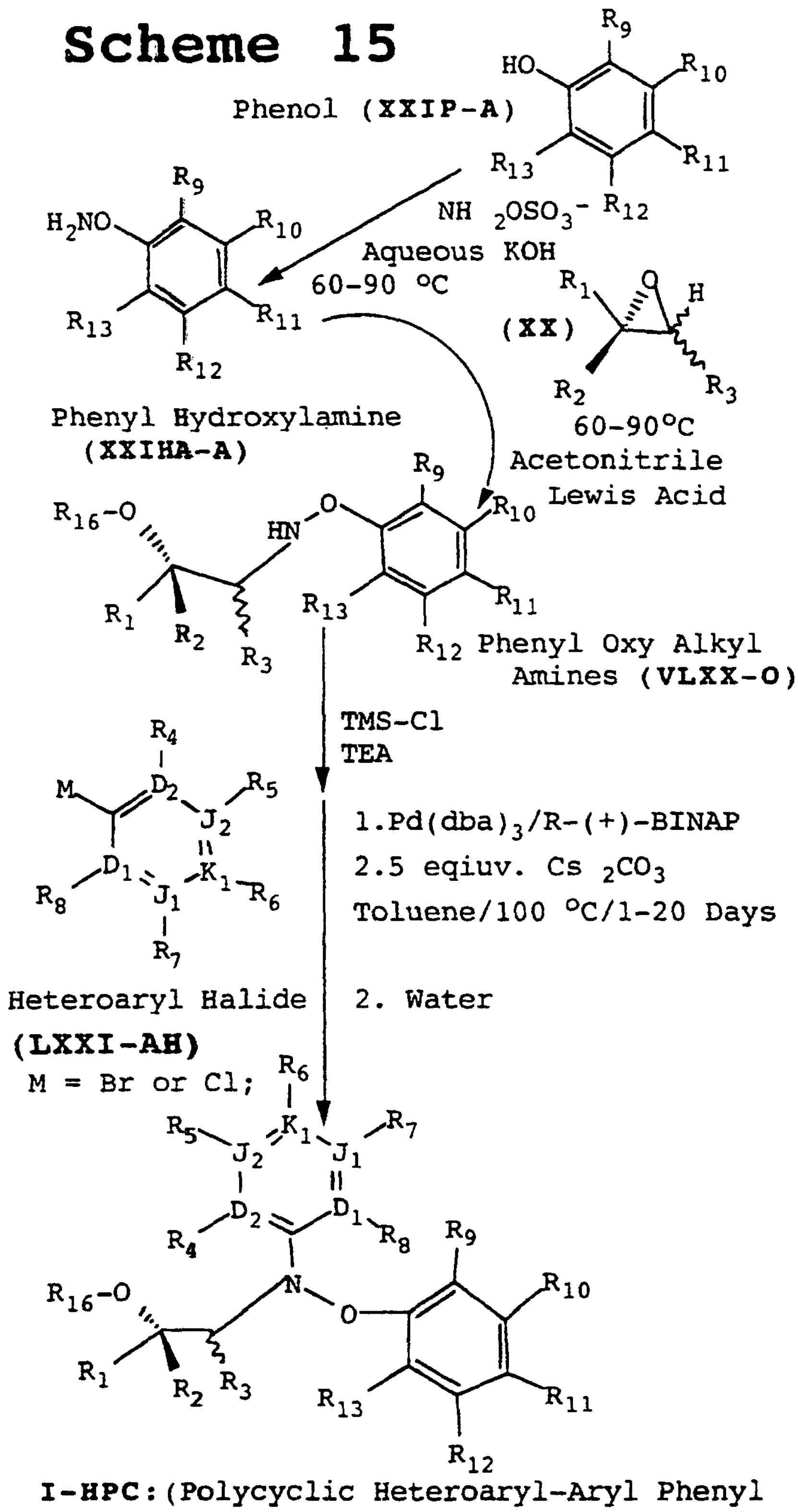


**I-HPC:** (Polycyclic Heteroaryl-Aryl Phenyl (R)-Chiral Halogenated 1-Substiutedamino-2-Propanols when $R_{16}$ = H and Y = O) NOTE: Diaryl **(I-CP)** and Diheteroaryl **(I-HP)** Analogs Can Be Prepared by Using Aryl Bromide and Heteroaryl-OH,respectively.

The following examples are provided to illustrate the present invention and are not intended to limit the scope thereof. Without further elaboration, it is believed that one skilled in the art can, using the preceding descriptions, utilize the present invention to its fullest extent. Therefore the following preferred specific embodiments are to be construed as merely illustrative and not limitative of the remainder of the disclosure in any way whatsoever. Compounds containing multiple variations of the structural modifications illustrated in the preceding schemes or the following Examples are also contemplated. Those skilled in the art will readily understand that known variations of the conditions and processes of the following preparative procedures can be used to prepare these compounds.

One skilled in the art may use these generic methods to prepare the following specific examples, which have been or may be property characterized by $^1$H NMR and mass spectrometry. These compounds also may be formed in vivo.

The following examples contain detailed descriptions of the methods of preparation of compounds of the invention. These detailed descriptions fall within the scope and are presented for illustrative purposes only and are not intended as a restriction on the scope of the invention. All parts are by weight and temperatures are Degrees centigrade unless otherwise indicated.

**EXAMPLE 1**

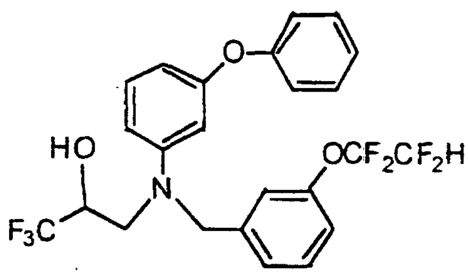

**(2*R*,*S*)-3-[(3-phenoxyphenyl)[[3-(1,1,2,2-tetrafluoroethoxy)phenyl]methyl]amino]-1,1,1-trifluoro-2-propanol**

**EX-1A**) To a solution of 3-(1,1,2,2-tetrafluoroethoxy)toluene (50 g, 0.24 mol) and *N*-bromosuccinimide (42.75 g, 0.24 mol) in 100 mL of carbon tetrachloride under nitrogen was added 2,2'-azobisisobutyronitrile (0.71 g, 0.004 mol). The resultant mixture was refluxed for 2 h, then cooled to room temperature and quenched with 300 mL of water. The organic layer was collected, washed with water and brine, dried over $MgSO_4$, and concentrated *in vacuo* to give 66.0 g (96%) of the desired crude 3-(1,1,2,2-tetrafluoroethoxy)bromomethylbenzene product as a yellow oil. $^1$H NMR indicates that this oil is a mixture of products: 7% dibrominated, 67% monobrominated, and 20% starting material. The crude product was used without further purification. ESMS *m*/*z* = 287 $[M+H]^+$.

**EX-1B**) The crude product (56 g, 0.14 mol) from **EX-1A** in 200 mL of cyclohexane was added dropwise under nitrogen to a solution of 3-phenoxyaniline (89 g, 0.480 mol) in 500 mL of cyclohexane. The reaction mixture was refluxed overnight, then cooled to room temperature and diluted with water and diethyl ether. The layers were separated, and the aqueous layer was extracted with diethyl ether. The combined organic layers were dried over $MgSO_4$ and concentrated *in vacuo* to give a dark oil. The crude product was purified by column chromatography on silica gel eluting with 1:4 ethyl acetate in hexane to afford 44.96 g (83%) of the desired *N*-(3-phenoxyphenyl)-[[3-(1,1,2,2-tetrafluoroethoxy) phenyl]methyl]amine product as a yellow oil. ESMS *m*/*z* = 392 $[M+H]^+$.

To a mixture of the amine product (15.0 g, 0.038 mol) from **EX-1B** and 1,1,1-trifluoro-2,3-epoxypropane (8.58 g. 0.077 mol. TCI) was added a suspension of ytterbium (III) trifluoromethanesulfonate (2.37 g. 0.0031 mol) in 15 mL of acetonitrile. The resulting mixture was heated at 50 °C in a sealed glass vial for 1.5 h. The reaction mixture was cooled to room temperature then diluted with water and ethyl acetate and extracted. The organic layers were combined, dried over $MgSO_4$, and concentrated *in vacuo*. The crude product was purified by column chromatography on silica gel eluting with 1:4 ethyl acetate in hexane to afford 12.03 g (62%) of the desired (2*RS*)-3-[(3-phenoxyphenyl)[ [3-(1,1,2,2-tetrafluoroethoxy) phenyl]methyl]amino]-1,1,1-trifluoro-2-propanol product as a yellow oil. Anal. calcd. for $C_{24}H_{20}F_7NO_3$: C, 57.26; H, 4.00; N, 2.78. Found: C, 56.96; H, 4.35; N, 2.69. HRMS calcd. 504.1410 $[M+H]^+$, found: 504.1431. $^1$H NMR ($CDCl_3$) δ 7.28 (m, 4H), 7.14 (t, 1H), 7.07, (m, 3H), 7.00 (s, 1H), 6.94 (d, 2H), 6.46 (dd, 1H), 6.38 (dd. 1H), 6.35 (t, 1H), 5.84 (t, 1H), 4.60 (t, 2H), 4.36 (m, 1H), 3.82 (d, 1H), 3.48 (m, 1H), 2.51 1 (s, 1H). $^{19}$F NMR ($CDCl_3$) δ -79.0 (s, 3F). -88.21 (d. 2F), -137.05 (dd. 2F).

**EXAMPLE 2**

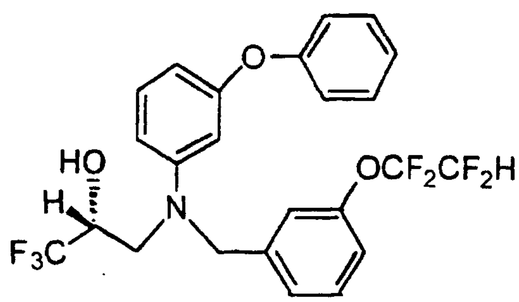

**(2*R*)-3-[(3-phenoxyphenyl)[[3-(1,1,2,2-tetrafluoroethoxy)phenyl]methyl]amino]- 1,1,1-trifluoro-2-propanol**

On a Chiralpak AD HPLC column, (2*RS*)-3-[(3-phenoxyphenyl)[[3-(1,1,2,2-tetrafluoroethoxy)phenyl]methyl] amino]-1,1,1-trifluoro-2-propanol (12.2 g, 0.024 mol) from **EX-1** was purified by chiral chromatography to give 1.4 g (0.003 mol. 12%) of (2*R*)-3-[(3-phenoxyphenyl)[[3-(1,1,2,2-tetrafluoroethoxy)phenyl]methyl] amino]-1,1,1-trifluoro-2-propanol as a light yellow oil. Chiral purification was accomplished by eluting with 1:9 isopropanol in heptane at 1.0 mL/min with 250 nm UV detection. The product eluted at 8.43 min. $[\alpha]_{589}$ = +16.8.0 (c 0.125 g/dL, $CH_3CN$), $[\alpha]_{365}$ = +84.0 (c 0.125, $CH_3CN$). Anal. calcd. for $C_{24}H_{20}F_7NO_3$: C, 57.26; H, 4.00; N, 2.78. Found: C, 56.96; H, 4.35; N, 2.69. HRMS calcd.: 504.1410 $[M+H]^+$, found: 504.1388. $^1H$ NMR ($CDCl_3$) δ 7.28 (m, 4H), 7.14 (t, 1H), 7.07, (m, 3H), 7.00 (s, 1H), 6.94 (d, 2H), 6.46 (dd. 1H), 6.38 (dd. 1H), 6.35 (t, 1H), 5.84 (t, 1H), 4.60 (t, 2H), 4.36 (m, 1H), 3.82 (d, 1H), 3.48 (m, 1H), 2.51 (s, 1H). $^{19}F$ NMR ($CDCl_3$) δ -79.0 (s, 3F), -88.21 (d, 2F), -137.05 (dd, 2F).

**EXAMPLE 3**

(Included for reference and/or comparison only)

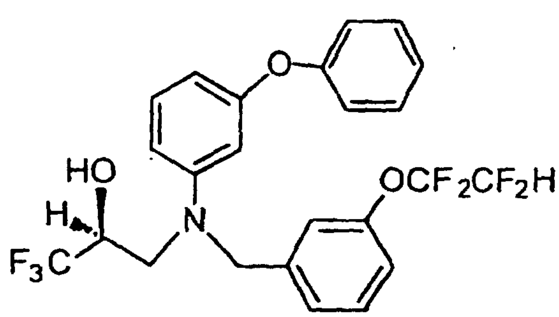

**(2S)-3-[(3-phenoxyphenyl)[[3-(1,1,2,2-tetrafluoroethoxy)phenyl]methyl]amino]-1,1,1-trifluoro-2-propanol**

On a Chiralpak AD HPLC column. (2*RS*)-3-[(3-phenoxyphenyl)[[3-(1,1,2,2-tetrafluoroethoxy)phenyl]methyl] amino]-1,1,1-trifluoro-2-propanol (12.2 g, 0.024 mol) from **EX-1** was purified by chiral chromatography to give 10.5 g (0.021 mol, 86%) of (2*S*)-3-[(3-phenoxyphenyl)[[3-(1,1,2,2-tetrafluoroethoxy)phenyl] methyl]amino]-1,1,1-trifluoro-2-propanol as a light yellow oil. Chiral purification was accomplished by eluting with 1:9 isopropanol in heptane at 1.0 mL/min with 250 nm UV detection. The product eluted at 6.36 min. $[\alpha]_{589}$ = -17.0 (c 0.265 g/dL, $CH_3CN$), $[\alpha]_{365}$ = -85.7 (c 0.265, $CH_3CN$). Anal. calcd. For $C_{24}H_{20}F_7NO_3$: C, 57.26; H, 4.00; N, 2.78. Found: C, 56.96; H, 4.35; N, 2.69. HRMS calcd.: 504.1410 $[M+H]^+$, found: 504.1431. $^1H$ NMR ($CDCl_3$) δ 7.28 (m, 4H), 7.14 (t, 1H), 7.07, (m, 3H), 7.00 (s, 1H), 6.94 (d, 2H), 6.46 (dd, 1H), 6.38 (dd, 1H), 6.35 (t, 1H), 5.84 (t, 1H), 4.60 (t, 2H), 4.36 (m, 1H), 3.82 (d, 1H), 3.48 (m, 1H), 2.51 (s, 1H). $^{19}F$ NMR ($CDCl_3$) δ -79.0 (s, 3F), -88.21 (d, 2F), - 137.05 (dd. 2F).

**EXAMPLE 4**

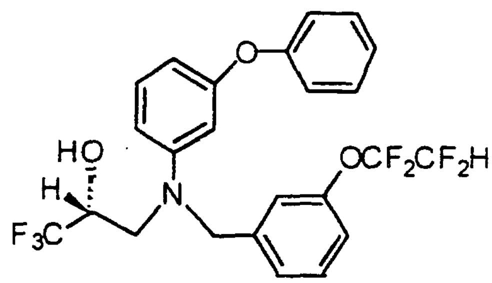

**(2*R*)-3-[(3-phenoxyphenyl)[[3-(1,1,2,2-tetrafluoroethoxy)phenyl]methyl]amino]-1,1,1-trifluoro-2-propanol**

Using a procedure adopted from H.C.Brown et al. (*J. Org. Chem.* **60**, 41-46. (1995)). *R*-(+)-1,1,1-trifluoro-2,3-epoxypropane was prepared beginning with the transfer of (+)-*B*-chlorodiisopinocampheylborane ((+)-DIP-Cl, 1.2 kg, 3.74 mol) to a 5 L three neck flask containing 5 L of ether under nitrogen. Anhydrous ether (5 L) was added. and the mixture was stirred until the solids dissolved and the temperature equilibrated to 0 °C. Then 3-bromotrifluoroacetone (326 mL, 3.14 mol) was added. and the reaction was stirred for 72 h while maintaining the temperature between -4 and +5 °C. The reaction was followed by $^{19}F$ NMR by removing an aliquot (20 μL), quenching with anhydrous methanol (0.6 mL), and referencing to external $D_2O$. The reduction was 68 % complete after 48 h. The ether was removed under vacuum (100 torr to 0.1 torr), leaving a pale, viscous oil. A 5 L 3-neck flask equipped with stirrer. dropping funnel, and short-path distillation head with chilled receiver was charged with 50% (w/w) aqueous NaOH and heated to 40 °C. With external heat removed. the quenched reduction mixture was added dropwise to the aqueous NaOH, with the rate controlled to maintain the pot temperature below 65 °C. The product epoxide formed immediately, distilling over with a head temperature of 32-42 °C. A yellow-orange solid byproduct was broken up by stirring and some foaming was observed. When the distillation was complete. 145 g (43%) of the desired *R*-(+)-1,1,1-trifluoro-2,3-epoxy-propane product was obtained as a clear, colorless oil. $^1H$ NMR ($C_6D_6$) δ 2.50 (m, 1H, $CF_3CH$), 2.15 (dd, 1H, J = 2.10, 5.01 Hz), 1.75 (m, 1H). $^{19}F$ NMR ($C_6D_6$) δ -75.4 (d, J = 4.7 Hz). Chiral GC/MS analysis was performed on the corresponding diethylamine adduct using a gamma cyclodextrin column (Supelco gammadex120 G-cyclodextrin fused silica): 4 drops of the epoxide, *R*-(+)-1,1,1-trifluoro-2,3-epoxypropane, and 4 drops of diethylamine were heated briefly in a sealed vial, cooled. diluted with methyl *t*-butyl ether, and analyzed. Found: two gc peaks: 10.97 min and 11.11 min (ratio 1: 230; 99% ee), where the *R*-product predominated. MS calcd. for $C_7H_{14}F_3NO$: *m/z* = 186 $[M+H]^+$, found: 186. for both gc peaks. In contrast, the diethylamine adduct obtained with the TCI trifluoromethyl-oxirane (lot OGH01) from **EX-1**, gave 2 peaks with identical MS signals *m/z* = 186, 10.96 min and 11.12 min (ratio 8.5:1; 79% ee), where the *S*-product predominated.

To a mixture *N*-(3-phenoxyphenyl)-[[3-(1,1,2,2-tetrafluoroethoxy)phenyl] methyl]-amine from **EX-1B** (1.48 g, 0.0038 mol) and *R*-(+)-1,1,1-trifluoro-2,3-epoxypropane (0.64 g, 0.0057 mol) was added a suspension of ytterbium (III) trifluoro-methanesulfonate (0.23 g, 0.0004 mol) in 1.5 mL of acetonitrile. The resulting mixture was heated at 50 °C in a sealed glass tube for 1.5 h. The reaction mixture was cooled to room temperature then diluted with water and ethyl acetate and extracted. The organic layers were combined, dried over $MgSO_4$, and concentrated *in vacuo*. The crude product was purified by column chromatography on silica gel eluting with 1:4 ethyl acetate in hexane to afford 1.2 g (63%) of the desired (2*R*)-3-[(3-phenoxyphenyl)-[[3-(1,1,2,2-tetrafluoroethoxy)phenyl]methyl]amino]-1,1,1-trifluoro-2-propanol product as a pure yellow oil (>96% ee by chiral HPLC analysis), which was identical in all respects to **EX-2.** Anal. calcd. for $C_{24}H_{20}F_7NO_3$: C, 57.26; H, 4.00; N, 2.78. found: C, 56.96; H, 4.35; N, 2.69. HRMS calcd.: 504.1410 $[M+H]^+$. found: 504.1431. $^1H$ NMR ($CDCl_3$) δ 7.28 (m, 4H), 7.14 (t, 1H), 7.07, (m. 3H), 7.00 (s, 1H), 6.94 (d, 2H), 6.46 (dd, 1H), 6.38 (dd, 1H), 6.35 (t, 1H), 5.84 (t, 1H), 4.60 (t, 2H), 4.36 (m, 1H), 3.82 (d, 1H), 3.48 (m, 1H), 2.51 (s, 1H), $^{19}F$ NMR ($CDCl_3$) δ -79.0 (s, 3F), -88.21 (d, 2F), -137.05 (dd, 2F).

Additional examples can be prepared by one skilled in the art using similiar methods and commercially available epoxides. For example, 3-[(3-phenoxyphenyl)[[3-(trifluoromethoxy)phenyl]methyl]amino]-1-chloro-2-propanols can be prepared from the reaction of *N*-(3-phenoxyphenyl)-[[3-(trifluoromethoxy)phenyl]methyl]amine with either (*R*)-epichlorohydrin or (*S*)-epichlorohydrin, as illustrated in Example Table 1.

Example Table 1. 3-[(3-phenoxyphenyl)[[3-(trifluoromethoxy)phenyl]methyl]amino]-1-chloro-2-propanols.

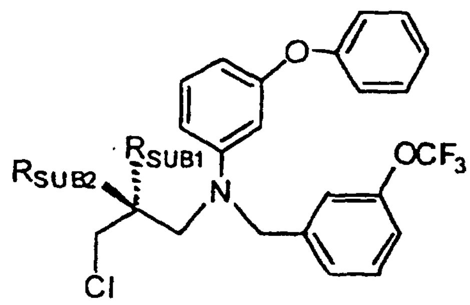

| Ex. No. | $R_{SUB1}$ | $R_{SUB2}$ | Calculated Mass $[M+H]^+$ | Observed Mass $[M+H]^+$ |
|---|---|---|---|---|
| 5 | OH | H | 452.1240 | 452.1245 |
| 6* | H | OH | 452.1240 | 452.1259 |

*Included for reference and/or comparison only.

EXAMPLE 7

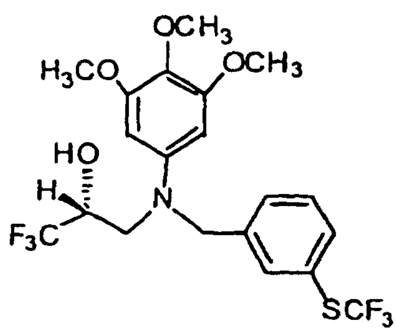

**(2*R*)-3-[(3,4,5-trimethoxyphenyl)[[3-(trifluoromethylthio)phenyl]methyl]amino]-1,1,1-trifluoro-2-propanol**

**EX-45A)** To a 1,2-dichloroethane (12 mL) solution of 3,4,5-trimethoxyaniline (0.80 g, 4.4 mmol) was added (3-trifluoromethylthio)benzaldehyde (0.90 g, 4.4 mmol), $NaB(OAc)_3H$ (1.20 g, 5.66 mmol) and acetic acid (0.26 mL, 4.5 mmol). The cloudy solution was stirred at room temperature for 1 h. The reaction mixture was poured into water and extracted with dichloromethane. The organic layer was washed with saturated $NaHCO_3$ and brine, dried ($MgSO_4$) and evaporated to give 1.58 g (96%) of the desired *N*-(3,4,5-trimethoxyphenyl)[[3-trifluoromethylthiophenyl]methyl] amine product as an off-white solid. MS: *m*/*z* = 373.8 $[M+H]^+$.

To an acetonitrile (3.2 mL) solution of amine (1.20 g, 32 mmol) from **EX-45A** was added *R*-(+)-1,1,1-trifluoro-2,3-epoxypropane (0.55 mL 6.4 mmol) from **EX-4** and $Yb(OTf)_3$ (0.40 g, 0.64 mmol). The cloudy solution was stirred in a sealed flask at 50 °C for 18 h. The cooled reaction mixture was diluted with diethyl ether and washed with water and brine. The organic layer was dried ($MgSO_4$) and evaporated to an oil. Purification by flash column chromatography

on silica gel eluting with 20% ethyl acetate in hexane gave an oil which was triturated with hexanes to give a white solid. The precipitate was isolated by filtration and dried *in vacuo* to give 0.82 g (53 %) of the desired (2*R*)-3-[(3,4,5-trimethoxyphenyl)[[3-(trifluoromethylthio)phenyl]methyl]-amino]-1,1,1-trifluoro-2-propanol product as a white solid. m.p. 88.9-89.1 °C (95% ee by chiral HPLC). Anal. calcd. for $C_{20}H_{21}NO_4SF_6$: C. 49.48; H, 4.36; N, 2.89. Found: C, 49.29; H, 4.21; N, 2.81. HRMS calcd.: 486.1174 $[M+H]^+$, found: 486.1158. $^1H$ NMR ($C_6D_6$) δ 3.10 (d, 1H), 3.18 (dd, 1H), 3.32 (s, 6H), 3.53 (d, 1H), 3.64 (s, 3H), 4.01 (m, 1H), 4.21 (dd, 2H), 5.70 (s, 2H), 6.80 (t, 1H), 6.94 (d, 1H), 7.23 (d, 1H), 7.37 (s, 1H). $[\alpha]_{589}$ = +26.8 (c 1.099 g/dL, $CHCl_3$).

**EXAMPLE 8**

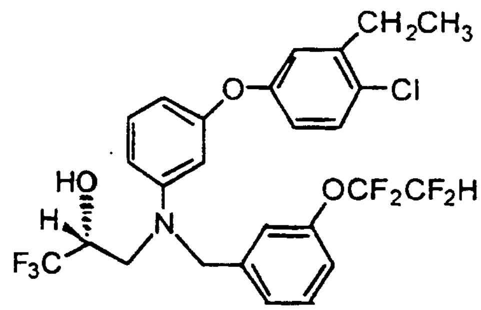

**(2*R*)-3-[[3-(4-chloro-3-ethylphenoxy)phenyl][[3-(1,1,2,2-tetrafluoroethoxy)phenyl]methyl]amino]-1,1,1-trifluoro-2-propanol**

**EX-8A)** To a solution of 1.3-dinitrobenzene (16.8 g. 0.1 mol) and 4-chloro-3-ethylphenol (15.6 g. 0.1 mol) in 200 mL of dimethylsulfoxide was added cesium carbonate (65 g, 0.2 mol). The reaction mixture was heated at 100 °C under nitrogen overnight then cooled to room temperature. The reaction mixture was filtered through celite then rinsed with diethyl ether and a small amount of water. The filtrate was extracted several times with diethyl ether. The organic layers were combined. washed with water and brine, dried over $MgSO_4$, and concentrated *in vacuo* to give 21.8 g (78%) of the desired 3-(4-chloro-3-ethylphenoxy)-1-nitrobenzene product as a dark orange oil, which was greater than 90% pure by reverse phase HPLC analysis. HRMS calcd. for $C_{14}H_{12}ClNO_3$: 295.0849 $[M+NH_4]^+$, found 295.0862.

**EX-8B**) To a solution of 3-(4-chloro-3-ethylphenoxy)-1-nitrobenzene (10 g, 0.036 mol) from **EX-8A** in 400 mL of glacial acetic acid and 1 mL of water was added zinc metal (20 g, 0.305 mol) at room temperature, and the resultant mixture was stirred for 1 h. The reaction mixture was filtered through celite. The filtrate was neutralized with ammonium hydroxide and extracted with diethyl ether. The organic layer was washed with water and brine, dried over $MgSO_4$, and concentrated *in vacuo* to give 10 g (100%) of the desired 3-(4-chloro-3-ethylphenoxy)aniline product as a dark orange oil, which was greater than 90% pure by reverse phase HPLC analysis. HRMS calcd. for $C_{14}H_{14}ClNO$: 248.0842 $[M+H]^+$, found: 248.0833.

**EX-8C**) To a solution of 3-(4-chloro-3-ethylphenoxy)aniline (2.0 g, 8.1 mmol) from **EX-8B** and 3-(1,1,2,2-tetrafluoroethoxy)benzaldehyde (1.6 g. 7.3 mmol) in 30 mL of dichloroethane was added sodium triacetoxyborohydride (2.0 g, 9.7 mmol) and glacial acetic acid (0.51 mL. 8.9 mmol). The reaction mixture was stirred at room temperature for 1 h then quenched with water and extracted with diethyl ether. The organic layer was washed with water and brine. dried over $MgSO_4$, and concentrated *in vacuo* to give 3.5 g (95%) of the desired *N*-[(4-chloro-3-ethylphenoxy)phenyl]-3-[[3-(1,1,2,2-tetrafluoroethoxy)phenyl]methyl] amine product as a brown oil, which was greater than 90% pure by reverse phase HPLC analysis. HRMS calcd, for $C_{23}H_{20}ClF_4NO_2$: 454.1197 $[M+H]^+$, found: 454.1220.

A solution of *N*-[(4-chloro-3-ethylphenoxy)phenyl]-3-[[3-(1,1,2,2-tetrafluoroethoxy)-phenyl]methyl]amine (1.8 g, 4.0 mmol) from **EX-8C,** *R*-(+)-1,1,1-trifluoro-2,3-epoxy-propane (0.64 g, 0.0057 mol) from **EX-4,** and ytterbium (III) trifluoromethanesulfonate (0.25 g, 0.4 mmol) in 1.5 mL of acetonitrile was heated at 40 °C in a sealed glass tube for 1 h. The reaction mixture was cooled to room temperature then diluted with water and diethyl ether and extracted. The ether layer was washed with water and brine, dried over $MgSO_4$, and concentrated *in vacuo* The crude product was purified by column chromatography on silica gel eluting with 1:7:0.01 of ethyl acetate:hexane:ammonium hydroxide to afford 1.5 g (66%) of the desired (2*R*)-3-[[3-(4-chloro-3-ethylphenoxy)phenyl][[3-(1,1,2,2-tetrafluoroethoxy)phenyl] methyl]amino]-1,1,1-tri-fluoro-2-propanol product as a yellow oil (96% ee by chiral HPLC analysis). $[\alpha]_{589}^{25}$ = + 36.9 (c 1.044g%, $CHCl_3$). $[\alpha]_{365}^{25}$ = + 189.7 (c 1.044g%, $CHCl_3$). The refractive index @ 25 °C is 1.5275. Anal. calcd. for

$C_{26}H_{23}ClF_7NO_3$: C, 55.18; H, 4.10; N, 2.48. found: C, 54.92; H, 4.05; N, 2.33. HRMS calcd.: 566.1330 $[M+H]^+$, found: 566.1323. $^1H$ NMR ($CDCl_3$) δ 7.30 (t, 1H), 7.20 (d, 1H), 7.15 (t, 1H), 7.08 (t, 2H). 7.00 (s, 1H), 6.86 (d, 1H), 6.68 (dd, 1H), 6.48 (dd, 1H), 6.36 (dd, 1H), 6.34 (t, 1H), 5.81 (tt, 1H), 4.62 (s, 2H), 4.32 (m, 1H), 3.84 (dd, 1H), 3.55 (dd, 1H), 2.67 (q, 2H), 2.45 (bs, 1H), 1.17 (t, 3H). $^{19}F$ NMR ($CDCl_3$) δ -79.22 (d, 3F), -88.57 (m, 2F), -137.16 (dt, 2F).

Additional examples of (2*R*)-3-[[3-(substituted-phenoxy)phenyl][[3-(1,1,2,2-tetrafluoroethoxy)phenyl]methyl]amino]-1,1,1-trifluoro-2-propanols and (2*R*)-3-[[3-(4-chloro-3-ethylphenoxy)phenyl][[3-substituted-phenyl]methyl]amino]-1,1,1-trifluoro-2-propanols can be prepared by one skilled in the art using similar methods, as shown in Example Tables 2 and 3, respectively.

Example Table 2. (2*R*)-3-[[3-(Substituted-phenoxy)phenyl][[3-(1,1,2,2-tetrafluoroethoxy)-phenyl]methyl]amino]-1,1,1-trifluoro-2-propanols.

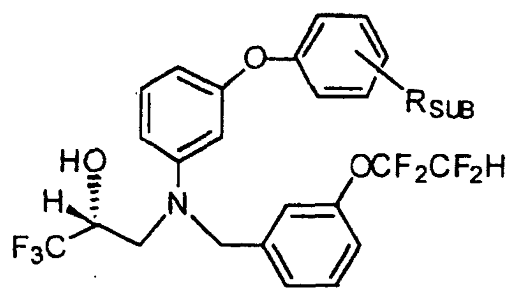

| Ex. No. | $R_{SUB}$ | Calculated Mass $[M+H]^+$ | Observed Mass $[M+H]^+$ |
|---|---|---|---|
| 9 | 4-methyl | 518.1566 | 518.1587 |
| 10 | 3-isopropyl | 546.1879 | 546.1900 |
| 11 | 3-ethyl | 532.1723 | 532.1713 |

Example Table 3. (2*R*)-3-[[3-(4-Chloro-3-ethylphenoxy)phenyl][[3-substituted-phenyl]methyl]amino]-1,1,1-trifluoro-2-propanols.

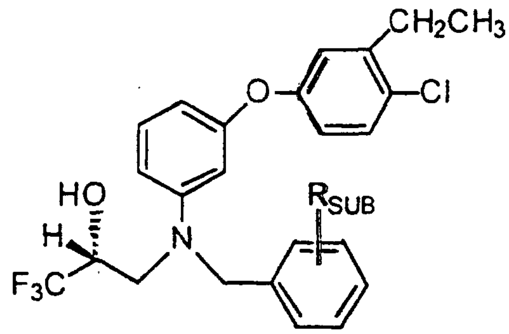

| Ex. No. | $R_{SUB}$ | Calculated Mass $[M+H]^+$ | Observed Mass $[M+H]^+$ |
|---|---|---|---|
| 12 | 3-trifluoromethoxy | 534.1271 | 534.1309 |
| 13 | 3-trifluoromethyl, 4-fluoro | 536.1228 | 536.1265 |
| 14 | 2-fluoro, 4-trifluoromethyl | 536.1228 | 536.1241 |
| 15 | 2-trifluoromethyl, 4-fluoro | 536.1228 | 536.1245 |
| 16 | 2-fluoro, 5-trifluoromethyl | 536.1228 | 536.1252 |
| 17 | 2-fluoro, 6-trifluoromethyl | 536.1228 | 536.1199 |

**EXAMPLE 18**

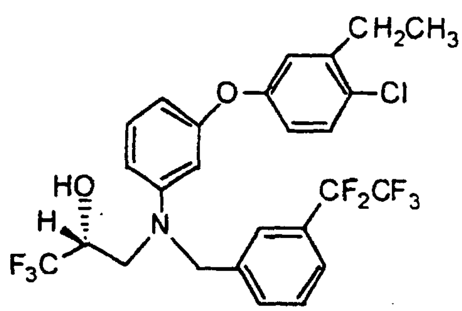

**(2*R*)-3-[[3-(4-chloro-3-ethylphenoxy)phenyl][[13-(1,1,1,2,2-pentafluoroethyl)phenyl]methyl]amino]-1,1,1-trifluoro-2-propanol**

**EX-18A**) Sodium pentafluoroethyl propionate (8.4 g, 50 mmol) and 3-iodotoluene (5.5 g, 25 mmol) were dissolved in anhydrous DMF (300 mL) under nitrogen. CuI (9.5 g, 50 mmol) was added, and the mixture was heated to 160 °C under nitrogen for 4 h, at which time a 15 mL fraction of a mixture of DMF and 3-pentafluoroethyl toluene was collected. The distillate was diluted with $Et_2O$ and was washed with brine. The ether layer was dried over $MgSO_4$, filtered and concentrated *in vacuo* to give 5.25 g (55%) of the desired 3-pentafluoroethyl-toluene product as a colorless oil. $^1H$ NMR ($CDCl_3$) δ 7.36 (m, 4H), 2.40 (s, 3H). $^{19}F$ NMR ($CDCl_3$) δ -85.2 (s, 3F), -115.2 (s, 2F).

**EX-18B**) The 3-pentafluoroethyl-toluene (2.9 g, 13.8 mmol) product from **EX-18A** and *N*-bromosuccinimide (2.5 g, 13.8 mmol) were dissolved in $CCl_4$ (25 mL). AIBN (50 mg, 0.3 mmol) was added, and the mixture was refluxed for 3.5 h under $N_2$. The reaction mixture was cooled to room temperature and diluted with water. The layers were separated, and the organic layer was washed with brine, dried with anhydrous $MgSO_4$, filtered, and concentrated *in vacuo* to give 3.4 g (87%) of a colorless oil. The $^1H$ NMR spectrum indicated that the crude product contained 3-pentafluoroethyl-benzylbromide (70%), the benzyldibromide (10%) and pentafluoroethyl toluene (20%). $^1H$ NMR ($CDCl_3$) δ 7.60 (m. 2H), 7.50 (m, 2H), 4.50 (s, 2H). $^{19}F$ NMR ($CDCl_3$) δ -85.1 (s, 3F), -115.4 (s, 2F).

**EX-18C**) A solution of 3-(4-chloro-3-ethylphenoxy)aniline (1.7 g. 6.9 mmol) was prepared in cyclohexane (13 mL). A solution of crude 3-pentafluoroethyl benzylbromide (1g, 3.5 mmol) product from **EX-18B** in cyclohexane (10 mL) was added dropwise under nitrogen over 3 min. The reaction mixture was refluxed under $N_2$ for 24 h and then was cooled to room temperature. The mixture was diluted with $Et_2O$ and saturated aqueous $NaHCO_3$. The layers were separated, and the aqueous layer was extracted with $Et_2O$. The organic layer was washed with brine, dried with anhydrous $MgSO_4$, filtered and concentrated *in vacuo*. The residue was purified by chromatography on silica gel eluting with hexanes in ethyl acetate (95:5) which gave 0.56 g (35%) of the desired *N*-[3-(4-chloro-3-ethylphenoxy)phenyl][ [3-(pentafluoro-ethyl)phenyl]methyl]amine product as a brown oil. $^1H$ NMR ($CDCl_3$) δ 7.53 (m, 4H), 7.27 (d, 1H), 7.15 (t, 1H), 6.93 (d, 1H), 6.77 (dd, 1H), 6.41 (tt, 2H), 6.30 (t, 1H), 4.41 (s, 2H), 2.73 (q, 2H), 1.23 (t, 3H). $^{13}C$ NMR ($CDCl_3$) δ 158.6, 156.1, 143.4, 141.3, 140.2, 131.3, 130.7, 130.4, 129.4, 128.1, 120.4, 117.8, 108.8. 103.9, 48.5, 27.5, 14.1. $^{19}F$ NMR ($CDCl_3$) δ -85.1 (s, 3F), -115.2 (s, 2F). HRMS calcd. for $C_{23}H_{19}ClF_5NO$: 456.1154 $[M+H]^+$ , found: 456.1164.

The *N*-[3-(4-chloro-3-ethylphenoxy)phenyl][[3-(pentafluoroethyl)phenyl]-methyl]amine (0.4 g, 0.88 mmol) product of **EX-18C** was dissolved in anhydrous acetonitrile (1.5 mL). *R*-(+)-1,1,1-trifluoro-2,3-epoxypropane (0.22 g, 1.94 mmol) and $Yb(OTf)_3$ (22 mg, 0.035 mmol) were added. and the reaction mixture was stirred under $N_2$ at 45 °C in a sealed glass tube for 15 h. The reaction mixture was then cooled to room temperature and diluted with $Et_2O$ and saturated aqueous $NaHCO_3$. The layers were separated and the aqueous layer was extracted with $Et_2O$. The ether layers were combined, washed with brine, dried with anhydrous $Na_2SO_4$, filtered, and concentrated *in vacuo*. The viscous oil was adsorbed onto silica gel and purified by column chromatography eluting with hexanes in ethyl acetate (95:5) which gave 0.32 g (64%) of the desired (2R)-3-[(4-chloro-3-ethylphenoxy)phenyl[[3-(pentafluoroethyl)phenyl]-methyl]amino]-1,1,1-trifluoro-2-propanol product as a viscous. colorless oil. $^1H$ NMR ($CDCl_3$) δ 7.47 (m. 4H), 7.23 (m. 3H), 6.90 (d, 1H), 6.72 (dd, 1H), 6.52 (d, 1H), 6.42 (m, 2H), 4.73 (s, 2H), 4.39 (m, 1H), 3.91 (dd, 1H), 3.58 (m, 2H), 2.73 (q, 2H), 2.57 (s, 1H), 1.22 (t, 3H). $^{19}F$ NMR ($CDCl_3$) δ -79.2 (s, 3F), -84.9 (s, 3F), -115.2 (s, 2F). HRMS calcd. for $C_{26}H_{22}ClF_8NO_2$: 568.1290 $[M+H]^+$, found: 568.1296.

**EXAMPLE 19**

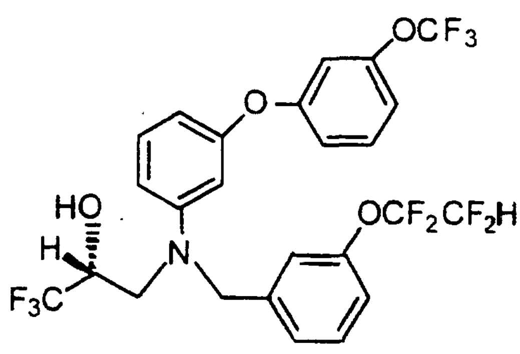

**(2*R*)-3-[[3-(3-trifluoromethoxyphenoxy)phenyl][[3-(1,1,2,2-tetrafluoroethoxy)phenyl]methyl]amino]-1,1,1-trifluoro-2-propanol**

**EX-19A**) To a solution of 1,3-dinitrobenzene (4.5 g, 0.03 mol) and 3-trifluoromethoxy-phenol (4.8 g, 0.03 mol) in 54 mL of dimethylsulfoxide was added cesium carbonate (21.8 g, 0.07 mol). The reaction mixture was heated at 100 °C under nitrogen overnight then cooled to room temperature. The reaction mixture was diluted with water and extracted with diethyl ether several times. The organic layers were combined, washed with 1 N HCl and water. dried over $MgSO_4$, and concentrated *in vacuo*. The crude product was purified by column chromatography on silica gel eluting with 1:9 ethyl acetate in hexane to afford 3.0 g (38%) of the desired 3-(3-trifluoro-methoxyphenoxy)nitrobenzene product as a yellow-orange liquid which was 85% pure by reverse phase HPLC analysis. This material was carried on without further purification.

**EX-19B**) To a solution of 3-(3-trifluoromethoxyphenoxy)nitrobenzene (3.0 g, 0.01 mol) from **EX-19A** in 100 mL of glacial acetic acid was added zinc metal (6.6 g, 0.1 mol) at room temperature, and the resultant mixture was stirred for 1 h. The reaction mixture was filtered through celite. The filtrate was neutralized with ammonium hydroxide and extracted with diethyl ether then ethyl acetate. The combined organic layers were dried over $MgSO_4$ and concentrated *in vacuo*. The crude product was purified by column chromatography on silica gel eluting with 1:9 ethyl acetate in hexane to afford 1.2 g (44%) of the desired 3-(3-trifluoromethoxyphenoxy)aniline product as a yellow oil which was 98% pure by reverse phase HPLC analysis. Anal. calcd. for $C_{13}H_{10}F_3NO_2$: C, 58.00; H, 3.74: N, 5.20. found: C, 57.68; H, 3.57; N, 5.14. HRMS calcd.: 270.0742 $[M+H]^+$, found: 270.0767.

**EX-19C)** To a solution of 3-(3-trifluoromethoxyphenoxy)aniline (1.0 g. 3.7 mmol) from **EX-19B** and 3-(1,1,2,2-tetrafluoroethoxy)benzaldehyde (0.83 g. 3.7 mmol) in 18.5 mL of dichloroethane was added sodium triacetoxyborohydride (1.0 g, 4.7 mmol) and glacial acetic acid (0.25 mL, 4.3 mmol). The reaction mixture was stirred at room temperature overnight then quenched with saturated aqueous sodium bicarbonate and extracted with methylene chloride. The organic layer was dried over $MgSO_4$ and concentrated *in vacuo* to give 1.8 g (100%) of the desired [3-(3-trifluoromethoxy-phenoxy)phenyl][[3-(1,1,2,2-tetrafluoroethoxy)phenyl]methyl] amine product as a yellow oil. which was greater than 90% pure by reverse phase HPLC analysis. HRMS calcd. for $C_{22}H_{16}F_7NO_3$: 476.1097 $[M+H]^+$, found: 476.1069. This material was carried on to the next step without further purification.

A solution of [3-(3-trifluoromethoxyphenoxy)phenyl][[3-(1,1,2,2-tetrafluoroethoxy)-phenyl]methyl]amine (1.8 g, 3.7 mmol) from **EX-19C,** *R*-(+)-1,1,1-trifluoro-2,3-epoxy-propane (0.57 g, 5.2 mmol), and ytterbium (III) trifluoromethanesulfonate (0.24 g, 0.38 mmol) in 2.0 mL of acetonitrile was heated at 40 °C in a sealed glass tube overnight. At this time reverse phase HPLC analysis indicated that the reaction was only 50% complete. Additional ytterbium (III) trifluoromethanesulfonate and *R*-(+)-1,1,1-trifluoro-2,3-epoxypropane (0.26 g. 2,3 mmol) were added to the reaction mixture and again heated at 40 °C in a sealed glass tube for 48 h. The reaction mixture was cooled to room temperature then diluted with water and methylene chloride and extracted. The organic layer was washed with brine, dried over $MgSO_4$, and concentrated *in vacuo*. The crude product was purified by reverse phase HPLC eluting with 30% to 90% acetonitrile in water to afford 1.25 g (23%) of the desired (2*R*)-3-[[3-(3-trifluoromethoxyphenoxy) phenyl][[3-(1,1,2,2-tetrafluoroethoxy)phenyl]-methyl]amino]-1,1,1-trifluoro-2-propanol product as yellow-brown oil (90% ee by chiral HPLC analysis). HRMS calcd. for $C_{25}H_{19}F_{10}NO_4$: 588.1233 $[M+H]^+$, found: 588.1225, $^1H$ NMR ($CDCl_3$) δ 7.35-7.18 (m, 3H), 7.12 (t, 2H), 7.01 (s, 1H), 6.93 (d, 1H), 6.85 (d, 1H), 6.82 (s, 1H), 6.56 (dd, 1H), 6.47 (dd, 1H), 6.41 (s, 1H), 5.88 (t, 1H), 4.66 (s, 2H), 4.35 (m, 1H), 3.86 (d, 1H), 3.59 (dd, 1H), 2.02 (s, 1H). $^{19}F$ NMR ($CDCl_3$) δ -58.31 (s, 3F), -79.24 (d, 3F), -88.57 (m. 2F), - 137.16 (dt. 2F).

**EXAMPLE 20**

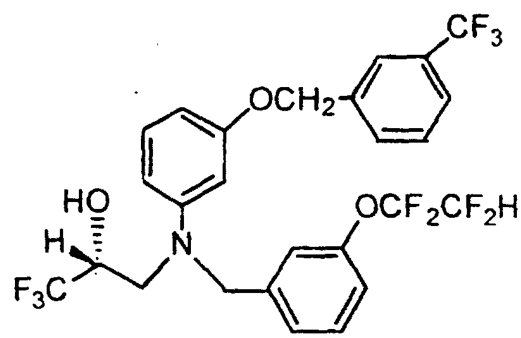

**(2*R*)-3-[[[3-(1,1,2,2-tetrafluoroethoxy)phenyl]methyl][3-[[3-(trifluoromethyl)-phenyl]methoxy]phenyl]amino]-1,1,1-trifluoro-2-propanol**

**EX-20A**) To a solution of 3-aminophenol (4.91 g. 45.0 mmol) and 3-(1,1,2.2-tetrafluoroethoxy)benzaldehyde (10.0 g, 45.0 mmol) in 100 mL of 1,2-dichloroethane was added sodium triacetoxyborohydride (14.28 g 67.5 mmol) and glacial acetic acid (2.7 mL. 47.3 mmol). The reaction mixture was stirred at room temperature for 6 h then quenched with water and extracted with dichloromethane. The organic layer was washed with saturated aqueous sodium bicarbonate, dried over $MgSO_4$, and concentrated *in vacuo* to give 11.82 g (83%) of the desired 3-[[[3-(1,1,2,2-tetrafluoroethoxy)phenyl]methyl]amino]phenol product as a dark orange oil. $^1$H NMR (acetone-$d_6$) δ 7.01-7.38 (m, 5H), 6.26-6.44 (m. 3H), 6.08 (t, 1H), 5.88 (tt, 1H), 4.34 (s, 2H).

**EX-20B**) A solution of 3-[[[3-(1,1,2,2-tetrafluoroethoxy)phenyl]methyl]amino] phenol (5.1 g, 16.2 mmol) from **EX-20A,** *R*-(+)-1,1,1-trifluoro-2,3-epoxypropane (1.5 mL, 17.4 mmol), and ytterbium trifluoromethanesulfonate (1.0 g, 10 mol%) in 10 mL of acetonitrile was heated at 50 °C in a sealed glass tube for 4 h. The reaction mixture was cooled to room temperature, then diluted with water and diethyl ether and extracted. The ether layer was washed with saturated aqueous sodium bicarbonate and brine, dried over $MgSO_4$, and concentrated *in vacuo* to give 5.64 g (81%) of the desired (2*R*)-3-[[[3-(1,1,2,2-tetrafluoroethoxy] phenyl]methyl][3,3,3-trifluoro-2-hydroxy-propyl)amino]phenol product as a yellow oil. $^1$H NMR (acetone-$d_6$) δ 7.41 (t, 1H), 7.23 (d, 1H), 7.16-7.20 (m, 2H), 6.97 (t, 1H), 6.42 (tt, 1H), 6.18-6.24 (m, 3H), 4.77 (s, 2H), 4.43-4.48 (m, 1H), 3.58 (dd, 1H), 3.39 (dd, 1H).

To a solution of (2*R*)-3-[[[3-(1,1,2,2-tetrafluoroethoxy]phenyl]methyl][3,3,3-trifluoro-2-hydroxypropyl)amino] phenol (100 mg, 0.23 mmol) from **EX-20B** and 3-trifluoromethylbenzyl bromide (70.0 mg. 0.27 mmol) in 2.5 mL of acetone was added cesium carbonate (100 mg, 0.31 mmol). The reaction mixture was heated at 60 °C for 18 h then cooled to room temperature. The reaction mixture was filtered through celite, and the filtrate was concentrated. The residue was purified by reverse phase HPLC eluting with 50% to 90% acetonitrile in water to afford 63.3 mg (45%) of the desired (2*R*)-3-[[[3-(1,1,2,2-tetrafluoroethoxy)phenyl]methyl][3-[[3-(trifluoro-methyl)phenyl]methoxy]phenyl]amino]-1,1,1-trifluoro-2-propanol product as an orange oil. HRMS calcd. for $C_{26}H_{21}F_{10}NO_3$: 586.1440 $[M+H]^+$, found: 586.1419. $^1$H NMR (acetone-$d_6$) δ 7.61-7.82 (m, 4H), 7.41 (t, 1H), 7.25 (d, 1H), 7.10-7.21 (m, 3H), 6.34-6.67 (m, 4H), 5.73 (d, 1H), 5.19 (s, 2H), 4.82 (s, 2H), 4.34-4.48 (m, 1H), 3.99 (dd, 1H), 3.68 (dd, 1H).

Additional examples of (2*R*)-3-[[[3-(1,1,2,2-tetrafluoroethoxy)phenyl]methyl]-[3-[[aryl]methoxy]phenyl]amino]-1,1,1-trifluoro-2-propanols are prepared by one skilled in the art using similar methods. as shown in, Example Table 4.

Example Table 4. (*2R*)-3-[[[3-(1,1,2.2-tetrafluoroethoxy)phenyl]methyl][3-[[aryl]methoxy]phenyl]amino]-1.1.1-trifluoro-2-propanols.

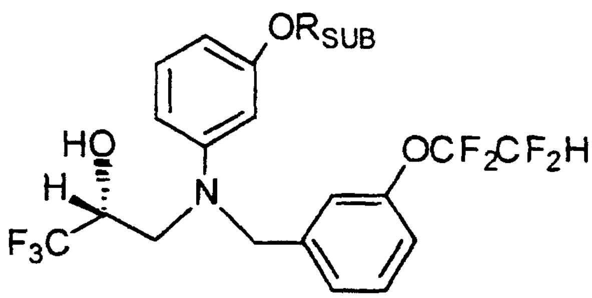

| Ex. No. | $R_{SUB}$ | Calculated Mass $[M+H]^+$ | Observed Mass $[M+H]^+$ |
|---|---|---|---|
| 21 | 3,5-difluorobenzyl | 554.1378 | 554.1352 |
| 22 | 3-trifluoromethoxybenzyl | 602.1389 | 602.1390 |
| 23 | 3-isopropyl | 470.1566 | 464.1601 |

**EXAMPLE 24**

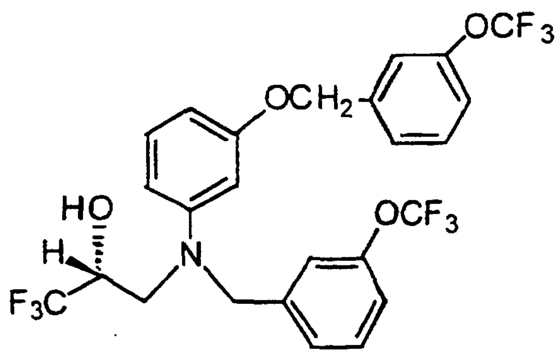

**(2*R*)-3-[[3-[[3-(trifluoromethoxy)phenyl]methoxy]phenyl][[3-(trifluoromethoxy)-phenyl]methyl]amino]-1,1,1-trifluoro-2-propanol**

(2*R*)-3-[[3-[[3-(trifluoromethoxy)phenyl]methoxy]phenyl][[3-(trifluoromethoxy)phenyl]methyl]amino]-1,1,1-trifluoro-2-propanol can be prepared by one skilled in the art using similar methods starting from 3-(trifluoromethoxy)-benzaldehyde. HRMS calcd. for $C_{25}H_{20}F_9NO_4$: 570.1327 $[M+H]^+$, found: 570.1325. $^1H$ NMR (acetone-$d_6$) δ 7.43 (t, 1H), 7.32 (d, 1H), 7.18-7.23 (m, 2H), 7.01-7.16 (m, 3H), 6.92-7.00 (m, 1H), 6.38-6.45 (m, 3H), 5.12 (s, 2H), 4.81 (s, 2H), 4.41-4.53 (m, 1H), 3.98 (dd, 1H), 3.63 (dd, 1H).

Additional examples of (2*R*)-3-[[3-[[aryl]methoxy]phenyl][[3-(trifluoromethoxy)phenyl]methyl]amino]-1,1,1-tri-

fluoro-2-propanols can be prepared by one skilled in the art using similar methods, as shown in Example Table 5.

Example Table 5. (*2R*)- 3-[[3-[[aryl]methoxy]phenyl][[3-(trifluoromethoxy)-phenyl]methyl]amino]-1,1,1-trifluoro-2-propanols.

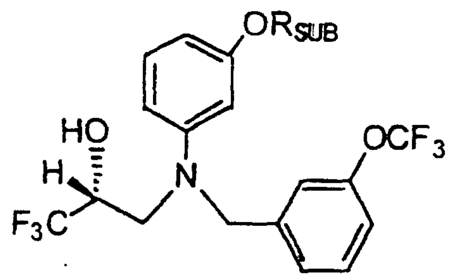

| Ex. No. | $R_{SUB}$ | Calculated Mass $[M+H]^+$ | Observed Mass $[M+H]^+$ |
|---|---|---|---|
| 25 | 4-trifluoromethoxybenzyl | 570.1327 | 570.1299 |
| 26 | 3,5-di(trifluoromethyl)benzyl | 622.1252 | 622.1252 |
| 27 | 3-trifluoromethylbenzyl | 554.1378 | 554.1369 |
| 28 | 3,5-difluorobenzyl | 522.1315 | 522.1259 |
| 29 | benzyl | 486.1504 | 486.1504 |
| 30 | isopropyl | 438.1504 | 438.1509 |
| 31 | cyclohexylmethyl | 492.1973 | 492.1973 |
| 32 | cyclopentyl | 464.1660 | 464.1641 |

**EXAMPLE 33**

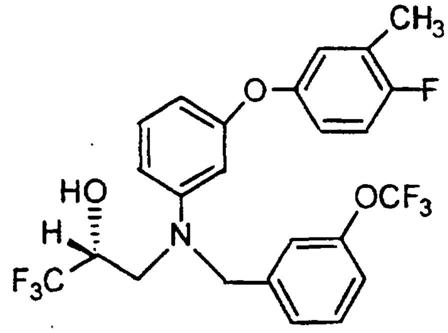

**(2*R*)-3-[[3-(4-fluoro-3-methylphenoxy)phenyl][[3-(trifluoromethoxy)phenyl]methyl]amino]-1,1,1-trifluoro-2-propanol**

**EX-33A**) To a solution of 3-bromoaniline (5.7 mL, 52.6 mmol) and 3-trifluoromethoxybenzaldehyde (10.0 g, 52.6 mmol) in 135 mL of dichloroethane was added sodium triacetoxyborohydride (14.5 g, 68.4 mmol) and glacial acetic acid (3.1 mL, 54.7 mmol). The reaction was stirred at room temperature for 2 h, then quenched with water and extracted with dichloromethane. The organic layer was washed with saturated aqueous sodium bicarbonate, dried over $MgSO_4$, and concentrated *in vacuo*. The crude product was purified by column chromatography on silica gel eluting with 1:9 ethyl acetate in hexane to give 14.3 g (78%) of the desired of *N*-(3-bromophenyl)[[3-(trifluoromethoxy) phenyl] methyl]amine product as a dark brown oil. HRMS calcd. for $C_{14}H_{11}BrF_3NO$: 346.0055 $[M+H]^+$, found: 346.0052.

**EX-33B**) A solution of of *N*-(3-bromophenyl)[[3-(trifluoromethoxy)phenyl]methyl]-amine (10.0 g, 28.9 mmol) from **EX-33A,** *R*-(+)-1,1,1-trifluoro-2,3-epoxypropane (4.2 g, 37.6 mmol), and ytterbium (III) trifluoromethanesulfonate (1.79 g, 2.89 mmol) in 27 mL of acetonitrile was heated at 50 °C in a sealed glass tube overnight. The reaction mixture was cooled to room temperature and filtered through celite. The crude product was purified by column chromatography on silica gel eluting with 2:3 dichloromethane in hexane to afford 11.9 g (90%) of the desired (2*R*)-3-[[(3-bromophenyl)][[3-(trifluoromethoxy)phenyl]methyl]amino]-1,1,1-trifluoro-2-propanol product as a brown oil (98% ee by chiral HPLC analysis). HRMS calcd. for $C_{17}H_{14}BrF_6NO_2$: 458.0190 $[M+H]^+$, found: 458.0197.

A suspension of 4-fluoro-3-methylphenol (98.0 µL, 0.88 mmol) and cesium carbonate (319.5 mg, 0.98 mmol) in 1 mL of *N*,*N*-dimethylacetamide was preheated at 60 °C for 5 minutes. To this solution was added 4 mL of a stock solution containing (2*R*)-3-[[(3-bromophenyl)][[3-(trifluoromethoxy)phenyl] methyl]amino]-1,1,1-trifluoro-2-propanol (200 mg, 0.437 mmol) from **EX-33B,** 1-naphthoic acid (164 mg, 0.95 mmol). copper(I) trifluoromethansulfonate benzene complex (21.8 mg, 0.0434 mmol), 4 Å sieves (105 mg), and 4 mL of toluene. The reaction mixture was stirred at 105 °C for 3 weeks and 2 days. During that time, additional cesium carbonate and catalyst were added (a spatula tip of each) to the reaction three different times. The reaction was cooled to room temperature, filtered through celite, and the solvent was evaporated. The residue was purified by reverse phase HPLC eluting with 35% to 90% acetonitrile in water to afford 50.5 mg (23%) of the desired (2*R*)-3-[[3-(4-fluoro-3-methylphenoxy)phenyl][[3-(trifluoromethoxy)phenyl] methyl]amino]-1,1,1-trifluoro-2-propanol product as an orange oil. HRMS calcd. for $C_{24}H_{20}F_7NO_3$: 504.1410 $[M+H]^+$, found: 504.1389. $^1H$ NMR (acetone-$d_6$) δ 7.44 (t, 1H), 7.24 (d, 1H), 7.08-7.21 (m, 3H), 6.98 (t, 1H), 6.75-6.85 (m, 1H), 6.68-6.74 (m, 1H), 6.53 (d, 1H), 6.21-6.34 (m, 2H), 4.79 (t, 2H), 4.46-4.53 (m, 1H), 3.95 (dd, 1H), 2.61-2.72 (m, 1H), 2.20 (s, 3H).

Additional examples (2*R*)-3-[[(aryloxy)phenyl][[3-(trifluoromethoxy )phenyl] methyl]amino]-1,1,1-trifluoro-2-propanols can be prepared by one skilled in the art using similar methods, as shown in Example Table 6.

Example Table 6. (2*R*)-3-[[(aryloxy)phenyl][[3-(trifluoromethoxy)phenyl]-methyl]amino]-1,1,1-trifluoro-2-propanols.

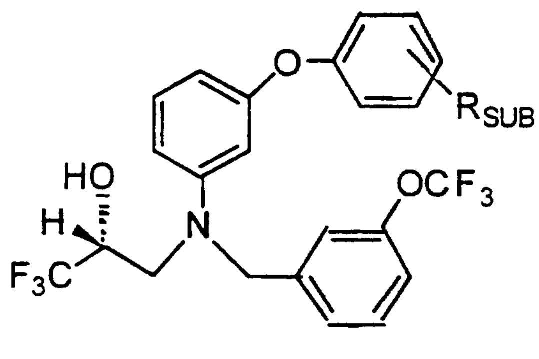

| Ex. No. | $R_{SUB}$ | Calculated Mass $[M+H]^+$ | Observed Mass $[M+H]^+$ |
|---|---|---|---|
| 34 | 3-trifluoromethoxy | 556.1170 | 556.1180 |
| 35 | 3-isopropyl | 514.1817 | 514.1823 |
| 36 | 3,4-dimethyl | 500.1660 | 500.1654 |
| 37 | 4-chloro-3-methyl | 520.1114 | 520.1129 |
| 38 | 3-*tert*-butyl | 528.1973 | 528.1942 |
| 39 | 3,4-dichloro | 540.0568 | 540.0567 |
| 40 | 3,4-($CH_2CH_2CH_2CH_2$)- | 526.1817 | 526.1788 |

**EXAMPLE 41**

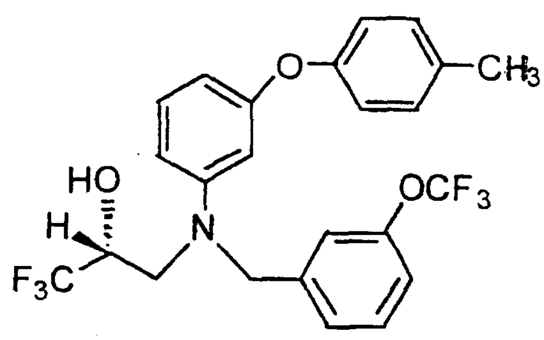

**(2*R*)-3-[[3-(4-methylphenoxy)phenyl][[3-(trifluoromethoxy)phenyl] methyl]amino]-1,1,1-trifluoro-2-propanol**

**EX-41A**) To a solution of *p*-cresol (5.76 g, 0.053 mol) and 1,3-dinitrobenzene (8.97 g, 0.053 mol) in 100 mL of dimethylsulfoxide was added cesium carbonate (43.4 g, 0.133 mol). The reaction mixture was heated at 100 °C for

18 h, then cooled to room temperature, quenched with water, and extracted with diethyl ether. The organic layers were combined, washed with 0.1 N HCl and water, dried over $MgSO_4$ and concentrated *in vacuo*. The crude product was purified by column chromatography on silica gel eluting with 1:4 ethyl acetate in hexane to afford 8.0 g (66%) of the desired 3-(4-methylphenoxy)nitrobenzene product as a yellow oil. $^1H$ NMR ($CDCl_3$) δ 7.83 (s, 1H), 7.64 (t, 1H), 7.32 (d, 1H), 7.18 (d, 1H), 7.09 (d, 2H), 6.8 (d, 2H), 2.20 (s, 1H).

**EX-41B)** A solution of 3-(4-methylphenoxy)nitrobenzene (8.0 g, 0.035 mol) from **EX-41A** in 25 mL of ethanol under nitrogen was charged with 10% palladium on carbon (0.80 g). The resulting mixture was hydrogenated for 4 h at room temperature and 45 psi. The reaction mixture was filtered through celite and concentrated *in vacuo* to give 6.7 g (96%) of the desired 3-(4-methylphenoxy)aniline product as a yellow oil. ESMS *m*/*z* = 200 $[M+H]^+$ confirmed the desired $C_{13}H_{13}NO$ product and the complete consumption of starting material.

**EX-41C**) To a solution of 3-(4-methylphenoxy)aniline (2.91 g, 0.015 mol) from **EX-41B**, and 3-(trifluoromethoxy)benzyaldehyde (3.24 g, 0.015 mol) in 50 mL dichloroethane was added sodium triacetoxyborohydride (4.02 g, 0.019 mol) and glacial acetic acid (0.99 g, 0.017 mol). The reaction mixture was stirred at room temperature for 18 h, then quenched with saturated aqueous sodium bicarbonate and extracted with dichloromethane. The organic layers were combined, dried over $MgSO_4$ and concentrated *in vacuo* to give 5.38 g (91%) of the desired *N*-[3-(4-methylphenoxy)-phenyl)]-[[3-(trifluoromethoxy)phenyl]methyl]amine product as an orange oil. ESMS *m*/*z* = 374 $[M+H]^+$ confirmed the desired $C_{21}H_{18}NO_2F_3$ product and the complete consumption of starting material.

To a mixture of *N*-[3-(4-methylphenoxy)phenyl)]-[[3-(trifluoromethoxy)phenyl]-methyl]amine(1.3 g, 0.0035 mol) from **EX-41C** and *R*-(+)-1,1,1-trifluoro-2,3-epoxypropane (0.59 g, 0.0053 mol) was added a suspension of ytterbium (III) trifluoromethanesulfonate (0.22 g, 0.0004 mol) in 1.3 mL of acetonitrile. The resulting mixture was heated at 50 °C in a sealed glass tube for 18 h. The reaction mixture was cooled to room temperature, then diluted with water and extracted with ethyl acetate. The crude product was purified by column chromatography on silica gel eluting with 1:4 ethyl acetate in hexane to afford 1.03 g (61%) of the desired (2*R*)-3-[3-(4-methyl-phenoxy)phenyl)[[3-(trifluoromethoxy)phenyl] methyl]amino]-1,1,1-trifluoro-2-propa-nol product as a pure yellow oil. Anal. calcd. for $C_{24}H_{21}F_6NO_3$: C, 59.38; H, 4.36; N, 2.89. Found: C, 59.17; H, 4.62; N, 2.80. HRMS calcd.: 486.1504 $[M+H]^+$, found: 486.1513, $^1H$ NMR ($C_6D_6$) δ 6.82 (m, 8H), 6.60 (dd, 1H), 6.42 (dd, 1H), 6.38 (s, 1H),. 6.18 (dd, 1H), 4.00 (s, 2H), 3.63 (m, 1H), 3.40 (d, 1H), 3.02 (m, 1H), 2.00 (s, 3H), 1.40 (d, 1H). $^{19}F$ NMR ($C_6D_6$) δ -57.98 (s, 3F), -78.50 (s. 3F).

Additional examples of (2*R*)-3-[3-(substituted-phenoxy)phenyl]-[[3-(trifluoro-methoxy)phenyl]methyl]amino]-1,1,1-trifluoro-2-propanols can prepared by one skilled in the art using similar methods. as shown in Example Table 7.

Example Table 7. (2*R*)- 3-[3-(substituted-phenoxy)phenyl][[3-(trifluoromethoxy)-phenyl]methyl]amino]-1.1.1-trifluoro-2-propanols.

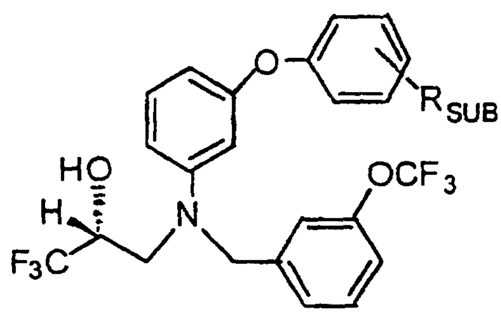

| Ex. No. | RSUB | Calculated Mass $[M+H]^+$ | Observed Mass $[M+H]^+$ |
|---|---|---|---|
| 42 | 4-fluoro | 490.1253 | 490.1238 |

**EXAMPLE 43**

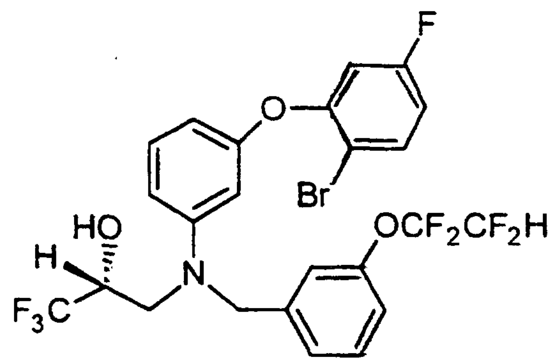

**(2*R*)-3-[[3-(2-bromo-5-fluorophenoxy)phenyl][[3-(1,1,2,2-tetrafluoroethoxy)phenyl]methyl]amino]-1,1,1-trifluoro-2-propanol**

**EX-43A**) To a solution of 3-aminophenol (5 g, 46 mmol), 1-bromo-2,4-difluorobenzene (10 g, 50 mmol) and $Cs_2CO_3$ (16 g, 50 mmol) in 25 mL of dimethylformamide was added solid $(CuOTf)_2C_6H_6$ (100 mg), and the mixture was stirred under nitrogen at 85 °C for 22 h, at which time HPLC analysis indicated that the reaction had gone to completion and formed two products. The DMF was removed under reduced pressure. The residue was diluted with ether and filtered through a celite pad. The pad was washed with ether and a small amount of water. The mixture was extracted with ether several times. The combined ether layers were washed with water and brine, then dried over $MgSO_4$. The dried organic layer was evaporated to give 10.2 g (80%) of the desired product, which consisted of a 11:1 ratio of 3-(2-bromo-5-fluoro-phenoxy)aniline and 3-(4-bromo-3-fluorophenoxy)aniline. The crude product was purified by flash column chromatography on silica gel eluting with 1:7:0.01 of ethyl acetate:hexane:ammonium hydroxide to give 8.8 g (68%) of the desired product as a yellow oil, which was a 25:1 ratio of 3-(2-bromo-5-fluorophenoxy)aniline and 3-(4-bromo-3-fluorophenoxy)aniline. HRMS calcd. for $C_{12}H_9NOFBr$: 281.9930 $[M+H]^+$, found: 281.9950.

**EX-43B**) The 3-(2-bromo-5-fluorophenoxy)aniline (1.39 g, 4.95 mmol) product from **EX-43A** and 3-(1,1,2,2-tetrafluoroethoxy)benzaldehyde (1.0 g, 4.5 mmol) were dissolved in 15 mL of dichloroethane and acetic acid (0.30 mL, 5.4 mmol), then solid $NaBH(OAc)_3$ (1.26 g, 5.9 mmol) was added. The mixture was stirred at room temperature for 1 h, then quenched with water and extracted with ether. The ether layer was washed with water and brine, then dried over $MgSO_4$, and evaporated to give 2.1 g (97%) of crude product, which was purified by flash column chromatography on silica gel eluting with 1:7:0.01 of ethyl acetate:hexane:ammonium hydroxide to give 2.0 g (91%) of the desired 3-[3-(2-bromo-5-fluoro-phenoxy)phenyl][[3-(1,1,2,2-tetrafluoroethoxy)phenyl]methyl]amine product. as a light yellow oil, > 90% pure by HPLC analysis. HRMS calcd. for $C_{21}H_{15}NO_2BrF_5$: 488.0285 $[M+H]^+$, found: 488.0269.

The 3-[3-(2-bromo-5-fluorophenoxy)phenyl][[3-(1,1,2,2-tetrafluoroethoxy)-phenyl]-methyl]amine (0.5 g, 2.0 mmol) product from **EX-43B** and *R*-(+)-1,1,1-trifluoro-2,3-epoxypropane (0.17 g, 2.0 mmol) from **EX-4** were dissolved in 0.5 mL of acetonitrile. Ytterbium (III) trifluoromethanesulfonate (0.06 g, 0.1 mmol) was added, and the stirred solution was warmed to 40 °C for 1 h, at which time HPLC analysis indicated that no secondary amine starting material remained. The reaction was quenched with water and extracted with ether. The ether layer was washed with water and brine. then dried over $MgSO_4$. The crude product was purified by flash column chromatography on silica gel eluting with 1:7:0.01 of ethyl acetate:hexane:ammonium hydroxide to give 0.4 g (67%) of the desired *R*-(+)-3-[[3-(2-bromo-5-fluorophenoxy)phenyl][[3-(1,1,2,2-tetrafluoroethoxy)phenyl]methyl]amino]-1,1,1-trifluoro-2-propanol product as a light yellow oil (> 84% ee by chiral HPLC analysis). Anal. calcd. for $C_{24}H_{18}BrF_8NO_3$: C, 48.02; H, 3.02; N, 2.33. found: C, 48.07; H, 3.14: N. 2.31. HRMS calcd.: 600.0420 $[M+H]^+$, found: 600.0386. $^1H$ NMR ($CDCl_3$) δ 7.5 0 (dd, 1H), 7.30 (t, 1H), 7.18 (t, 1H), 7.07 (t, 2H), 6.99 (s, 1H), 6.70 (dt, 1H), 6.56 (dd, 1H), 6.52 (dd, 1H), 6.38 (dd, 1H), 6.32 (m, 1H), 5.87 (tt, 1H,), 4.65 (d, 2H). 4.33 (m, 1H), 3.85 (dd, 1H), 3.56 (dd, 1H), 2.48 (bs, 1H). NOE difference spectra confirmed that the isolated material was the indicated *N*-[3-(2-bromo-5-fluorophenoxy)phenyl]-3-aminopropanol product. $^{19}F$ NMR ($CDCl_3$) δ -79.24 (d, 3F), -88.57 (m, 2F), -112.04 (q, 1H), -137.16 (dt, 2F).

**EXAMPLE 44**

(Included for reference and/or comparison only)

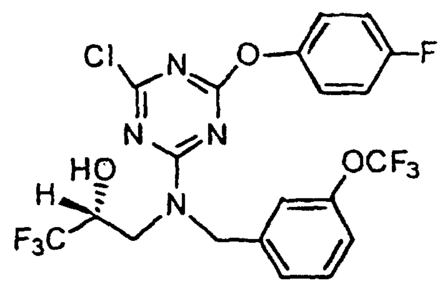

**(2*R*)-*N*-[2-chloro-6-(p-fluorophenoxy)-1,3,5-triazin-4-yl]-3-[[[3-(trifluoromethoxy)phenyl]methyl]amino]-1,1,1-trifluoro-2-propanol**

**EX-44A)** 3-Trifluoromethoxybenzenemethanamine (1.15g, 6 mmol) and *R*-(+)-1,1,1-trifluoro-2,3-epoxypropane (0.67 g, 6 mmol) were combined and stirred at 80 °C for 1.5 h. The mixture was cooled to room temperature, and the resulting solid was recrystallized from hot hexanes. The white solid was isolated by vacuum filtration and washed with cold hexanes to give 0.67 g (37%) of pure (2*R*)-3-[[[3-(trifluoro-methoxy)phenyl]methyl]amino]-1,1,1-trifluoro-2-propanol. $^1H$ NMR ($CDCl_3$) δ 7.37 (t, 1H), 7.24 (d, 1H), 7.15 (m, 2H), 3.99 (m, 1H), 3.85 (d, 2H), 2.98 (dd, 1H), 2.88 (dd, 1H), 2.79 (s, 1H). $^{19}F$ NMR ($CDCl_3$) δ - 58.19 (s, 3F), -78.88 (s, 3F). HRMS calcd, for $C_{11}H_{11}F_6NO_2$: 304.0772 $[M+H]^+$, found: 304.0794.

**EX-44B**) To a solution of p-fluorophenol 1.00 g (8.92 mmol) in 30 mL of tetrahydrofuran at 0 °C was added a 60% dispersion of sodium hydride in mineral oil (0.36 g, 8.92 mmol). After 30 min, cyanuric chloride (1.64 g, 8.92 mmol) was added as a heterogeneous mixture in tetrahydrofuran at 0 °C. The reaction mixture was allowed to slowly warm to room temperature. After 14 h, the mixture was cooled to 0 °C. and a saturated aqueous $NH_4Cl$ solution was added. The aqueous solution was extracted with diethyl ether (3 x 50 mL). The combined ether extracts were washed with brine. dried ($MgSO_4$), and concentrated *in vacuo* to afford 1.34 g (58%) of the desired 2.4-dichloro-6-(4-fluorophenoxy)-1,3,5-triazine product as an off white solid which was taken on to the next step without purification. MS *m*/*z* = 260 $[M+H]^+$.

To a stirred solution of aminopropanol from **EX-44A** (0.100 g, 0.330 mmol) in *N*,*N*-dimethylformamide at 0 °C was added the 2,4-dichloro-(4-fluorophenoxy)-1,3,5-triazine ether product from **EX-44B** (0.086 g, 0.330 mmol) as a solution in *N*,*N*-di-methylformamide. The reaction mixture was allowed to slowly warm to room temperature. After 14 h, the reaction mixture was cooled to 0 °C, and a saturated aq. $NaHCO_3$ solution was added. After stirring the reaction mixture for 30 min at room temperature, the aqueous layer was extracted with ether (3 x 30 mL). The combined ether extracts were washed with brine, dried ($MgSO_4$), and concentrated *in vacuo* to give a yellow oil. The crude residue was purified by column chromatography on silica gel eluting with 20 % ethyl acetate in hexanes to give 0.075 g (43%) of the desired (2*R*)-*N*-[2-chloro-6-(p-fluorophenoxy)-1,3,5-triazin-4-yl]-3-[[[3-(trifluoromethoxy)-phenyl]methyl]amino]-1,1,1-trifluoro-2-propanol product as a pale yellow oil. HRMS calcd. for $C_{20}H_{14}ClF_7N_4O_3$: 526.0643 $[M^+]$, found: 526.0632. $^1H$ NMR ($C_6D_6$) δ 6.95 (s, 1H), 6.63 (m, 14H), 4.74 (d, 1H), 4.37 (d, 1H), 4.16 (d, 1H), 4.00 (d, 2H), 3.73 (m, 1H), 3.48 (m, 2H), 3.26 (m, 2H), 3.12 (m, 2H).

Based on the preceding procedures, additional substituted (2*R*)-3-[(*N*-aryl)-[[aryl]methyl]amino]-1,1,1-trifluoro-2-propanols are prepared by one skilled in the art using similar methods, as shown in Example Table 8. Substituted (3*R*)-4-[*N*-(aryl)-[(aryl)methyl]amino]-1,1,1,2,2-pentafluoro-3-butanols are prepared by one skilled in the art using similar methods, as shown in Example Table 9. Substituted (2*R*)-3-[*N*-(aryl)[(aryl)oxy]amino]-1,1,1-trifluoro-2-propanois are prepared by one skilled in the art using similar methods, as shown in Example Table 10. Substituted (2*R*)-3-[*N*-(aryl) -[(aryl)methyl]amino]-1,1-difluoro-1-chloro-2-propanols are prepared by one skilled in the art using similar methods, as shown in Example Table 11. Substituted (2*R*)-3-[*N*,*N'*-(diaryl)amino]-1,1,1-trifluoro-2-propanols are prepared by one skilled in the art using similar methods. as shown in Example Table 12.

Example Table 8. Substituted (2*R*)-3-[*N*-(aryl)-[(aryl)methyl]amino]-1,1,1-trifluoro-2-propanols.

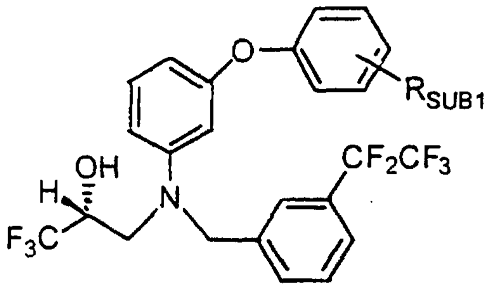

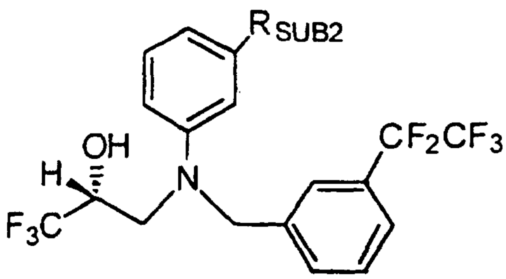

| Ex. No. | $R_{SUB1}$ |
|---|---|
| 45 | 3-isopropyl |
| 46 | 2-Cl, 3-Cl |
| 47 | 3-$CF_3O$ |
| 48 | 4-F |
| 49 | 4-$CH_3$ |
| 50 | 2-F, 5-Br |
| 51 | 3-$CF_3CF_2$ |
| 52 | 3-$CH_3CH_2$ |
| 53 | 3-$CH_3$, 5-$CH_3$ |
| 54 | 3-$(CH_3)_3C$ |
| 55 | 4-F, 3-$CH_3$ |
| 56 | 3-Cl, 4-Cl |
| 57 | 3,4-$(CH_2)_4$ |
| 58 | 3-$HCF_2CF_2O$ |
| 59 | 3-$CHF_2O$ |
| 60 | 3-$(CH_3)_2N$ |
| 61 | 3-cyclopropyl |
| 62 | 3-(2-furyl) |
| 63 | 3-$CF_3CF_2$ |
| 64 | 4-$NH_2$ |
| 65 | 3-$CH_3$, 4-$CH_3$, 5-$CH_3$ |

| Ex. No. | $R_{SUB2}$ |
|---|---|
| 69 | 3-$CF_3O$-benzyloxy |
| 70 | 3-$CF_3$-benzyloxy |
| 71 | 3-F, 5-F-benzyloxy |
| 72 | cyclohexylmethyleneoxy |
| 73 | benzyloxy |
| 74 | 3-$CF_3$, 5-$CF_3$-benzyloxy |
| 75 | 4-$CF_3O$-benzyloxy |
| 76 | 4-$CH_3CH_2$-benzyloxy |
| 77 | isopropoxy |
| 78 | 3-$CF_3$-benzyl |
| 79 | isopropylthio |
| 80 | cyclopentoxy |
| 81 | 3-Cl-5-pyridinyloxy |
| 82 | 3-$CF_3S$-benzyloxy |
| 83 | 3-$CH_3$, 4-$CH_3$-benzyloxy |
| 84 | 2-F, 3-$CF_3$-benzyloxy |
| 85 | 3-F, 5-$CF_3$-benzyloxy |
| 86 | 4-$(CH_3)_2CH$-benzyloxy |
| 87 | 1-phenylethoxy |
| 88 | 4-F, 3-$CH_3$-benzoyl |
| 89 | 3-$CF_3$-phenyl |

Example Table 8 (continued). Substituted (2*R*)-3-[*N*-(aryl)-[(aryl)methyl] amino]-1,1,1-trifluoro-2-propanols.

| Ex. No. | $R_{SUB1}$ |
|---|---|
| 66 | $4\text{-}CH_3CH_2CH_2O$ |
| 67 | $3\text{-}CF_3$ |
| 68 | $2\text{-}NO_2$ |

| Ex. No. | $R_{SUB2}$ |
|---|---|
| 90 | $4\text{-}CH_3O$-phenylamino |
| 91 | cyclopropoxy |
| 92 | $4\text{-}NO_2$-phenylthio |

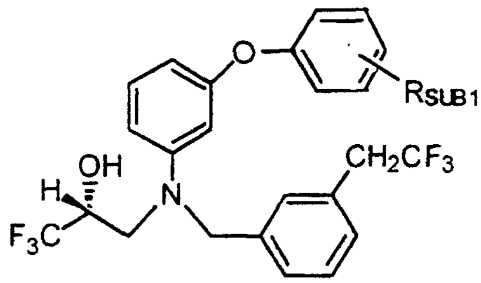

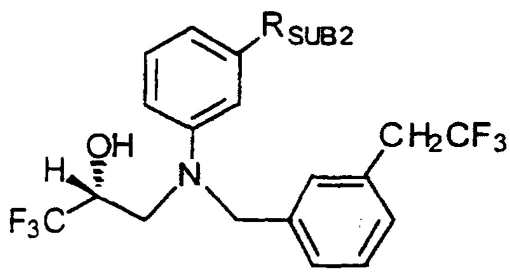

| Ex. No. | $R_{SUB1}$ |
|---|---|
| 93 | 3-isopropyl |
| 94 | 2-Cl, 3-Cl |
| 95 | $3\text{-}CF_3O$ |
| 96 | 4-F |
| 97 | $4\text{-}CH_3$ |
| 98 | 2-F, 5-Br |
| 99 | 4-Cl, $3\text{-}CH_3CH_2$ |
| 100 | $3\text{-}CH_3CH_2$ |
| 101 | $3\text{-}CH_3$, $5\text{-}CH_3$ |
| 102 | $3\text{-}(CH_3)_3C$ |
| 103 | 4-F, $3\text{-}CH_3$ |
| 104 | 3-Cl, 4-Cl |
| 105 | $3,4\text{-}(CH_2)_4$ |
| 106 | $3\text{-}HCF_2CF_2O$ |
| 107 | $3\text{-}CHF_2O$ |

| Ex. No. | $R_{SUB2}$ |
|---|---|
| 117 | $3\text{-}CF_3O$-benzyloxy |
| 118 | $3\text{-}CF_3$-benzyloxy |
| 119 | 3-F, 5-F-benzyloxy |
| 120 | cyclohexylmethyleneoxy |
| 121 | benzyloxy |
| 122 | $3\text{-}CF_3$, $5\text{-}CF_3$-benzyloxy |
| 123 | $4\text{-}CF_3O$-benzyloxy |
| 124 | $4\text{-}CH_3CH_2$-benzyloxy |
| 125 | isopropoxy |
| 126 | $3\text{-}CF_3$-benzyl |
| 127 | isopropylthio |
| 128 | cyclopentoxy |
| 129 | 3-Cl-5-pyridinyloxy |
| 130 | $3\text{-}CF_3S$-benzyloxy |
| 131 | $3\text{-}CH_3$, $4\text{-}CH_3$-benzyloxy |

Example Table 8 (continued). Substituted (2*R*)-3-[*N*-(aryl)-[(aryl)methyl] amino]-1,1,1-trifluoro-2-propanols.

| Ex. No. | $R_{SUB1}$ |
|---|---|
| 108 | 3-$(CH_3)_2N$ |
| 109 | 3-cyclopropyl |
| 110 | 3-(2-furyl) |
| 111 | 3-$CF_3CF_2$ |
| 112 | 4-$NH_2$ |
| 113 | 3-$CH_3$, 4-$CH_3$, 5-$CH_3$ |
| 114 | 4-$CH_3CH_2CH_2O$ |
| 115 | 3-$CF_3$ |
| 116 | 2-$NO_2$ |

| Ex. No. | $R_{SUB2}$ |
|---|---|
| 132 | 2-F, 3-$CF_3$-benzyloxy |
| 133 | 3-F, 5-$CF_3$-benzyloxy |
| 134 | 4-$(CH_3)_2CH$-benzyloxy |
| 135 | 1-phenylethoxy |
| 136 | 4-F, 3-$CH_3$-benzoyl |
| 137 | 3-$CF_3$-phenyl |
| 138 | 4-$CH_3O$-phenylamino |
| 139 | cyclopropoxy |
| 140 | 4-$NO_2$-phenylthio |

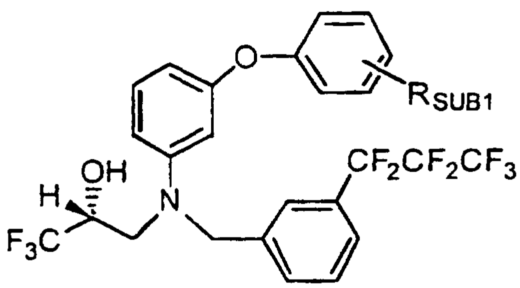

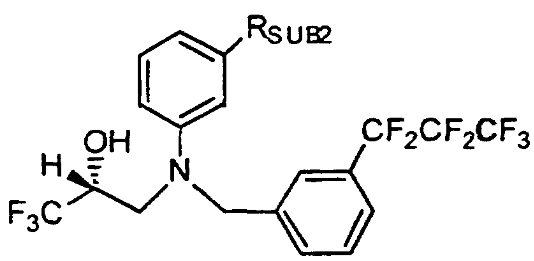

| Ex. No. | $R_{SUB1}$ |
|---|---|
| 141 | 3-isopropyl |
| 142 | 2-Cl, 3-Cl |
| 143 | 3-$CF_3O$ |
| 144 | 4-F |
| 145 | 4-$CH_3$ |
| 146 | 2-F, 5-Br |
| 147 | 4-Cl, 3-$CH_3CH_2$ |
| 148 | 3-$CH_3CH_2$ |

| Ex. No. | $R_{SUB2}$ |
|---|---|
| 165 | 3-$CF_3O$-benzyloxy |
| 166 | 3-$CF_3$-benzyloxy |
| 167 | 3-F, 5-F-benzyloxy |
| 168 | cyclohexylmethyleneoxy |
| 169 | benzyloxy |
| 170 | 3-$CF_3$, 5-$CF_3$-benzyloxy |
| 171 | 4-$CF_3O$-benzyloxy |
| 172 | 4-$CH_3CH_2$-benzyloxy |

Example Table 8 (continued). Substituted (2*R*)-3-[*N*-(aryl)-[(aryl)methyl] amino]-1,1,1-trifluoro-2-propanols.

| Ex. No. | RSUB1 | Ex. No. | RSUB2 |
|---|---|---|---|
| 149 | 3-$CH_3$, 5-$CH_3$ | 173 | isopropoxy |
| 150 | 3-$(CH_3)_3C$ | 174 | 3-$CF_3$-benzyl |
| 151 | 4-F, 3-$CH_3$ | 175 | isopropylthio |
| 152 | 3-Cl, 4-Cl | 176 | cyclopentoxy |
| 153 | 3,4-$(CH_2)_4$ | 177 | 3-Cl-5-pyridinyloxy |
| 154 | 3-$HCF_2CF_2O$ | 178 | 3-$CF_3S$-benzyloxy |
| 155 | 3-$CHF_2O$ | 179 | 3-$CH_3$, 4-$CH_3$-benzyloxy |
| 156 | 3-$(CH_3)_2N$ | 180 | 2-F, 3-$CF_3$-benzyloxy |
| 157 | 3-cyclopropyl | 181 | 3-F, 5-$CF_3$-benzyloxy |
| 158 | 3-(2-furyl) | 182 | 4-$(CH_3)_2CH$-benzyloxy |
| 159 | 3-$CF_3CF_2$ | 183 | 1-phenylethoxy |
| 160 | 4-$NH_2$ | 184 | 4-F, 3-$CH_3$-benzoyl |
| 161 | 3-$CH_3$, 4-$CH_3$, 5-$CH_3$ | 185 | 3-$CF_3$-phenyl |
| 162 | 4-$CH_3CH_2CH_2O$ | 186 | 4-$CH_3O$-phenylamino |
| 163 | 3-$CF_3$ | 187 | cyclopropoxy |
| 164 | 2-$NO_2$ | 188 | 4-$NO_2$-phenylthio |

Example Table 8 (continued). Substituted (2*R*)-3-[*N*-(aryl)-[(aryl)methyl] amino]-1,1,1-trifluoro-2-propanols.

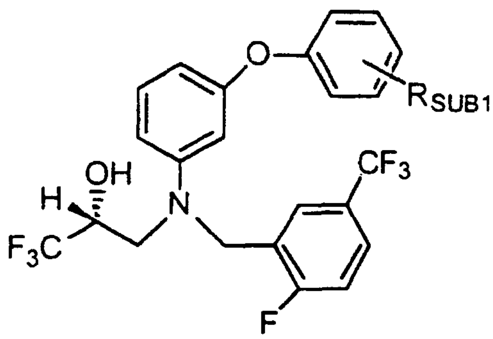

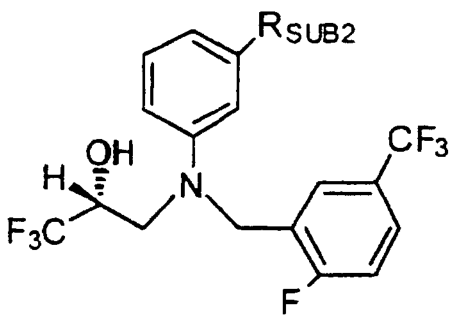

| Ex. No. | $R_{SUB1}$ |
|---|---|
| 189 | 3-isopropyl |
| 190 | 2-Cl, 3-Cl |
| 191 | 3-$CF_3O$ |
| 192 | 4-F |
| 193 | 4-$CH_3$ |
| 194 | 2-F, 5-Br |
| 195 | 4-Cl, 3-$CH_3$ |
| 196 | 3-$CH_3CH_2$ |
| 197 | 3-$CH_3$, 5-$CH_3$ |
| 198 | 3-$(CH_3)_3C$ |
| 199 | 4-F, 3-$CH_3$ |
| 200 | 3-Cl, 4-Cl |
| 201 | 3,4-$(CH_2)_4$ |
| 202 | 3-$HCF_2CF_2O$ |
| 203 | 3-$CHF_2O$ |
| 204 | 3-$(CH_3)_2N$ |
| 205 | 3-cyclopropyl |
| 206 | 3-(2-furyl) |
| 207 | 3-$CF_3CF_2$ |

| Ex. No. | $R_{SUB2}$ |
|---|---|
| 213 | 3-$CF_3O$-benzyloxy |
| 214 | 3-$CF_3$-benzyloxy |
| 215 | 3-F, 5-F-benzyloxy |
| 216 | cyclohexylmethyleneoxy |
| 217 | benzyloxy |
| 218 | 3-$CF_3$, 5-$CF_3$-benzyloxy |
| 219 | 4-$CF_3O$-benzyloxy |
| 220 | 4-$CH_3CH_2$-benzyloxy |
| 221 | isopropoxy |
| 222 | 3-$CF_3$-benzyl |
| 223 | isopropylthio |
| 224 | cyclopentoxy |
| 225 | 3-Cl-5-pyridinyloxy |
| 226 | 3-$CF_3S$-benzyloxy |
| 227 | 3-$CH_3$, 4-$CH_3$-benzyloxy |
| 228 | 2-F, 3-$CF_3$-benzyloxy |
| 229 | 3-F, 5-$CF_3$-benzyloxy |
| 230 | 4-$(CH_3)_2CH$-benzyloxy |
| 231 | 1-phenylethoxy |

Example Table 8 (continued). Substituted (2*R*)-3-[*N*-(aryl)-[(aryl)methyl] amino]-1,1,1-trifluoro-2-propanols.

| Ex. No. | $R_{SUB1}$ |
|---|---|
| 208 | 4-$NH_2$ |
| 209 | 3-$CH_3$, 4-$CH_3$, 5-$CH_3$ |
| 210 | 4-$CH_3CH_2CH_2O$ |
| 211 | 3-$CF_3$ |
| 212 | 2-$NO_2$ |

| Ex. No. | $R_{SUB2}$ |
|---|---|
| 232 | 4-F, 3-$CH_3$-benzoyl |
| 233 | 3-$CF_3$-phenyl |
| 234 | 4-$CH_3O$-phenylamino |
| 235 | cyclopropoxy |
| 236 | 4-$NO_2$-phenylthio |

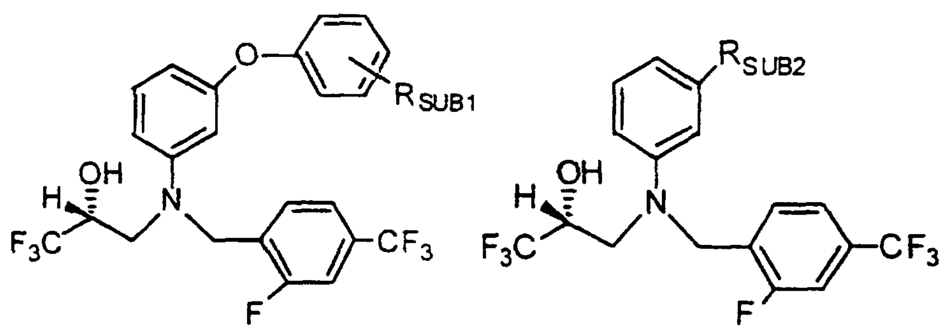

| Ex. No. | $R_{SUB1}$ |
|---|---|
| 237 | 3-isopropyl |
| 238 | 2-Cl, 3-Cl |
| 239 | 3-$CF_3O$ |
| 240 | 4-F |
| 241 | 4-$CH_3$ |
| 242 | 2-F, 5-Br |
| 243 | 4-Cl, 3-$CH_3$ |
| 244 | 3-$CH_3CH_2$ |
| 245 | 3-$CH_3$, 5-$CH_3$ |
| 246 | 3-$(CH_3)_3C$ |
| 247 | 4-F, 3-$CH_3$ |
| 248 | 3-Cl, 4-Cl |

| Ex. No. | $R_{SUB2}$ |
|---|---|
| 261 | 3-$CF_3O$-benzyloxy |
| 262 | 3-$CF_3$-benzyloxy |
| 263 | 3-F, 5-F-benzyloxy |
| 264 | cyclohexylmethyleneoxy |
| 265 | benzyloxy |
| 266 | 3-$CF_3$, 5-$CF_3$-benzyloxy |
| 267 | 4-$CF_3O$-benzyloxy |
| 268 | 4-$CH_3CH_2$-benzyloxy |
| 269 | isopropoxy |
| 270 | 3-$CF_3$-benzyl |
| 271 | isopropylthio |
| 272 | cyclopentoxy |

Example Table 8 (continued). Substituted (2*R*)-3-[*N*-(aryl)-[(aryl)methyl] amino]-1,1,1-trifluoro-2-propanols.

| Ex. No. | $R_{SUB1}$ |
|---|---|
| 249 | 3,4-$(CH_2)_4$ |
| 250 | 3-$HCF_2CF_2O$ |
| 251 | 3-$CHF_2O$ |
| 252 | 3-$(CH_3)_2N$ |
| 253 | 3-cyclopropyl |
| 254 | 3-(2-furyl) |
| 255 | 3-$CF_3CF_2$ |
| 256 | 4-$NH_2$ |
| 257 | 3-$CH_3$, 4-$CH_3$, 5-$CH_3$ |
| 258 | 4-$CH_3CH_2CH_2O$ |
| 259 | 3-$CF_3$ |
| 260 | 2-$NO_2$ |

| Ex. No. | $R_{SUB2}$ |
|---|---|
| 273 | 3-Cl-5-pyridinyloxy |
| 274 | 3-$CF_3S$-benzyloxy |
| 275 | 3-$CH_3$, 4-$CH_3$-benzyloxy |
| 276 | 2-F, 3-$CF_3$-benzyloxy |
| 277 | 3-F, 5-$CF_3$-benzyloxy |
| 278 | 4-$(CH_3)_2CH$-benzyloxy |
| 279 | 1-phenylethoxy |
| 280 | 4-F, 3-$CH_3$-benzoyl |
| 281 | 3-$CF_3$-phenyl |
| 282 | 4-$CH_3O$-phenylamino |
| 283 | cyclopropoxy |
| 284 | 4-$NO_2$-phenylthio |

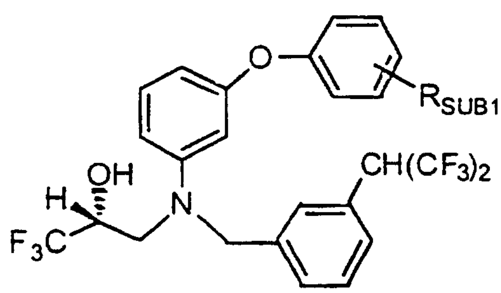

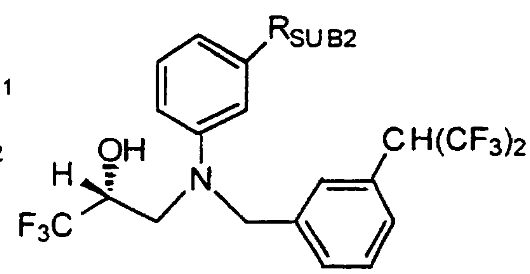

| Ex. No. | $R_{SUB1}$ |
|---|---|
| 285 | 3-isopropyl |
| 286 | 2-Cl, 3-Cl |
| 287 | 3-$CF_3O$ |
| 288 | 4-F |
| 289 | 4-$CH_3$ |

| Ex. No. | $R_{SUB2}$ |
|---|---|
| 309 | 3-$CF_3O$-benzyloxy |
| 310 | 3-$CF_3$-benzyloxy |
| 311 | 3-F, 5-F-benzyloxy |
| 312 | cyclohexylmethyleneoxy |
| 313 | benzyloxy |

Example Table 8 (continued). Substituted (2*R*)-3-[*N*-(aryl)-[(aryl)methyl] amino]-1,1,1-trifluoro-2-propanols.

| Ex. No. | $R_{SUB1}$ | Ex. No. | $R_{SUB2}$ |
|---|---|---|---|
| 290 | 2-F, 5-Br | 314 | 3-$CF_3$, 5-$CF_3$-benzyloxy |
| 291 | 4-Cl, 3-$CH_3CH_2$ | 315 | 4-$CF_3O$-benzyloxy |
| 292 | 3-$CH_3CH_2$ | 316 | 4-$CH_3CH_2$-benzyloxy |
| 293 | 3-$CH_3$, 5-$CH_3$ | 317 | isopropoxy |
| 294 | 3-$(CH_3)_3C$ | 318 | 3-$CF_3$-benzyl |
| 295 | 4-F, 3-$CH_3$ | 319 | isopropylthio |
| 296 | 3-Cl, 4-Cl | 320 | cyclopentoxy |
| 297 | 3,4-$(CH_2)_4$ | 321 | 3-Cl-5-pyridinyloxy |
| 298 | 3-$HCF_2CF_2O$ | 322 | 3-$CF_3S$-benzyloxy |
| 299 | 3-$CHF_2O$ | 323 | 3-$CH_3$, 4-$CH_3$-benzyloxy |
| 300 | 3-$(CH_3)_2N$ | 324 | 2-F, 3-$CF_3$-benzyloxy |
| 301 | 3-cyclopropyl | 325 | 3-F, 5-$CF_3$-benzyloxy |
| 302 | 3-(2-furyl) | 326 | 4-$(CH_3)_2CH$-benzyloxy |
| 303 | 3-$CF_3CF_2$ | 327 | 1-phenylethoxy |
| 304 | 4-$NH_2$ | 328 | 4-F, 3-$CH_3$-benzoyl |
| 305 | 3-$CH_3$, 4-$CH_3$, 5-$CH_3$ | 329 | 3-$CF_3$-phenyl |
| 306 | 4-$CH_3CH_2CH_2O$ | 330 | 4-$CH_3O$-phenylamino |
| 307 | 3-$CF_3$ | 331 | cyclopropoxy |
| 308 | 2-$NO_2$ | 332 | 4-$NO_2$-phenylthio |

Example Table 8 (continued). Substituted (2*R*)-3-[*N*-(aryl)-[(aryl)methyl] amino]-1,1,1-trifluoro-2-propanols.

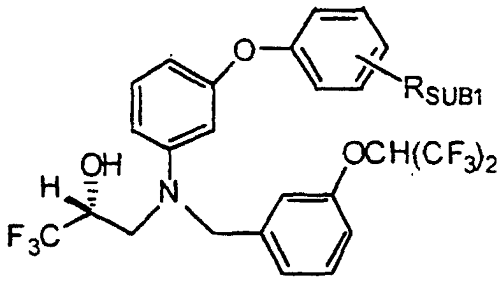

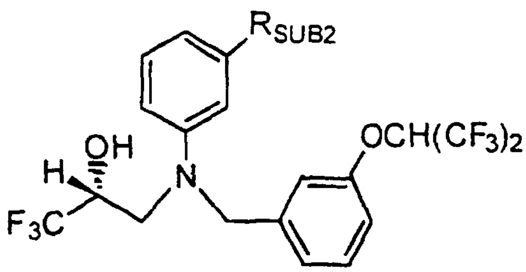

| Ex. No. | $R_{SUB1}$ | Ex. No. | $R_{SUB2}$ |
|---|---|---|---|
| 333 | 3-isopropyl | 357 | 3-$CF_3$O-benzyloxy |
| 334 | 2-Cl, 3-Cl | 358 | 3-$CF_3$-benzyloxy |
| 335 | 3-$CF_3$O | 359 | 3-F, 5-F-benzyloxy |
| 336 | 4-F | 360 | cyclohexylmethyleneoxy |
| 337 | 4-$CH_3$ | 361 | benzyloxy |
| 338 | 2-F, 5-Br | 362 | 3-$CF_3$, 5-$CF_3$-benzyloxy |
| 339 | 4-Cl, 3-$CH_3CH_2$ | 363 | 4-$CF_3$O-benzyloxy |
| 340 | 3-$CH_3CH_2$ | 364 | 4-$CH_3CH_2$-benzyloxy |
| 341 | 3-$CH_3$, 5-$CH_3$ | 365 | isopropoxy |
| 342 | 3-$(CH_3)_3C$ | 366 | 3-$CF_3$-benzyl |
| 343 | 4-F, 3-$CH_3$ | 367 | isopropylthio |
| 344 | 3-Cl, 4-Cl | 368 | cyclopentoxy |
| 345 | 3,4-$(CH_2)_4$ | 369 | 3-Cl-5-pyridinyloxy |
| 346 | 3-$HCF_2CF_2O$ | 370 | 3-$CF_3$S-benzyloxy |
| 347 | 3-$CHF_2O$ | 371 | 3-$CH_3$, 4-$CH_3$-benzyloxy |
| 348 | 3-$(CH_3)_2N$ | 372 | 2-F, 3-$CF_3$-benzyloxy |
| 349 | 3-cyclopropyl | 373 | 3-F, 5-$CF_3$-benzyloxy |
| 350 | 3-(2-furyl) | 374 | 4-$(CH_3)_2CH$-benzyloxy |
| 351 | 3-$CF_3CF_2$ | 375 | 1-phenylethoxy |
| 352 | 4-$NH_2$ | 376 | 4-F, 3-$CH_3$-benzoyl |

Example Table 8 (continued). Substituted (2*R*)-3-[*N*-(aryl)-[(aryl)methyl] amino]-1,1,1-trifluoro-2-propanols.

| Ex. No. | $R_{SUB1}$ |
|---|---|
| 353 | 3-$CH_3$, 4-$CH_3$, 5-$CH_3$ |
| 354 | 4-$CH_3CH_2CH_2O$ |
| 355 | 3-$CF_3$ |
| 356 | 2-$NO_2$ |

| Ex. No. | $R_{SUB2}$ |
|---|---|
| 377 | 3-$CF_3$-phenyl |
| 378 | 4-$CH_3O$-phenylamino |
| 379 | cyclopropoxy |
| 380 | 4-$NO_2$-phenylthio |

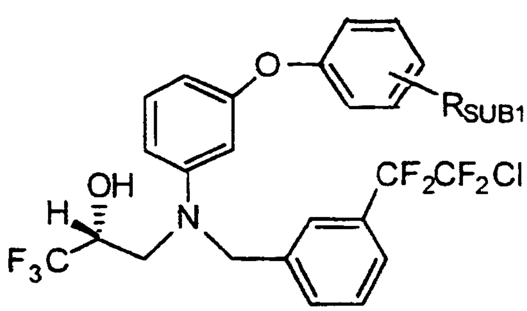

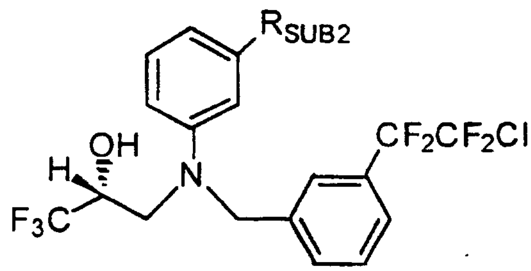

| Ex. No. | $R_{SUB1}$ |
|---|---|
| 381 | 3-isopropyl |
| 382 | 2-Cl, 3-Cl |
| 383 | 3-$CF_3O$ |
| 384 | 4-F |
| 385 | 4-$CH_3$ |
| 386 | 2-F, 5-Br |
| 387 | 4-Cl, 3-$CH_3CH_2$ |
| 388 | 3-$CH_3CH_2$ |
| 389 | 3-$CH_3$, 5-$CH_3$ |
| 390 | 3-$(CH_3)_3C$ |
| 391 | 4-F, 3-$CH_3$ |
| 392 | 3-Cl, 4-Cl |
| 393 | 3,4-$(CH_2)_4$ |

| Ex. No. | $R_{SUB2}$ |
|---|---|
| 405 | 3-$CF_3O$-benzyloxy |
| 406 | 3-$CF_3$-benzyloxy |
| 407 | 3-F, 5-F-benzyloxy |
| 408 | cyclohexylmethyleneoxy |
| 409 | benzyloxy |
| 410 | 3-$CF_3$, 5-$CF_3$-benzyloxy |
| 411 | 4-$CF_3O$-benzyloxy |
| 412 | 4-$CH_3CH_2$-benzyloxy |
| 413 | isopropoxy |
| 414 | 3-$CF_3$-benzyl |
| 415 | isopropylthio |
| 416 | cyclopentoxy |
| 417 | 3-Cl-5-pyridinyloxy |

Example Table 8 (continued). Substituted (2*R*)-3-[*N*-(aryl)-[(aryl)methyl] amino]-1,1,1-trifluoro-2-propanols.

| Ex. No. | $R_{SUB1}$ |
|---|---|
| 394 | 3-$HCF_2CF_2O$ |
| 395 | 3-$CHF_2O$ |
| 396 | 3-$(CH_3)_2N$ |
| 397 | 3-cyclopropyl |
| 398 | 3-(2-furyl) |
| 399 | 3-$CF_3CF_2$ |
| 400 | 4-$NH_2$ |
| 401 | 3-$CH_3$, 4-$CH_3$, 5-$CH_3$ |
| 402 | 4-$CH_3CH_2CH_2O$ |
| 403 | 3-$CF_3$ |
| 404 | 2-$NO_2$ |

| Ex. No. | $R_{SUB2}$ |
|---|---|
| 418 | 3-$CF_3S$-benzyloxy |
| 419 | 3-$CH_3$, 4-$CH_3$-benzyloxy |
| 420 | 2-F, 3-$CF_3$-benzyloxy |
| 421 | 3-F, 5-$CF_3$-benzyloxy |
| 422 | 4-$(CH_3)_2CH$-benzyloxy |
| 423 | 1-phenylethoxy |
| 424 | 4-F, 3-$CH_3$-benzoyl |
| 425 | 3-$CF_3$-phenyl |
| 426 | 4-$CH_3O$-phenylamino |
| 427 | cyclopropoxy |
| 428 | 4-$NO_2$-phenylthio |

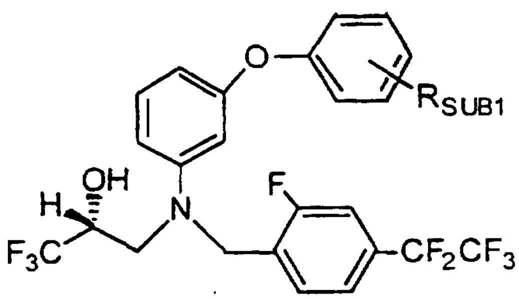

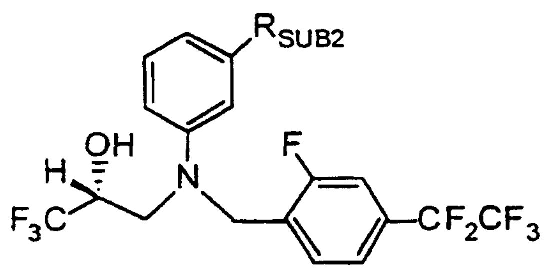

| Ex. No. | $R_{SUB1}$ |
|---|---|
| 429 | 3-isopropyl |
| 430 | 2-Cl, 3-Cl |
| 431 | 3-$CF_3O$ |
| 432 | 4-F |
| 433 | 4-$CH_3$ |
| 434 | 2-F, 5-Br |

| Ex. No. | $R_{SUB2}$ |
|---|---|
| 453 | 3-$CF_3O$-benzyloxy |
| 454 | 3-$CF_3$-benzyloxy |
| 455 | 3-F, 5-F-benzyloxy |
| 456 | cyclohexylmethyleneoxy |
| 457 | benzyloxy |
| 458 | 3-$CF_3$, 5-$CF_3$-benzyloxy |

Example Table 8 (continued). Substituted (2*R*)-3-[*N*-(aryl)-[(aryl)methyl] amino]-1,1,1-trifluoro-2-propanols.

| Ex. No. | $R_{SUB1}$ | Ex. No. | $R_{SUB2}$ |
|---|---|---|---|
| 435 | 4-Cl, 3-$CH_3CH_2$ | 459 | 4-$CF_3O$-benzyloxy |
| 436 | 3-$CH_3CH_2$ | 460 | 4-$CH_3CH_2$-benzyloxy |
| 437 | 3-$CH_3$, 5-$CH_3$ | 461 | isopropoxy |
| 438 | 3-$(CH_3)_3C$ | 462 | 3-$CF_3$-benzyl |
| 439 | 4-F, 3-$CH_3$ | 463 | isopropylthio |
| 440 | 3-Cl, 4-Cl | 464 | cyclopentoxy |
| 441 | 3,4-$(CH_2)_4$ | 465 | 3-Cl-5-pyridinyloxy |
| 442 | 3-$HCF_2CF_2O$ | 466 | 3-$CF_3S$-benzyloxy |
| 443 | 3-$CHF_2O$ | 467 | 3-$CH_3$, 4-$CH_3$-benzyloxy |
| 444 | 3-$(CH_3)_2N$ | 468 | 2-F, 3-$CF_3$-benzyloxy |
| 445 | 3-cyclopropyl | 469 | 3-F, 5-$CF_3$-benzyloxy |
| 446 | 3-(2-furyl) | 470 | 4-$(CH_3)_2CH$-benzyloxy |
| 447 | 3-$CF_3CF_2$ | 471 | 1-phenylethoxy |
| 448 | 4-$NH_2$ | 472 | 4-F, 3-$CH_3$-benzoyl |
| 449 | 3-$CH_3$, 4-$CH_3$, 5-$CH_3$ | 473 | 3-$CF_3$-phenyl |
| 450 | 4-$CH_3CH_2CH_2O$ | 474 | 4-$CH_3O$-phenylamino |
| 451 | 3-$CF_3$ | 475 | cyclopropoxy |
| 452 | 2-$NO_2$ | 476 | 4-$NO_2$-phenylthio |

Example Table 8 (continued). Substituted (2*R*)-3-[*N*-(aryl)-[(aryl)methyl] amino]-1,1,1-trifluoro-2-propanols.

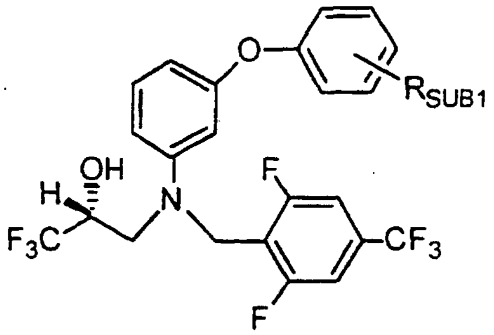

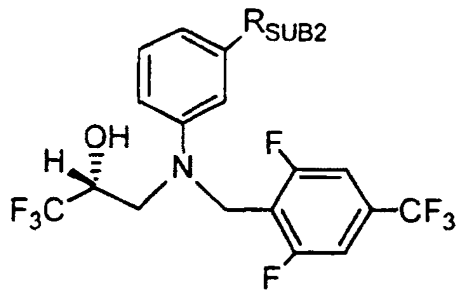

| Ex. No. | $R_{SUB1}$ |
|---|---|
| 477 | 3-isopropyl |
| 478 | 2-Cl, 3-Cl |
| 479 | 3-$CF_3O$ |
| 480 | 4-F |
| 481 | 4-$CH_3$ |
| 482 | 2-F, 5-Br |
| 483 | 4-Cl, 3-$CH_3CH_2$ |
| 484 | 3-$CH_3CH_2$ |
| 485 | 3-$CH_3$, 5-$CH_3$ |
| 486 | 3-$(CH_3)_3C$ |
| 487 | 4-F, 3-$CH_3$ |
| 488 | 3-Cl, 4-Cl |
| 489 | 3,4-$(CH_2)_4$ |
| 490 | 3-$HCF_2CF_2O$ |
| 491 | 3-$CHF_2O$ |
| 492 | 3-$(CH_3)_2N$ |
| 493 | 3-cyclopropyl |
| 494 | 3-(2-furyl) |
| 495 | 3-$CF_3CF_2$ |

| Ex. No. | $R_{SUB2}$ |
|---|---|
| 501 | 3-$CF_3O$-benzyloxy |
| 502 | 3-$CF_3$-benzyloxy |
| 503 | 3-F, 5-F-benzyloxy |
| 504 | cyclohexylmethyleneoxy |
| 505 | benzyloxy |
| 506 | 3-$CF_3$, 5-$CF_3$-benzyloxy |
| 507 | 4-$CF_3O$-benzyloxy |
| 508 | 4-$CH_3CH_2$-benzyloxy |
| 509 | isopropoxy |
| 510 | 3-$CF_3$-benzyl |
| 511 | isopropylthio |
| 512 | cyclopentoxy |
| 513 | 3-Cl-5-pyridinyloxy |
| 514 | 3-$CF_3S$-benzyloxy |
| 515 | 3-$CH_3$, 4-$CH_3$-benzyloxy |
| 516 | 2-F, 3-$CF_3$-benzyloxy |
| 517 | 3-F, 5-$CF_3$-benzyloxy |
| 518 | 4-$(CH_3)_2CH$-benzyloxy |
| 519 | 1-phenylethoxy |

Example Table 8 (continued). Substituted (2*R*)-3-[*N*-(aryl)-[(aryl)methyl] amino]-1,1,1-trifluoro-2-propanols.

| Ex. No. | $R_{SUB1}$ |
|---|---|
| 496 | 4-$NH_2$ |
| 497 | 3-$CH_3$, 4-$CH_3$, 5-$CH_3$ |
| 498 | 4-$CH_3CH_2CH_2O$ |
| 499 | 3-$CF_3$ |
| 500 | 2-$NO_2$ |

| Ex. No. | $R_{SUB2}$ |
|---|---|
| 520 | 4-F, 3-$CH_3$-benzoyl |
| 521 | 3-$CF_3$-phenyl |
| 522 | 4-$CH_3O$-phenylamino |
| 523 | cyclopropoxy |
| 524 | 4-$NO_2$-phenylthio |

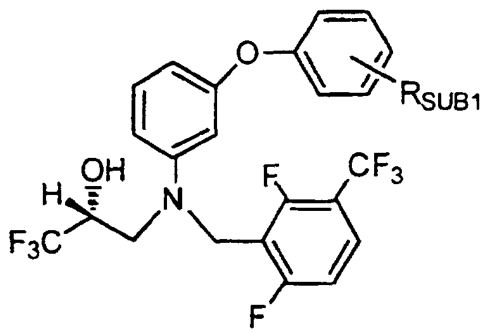

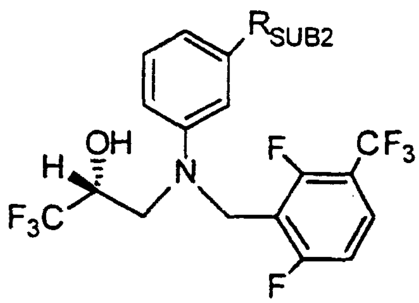

| Ex. No. | $R_{SUB1}$ |
|---|---|
| 525 | 3-isopropyl |
| 526 | 2-Cl, 3-Cl |
| 527 | 3-$CF_3O$ |
| 528 | 4-F |
| 529 | 4-$CH_3$ |
| 530 | 2-F, 5-Br |
| 531 | 4-Cl, 3-$CH_3CH_2$ |
| 532 | 3-$CH_3CH_2$ |
| 533 | 3-$CH_3$, 5-$CH_3$ |
| 534 | 3-$(CH_3)_3C$ |
| 535 | 4-F, 3-$CH_3$ |
| 536 | 3-Cl, 4-Cl |

| Ex. No. | $R_{SUB2}$ |
|---|---|
| 549 | 3-$CF_3O$-benzyloxy |
| 550 | 3-$CF_3$-benzyloxy |
| 551 | 3-F, 5-F-benzyloxy |
| 552 | cyclohexylmethyleneoxy |
| 553 | benzyloxy |
| 554 | 3-$CF_3$, 5-$CF_3$-benzyloxy |
| 555 | 4-$CF_3O$-benzyloxy |
| 556 | 4-$CH_3CH_2$-benzyloxy |
| 557 | isopropoxy |
| 558 | 3-$CF_3$-benzyl |
| 559 | isopropylthio |
| 560 | cyclopentoxy |

Example Table 8 (continued). Substituted (2*R*)-3-[*N*-(aryl)-[(aryl)methyl] amino]-1,1,1-trifluoro-2-propanols.

| Ex. No. | RSUB1 |
|---|---|
| 537 | 3,4-$(CH_2)_4$ |
| 538 | 3-$HCF_2CF_2O$ |
| 539 | 3-$CHF_2O$ |
| 540 | 3-$(CH_3)_2N$ |
| 541 | 3-cyclopropyl |
| 542 | 3-(2-furyl) |
| 543 | 3-$CF_3CF_2$ |
| 544 | 4-$NH_2$ |
| 545 | 3-$CH_3$, 4-$CH_3$, 5-$CH_3$ |
| 546 | 4-$CH_3CH_2CH_2O$ |
| 547 | 3-$CF_3$ |
| 548 | 2-$NO_2$ |

| Ex. No. | RSUB2 |
|---|---|
| 561 | 3-Cl-5-pyridinyloxy |
| 562 | 3-$CF_3S$-benzyloxy |
| 563 | 3-$CH_3$, 4-$CH_3$-benzyloxy |
| 564 | 2-F, 3-$CF_3$-benzyloxy |
| 565 | 3-F, 5-$CF_3$-benzyloxy |
| 566 | 4-$(CH_3)_2CH$-benzyloxy |
| 567 | 1-phenylethoxy |
| 568 | 4-F, 3-$CH_3$-benzoyl |
| 569 | 3-$CF_3$-phenyl |
| 570 | 4-$CH_3O$-phenylamino |
| 571 | cyclopropoxy |
| 572 | 4-$NO_2$-phenylthio |

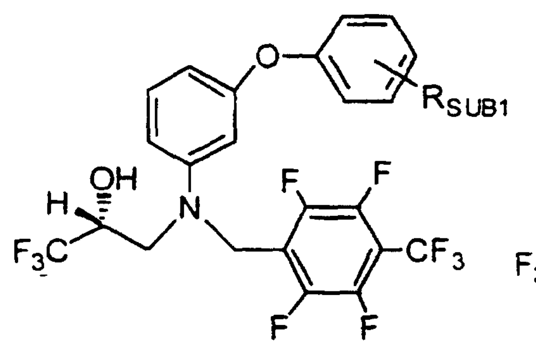

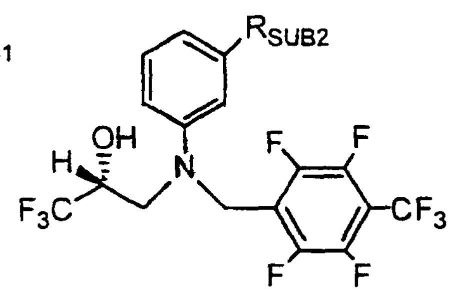

| Ex. No. | RSUB1 |
|---|---|
| 573 | 3-isopropyl |
| 574 | 2-Cl, 3-Cl |
| 575 | 3-$CF_3O$ |
| 576 | 4-F |

| Ex. No. | RSUB2 |
|---|---|
| 597 | 3-$CF_3O$-benzyloxy |
| 598 | 3-$CF_3$-benzyloxy |
| 599 | 3-F, 5-F-benzyloxy |
| 600 | cyclohexylmethyleneoxy |

Example Table 8 (continued). Substituted (2*R*)-3-[*N*-(aryl)-[(aryl)methyl] amino]-1,1,1-trifluoro-2-propanols.

| Ex. No. | $R_{SUB1}$ | Ex. No. | $R_{SUB2}$ |
|---|---|---|---|
| 577 | 4-$CH_3$ | 601 | benzyloxy |
| 578 | 2-F, 5-Br | 602 | 3-$CF_3$, 5-$CF_3$-benzyloxy |
| 579 | 4-Cl, 3-$CH_3CH_2$ | 603 | 4-$CF_3O$-benzyloxy |
| 580 | 3-$CH_3CH_2$ | 604 | 4-$CH_3CH_2$-benzyloxy |
| 581 | 3-$CH_3$, 5-$CH_3$. | 605 | isopropoxy |
| 582 | 3-$(CH_3)_3C$ | 606 | 3-$CF_3$-benzyl |
| 583 | 4-F, 3-$CH_3$ | 607 | isopropylthio |
| 584 | 3-Cl, 4-Cl | 608 | cyclopentoxy |
| 585 | 3,4-$(CH_2)_4$ | 609 | 3-Cl-5-pyridinyloxy |
| 586 | 3-$HCF_2CF_2O$ | 610 | 3-$CF_3S$-benzyloxy |
| 587 | 3-$CHF_2O$ | 611 | 3-$CH_3$, 4-$CH_3$-benzyloxy |
| 588 | 3-$(CH_3)_2N$ | 612 | 2-F, 3-$CF_3$-benzyloxy |
| 589 | 3-cyclopropyl | 613 | 3-F, 5-$CF_3$-benzyloxy |
| 590 | 3-(2-furyl) | 614 | 4-$(CH_3)_2CH$-benzyloxy |
| 591 | 3-$CF_3CF_2$ | 615 | 1-phenylethoxy |
| 592 | 4-$NH_2$ | 616 | 4-F, 3-$CH_3$-benzoyl |
| 593 | 3-$CH_3$, 4-$CH_3$, 5-$CH_3$ | 617 | 3-$CF_3$-phenyl |
| 594 | 4-$CH_3CH_2CH_2O$ | 618 | 4-$CH_3O$-phenylamino |
| 595 | 3-$CF_3$ | 619 | cyclopropoxy |
| 596 | 2-$NO_2$ | 620 | 4-$NO_2$-phenylthio |

Example Table 8 (continued). Substituted (2*R*)-3-[*N*-(aryl)-[(aryl)methyl] amino]-1,1,1-trifluoro-2-propanols.

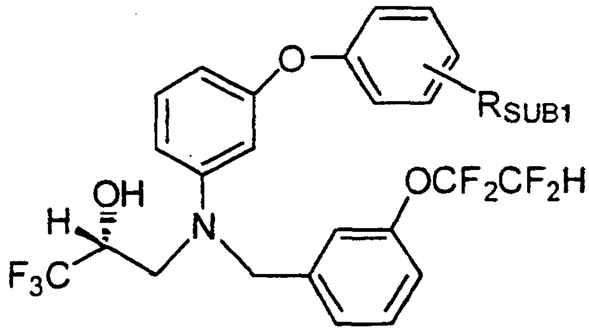

| Ex. No. | RSUB1 | Calculated Mass $[M+H]^+$ | Observed Mass $[M+H]^+$ |
|---|---|---|---|
| 621 | 4-F | 522.1315 | 522.1297 |
| 622 | 2-Cl, 3-Cl | 572.0630 | 572.0653 |
| 623 | 2-F, 5-Br | 600.0420 | 600.0404 |
| 624 | 4-Cl, 3-$CH_3$ | 551.1098 | 551.1101 |
| 625 | 3-$CH_3$, 5-$CH_3$ | 532.1722 | 532.1705 |
| 626 | 3-$(CH_3)_3C$ | 560.2035 | 560.2055 |
| 627 | 4-F, 3-$CH_3$ | 536.1471 | 536.1480 |
| 628 | 3-Cl, 4-Cl | 572.0630 | 572.0630 |
| 629 | 3,4-$(CH_2)_4$ | 558.1879 | 558.1881 |
| 630 | 3-$HCF_2CF_2O$ | | |
| 631 | 3-$CHF_2O$ | | |
| 632 | 3-$(CH_3)_2N$ | 547.1831 | 547.1844 |
| 633 | 3-cyclopropyl | | |
| 634 | 3-(2-furyl) | | |
| 635 | 3-$CF_3CF_2$ | | |
| 636 | 3-cyclopentyl | | |
| 637 | 4-$NH_2$ | 519.1519 | 519.1529 |

Example Table 8 (continued). Substituted (2*R*)-3-[*N*-(aryl)-[(aryl)methyl]amino]-1,1,1-trifluoro-2-propanols.

| Ex. No. | RSUB1 | Calculated Mass $[M+H]^+$ | Observed Mass $[M+H]^+$ |
|---|---|---|---|
| 638 | 3-$CH_3$, 4-$CH_3$, 5-$CH_3$ | 546.1879 | 546.1901 |
| 639 | 4-$CH_3CH_2O$ | 547.1594 | 547.1594 |
| 640 | 3-$CF_3$ | | |
| 641 | 2-$NO_2$ | 549.1260 | 549.1235 |
| 642 | 3,4-dimethyl | 531.1644 | 531.1649 |
| 643 | 3-methyl, 5-ethyl | 546.1879 | 546.1899 |
| 644 | 3-methyl | 517.1488 | 517.1493 |
| 645 | 2,3-difluoro | 540.1221 | 540.1182 |
| 646 | 4-$CF_3$ | 572.1282 | 572.1268 |
| 647 | 2-fluoro, 3-$CF_3$ | 590.1189 | 590.1184 |
| 648 | 2-fluoro, 4-$CF_3$ | 590.1189 | 590.1155 |
| 649 | 2-chloro, 4-fluoro | 556.0925 | 556.0891 |
| 650 | 4-*n*-propyl | 546.1879 | 546.1878 |
| 651 | 3-chloro, 4-fluoro | 556.0925 | 556.0932 |
| 652 | 2,4-difluoro | 540.1221 | 540.1194 |
| 653 | 3,5-difluoro | 540.1221 | 540.1217 |
| 654 | 3,4-difluoro | 540.1221 | 540.1248 |
| 655 | 3-fluoro | 522.1315 | 522.1337 |
| 656 | 2-chloro | 538.1019 | 538.1021 |
| 657 | 2-fluoro | 522.1315 | 522.1310 |
| 658 | 2,5-difluoro | 540.1221 | 540.1255 |
| 659 | 4-chloro, 2-fluoro | 556.0926 | 556.0954 |
| 660 | 2,4-dichloro | 572.0630 | 572.0667 |
| 661 | 2-fluoro, 3-$CH_3$ | | |
| 662 | 4-chloro | 537.0942 | 537.0944 |

Example Table 8 (continued). Substituted (2*R*)-3-[*N*-(aryl)-[(aryl)methyl] amino]-1,1,1-trifluoro-2-propanols.

| Ex. No. | $R_{SUB1}$ | Calculated Mass $[M+H]^+$ | Observed Mass $[M+H]^+$ |
|---|---|---|---|
| 663 | 4-isopropyl, 3-methyl | 560.2035 | 560.2035 |
| 664 | 2,3,4-trifluoro | 558.1127 | 558.1161 |
| 665 | 2,3,5-trifluoro | 558.1127 | 558.1109 |
| 666 | 4-propoxy | 562.1828 | 562.1803 |
| 667 | 4-isopropyl | 546.1879 | 546.1899 |
| 668 | $4-CF_3O-$ | 588.1233 | 588.1241 |
| 669 | 4-butoxy | 576.1958 | 576.1969 |
| 670 | 3-methyl, $4-CH_3S-$ | 564.1443 | 564.1476 |
| 671 | 4-nitro | 549.1260 | 549.1306 |
| 672 | $3-CF_3S-$ | | |
| 673 | 4-chloro, 3-fluoro | 556.0925 | 556.0933 |
| 674 | 3,5-dimethoxy | 564.1623 | 564.1617 |
| 675 | 4-bromo | 582.0716 | 582.0473 |
| 676 | 4-*sec*-butyl | 560.2035 | 560.2051 |
| 677 | 3-fluoro-2-nitro | 567.1166 | 567.1135 |
| 678 | 3-methoxy | 533.1437 | 533.1450 |
| 679 | 4-bromo-2-nitro | 627.0366 | 627.0375 |
| 680 | 4-cyano | 529.1362 | 529.1364 |
| 681 | $4-CH_3S-$ | 550.1209 | 550.1251 |
| 682 | $3,4-(CH=CH)_2$ | 554.1566 | 554.1578 |
| 683 | $4-CH_3CH_2NH-$ | 547.1832 | 547.1819 |
| 684 | 4-propionyl | 560.1672 | 560.1694 |
| 685 | 3-phenyl | 580.1723 | 580.1772 |
| 686 | 4-cyclopentyl | 572.2035 | 572.2029 |

Example Table 8 (continued). Substituted (2*R*)-3-[*N*-(aryl)-[(aryl)methyl] amino]-1,1,1-trifluoro-2-propanols.

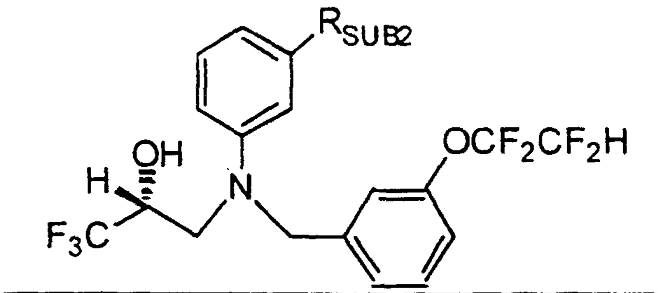

| Ex. No. | $R_{SUB2}$ | Calculated Mass $[M+H]^+$ | Observed Mass $[M+H]^+$ |
|---|---|---|---|
| 687 | 6-methyl-3-pyridinyloxy | 518.1440 | 518.1452 |
| 688 | 5-chloro-3-pyridinyloxy | 539.0972 | 539.1002 |
| 689 | 3-pyridinyloxy | 505.1362 | 505.1369 |
| 690 | 2-methyl-3-pyridinyloxy | 519.1518 | 519.1517 |
| 691 | 5-indolinyloxy | 543.1519 | 543.1630 |
| 692 | 4-fluoro-2-pyridinyloxy | 523.1268 | 523.1243 |
| 693 | 2-cyano-3-pyridinyloxy | 530.1315 | 530.1300 |
| 694 | 5-bromo-2-pyridinyloxy | 583.0667 | 583.0405 |
| 695 | 3-$CF_3$-2-pyridinyloxy | 573.1236 | 573.1205 |
| 696 | 2-pyridinylmethyleneoxy | 519.1519 | 519.1522 |
| 697 | cyclohexylmethyleneoxy | 524.2036 | 524.2028 |
| 698 | isopropoxy | 470.1488 | 470.1565 |
| 699 | cyclopentyloxy | 496.1723 | 496.1719 |
| 700 | *neo*-pentoxy | 498.1879 | 498.1845 |
| 701 | 4-(methoxycarbonyl)-butoxy | 542.1777 | 542.1827 |
| 702 | trifluoromethoxy | 496.0971 | 496.0959 |
| 703 | 2-methylpropoxy | 484.1723 | 484.1718 |
| 704 | 2-methoxyethoxy | 486.1515 | 486.1537 |
| 705 | 2-oxobutoxy | 498.1515 | 498.1529 |
| 706 | cyclohexyloxy | 510.1880 | 510.1910 |

Example Table 8 (continued). Substituted (2*R*)-3-[*N*-(aryl)-[(aryl)methyl] amino]-1,1,1-trifluoro-2-propanols.

| Ex. No. | RSUB2 | Calculated Mass [M+H]+ | Observed Mass [M+H]+ |
|---|---|---|---|
| 707 | (methoxycarbonyl)methoxy | 500.1308 | 500.1297 |
| 708 | 4-tetrahydropyranyloxy | 512.1672 | 512.1631 |
| 709 | 1-phenylethoxy | 532.1723 | 532.1711 |
| 710 | 3-$CF_3O$-benzyloxy | 602.1389 | 602.1380 |
| 711 | 3-trifluoromethyl-benzyloxy | 586.1440 | 586.1419 |
| 712 | 3,5-dimethyl-benzyloxy | 546.1879 | 546.1890 |
| 713 | 3-bromo-benzyloxy | 596.0671 | 596.0641 |
| 714 | 3-$CF_3S$-benzyloxy | 618.1161 | 618.1151 |
| 715 | 3,4-dimethyl-benzyloxy | 546.1879 | 546.1881 |
| 716 | 3,5-difluoro-benzyloxy | 554.1378 | 554.1390 |
| 717 | 2-fluoro-3-$CF_3$-benzyloxy | 604.1346 | 604.1329 |
| 718 | benzyloxy | 518.1566 | 518.1578 |
| 719 | 3,5-$(CF_3)_2$-benzyloxy | 654.1314 | 654.1308 |
| 720 | 3-fluoro-5-$CF_3$-benzyloxy | 604.1346 | 604.1309 |
| 721 | 4-$CF_3O$-benzyloxy | 602.1389 | 602.1383 |
| 722 | 3-chloro-benzyloxy | 552.1176 | 552.1157 |
| 723 | 4-ethyl-benzyloxy | 546.1879 | 546.1862 |
| 724 | 3-methyl-benzyloxy | 532.1723 | 532.1692 |
| 725 | 2-fluoro-benzyloxy | 536.1472 | 536.1465 |
| 726 | 2,3-difluoro-benzyloxy | 554.1378 | 554.1364 |
| 727 | 4-isopropyl-benzyloxy | 560.2036 | 560.2020 |
| 728 | 4-methyl-benzyloxy | 532.1723 | 532.1729 |
| 729 | 4-bromo-benzyloxy | 596.0671 | 596.0669 |
| 730 | 4-$CF_3$-benzyloxy | 586.1440 | 586.1400 |
| 731 | 4-fluoro-benzyloxy | 536.1472 | 536.1454 |

Example Table 8 (continued). Substituted (2*R*)-3-[*N*-(aryl)-[(aryl)methyl] amino]-1,1,1-trifluoro-2-propanols.

| Ex. No. | RSUB2 | Calculated Mass [M+H]+ | Observed Mass [M+H]+ |
|---|---|---|---|
| 732 | 3-iodo-benzyloxy | 644.0533 | 644.0517 |
| 733 | 4-$CF_3S$-benzyloxy | 618.1161 | 618.1165 |
| 734 | 4-$CF_2HO$-benzyloxy | 584.1483 | 584.1480 |
| 735 | 4-fluoro-3-$CF_3$-benzyloxy | 604.1346 | 604.1336 |
| 736 | 2,3,5-trifluoro-benzyloxy | 572.1284 | 572.1276 |
| 737 | 4-chloro-benzyloxy | 552.1176 | 552.1188 |
| 738 | 2,5-difluoro-benzyloxy | 554.1378 | 554.1350 |
| 739 | 3-chloro-2-fluoro-benzyloxy | 570.1082 | 570.1069 |
| 740 | 2,4-$(CF_3)_2$-benzyloxy | 654.1314 | 654.1321 |
| 741 | 3,5-dichloro-benzyloxy | 586.1787 | 586.1378 |
| 742 | 3-methoxy-benzyloxy | 548.1672 | 548.1676 |
| 743 | 4-cyano-benzyloxy | 543.1519 | 543.1517 |
| 744 | 4-*tert*-butyl-benzyloxy | 574.2192 | 574.2163 |
| 745 | isopropylthio | 486.1338 | 486.1351 |
| 746 | 4-nitrophenylthio | 565.1032 | 565.1034 |
| 747 | 4-acetylphenylthio | 562.1287 | 562.1261 |
| 748 | (4-chloro-thien-2-yl)-methylthio | 574.0512 | 574.0523 |
| 749 | 4-methoxy-phenylamino | 532.1597 | 532.1592 |
| 750 | 3-methoxy-phenylamino | 532.1597 | 532.1593 |
| 751 | 4-chloro-phenylamino | 536.1102 | 536.1125 |
| 752 | 4-*n*-propyl-phenylamino | 544.1961 | 544.1959 |
| 753 | 3-cyano-phenylamino | 527.1444 | 527.1448 |
| 754 | 3-$CF_3$-benzyl | 570.1413 | 570.1480 |
| 755 | 3-methyl-4-fluoro-benzyl | 534.1679 | 534.1688 |
| 756 | 3-$CF_3$-phenyl | 556.1334 | 556.1339 |

Example Table 8 (continued). Substituted (2*R*)-3-[*N*-(aryl)-[(aryl)methyl] amino]-1,1,1-trifluoro-2-propanols.

| Ex. No. | RSUB2 | Calculated Mass $[M+H]^+$ | Observed Mass $[M+H]^+$ |
|---|---|---|---|
| 757 | 2,4-dichloro-phenyl | 556.0681 | 556.0651 |
| 758 | 3-methoxybenzyl | 532.1723 | 532.1705 |
| 759 | 4-methoxyphenyl | 518.1566 | 518.1533 |
| 760 | 3-chloro-4-fluoro-phenyl | 540.0976 | 540.0957 |
| 761 | 4-fluoro-3-methyl-benzoyl | 548.1410 | 548.1441 |
| 762 | 3-chlorobenzyl | 536.1227 | 536.1218 |
| 763 | 3,4-dimethylbenzyl | 530.1930 | 530.1887 |
| 764 | 3,5-dichlorobenzyl | 570.0838 | 570.0801 |
| 765 | 2,3,4-trifluorophenyl | 542.1177 | 542.1152 |
| 766 | 3-chloro-4-fluoro-benzyl | 554.1133 | 554.1108 |
| 767 | 4-fluoro-3-methyl-phenyl | 520.1523 | 520.1494 |
| 768 | 3-methyl-4-chloro-benzyl | 550.1384 | 550.1380 |
| 769 | 2-methylpropanoyl | 482.1566 | 482.1576 |
| 770 | 4-methylthiobenzyl | 548.1494 | 548.1503 |
| 771 | 4-fluorophenyl | 506.1366 | 506.1336 |
| 772 | 4-chlorophenyl | 522.1071 | 522.1049 |
| 773 | 3-methoxyphenyl | 518.1566 | 518.1544 |
| 774 | 4-methylbenzyl | 516.1774 | 516.1769 |
| 775 | 1-hydroxy-2-methyl-propyl | 484.1723 | 484.1725 |
| 776 | benzyl | 502.1617 | 502.1609 |
| 777 | 2-$CF_3$-phenyl | 556.1334 | 556.1286 |
| 778 | 3,4-dichlorophenyl | 556.0681 | 556.0698 |
| 779 | benzoyl | 516.1410 | 516.1383 |
| 780 | 4-fluorobenzoyl | 534.1315 | 534.1273 |
| 781 | N-piperidinyl | 494.1804 | 494.1804 |
| 782 | phenyl | 488.1460 | 488.1457 |
| 783 | thien-2-yl | 494.1024 | 494.0987 |

Example Table 8 (continued). Substituted (2*R*)-3-[*N*-(aryl)-[(aryl)methyl] amino]-1,1,1-trifluoro-2-propanols.

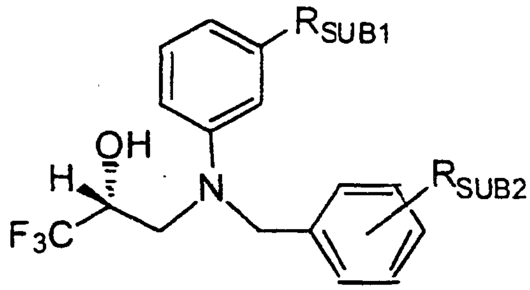

| Ex. No. | RSUB1 | RSUB2 | Calculated Mass $[M+H]^+$ | Observed Mass $[M+H]^+$ |
|---|---|---|---|---|
| 784 | phenoxy | 3-cyclopentyl | 456.2150 | 456.2143 |
| 785 | phenoxy | 3-isopropoxy | 446.1943 | 446.1936 |
| 786 | phenoxy | 3-$CF_3S$ | 488.1119 | 488.1116 |
| 787 | 4-F-phenoxy | 3-$CF_3S$ | 505.0946 | 505.0927 |
| 788 | 4-F-phenoxy | 3-*sec*-butoxy | 478.2005 | 478.1880 |
| 789 | phenoxy | 3-$(CF_3)_2COH$- | 554.1378 | 554.1385 |
| 790 | 4-$CH_3$-phenoxy | 3-$CF_3S$ | 502.1275 | 502.1261 |
| 791 | phenoxy | 3-(2-furyl) | 454.1630 | 454.1635 |
| 792 | 4-F-phenoxy | 3-isopropoxy | 464.1849 | 464.1867 |
| 793 | phenoxy | 3-isobutyl | 444.2150 | 444.2157 |
| 794 | phenoxy | 3-*tert*-butoxy | 460.2100 | 460.2103 |
| 795 | 4-F-phenoxy | 3-$CH_3CH_2O$- | 450.1692 | 450.1682 |
| 796 | 4-F-phenoxy | 3-$CF_3O$- | 490.1253 | 490.1211 |
| 797 | phenoxy | 4-F-3-(2-furyl)- | 472.1536 | 472.1530 |
| 798 | 4-F-phenoxy | 3-*n*-propoxy- | 464.1849 | 464.1820 |
| 799 | 4-F-phenoxy | 3-cyclopentyloxy- | 490.2005 | 490.1998 |
| 800 | phenoxy | 3-(3-furyl)- | 454.1630 | 454.1646 |
| 801 | 4-F-phenoxy | 3-cyclopropyl-methyleneoxy | 476.1849 | 476.1857 |
| 802 | phenoxy | 3-$CF_3CH_2O$- | 486.1504 | 486.1498 |

Example Table 9. (3*R*)-4-[*N*-(aryl)-[(aryl)methyl]amino]-1,1,1,2,2-pentafluoro-3-butanols.

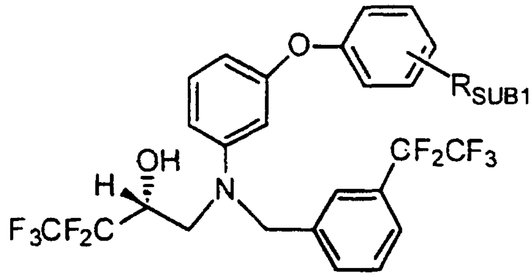

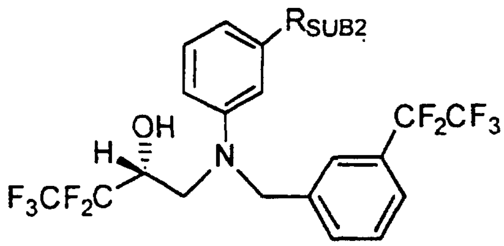

| Ex. No. | $R_{SUB1}$ |
|---|---|
| 803 | 3-isopropyl |
| 804 | 2-Cl, 3-Cl |
| 805 | 3-$CF_3O$ |
| 806 | 4-F |
| 807 | 4-$CH_3$ |
| 808 | 2-F, 5-Br |
| 809 | 4-Cl, 3-$CH_3CH_2$ |
| 810 | 3-$CH_3CH_2$ |
| 811 | 3-$CH_3$, 5-$CH_3$ |
| 812 | 3-$(CH_3)_3C$ |
| 813 | 4-F, 3-$CH_3$ |
| 814 | 3-Cl, 4-Cl |
| 815 | 3,4-$(CH_2)_4$ |
| 816 | 3-$HCF_2CF_2O$ |
| 817 | 3-$CHF_2O$ |
| 818 | 3-$(CH_3)_2N$ |
| 819 | 3-cyclopropyl |
| 820 | 3-(2-furyl) |
| 821 | 3-$CF_3CF_2$ |
| 822 | 4-$NH_2$ |
| 823 | 3-$CH_3$, 4-$CH_3$, 5-$CH_3$ |
| 824 | 4-$CH_3CH_2CH_2O$ |

| Ex. No. | $R_{SUB2}$ |
|---|---|
| 827 | 3-$CF_3O$-benzyloxy |
| 828 | 3-$CF_3$-benzyloxy |
| 829 | 3-F, 5-F-benzyloxy |
| 830 | cyclohexylmethyleneoxy |
| 831 | benzyloxy |
| 832 | 3-$CF_3$, 5-$CF_3$-benzyloxy |
| 833 | 4-$CF_3O$-benzyloxy |
| 834 | 4-$CH_3CH_2$-benzyloxy |
| 835 | isopropoxy |
| 836 | 3-$CF_3$-benzyl |
| 837 | isopropylthio |
| 838 | cyclopentoxy |
| 839 | 3-Cl-5-pyridinyloxy |
| 840 | 3-$CF_3S$-benzyloxy |
| 841 | 3-$CH_3$, 4-$CH_3$-benzyloxy |
| 842 | 2-F, 3-$CF_3$-benzyloxy |
| 843 | 3-F, 5-$CF_3$-benzyloxy |
| 844 | 4-$(CH_3)_2CH$-benzyloxy |
| 845 | 1-phenylethoxy |
| 846 | 4-F, 3-$CH_3$-benzoyl |
| 847 | 3-$CF_3$-phenyl |
| 848 | 4-$CH_3O$-phenylamino |

Example Table 9. (3*R*)-4-[*N*-(aryl)-[(aryl)methyl]amino]-1,1,1,2,2-pentafluoro-3-butanols (Continued).

| Ex. No. | $R_{SUB1}$ |
|---|---|
| 825 | 3-$CF_3$ |
| 826 | 2-$NO_2$ |

| Ex. No. | $R_{SUB2}$ |
|---|---|
| 849 | cyclopropoxy |
| 850 | 4-$NO_2$-phenylthio |

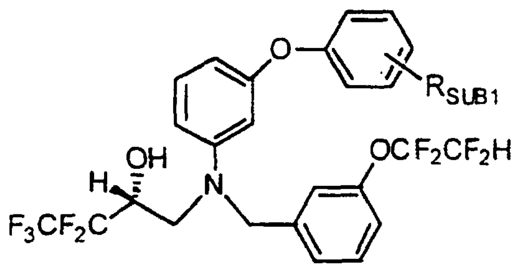

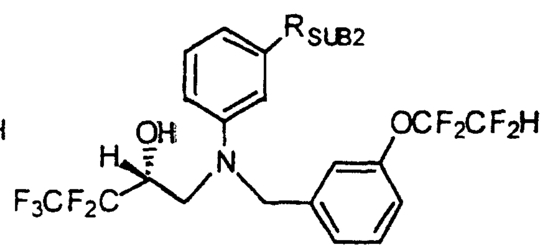

| Ex. No. | $R_{SUB1}$ |
|---|---|
| 851 | 3-isopropyl |
| 852 | 2-Cl, 3-Cl |
| 853 | 3-$CF_3O$ |
| 854 | 4-F |
| 855 | 4-$CH_3$ |
| 856 | 2-F, 5-Br |
| 857 | 4-Cl, 3-$CH_3CH_2$ |
| 858 | 3-$CH_3CH_2$ |
| 859 | 3-$CH_3$, 5-$CH_3$ |
| 860 | 3-$(CH_3)_3C$ |
| 861 | 4-F, 3-$CH_3$ |
| 862 | 3-Cl, 4-Cl |
| 863 | 3,4-$(CH_2)_4$ |
| 864 | 3-$HCF_2CF_2O$ |
| 865 | 3-$CHF_2O$ |
| 866 | 3-$(CH_3)_2N$ |
| 867 | 3-cyclopropyl |
| 868 | 3-(2-furyl) |

| Ex. No. | $R_{SUB2}$ |
|---|---|
| 875 | 3-$CF_3O$-benzyloxy |
| 876 | 3-$CF_3$-benzyloxy |
| 877 | 3-F, 5-F-benzyloxy |
| 878 | cyclohexylmethyleneoxy |
| 879 | benzyloxy |
| 880 | 3-$CF_3$, 5-$CF_3$-benzyloxy |
| 881 | 4-$CF_3O$-benzyloxy |
| 882 | 4-$CH_3CH_2$-benzyloxy |
| 883 | isopropoxy |
| 884 | 3-$CF_3$-benzyl |
| 885 | isopropylthio |
| 886 | cyclopentoxy |
| 887 | 3-Cl-5-pyridinyloxy |
| 888 | 3-$CF_3S$-benzyloxy |
| 889 | 3-$CH_3$, 4-$CH_3$-benzyloxy |
| 890 | 2-F, 3-$CF_3$-benzyloxy |
| 891 | 3-F, 5-$CF_3$-benzyloxy |
| 892 | 4-$(CH_3)_2CH$-benzyloxy |

Example Table 9. (3*R*)-4-[*N*-(aryl)-[(aryl)methyl]amino]-1,1,1,2,2-pentafluoro-3-butanols (Continued).

| Ex. No. | $R_{SUB1}$ |
|---|---|
| 869 | 3-$CF_3CF_2$ |
| 870 | 4-$NH_2$ |
| 871 | 3-$CH_3$, 4-$CH_3$, 5-$CH_3$ |
| 872 | 4-$CH_3CH_2CH_2O$ |
| 873 | 3-$CF_3$ |
| 874 | 2-$NO_2$ |

| Ex. No. | $R_{SUB2}$ |
|---|---|
| 893 | 1-phenylethoxy |
| 894 | 4-F, 3-$CH_3$-benzoyl |
| 895 | 3-$CF_3$-phenyl |
| 896 | 4-$CH_3O$-phenylamino |
| 897 | cyclopropoxy |
| 898 | 4-$NO_2$-phenylthio |

Example Table 10. Substituted (2*R*)-3-[*N*-(aryl)-[(aryl)oxy]amino]-1,1,1-trifluoro-2-propanols.

(Included for reference and/or comparison only)

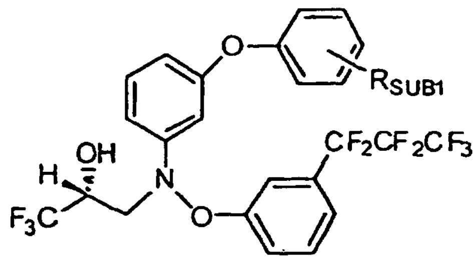

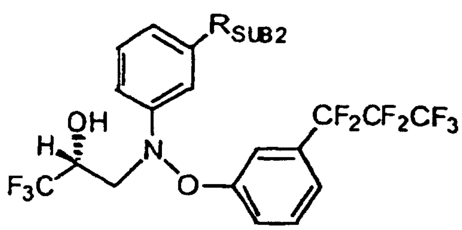

| Ex. No. | $R_{SUB1}$ | Ex. No. | $R_{SUB2}$ |
|---|---|---|---|
| 899 | 3-isopropyl | 923 | 3-$CF_3O$-benzyloxy |
| 900 | 2-Cl, 3-Cl | 924 | 3-$CF_3$-benzyloxy |
| 901 | 3-$CF_3O$ | 925 | 3-F, 5-F-benzyloxy |
| 902 | 4-F | 926 | cyclohexylmethyleneoxy |
| 903 | 4-$CH_3$ | 927 | benzyloxy |
| 904 | 2-F, 5-Br | 928 | 3-$CF_3$, 5-$CF_3$-benzyloxy |
| 905 | 4-Cl, 3-$CH_3CH_2$ | 929 | 4-$CF_3O$-benzyloxy |
| 906 | 3-$CH_3CH_2$ | 930 | 4-$CH_3CH_2$-benzyloxy |
| 907 | 3-$CH_3$, 5-$CH_3$ | 931 | isopropoxy |
| 908 | 3-$(CH_3)_3C$ | 932 | 3-$CF_3$-benzyl |
| 909 | 4-F, 3-$CH_3$ | 933 | isopropylthio |
| 910 | 3-Cl, 4-Cl | 934 | cyclopentoxy |
| 911 | 3,4-$(CH_2)_4$ | 935 | 3-Cl-5-pyridinyloxy |
| 912 | 3-$HCF_2CF_2O$ | 936 | 3-$CF_3S$-benzyloxy |
| 913 | 3-$CHF_2O$ | 937 | 3-$CH_3$, 4-$CH_3$-benzyloxy |
| 914 | 3-$(CH_3)_2N$ | 938 | 2-F, 3-$CF_3$-benzyloxy |
| 915 | 3-cyclopropyl | 939 | 3-F, 5-$CF_3$-benzyloxy |
| 916 | 3-(2-furyl) | 940 | 4-$(CH_3)_2CH$-benzyloxy |
| 917 | 3-$CF_3CF_2$ | 941 | 1-phenylethoxy |
| 918 | 4-$NH_2$ | 942 | 4-F, 3-$CH_3$-benzoyl |
| 919 | 3-$CH_3$, 4-$CH_3$, 5-$CH_3$ | 943 | 3-$CF_3$-phenyl |
| 920 | 4-$CH_3CH_2CH_2O$ | 944 | 4-$CH_3O$-phenylamino |
| 921 | 3-$CF_3$ | 945 | cyclopropoxy |

Example Table 10 (continued). Substituted (2*R*)-3-[*N*-(aryl)-[(aryl)oxy]amino]-1,1,1-trifluoro-2-propanols.

| Ex. No. | $R_{SUB1}$ |
|---|---|
| 922 | $2-NO_2$ |

| Ex. No. | $R_{SUB2}$ |
|---|---|
| 946 | $4-NO_2$-phenylthio |

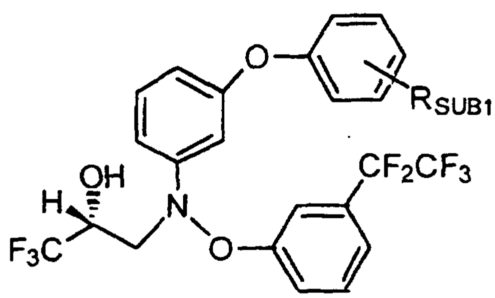

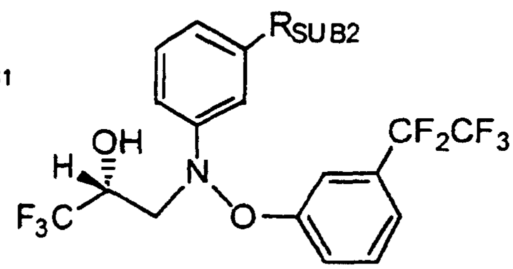

| Ex. No. | $R_{SUB1}$ |
|---|---|
| 947 | 3-isopropyl |
| 948 | 2-Cl, 3-Cl |
| 949 | $3-CF_3O$ |
| 950 | 4-F |
| 951 | $4-CH_3$ |
| 952 | 2-F, 5-Br |
| 953 | 4-Cl, $3-CH_3CH_2$ |
| 954 | $3-CH_3CH_2$ |
| 955 | $3-CH_3$, $5-CH_3$ |
| 956 | $3-(CH_3)_3C$ |
| 957 | 4-F, $3-CH_3$ |
| 958 | 3-Cl, 4-Cl |
| 959 | $3,4-(CH_2)_4$ |
| 960 | $3-HCF_2CF_2O$ |
| 961 | $3-CHF_2O$ |
| 962 | $3-(CH_3)_2N$ |
| 963 | 3-cyclopropyl |
| 964 | 3-(2-furyl) |

| Ex. No. | $R_{SUB2}$ |
|---|---|
| 971 | $3-CF_3O$-benzyloxy |
| 972 | $3-CF_3$-benzyloxy |
| 973 | 3-F, 5-F-benzyloxy |
| 974 | cyclohexylmethyleneoxy |
| 975 | benzyloxy |
| 976 | $3-CF_3$, $5-CF_3$-benzyloxy |
| 977 | $4-CF_3O$-benzyloxy |
| 978 | $4-CH_3CH_2$-benzyloxy |
| 979 | isopropoxy |
| 980 | $3-CF_3$-benzyl |
| 981 | isopropylthio |
| 982 | cyclopentoxy |
| 983 | 3-Cl-5-pyridinyloxy |
| 984 | $3-CF_3S$-benzyloxy |
| 985 | $3-CH_3$, $4-CH_3$-benzyloxy |
| 986 | 2-F, $3-CF_3$-benzyloxy |
| 987 | 3-F, $5-CF_3$-benzyloxy |
| 988 | $4-(CH_3)_2CH$-benzyloxy |

Example Table 10 (continued). Substituted (2*R*)-3-[*N*-(aryl)-[(aryl)oxy]amino]-1,1,1-trifluoro-2-propanols.

| Ex. No. | RSUB1 |
|---|---|
| 965 | 3-$CF_3CF_2$ |
| 966 | 4-$NH_2$ |
| 967 | 3-$CH_3$, 4-$CH_3$, 5-$CH_3$ |
| 968 | 4-$CH_3CH_2CH_2O$ |
| 969 | 3-$CF_3$ |
| 970 | 2-$NO_2$ |

| Ex. No. | RSUB2 |
|---|---|
| 989 | 1-phenylethoxy |
| 990 | 4-F, 3-$CH_3$-benzoyl |
| 991 | 3-$CF_3$-phenyl |
| 992 | 4-$CH_3O$-phenylamino |
| 993 | cyclopropoxy |
| 994 | 4-$NO_2$-phenylthio |

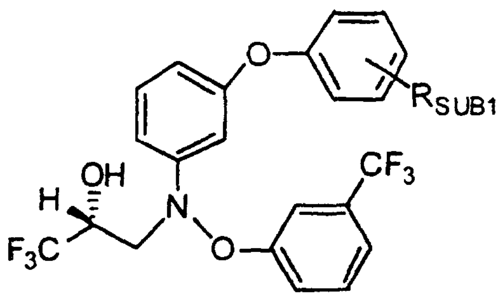

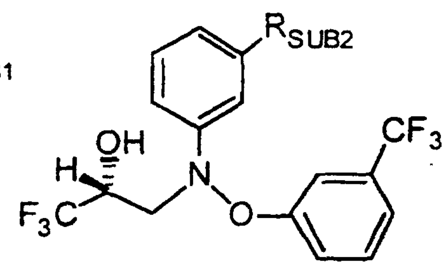

| Ex. No. | RSUB1 |
|---|---|
| 995 | 3-isopropyl |
| 996 | 2-Cl, 3-Cl |
| 997 | 3-$CF_3O$ |
| 998 | 4-F |
| 999 | 4-$CH_3$ |
| 1000 | 2-F, 5-Br |
| 1001 | 4-Cl, 3-$CH_3CH_2$ |
| 1002 | 3-$CH_3CH_2$ |
| 1003 | 3-$CH_3$, 5-$CH_3$ |
| 1004 | 3-$(CH_3)_3C$ |
| 1005 | 4-F, 3-$CH_3$ |
| 1006 | 3-Cl, 4-Cl |
| 1007 | 3,4-$(CH_2)_4$ |

| Ex. No. | RSUB2 |
|---|---|
| 1019 | 3-$CF_3O$-benzyloxy |
| 1020 | 3-$CF_3$-benzyloxy |
| 1021 | 3-F, 5-F-benzyloxy |
| 1022 | cyclohexylmethyleneoxy |
| 1023 | benzyloxy |
| 1024 | 3-$CF_3$, 5-$CF_3$-benzyloxy |
| 1025 | 4-$CF_3O$-benzyloxy |
| 1026 | 4-$CH_3CH_2$-benzyloxy |
| 1027 | isopropoxy |
| 1028 | 3-$CF_3$-benzyl |
| 1029 | isopropylthio |
| 1030 | cyclopentoxy |
| 1031 | 3-Cl-5-pyridinyloxy |

Example Table 10 (continued). Substituted (2*R*)-3-[*N*-(aryl)-[(aryl)oxy]amino]-1,1,1-trifluoro-2-propanols.

| Ex. No. | $R_{SUB1}$ |
|---|---|
| 1008 | 3-$HCF_2CF_2O$ |
| 1009 | 3-$CHF_2O$ |
| 1010 | 3-$(CH_3)_2N$ |
| 1011 | 3-cyclopropyl |
| 1012 | 3-(2-furyl) |
| 1013 | 3-$CF_3CF_2$ |
| 1014 | 4-$NH_2$ |
| 1015 | 3-$CH_3$, 4-$CH_3$, 5-$CH_3$ |
| 1016 | 4-$CH_3CH_2CH_2O$ |
| 1017 | 3-$CF_3$ |
| 1018 | 2-$NO_2$ |

| Ex. No. | $R_{SUB2}$ |
|---|---|
| 1032 | 3-$CF_3S$-benzyloxy |
| 1033 | 3-$CH_3$, 4-$CH_3$-benzyloxy |
| 1034 | 2-F, 3-$CF_3$-benzyloxy |
| 1035 | 3-F, 5-$CF_3$-benzyloxy |
| 1036 | 4-$(CH_3)_2CH$-benzyloxy |
| 1037 | 1-phenylethoxy |
| 1038 | 4-F, 3-$CH_3$-benzoyl |
| 1039 | 3-$CF_3$-phenyl |
| 1040 | 4-$CH_3O$-phenylamino |
| 1041 | cyclopropoxy |
| 1042 | 4-$NO_2$-phenylthio |

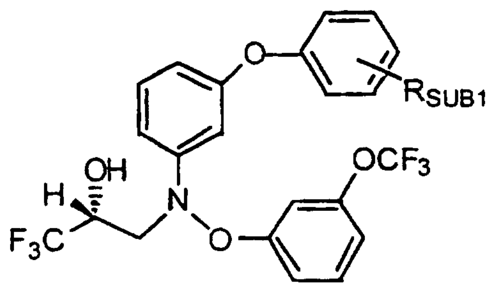

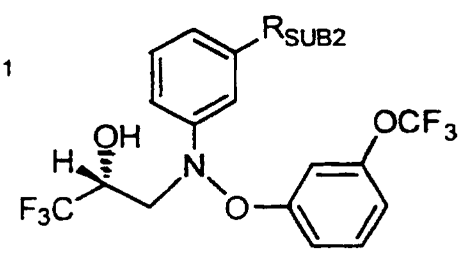

| Ex. No. | $R_{SUB1}$ |
|---|---|
| 1043 | 3-isopropyl |
| 1044 | 2-Cl, 3-Cl |
| 1045 | 3-$CF_3O$ |
| 1046 | 4-F |
| 1047 | 4-$CH_3$ |
| 1048 | 2-F, 5-Br |
| 1049 | 4-Cl, 3-$CH_3CH_2$ |
| 1050 | 3-$CH_3CH_2$ |

| Ex. No. | $R_{SUB2}$ |
|---|---|
| 1067 | 3-$CF_3O$-benzyloxy |
| 1068 | 3-$CF_3$-benzyloxy |
| 1069 | 3-F, 5-F-benzyloxy |
| 1070 | cyclohexylmethyleneoxy |
| 1071 | benzyloxy |
| 1072 | 3-$CF_3$, 5-$CF_3$-benzyloxy |
| 1073 | 4-$CF_3O$-benzyloxy |
| 1074 | 4-$CH_3CH_2$-benzyloxy |

Example Table 10 (continued). Substituted (2*R*)-3-[*N*-(aryl)-[(aryl)oxy]amino]-1,1,1-trifluoro-2-propanols.

| Ex. No. | RSUB1 |
|---|---|
| 1051 | 3-$CH_3$, 5-$CH_3$ |
| 1052 | 3-$(CH_3)_3C$ |
| 1053 | 4-F, 3-$CH_3$ |
| 1054 | 3-Cl, 4-Cl |
| 1055 | 3,4-$(CH_2)_4$ |
| 1056 | 3-$HCF_2CF_2O$ |
| 1057 | 3-$CHF_2O$ |
| 1058 | 3-$(CH_3)_2N$ |
| 1059 | 3-cyclopropyl |
| 1060 | 3-(2-furyl) |
| 1061 | 3-$CF_3CF_2$ |
| 1062 | 4-$NH_2$ |
| 1063 | 3-$CH_3$, 4-$CH_3$, 5-$CH_3$ |
| 1064 | 4-$CH_3CH_2CH_2O$ |
| 1065 | 3-$CF_3$ |
| 1066 | 2-$NO_2$ |

| Ex. No. | RSUB2 |
|---|---|
| 1075 | isopropoxy |
| 1076 | 3-$CF_3$-benzyl |
| 1077 | isopropylthio |
| 1078 | cyclopentoxy |
| 1079 | 3-Cl-5-pyridinyloxy |
| 1080 | 3-$CF_3S$-benzyloxy |
| 1081 | 3-$CH_3$, 4-$CH_3$-benzyloxy |
| 1082 | 2-F, 3-$CF_3$-benzyloxy |
| 1083 | 3-F, 5-$CF_3$-benzyloxy |
| 1084 | 4-$(CH_3)_2CH$-benzyloxy |
| 1085 | 1-phenylethoxy |
| 1086 | 4-F, 3-$CH_3$-benzoyl |
| 1087 | 3-$CF_3$-phenyl |
| 1088 | 4-$CH_3O$-phenylamino |
| 1089 | cyclopropoxy |
| 1090 | 4-$NO_2$-phenylthio |

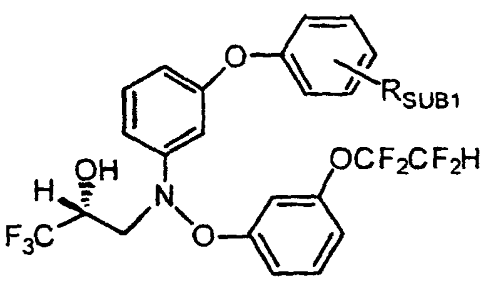

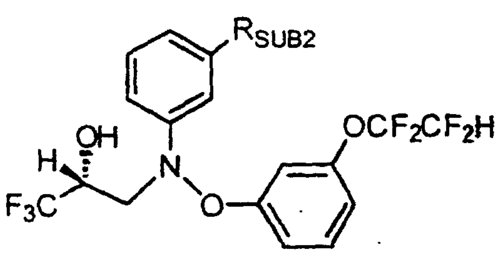

| Ex. No. | RSUB1 |
|---|---|
| 1091 | 3-isopropyl |
| 1092 | 2-Cl, 3-Cl |
| 1093 | 3-$CF_3O$ |
| 1094 | 4-F |

| Ex. No. | RSUB2 |
|---|---|
| 1115 | 3-$CF_3O$-benzyloxy |
| 1116 | 3-$CF_3$-benzyloxy |
| 1117 | 3-F, 5-F-benzyloxy |
| 1118 | cyclohexylmethyleneoxy |

Example Table 10 (continued). Substituted (2*R*)-3-[*N*-(aryl)-[(aryl)oxy]amino]-1,1,1-trifluoro-2-propanols.

| Ex. No. | $R_{SUB1}$ | Ex. No. | $R_{SUB2}$ |
|---|---|---|---|
| 1095 | 4-$CH_3$ | 1119 | benzyloxy |
| 1096 | 2-F, 5-Br | 1120 | 3-$CF_3$, 5-$CF_3$-benzyloxy |
| 1097 | 4-Cl, 3-$CH_3CH_2$ | 1121 | 4-$CF_3O$-benzyloxy |
| 1098 | 3-$CH_3CH_2$ | 1122 | 4-$CH_3CH_2$-benzyloxy |
| 1099 | 3-$CH_3$, 5-$CH_3$ | 1123 | isopropoxy |
| 1100 | 3-$(CH_3)_3C$ | 1124 | 3-$CF_3$-benzyl |
| 1101 | 4-F, 3-$CH_3$ | 1125 | isopropylthio |
| 1102 | 3-Cl, 4-Cl | 1126 | cyclopentoxy |
| 1103 | 3,4-$(CH_2)_4$ | 1127 | 3-Cl-5-pyridinyloxy |
| 1104 | 3-$HCF_2CF_2O$ | 1128 | 3-$CF_3S$-benzyloxy |
| 1105 | 3-$CHF_2O$ | 1129 | 3-$CH_3$, 4-$CH_3$-benzyloxy |
| 1106 | 3-$(CH_3)_2N$ | 1130 | 2-F, 3-$CF_3$-benzyloxy |
| 1107 | 3-cyclopropyl | 1131 | 3-F, 5-$CF_3$-benzyloxy |
| 1108 | 3-(2-furyl) | 1132 | 4-$(CH_3)_2CH$-benzyloxy |
| 1109 | 3-$CF_3CF_2$ | 1133 | 1-phenylethoxy |
| 1110 | 4-$NH_2$ | 1134 | 4-F, 3-$CH_3$-benzoyl |
| 1111 | 3-$CH_3$, 4-$CH_3$, 5-$CH_3$ | 1135 | 3-$CF_3$-phenyl |
| 1112 | 4-$CH_3CH_2CH_2O$ | 1136 | 4-$CH_3O$-phenylamino |
| 1113 | 3-$CF_3$ | 1137 | cyclopropoxy |
| 1114 | 2-$NO_2$ | 1138 | 4-$NO_2$-phenylthio |

Example Table 11. (2*R*)-3-[*N*-(aryl)-[(aryl)methyl]amino]-1,1-difluoro-1-chloro-2-propanols.

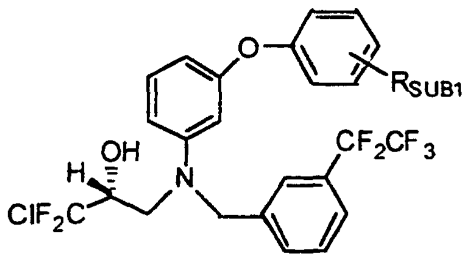

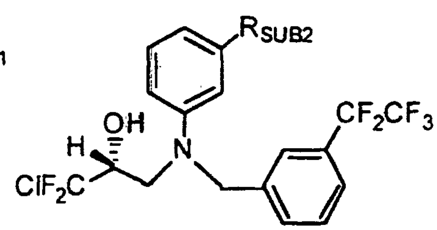

| Ex. No. | $R_{SUB1}$ |
|---|---|
| 1139 | 3-isopropyl |
| 1140 | 2-Cl, 3-Cl |
| 1141 | 3-$CF_3O$ |
| 1142 | 4-F |
| 1143 | 4-$CH_3$ |
| 1144 | 2-F, 5-Br |
| 1145 | 4-Cl, 3-$CH_3CH_2$ |
| 1146 | 3-$CH_3CH_2$ |
| 1147 | 3-$CH_3$, 5-$CH_3$ |
| 1148 | 3-$(CH_3)_3C$ |
| 1149 | 4-F, 3-$CH_3$ |
| 1150 | 3-Cl, 4-Cl |
| 1151 | 3,4-$(CH_2)_4$ |
| 1152 | 3-$HCF_2CF_2O$ |
| 1153 | 3-$CHF_2O$ |
| 1154 | 3-$(CH_3)_2N$ |
| 1155 | 3-cyclopropyl |
| 1156 | 3-(2-furyl) |
| 1157 | 3-$CF_3CF_2$ |
| 1158 | 4-$NH_2$ |
| 1159 | 3-$CH_3$, 4-$CH_3$, 5-$CH_3$ |
| 1160 | 4-$CH_3CH_2CH_2O$ |
| 1161 | 3-$CF_3$ |

| Ex. No. | $R_{SUB2}$ |
|---|---|
| 1163 | 3-$CF_3O$-benzyloxy |
| 1164 | 3-$CF_3$-benzyloxy |
| 1165 | 3-F, 5-F-benzyloxy |
| 1166 | cyclohexylmethyleneoxy |
| 1167 | benzyloxy |
| 1168 | 3-$CF_3$, 5-$CF_3$-benzyloxy |
| 1169 | 4-$CF_3O$-benzyloxy |
| 1170 | 4-$CH_3CH_2$-benzyloxy |
| 1171 | isopropoxy |
| 1172 | 3-$CF_3$-benzyl |
| 1173 | isopropylthio |
| 1174 | cyclopentoxy |
| 1175 | 3-Cl-5-pyridinyloxy |
| 1176 | 3-$CF_3S$-benzyloxy |
| 1177 | 3-$CH_3$, 4-$CH_3$-benzyloxy |
| 1178 | 2-F, 3-$CF_3$-benzyloxy |
| 1179 | 3-F, 5-$CF_3$-benzyloxy |
| 1180 | 4-$(CH_3)_2CH$-benzyloxy |
| 1181 | 1-phenylethoxy |
| 1182 | 4-F, 3-$CH_3$-benzoyl |
| 1183 | 3-$CF_3$-phenyl |
| 1184 | 4-$CH_3O$-phenylamino |
| 1185 | cyclopropoxy |

Example Table 11 (continued). (2*R*)-3-[*N*-(aryl)-[(aryl)methyl]amino]-1,1-difluoro-1-chloro-2-propanols.

| Ex. No. | RSUB1 |
|---|---|
| 1162 | 2-$NO_2$ |

| Ex. No. | RSUB2 |
|---|---|
| 1186 | 4-$NO_2$-phenylthio |

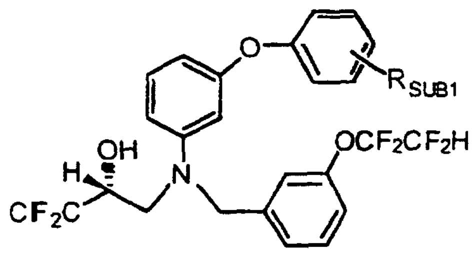

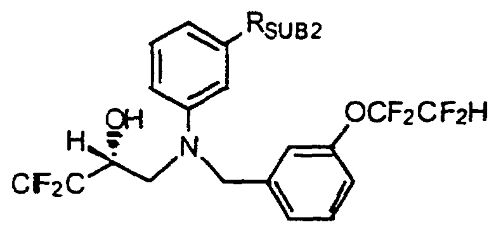

| Ex. No. | RSUB1 |
|---|---|
| 1187 | 3-isopropyl |
| 1188 | 2-Cl, 3-Cl |
| 1189 | 3-$CF_3O$ |
| 1190 | 4-F |
| 1191 | 4-$CH_3$ |
| 1192 | 2-F, 5-Br |
| 1193 | 4-Cl, 3-$CH_3CH_2$ |
| 1194 | 3-$CH_3CH_2$ |
| 1195 | 3-$CH_3$, 5-$CH_3$ |
| 1196 | 3-$(CH_3)_3C$ |
| 1197 | 4-F, 3-$CH_3$ |
| 1198 | 3-Cl, 4-Cl |
| 1199 | 3,4-$(CH_2)_4$ |
| 1200 | 3-$HCF_2CF_2O$ |
| 1201 | 3-$CHF_2O$ |
| 1202 | 3-$(CH_3)_2N$ |
| 1203 | 3-cyclopropyl |
| 1204 | 3-(2-furyl) |
| 1205 | 3-$CF_3CF_2$ |

| Ex. No. | RSUB2 |
|---|---|
| 1211 | 3-$CF_3O$-benzyloxy |
| 1212 | 3-$CF_3$-benzyloxy |
| 1213 | 3-F, 5-F-benzyloxy |
| 1214 | cyclohexylmethyleneoxy |
| 1215 | benzyloxy |
| 1216 | 3-$CF_3$, 5-$CF_3$-benzyloxy |
| 1217 | 4-$CF_3O$-benzyloxy |
| 1218 | 4-$CH_3CH_2$-benzyloxy |
| 1219 | isopropoxy |
| 1220 | 3-$CF_3$-benzyl |
| 1221 | isopropylthio |
| 1222 | cyclopentoxy |
| 1223 | 3-Cl-5-pyridinyloxy |
| 1224 | 3-$CF_3S$-benzyloxy |
| 1225 | 3-$CH_3$, 4-$CH_3$-benzyloxy |
| 1226 | 2-F, 3-$CF_3$-benzyloxy |
| 1227 | 3-F, 5-$CF_3$-benzyloxy |
| 1228 | 4-$(CH_3)_2CH$-benzyloxy |
| 1229 | 1-phenylethoxy |

Example Table 11 (continued). (2*R*)-3-[*N*-(aryl)-[(aryl)methyl]amino]-1,1-difluoro-1-chloro-2-propanols.

| Ex. No. | $R_{SUB1}$ |
|---|---|
| 1206 | 4-$NH_2$ |
| 1207 | 3-$CH_3$, 4-$CH_3$, 5-$CH_3$ |
| 1208 | 4-$CH_3CH_2CH_2O$ |
| 1209 | 3-$CF_3$ |
| 1210 | 2-$NO_2$ |

| Ex. No. | $R_{SUB2}$ |
|---|---|
| 1230 | 4-F, 3-$CH_3$-benzoyl |
| 1231 | 3-$CF_3$-phenyl |
| 1232 | 4-$CH_3O$-phenylamino |
| 1233 | cyclopropoxy |
| 1234 | 4-$NO_2$-phenylthio |

Example Table 12. (2*R*)-3-[*N*,*N*'-(diaryl)amino]-1,1,1-trifluoro-2-propanols.
(Included for reference and/or comparison only)

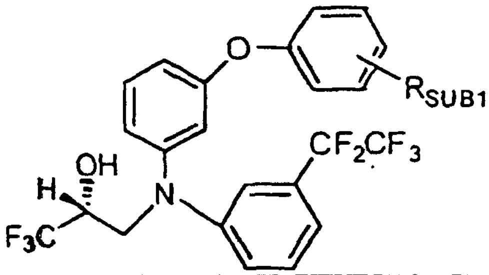

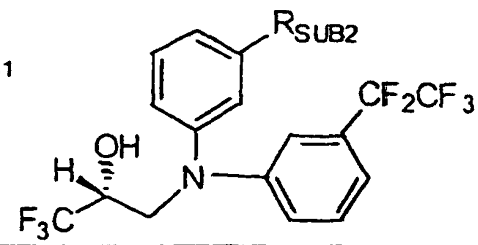

| Ex. No. | $R_{SUB1}$ |
|---|---|
| 1235 | 3-isopropyl |
| 1236 | 2-Cl, 3-Cl |
| 1237 | 3-$CF_3O$ |
| 1238 | 4-F |
| 1239 | 4-$CH_3$ |
| 1240 | 2-F, 5-Br |
| 1241 | 4-Cl, 3-$CH_3CH_2$ |
| 1242 | 3-$CH_3CH_2$ |
| 1243 | 3-$CH_3$, 5-$CH_3$ |
| 1244 | 3-$(CH_3)_3C$ |
| 1245 | 4-F, 3-$CH_3$ |
| 1246 | 3-Cl, 4-Cl |
| 1247 | 3,4-$(CH_2)_4$ |
| 1248 | 3-$HCF_2CF_2O$ |
| 1249 | 3-$CHF_2O$ |
| 1250 | 3-$(CH_3)_2N$ |
| 1251 | 3-cyclopropyl |
| 1252 | 3-(2-furyl) |
| 1253 | 3-$CF_3CF_2$ |
| 1254 | 4-$NH_2$ |
| 1255 | 3-$CH_3$, 4-$CH_3$, 5-$CH_3$ |
| 1256 | 4-$CH_3CH_2CH_2O$ |
| 1257 | 3-$CF_3$ |

| Ex. No. | $R_{SUB2}$ |
|---|---|
| 1259 | 3-$CF_3O$-benzyloxy |
| 1260 | 3-$CF_3$-benzyloxy |
| 1261 | 3-F, 5-F-benzyloxy |
| 1262 | cyclohexylmethyleneoxy |
| 1263 | benzyloxy |
| 1264 | 3-$CF_3$, 5-$CF_3$-benzyloxy |
| 1265 | 4-$CF_3O$-benzyloxy |
| 1266 | 4-$CH_3CH_2$-benzyloxy |
| 1267 | isopropoxy |
| 1268 | 3-$CF_3$-benzyl |
| 1269 | isopropylthio |
| 1270 | cyclopentoxy |
| 1271 | 3-Cl-5-pyridinyloxy |
| 1272 | 3-$CF_3S$-benzyloxy |
| 1273 | 3-$CH_3$, 4-$CH_3$-benzyloxy |
| 1274 | 2-F, 3-$CF_3$-benzyloxy |
| 1275 | 3-F, 5-$CF_3$-benzyloxy |
| 1276 | 4-$(CH_3)_2CH$-benzyloxy |
| 1277 | 1-phenylethoxy |
| 1278 | 4-F, 3-$CH_3$-benzoyl |
| 1279 | 3-$CF_3$-phenyl |
| 1280 | 4-$CH_3O$-phenylamino |
| 1281 | cyclopropoxy |

Example Table 12 (continued). (2*R*)-3-[*N*,*N*'-(diaryl)amino]-1,1,1-trifluoro-2-propanols.

| Ex. No. | RSUB1 | Ex. No. | RSUB2 |
|---|---|---|---|
| 1258 | 2-$NO_2$ | 1282 | 4-$NO_2$-phenylthio |

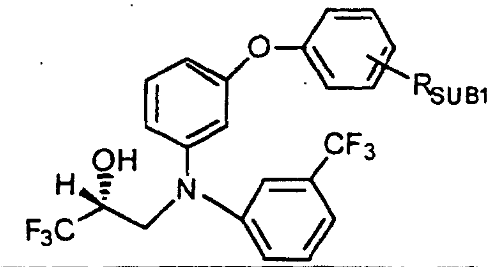

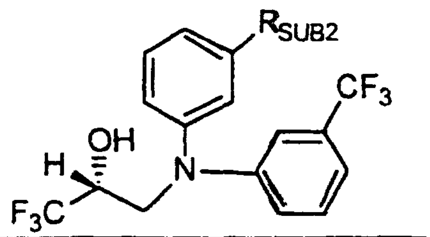

| Ex. No. | RSUB1 | Ex. No. | RSUB2 |
|---|---|---|---|
| 1283 | 3-isopropyl | 1307 | 3-$CF_3O$-benzyloxy |
| 1284 | 2-Cl, 3-Cl | 1308 | 3-$CF_3$-benzyloxy |
| 1285 | 3-$CF_3O$ | 1309 | 3-F, 5-F-benzyloxy |
| 1286 | 4-F | 1310 | cyclohexylmethyleneoxy |
| 1287 | 4-$CH_3$ | 1311 | benzyloxy |
| 1288 | 2-F, 5-Br | 1312 | 3-$CF_3$, 5-$CF_3$-benzyloxy |
| 1289 | 4-Cl, 3-$CH_3CH_2$ | 1313 | 4-$CF_3O$-benzyloxy |
| 1290 | 3-$CH_3CH_2$ | 1314 | 4-$CH_3CH_2$-benzyloxy |
| 1291 | 3-$CH_3$, 5-$CH_3$ | 1315 | isopropoxy |
| 1292 | 3-$(CH_3)_3C$ | 1316 | 3-$CF_3$-benzyl |
| 1293 | 4-F, 3-$CH_3$ | 1317 | isopropylthio |
| 1294 | 3-Cl, 4-Cl | 1318 | cyclopentoxy |
| 1295 | 3,4-$(CH_2)_4$ | 1319 | 3-Cl-5-pyridinyloxy |
| 1296 | 3-$HCF_2CF_2O$ | 1320 | 3-$CF_3S$-benzyloxy |
| 1297 | 3-$CHF_2O$ | 1321 | 3-$CH_3$, 4-$CH_3$-benzyloxy |
| 1298 | 3-$(CH_3)_2N$ | 1322 | 2-F, 3-$CF_3$-benzyloxy |
| 1299 | 3-cyclopropyl | 1323 | 3-F, 5-$CF_3$-benzyloxy |
| 1300 | 3-(2-furyl) | 1324 | 4-$(CH_3)_2CH$-benzyloxy |

Example Table 12 (continued). (2*R*)-3-[*N*,*N*'-(diaryl)amino]-1,1,1-trifluoro-2-propanols.

| Ex. No. | $R_{SUB1}$ |
|---|---|
| 1301 | 3-$CF_3CF_2$ |
| 1302 | 4-$NH_2$ |
| 1303 | 3-$CH_3$, 4-$CH_3$, 5-$CH_3$ |
| 1304 | 4-$CH_3CH_2CH_2O$ |
| 1305 | 3-$CF_3$ |
| 1306 | 2-$NO_2$ |

| Ex. No. | $R_{SUB2}$ |
|---|---|
| 1325 | 1-phenylethoxy |
| 1326 | 4-F, 3-$CH_3$-benzoyl |
| 1327 | 3-$CF_3$-phenyl |
| 1328 | 4-$CH_3O$-phenylamino |
| 1329 | cyclopropoxy |
| 1330 | 4-$NO_2$-phenylthio |

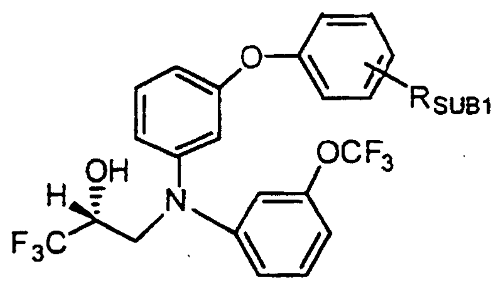

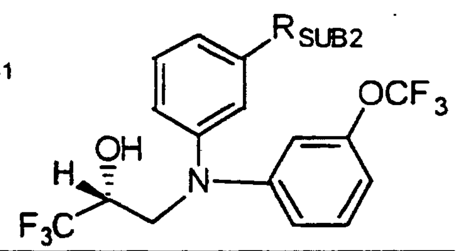

| Ex. No. | $R_{SUB1}$ |
|---|---|
| 1331 | 3-isopropyl |
| 1332 | 2-Cl, 3-Cl |
| 1333 | 3-$CF_3O$ |
| 1334 | 4-F |
| 1335 | 4-$CH_3$ |
| 1336 | 2-F, 5-Br |
| 1337 | 4-Cl, 3-$CH_3CH_2$ |
| 1338 | 3-$CH_3CH_2$ |
| 1339 | 3-$CH_3$, 5-$CH_3$ |
| 1340 | 3-$(CH_3)_3C$ |
| 1341 | 4-F, 3-$CH_3$ |
| 1342 | 3-Cl, 4-Cl |
| 1343 | 3,4-$(CH_2)_4$ |

| Ex. No. | $R_{SUB2}$ |
|---|---|
| 1355 | 3-$CF_3O$-benzyloxy |
| 1356 | 3-$CF_3$-benzyloxy |
| 1357 | 3-F, 5-F-benzyloxy |
| 1358 | cyclohexylmethyleneoxy |
| 1359 | benzyloxy |
| 1360 | 3-$CF_3$, 5-$CF_3$-benzyloxy |
| 1361 | 4-$CF_3O$-benzyloxy |
| 1362 | 4-$CH_3CH_2$-benzyloxy |
| 1363 | isopropoxy |
| 1364 | 3-$CF_3$-benzyl |
| 1365 | isopropylthio |
| 1366 | cyclopentoxy |
| 1367 | 3-Cl-5-pyridinyloxy |

Example Table 12 (continued). (2*R*)-3-[*N*,*N'*-(diaryl)amino]-1.1.1-trifluoro-2-propanols.

| Ex. No. | RSUB1 |
|---|---|
| 1344 | 3-$HCF_2CF_2O$ |
| 1345 | 3-$CHF_2O$ |
| 1346 | 3-$(CH_3)_2N$ |
| 1347 | 3-cyclopropyl |
| 1348 | 3-(2-furyl) |
| 1349 | 3-$CF_3CF_2$ |
| 1350 | 4-$NH_2$ |
| 1351 | 3-$CH_3$, 4-$CH_3$, 5-$CH_3$ |
| 1352 | 4-$CH_3CH_2CH_2O$ |
| 1353 | 3-$CF_3$ |
| 1354 | 2-$NO_2$ |

| Ex. No. | RSUB2 |
|---|---|
| 1368 | 3-$CF_3S$-benzyloxy |
| 1369 | 3-$CH_3$, 4-$CH_3$-benzyloxy |
| 1370 | 2-F, 3-$CF_3$-benzyloxy |
| 1371 | 3-F, 5-$CF_3$-benzyloxy |
| 1372 | 4-$(CH_3)_2CH$-benzyloxy |
| 1373 | 1-phenylethoxy |
| 1374 | 4-F, 3-$CH_3$-benzoyl |
| 1375 | 3-$CF_3$-phenyl |
| 1376 | 4-$CH_3O$-phenylamino |
| 1377 | cyclopropoxy |
| 1378 | 4-$NO_2$-phenylthio |

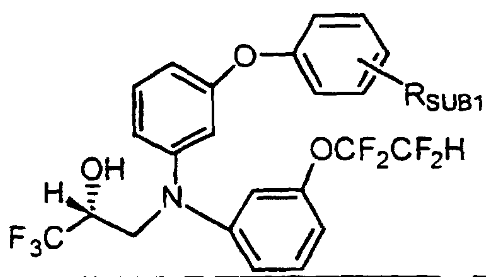

| Ex. No. | RSUB1 |
|---|---|
| 1379 | 3-isopropyl |
| 1380 | 2-Cl, 3-Cl |
| 1381 | 3-$CF_3O$ |
| 1382 | 4-F |
| 1383 | 4-$CH_3$ |
| 1384 | 2-F, 5-Br |
| 1385 | 4-Cl, 3-$CH_3CH_2$ |
| 1386 | 3-$CH_3CH_2$ |

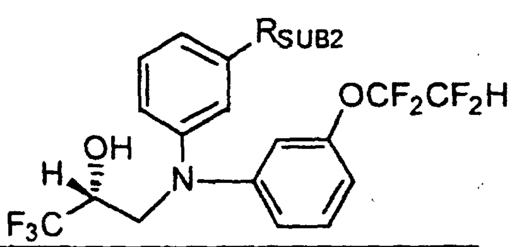

| Ex. No. | RSUB2 |
|---|---|
| 1403 | 3-$CF_3O$-benzyloxy |
| 1404 | 3-$CF_3$-benzyloxy |
| 1405 | 3-F, 5-F-benzyloxy |
| 1406 | cyclohexylmethyleneoxy |
| 1407 | benzyloxy |
| 1408 | 3-$CF_3$, 5-$CF_3$-benzyloxy |
| 1409 | 4-$CF_3O$-benzyloxy |
| 1410 | 4-$CH_3CH_2$-benzyloxy |

Example Table 12 (continued). (2*R*)-3-[*N*,*N'*-(diaryl)amino]-1,1,1-trifluoro-2-propanols.

| Ex. No. | $R_{SUB1}$ | Ex. No. | $R_{SUB2}$ |
|---|---|---|---|
| 1387 | 3-$CH_3$, 5-$CH_3$ | 1411 | isopropoxy |
| 1388 | 3-$(CH_3)_3C$ | 1412 | 3-$CF_3$-benzyl |
| 1389 | 4-F, 3-$CH_3$ | 1413 | isopropylthio |
| 1390 | 3-Cl, 4-Cl | 1414 | cyclopentoxy |
| 1391 | 3,4-$(CH_2)_4$ | 1415 | 3-Cl-5-pyridinyloxy |
| 1392 | 3-$HCF_2CF_2O$ | 1416 | 3-$CF_3S$-benzyloxy |
| 1393 | 3-$CHF_2O$ | 1417 | 3-$CH_3$, 4-$CH_3$-benzyloxy |
| 1394 | 3-$(CH_3)_2N$ | 1418 | 2-F, 3-$CF_3$-benzyloxy |
| 1395 | 3-cyclopropyl | 1419 | 3-F, 5-$CF_3$-benzyloxy |
| 1396 | 3-(2-furyl) | 1420 | 4-$(CH_3)_2CH$-benzyloxy |
| 1397 | 3-$CF_3CF_2$ | 1421 | 1-phenylethoxy |
| 1398 | 4-$NH_2$ | 1422 | 4-F, 3-$CH_3$-benzoyl |
| 1399 | 3-$CH_3$, 4-$CH_3$, 5-$CH_3$ | 1423 | 3-$CF_3$-phenyl |
| 1400 | 4-$CH_3CH_2CH_2O$ | 1424 | 4-$CH_3O$-phenylamino |
| 1401 | 3-$CF_3$ | 1425 | cyclopropoxy |
| 1402 | 2-$NO_2$ | 1426 | 4-$NO_2$-phenylthio |

## BIOASSAYS

### CETP Activity *In Vitro*

ASSAY OF CETP INHIBITION USING PURIFIED COMPONENTS (RECONSTITUTED BUFFER ASSAY)

The ability of compounds to inhibit CETP activity was assessed using an *in vitro* assay that measured the rate of transfer of radiolabeled cholesteryl ester ([$^3$H]CE) from HDL donor particles to LDL acceptor particles. Details of the assay are provided by Glenn. K. C. et al. (Glenn and Melton, "Quantification of Cholesteryl Ester Transfer Protein (CETP): A) CETP Activity and B) Immunochemical Assay of CETP Protein," *Meth. Enzymol.*, 263, 339-351 (1996)). Human recombinant CETP can be obtained from the serum-free conditioned medium of CHO cells transfected with a cDNA for CETP and purified as described by Wang, S. et al. (*J. Biol. Chem. 267*, 17487-17490 (1992)). To measure CETP activity, [$^3$H]CE-labeled-HDL, LDL, CETP and assay buffer (50 mM tris(hydroxymethyl)aminomethane, pH 7.4: 150 mM sodium chloride; 2 mM ethylenediamine-tetraacetic acid (EDTA); 1% bovine serum albumin) were incubated in a final volume of 200 µL, for 2 hours at 37 °C in 96 well plates. Inhibitors were included in the assay by diluting from a 10 mM DMSO stock solution into 16% (v/v) aqueous DMSO so that the final concentration of inhibitor was 800 µM. The inhibitors were then diluted 1:1 with CETP in assay buffer, and then 25 µL of that solution was mixed with 175 µL of lipoprotein pool for assay. Following incubation, LDL was differentially precipitated by the addition of 50 µL of 1% (w/v) dextran sulfate/0.5 M magnesium chloride, mixed by vortex, and incubated at room temperature for 10 minutes.

A potion of the solution (200 µL) was transferred to a filter plate (Millipore). After filtration, the radioactivity present in the precipitated LDL was measured by liquid scintillation counting. Correction for non-specific transfer or precipitation was made by including samples that do not contain CETP. The rate of [$^3$H]CE transfer using this assay was linear with respect to time and CETP concentration. up to 25-30% of [$^3$H]CE transferred.

The potency of test compounds was determined by performing the above described assay in the presence of varying concentrations of the test compounds and determining the concentration required for 50% inhibition of transfer of [$^3$H]CE from HDL to LDL. This value was defined as the $IC_{50}$. The $IC_{50}$ values determined from this assay are accurate when the $IC_{50}$ is greater than 10 nM. In the case where compounds have greater inhibitory potency, accurate measurements of $IC_{50}$ may be determined using longer incubation times (up to 18 hours) and lower final concentrations of CETP (< 50 nM).

Examples of $IC_{50}$ values determined by these methods are summarized in Table 9.

**ASSAY OF CETP INHIBITION IN HUMAN PLASMA**

Blood was obtained from healthy volunteers. recruited from the personnel of Monsanto Company, Saint Louis, MO. Blood was collected in tubes containing EDTA (EDTA plasma pool). The EDTA human plasma pool. previously stored at -20 °C, was thawed at room temperature and centrifuged for 5 minutes to remove any particulate matter. Tritiated HDL, radiolabeled in the cholesteryl ester moiety ([$^3$H]CE-HDL) as described by Morton and Zilversmit (J. Biol. Chem., 256, 11992-95 (1981)), was added to the plasma to a final concentration of 25 µg/mL cholesterol. Equal volumes (396 µL) of the plasma containing the [$^3$H]CE-HDL were added by pipette into micro tubes (Titertube®, Bio-Rad laboratories, Hercules, CA). Inhibitor compounds, dissolved as 20-50 mM stock solutions in DMSO, were serially diluted in DMSO (or an alternative solvent in some cases. such as dimethylformamide or ethanol). Four µL of each of the serial dilutions of inhibitor compounds or DMSO alone were then added to each of the tubes containing plasma (396 µL). After mixing, triplicate aliquots (100 µL) from each plasma tube were then transferred to wells of 96-well roundbottomed polystyrene microtiter plates (Corning, Corning, NY). Plates were sealed with plastic film and incubated at 37 °C for 4 hours. "Test" samples contained plasma with dilutions of inhibitor compounds. "Control" samples contained plasma with DMSO diluted to the same concentration as the test samples. but without inhibitor. "Blank" samples were prepared as "control" samples, but were left in the micro tubes at 4 °C for the 4 hour incubation and were then added to the microtiter wells at the end of the incubation period. VLDL and LDL were precipitated by the addition of 10 µL of precipitating reagent (1% (w/v) dextran sulfate (Dextralip50)/0.5 M magnesium chloride, pH 7.4) to all wells. The wells were mixed on a plate mixer and then incubated at ambient temperature for 10 min. The plates were then centrifuged at 1000 x g for 30 min at 10 °C. The supernatants (50 µL) from each well were then transferred to Picoplate™ 96 plate wells (Packard. Meriden, CT) containing Microscint™-40 (Packard, Meriden, CT). The plates were heat-sealed (TopSeal™-P, Packard, Meriden, CT) according to the manufacturer's directions and mixed for 30 min. Radioactivity was measured on a microplate scintillation counter (TopCount, Packard, Meriden, CT). The maximum percentage transfer in the control wells (% transfer) was determined using the following equation:

$$\% \text{ Transfer} = \frac{[dpm_{blank}\text{-}dpm_{control}] \times 100}{dpm_{blank}}$$

The percentage of transfer relative to the control (% control) was determined in the wells containing inhibitor compounds was determined as follows:

$$\% \text{ Control} = \frac{[dpm_{blank}\text{-}dpm_{test}] \times 100}{dpm_{blank}\text{-}dpm_{control}}$$

$IC_{50}$ values were then calculated from plots of % control versus concentration of inhibitor compound. $IC_{50}$ values were determined as the concentration of inhibitor compound inhibiting transfer of [$^3$H]CE from the supernatant [$^3$H]CE-HDL to the precipitated VLDL and LDL by 50% compared to the transfer obtained in the control wells.

Examples of plasma $IC_{50}$ values determined by these methods are summarized in Table 10.

**ASSAY OF CETP INHIBITION *IN VIVO*.**

Inhibition of CETP activity by a test compound can be determined by administering the compound to an animal by intravenous injection or oral gavage, measuring the amount of transfer of tritium-labeled cholesteryl ester ([$^3$H]CE) from HDL to VLDL and LDL particles, and comparing this amount of transfer with the amount of transfer observed in

control animals.

Male golden Syrian hamsters were maintained on a diet of chow containing 0.24% cholesterol for at least two weeks prior to the study. For animals receiving intravenous dosing immediately before the experiment, animals were anesthetized with pentobarbital. Anesthesia was maintained throughout the experiment. In-dwelling catheters were inserted into the jugular vein and carotid artery. At the start of the experiment all animals received 0.2 mL of a solution containing [$^3$H]CE-HDL into the jugular vein. [$^3$H]CE-HDL is a preparation of human HDL containing tritium-labeled cholesteryl ester, and was prepared according to the method of Glenn et al. (*Meth. Enzymol.,* 263, 339-351 (1996)). Test compound was dissolved as a 80 mM stock solution in vehicle (2% ethanol: 98% PEG 400, Sigma Chemical Company, St. Louis, Missouri, USA) and administered either by bolus injection or by continuous infusion. Two minutes after the [$^3$H]CE-HDL dose was administered, animals received 0.1 mL of the test solution injected into the jugular vein. Control animals received 0.1 mL of the intravenous vehicle solution without test compound. After 5 minutes, the first blood samples (0.5 mL) were taken from the carotid artery and collected in standard microtainer tubes containing ethylenediamine tetraacetic acid. Saline (0.5 mL) was injected to flush the catheter and replace blood volume. Subsequent blood samples were taken at two hours and four hours by the same method. Blood samples were mixed well and kept on ice until the completion of the experiment. Plasma was obtained by centrifugation of the blood samples at 4 °C. The plasma (50 μL) was treated with 5 μL of precipitating reagent (dextran sulfate, 10 g/L: 0.5 M magnesium chloride) to remove VLDL/LDL. After centrifugation, the resulting supernatant (25 μL) containing the HDL was analyzed for radioactivity using a liquid scintillation counter.

The percentage [$^3$H]CE transferred from HDL to LDL and VLDL (% transfer) was calculated based on the total radioactivity in equivalent plasma samples before precipitation. Typically, the amount of transfer from HDL to LDL and VLDL in control animals was 20% to 35% after 4 hours. The polyethylene glycol vehicle was determined to have no effect on CETP activity in this model.

Alternatively, conscious, non-anesthetized animals received an oral gavage dose of test compound as a suspension in 0.1% methyl cellulose in water. At a time determined for each compound at which plasma levels of the test substance reached their peak ($C_{max}$) after oral dosing, the animals were anesthetized with pentobarbital and then dosed with 0.2 mL of a solution containing [$^3$H]CE-HDL into the jugular vein as described above. Control animals received 0.25 mL of the vehicle solution without test compound by oral gavage. After 4 hours, the animals were sacrificed, blood samples were collected, and the percentage [$^3$H]CE transferred from HDL to LDL and VLDL (% transfer) assayed, as described above. The aqueous methyl cellulose vehicle was determined to have no effect on CETP activity in this model. Results from testing in this model are summarized in Table 11.

Alternatively, inhibition of CETP activity by a test compound was determined by administering the compound to mice which have been selected for expression of human CETP (hCETP) by transgenic manipulation (hCETP mice). Test compounds were administered by intravenous injection, or oral gavage and the amount of transfer of tritium-labeled cholesteryl ester ([$^3$H]CE) from HDL to VLDL and LDL particles was determined, and compared to the amount of transfer observed in control animals. C57Bl/6 mice that were homozygous for the hCETP gene were maintained on a high fat chow diet, such as TD 88051, as described by Nishina et al. (J Lipid Res., 31, 859-869 (1990)) for at least two weeks prior to the study. Mice received an oral gavage dose of test compound as a suspension in 0.1% methyl cellulose in water or an intravenous bolus injection of test compound in 10% ethanol and 90% polyethylene glycol. Control animals received the vehicle solution without test compound by oral gavage or by an intravenous bolus injection. At the start of the experiment all animals received 0.05 mL of a solution containing [$^3$H]CE-HDL into the tail vein. [$^3$H]CE-HDL is a preparation of human HDL containing tritium-labeled cholesteryl ester, and was prepared according to the method of Glenn et al. (*Meth. Enzymol*., 263, 339-351 (1996)). After 30 minutes, the animals were exsanguinated and blood collected in standard microtainer tubes containing ethylenediamine tetraacetic acid. Blood samples were mixed well and kept on ice until the completion of the experiment. Plasma was obtained by centrifugation of the blood samples at 4 °C. The plasma was separated and analyzed by gel filtration chromatography and the relative proportion of [$^3$H]CE in the VLDL. LDL and HDL regions was determined.

The percentage [$^3$H]CE transferred from HDL to LDL and VLDL (% transfer) was calculated based on the total radioactivity in equivalent plasma samples before precipitation. Typically, the amount of transfer from HDL to LDL and VLDL in control animals was 20% to 35% after 30 min. The polyethylene glycol and the aqueous methyl cellulose vehicles were determined to have no effect on CETP activity in this model. Results from testing in this model are summarized in Table 12.

**ASSAY OF PLASMA HDL ELEVATION *IN VIVO*.**

Syrian Golden hamsters were made hypercholesterolemic by feeding cholesterol supplemented chow for a minimum of two weeks, as described above. Test compounds were administered orally in selected aqueous or oil based vehicles for up to 1 week. Serum was obtained and analyzed by precipitation or size exclusion chromatography for the relative abundance of VLDL, LDL and HDL. Results from testing in this model are summarized in Table 13.

Alternatively, a strain of C57bl mouse was made to transgenicaly express human CETP. Plasma concentrations of hCETP ranged from 2-20 μg/ml. The hCETP mice were made hyperchotesterolemic by feeding cholesterol and fat supplemented chow for a minimum of two weeks. as described above. Test compounds were administered orally in selected aqueous or oil based vehicles for up to 1 week. Serum was obtained and analyzed by size exclusion chromatography for the relative abundance of VLDL, LDL and HDL. Results from testing in this model are summarized in Table 14.

Alternatively, cynomologous monkeys were maintained on a normal chow diet. The compound corresponding to example 8 was dissolved in a corn oil based vehicle and administered by oral gavage at 10 mpk q.d. for up to 11 days. Plasma levels of drug were detected throughout the experiment in treated animals at ranges of 0.1-1.5 μg/mL. Periodically, plasma samples were taken and analyzed for total cholesterol and HDL. After seven days, the treated animals exhibited a 2% increase in HDL and a 5% increase in total cholesterol, relative to vehicle-treated controls.

Alternatively, rabbits were maintained on a normal chow diet. The compound corresponding to example 8 was dissolved in a vehicle of ethanol:propylene glycol (1.5:18) and administered by Alzet pump at 30 mg/day/animal for up to 14 days. Plasma concentrations of drug were detected throughout the duration of the pump infusion in treated animals and averaged 1.2 μg/mL. Periodically, plasma samples were taken and analyzed for triglycerides, total cholesterol, and HDL. After fourteen days, the treated animals exhibited a 12% decrease in HDL, a 19% decrease in total cholesterol, as well as a 17% increase in triglycerides, compared to pre-dose levels.

Table 9. Inhibition of CETP Activity by Examples in Reconstituted Buffer Assay.

| Ex. No. | $IC_{50}$ (µM) | Ex. No. | $IC_{50}$ (µM) |
|---|---|---|---|
| 8 | 0.0008 | 42 | 0.38 |
| 11 | 0.001 | 27 | 0.44 |
| 19 | 0.004 | 26 | 0.53 |
| 9 | 0.008 | 29 | 0.72 |
| 10 | 0.012 | 3 * | 0.76 |
| 2 | 0.014 | 28 | 0.86 |
| 4 | 0.014 | 32 | 1.2 |
| 20 | 0.027 | 25 | 1.4 |
| 22 | 0.027 | 39 | 1.6 |
| 12 | 0.034 | 15 | 1.6 |
| 14 | 0.04 | 30 | 2.7 |
| 18 | 0.044 | 33B | 3.2 |
| 16 | 0.049 | 5 | 3.4 |
| 43 | 0.058 | 31 | 3.5 |
| 23 | 0.066 | 7 | 4.9 |
| 34 | 0.076 | 44 * | 6.8 |
| 41 | 0.086 | 17 | 18 |
| 21 | 0.11 | 6 * | 68 |
| 13 | 0.13 | 44A* | > 50 |
| 1 | 0.14 | | |
| 33 | 0.15 | | |
| 38 | 0.18 | | |
| 36 | 0.20 | | |
| 37 | 0.21 | | |
| 40 | 0.23 | | |
| 35 | 0.28 | | |
| 24 | 0.33 | | |

*Included for reference and/or comparison only.

Table 10.

| Inhibition of CETP Activity by Examples in Human Plasma Assay. | |
|---|---|
| **Ex. No.** | **$IC_{50}$ (μM)** |
| 8 | 0.049 |
| 11 | 0.072 |
| 10 | 0.11 |
| 22 | 0.14 |
| 19 | 0.19 |
| 20 | 0.3 |
| 18 | 0.44 |
| 14 | 0.59 |
| 9 | 0.62 |
| 2 | 0.65 |
| 4 | 0.65 |
| 16 | 0.77 |
| 12 | 0.79 |
| 34 | 1.4 |
| 43 | 1.5 |
| 23 | 2.0 |
| 1 | 5.6 |
| 41 | 7.2 |
| 42 | 11 |
| 3* | 20 |

*Included for reference and/or comparison only.

Table 11.

| Inhibition of CETP-mediated Transfer in Hamster | | |
|---|---|---|
| **Ex. No.** | **Single Oral Dose** | **% Inhibition of Transfer** |
| 8 | 10 mpk | 35 |

Table 12.

| Inhibition of CETP-mediated Transfer in hCETP Mice. | | |
|---|---|---|
| **Ex. No.** | **Single Oral Dose** | **% Inhibition of Transfer** |
| 8 | 60 mpk | 40 |

Table 13.

| Change in Lipoprotein Profile in Hamster. | | | | |
|---|---|---|---|---|
| **Ex. No.** | **Oral Dose qd, 5 days** | **% Change in Lipoprotein Profile** | | |
| | | **HDL** | **LDL** | **VLDL** |
| 8 | 30 mpk | 12 | -12 | -22 |

Table 14.

| Change in Lipoprotein Profile in hCETP Mice. | | | | |
|---|---|---|---|---|
| **Ex. No.** | **Oral Dose qd, 5 days** | **% Change in Lipoprotein Profile** | | |
| | | **HDL** | **LDL** | **VLDL** |
| 8 | 30 mpk | 12 | 20 | - |

## Claims

**1.** Compound of Formula M:

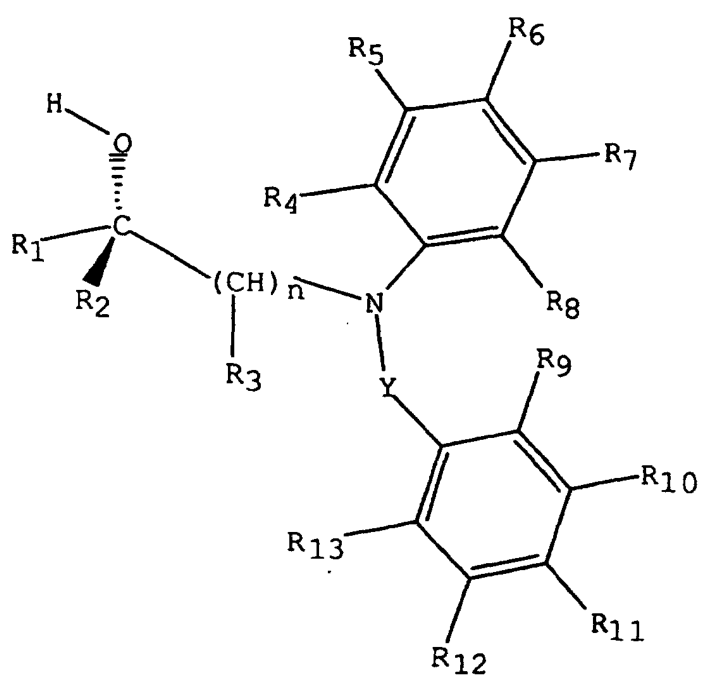

(M)

or a pharmaceutically acceptable salt thereof, wherein;
n is selected from the integers 1, 2, 3 and 4;
Y is $-(CH_2)_q-$ wherein q is 1 or 2;
$R_1$ is haloalkyl;
$R_2$ is hydrido;
$R_3$ is hydrido;
$R_4$, $R_8$, $R_9$, and $R_{13}$ are independently selected from the group consisting of hydrido, halo, haloalkyl, and alkyl;

$R_5$, $R_6$, $R_7$, $R_{10}$, $R_{11}$, and $R_{12}$ are independently selected from the group consisting of hydride, carboxy, heteroaralkylthio, heteroaralkoxy, cycloalkylamino, acylalkyl, acylalkoxy, aroylalkoxy, heterocyclyloxy, aralkylaryl, aralkyl, aralkenyl, aralkynyl, heterocyclyl, perhaloaralkyl, aralkylsulfonyl, aralkylsulfonylalkyl, aralkylsulfinyl, aralkylsulfinylalkyl, halocycloalkyl, halocycloalkenyl, cycloalkylsulfinyl, cycloalkylsulfinylalkyl, cycloalkylsulfonyl, cycloalkylsulfonylalkyl, heteroarylamino, N-heteroarylamino-N-alkylamino, heteroarylaminoalkyl, haloalkylthio, alkanoyloxy, alkoxy, alkoxyalkyl, heteroaralkoxy, cycloalkoxy, cycloalkenyloxy, cycloalkoxyalkyl, cycloalkylalkoxy, cycloalkenyloxyalkyl, halocycloalkoxy, halocycloalkoxyalkyl, halocycloalkenyloxy, halocycloalkenyloxyalkyl, hydroxy, amino, thio, nitro, C1-C6 alkylamino, alkylthio, alkylthioalkyl, arylamino, aralkylamino, arylthio, arylthioalkyl, heteroaralkoxyalkyl, alkylsulfinyl, alkylsulfinylalkyl, arylsulfinylalkyl, arylsulfonylalkyl, heteroarylsulfinylalkyl, heteroarylsulfonylalkyl, alkylsulfonyl, alkylsulfonylalkyl, haloalkylsulfinylalkyl, haloalkylsulfonylalkyl, alkylsulfonamido, alkylaminosulfonyl, amidosulfonyl, alkylamidosulfonyl, arylamidosulfonyl, arylsulfonamido, alkylarylamidosulfonyl, arylsulfinyl, arylsulfonyl, heteroarylthio, heteroarylsulfinyl, heteroarylsulfonyl, heterocyclylsulfonyl, heterocyclylthio, alkanoyl, alkenoyl, aroyl, heteroaroyl, aralkanoyl, heteroaralkanoyl, haloalkanoyl, alkyl, alkenyl, alkynyl, alkenyloxy, alkenyloxyalky, cycloalkyl, cycloalkylalkanoyl, cycloalkenyl, C3-C7 cycloalkylalkyl, C3-C7 cycloalkenylalkyl, halo, haloalkyl, haloalkenyl, haloalkoxy, hydroxyhaloalkyl, hydroxyaralkyl, hydroxyalkyl, hydoxyheteroaralkyl, haloalkoxyalkyl, aryl, heteroaralkynyl, aryloxy, aralkoxy, aryloxyalkyl, saturated heterocyclyl, partially saturated heterocyclyl, heteroaryl, heteroaryloxy, heteroaryloxyalkyl, heteroaralkyl, arylalkenyl, heteroarylalkenyl, carboxyalkyl, carboalkoxy, alkoxycarboxamido, alkylamidocarbonylamido, arylamidocarbonylamido, carboalkoxyalkyl, carboalkoxyalkenyl, carboaralkoxy, carboxamido, carboxamidoalkyl, cyano, carbohaloalkoxy, phosphono, phosphonoalkyl, diaralkoxyphosphono, and diaralkoxyphosphonoalkyl;

further, wherein when $R_7$ is aryloxy or aralkoxy, said aryloxy or aralkoxy may be substituted at one or more substitutable positions with one or more radicals selected from amino, halo, nitro, alkoxy, alkyl, cyano, cycloalkoxy, cycloalkyl, cycloalkylalkoxy, haloalkoxy, C1-C6 alkylamino, haloalkyl, alkanoyl, haloalkylthio, perhaloaralkyl, aralkylsulfonyl, aralkylsulfonylalkyl, halocycloalkyl, halocycloalkenyl, haloalkylthio, alkanoyloxy, alkoxy, alkoxyalkyl, haloalkoxylalkyl, cycloalkoxy, cycloalkoxyalkyl, cycloallcylalkoxy, hydroxy, thio, alkylthio, alkylthioalkyl, arylamino, aralkylamino, arylthio, arylthioalkyl, alkylsulfonyl, alkylsulfonylalkyl, haloalkylsulfonylalkyl, alkylsulfonamido, alkylaminosutfonyl, amidosulfonyl, monoalkyl amidosulfonyl, dialkyl amidosulfonyl, arylsulfonyl, alkenoyl, aroyl, aralkanoyl, haloalkanoyl, alkenyl, alkynyl, alkenyloxy, alkenyloxyalky, cycloalkylalkanoyl, C3-C7 cycloalkylalkyl, haloalkenyl, hydroxyhaloalkyl, hydroxyaralkyl, hydroxyalkyl, haloalkoxyalkyl, aryl, aralkyl, aryloxy, aralkoxy, aryloxyalkyl, carboxyalkyl, carboalkoxy, alkoxycarbonyl, carboaralkoxy, carboxamido, carboxamidoalkyl, carbohaloalkoxy, phosphono, phosphonoalkyl, diaralkoxyphosphono, and diaralkoxyphosphonoalkyl,

with the proviso that at least one of $R_4$, $R_5$, $R_6$, $R_7$, and $R_8$ is not hydrido,

and with the further proviso that at least one of $R_9$, $R_{10}$, $R_{11}$, $R_{12}$, and $R_{13}$ is not hydrido.

**2.** Compound of Claim 1 of Formula M:

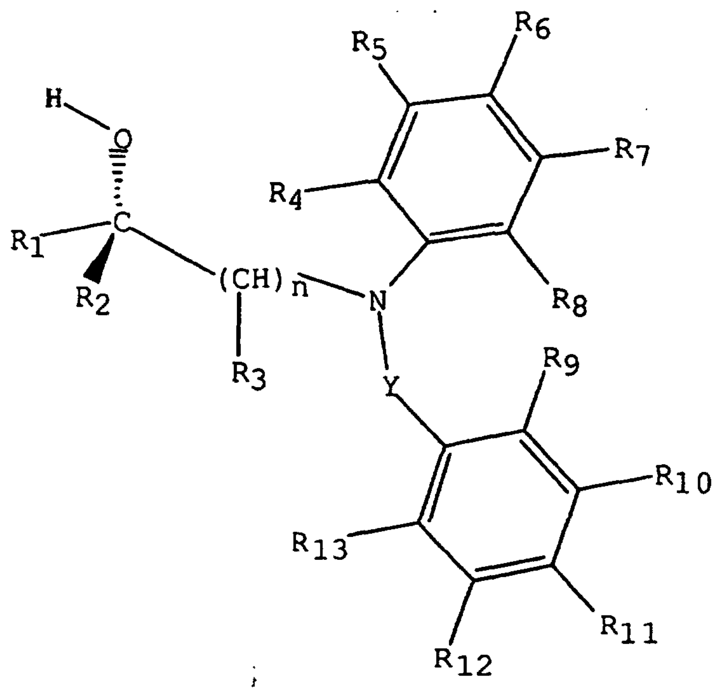

(M)

or a pharmaceutically acceptable salt thereof, wherein;

n is an integer selected from 1 through 3;

Y is $-(CH_2)_q-$ wherein q is 1 or 2;

$R_1$ is haloalkyl;

$R_2$ is hydrido;

$R_3$ is hydrido;

$R_4$, $R_8$, $R_9$, and $R_{13}$ are independently selected from the group consisting of hydride, halo, and haloalkyl;

$R_5$, $R_6$, $R_7$, $R_{10}$, $R_{11}$, and $R_{12}$ are independently selected from the group consisting of hydrido, heteroaralkoxy, acylalkyl, acylalkoxy, aroylalkoxy, heterocyclyloxy, aralkylaryl, aralkyl, perhaloaralkyl, aralkylsulfonyl, aralkylsulfonylalkyl, halocycloalkyl, cycloalkylsulfonyl, cycloalkylsulfonylalkyl, haloalkylthio, alkanoyloxy, alkoxy, alkoxyalkyl, heteroaralkoxy, cycloalkoxy, cycloalkoxyalkyl, cycloalkylalkoxy, halocycloalkoxy, halocycloalkoxyalkyl, hydroxy, amino, nitro, C1-C6 alkylamino, alkylthio, alkylthioalkyl, arylamino, aralkylamino, arylthio, arylsulfonylalkyl, alkylsulfonyl, alkylsulfonylalkyl, haloalkylsulfonylalkyl, alkylsulfonamido, alkylaminosulfonyl, amidosulfonyl, alkylamidosulfonyl, arylsulfonyl, alkanoyl, aroyl, aralkanoyl, heteroaralkanoyl, haloalkanoyl, alkyl, cycloalkyl, cycloalkylalkanoyl, C3-C7 cycloalkylalkyl, halo, haloalkyl, haloalkoxy, hydroxyhaloalkyl, hydroxyaralkyl, hydroxyalkyl, haloalkoxyalkyl, aryl, aryloxy, aralkoxy, aryloxyalkyl, heteroaryloxy, heteroaryloxyalkyl, carboxyalkyl, carboalkoxy, alkoxycarboxamido, carboalkoxyalkyl, carboaralkoxy, carboxamido, carboxamidoalkyl, cyano, carbohaloalkoxy, phosphono, phosphonoalkyl, diaralkoxyphosphono, and diaralkoxyphosphonoalkyl;

further, wherein when $R_7$ is aryloxy or aralkoxy, said aryloxy or aralkoxy may be substituted at one or more substitutable positions with one or more radicals selected from amino, halo, nitro, alkoxy, alkyl, cyano, cycloalkoxy, cycloalkyl, cycloalkylalkoxy, haloalkoxy, C1-C6 alkylamino, haloalkyl, alkanoyl and haloalkylthio,

with the proviso that at least one of $R_4$, $R_5$, $R_6$, $R_7$, and $R_8$ is not hydrido

and with the further proviso that at least one of $R_9$, $R_{10}$, $R_{11}$, $R_{12}$, and $R_{13}$ is not hydrido.

**3.** Compound of Claim 2 of Formula M:

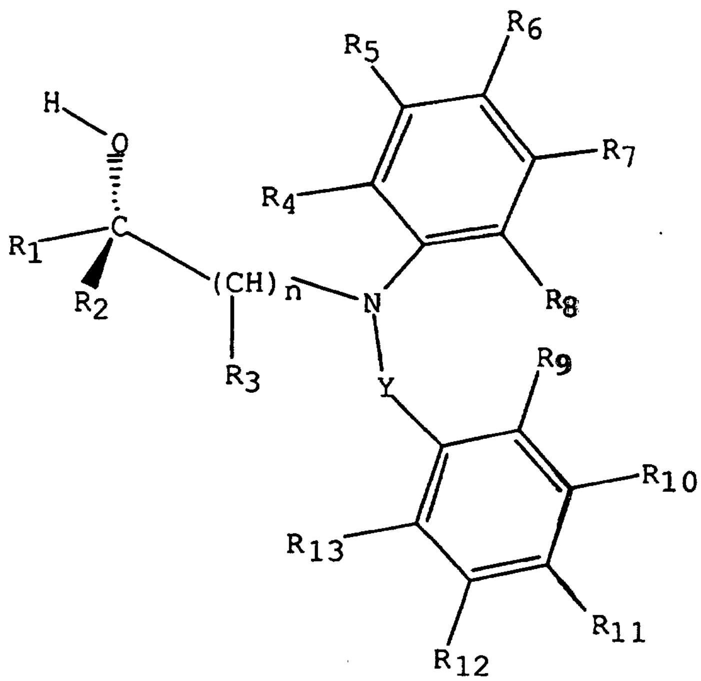

(M)

or a pharmaceutically acceptable salt thereof, wherein;

n is an integer selected from 1 through 3;

Y is $-(CH_2)_q-$ wherein q is 1 or 2;

$R_1$ is haloalkyl;

$R_2$ is hydrido;

$R_3$ is hydrido;

$R_4$, $R_8$, $R_9$, and $R_{13}$ are independently selected from the group consisting of hydrido, halo, and haloalkyl;

$R_5$, $R_6$, $R_7$, $R_{10}$, $R_{11}$, and $R_{12}$ are independently selected from the group consisting of hydrido, heteroaralkoxy, acylalkyl, acylalkoxy, aroylalkoxy, heterocyclyloxy, aralkylaryl, aralkyl, perhaloaralkyl, haloalkylthio, alkanoyloxy, alkoxy, alkoxyalkyl, heteroaralkoxy, cycloalkoxy, cycloalkoxyalkyl, cycloalkylalkoxy, halocycloalkoxy, halocycloalkoxyalkyl, alkylthio, alkylthioalkyl, arylthio, alkylsulfonyl, alkylsulfonylalkyl, haloalkylsulfonylalkyl, alkylsulfonamido, alkylaminosulfonyl, amidosulfonyl, alkylamidosulfonyl, arylsulfonyl, alkanoyl, haloalkanoyl, alkyl, cycloalkyl, C3-C7 cycloalkylalkyl, halo, haloalkyl, haloalkoxy, haloalkoxyalkyl, aryl, aryloxy, aralkoxy, aryloxyalkyl, heteroaryloxy, heteroaryloxyalkyl, carboalkoxy, carboalkoxyalkyl, carboaralkoxy and carbohaloalkoxy;

further, wherein when $R_7$ is aryloxy or aralkoxy, said aryloxy or aralkoxy may be substituted at one or more substitutable positions with one or more radicals selected from amino, halo, nitro, alkoxy, alkyl, cycloalkoxy, cycloalkyl, cycloalkylalkoxy, haloalkoxy, C1-C6 alkylamino, haloalkyl, alkanoyl and haloalkylthio.

**4.** Compound of Claim 3 of Formula "D"

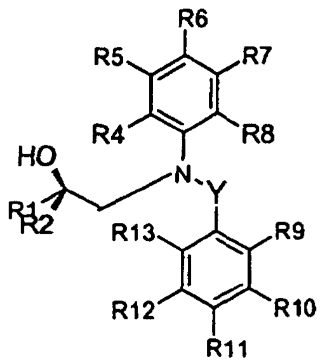

Formula "D"

wherein Y is $-CH_2-$;
wherein $R^1$ is haloalkyl;
wherein $R^2$ is hydrido;
wherein $R^4$ and $R^8$ are hydrido;
wherein $R^5$ and $R^6$ are selected from hydrido and alkoxy;
wherein $R^7$ is selected from aryloxy, arylalkoxy, 5,6,7,8-tetrahydronaphth-2-yloxy, alkoxy, cycloalkoxy, cycloalkylalkoxy and halo;
wherein said aryloxy and arylalkoxy groups in $R^7$ may be substituted at one or more substitutable positions with one or more radicals selected from halo, alkyl, alkoxy, haloalkoxy and haloalkyl;
wherein $R^9$ is selected from hydrido, halo and haloalkyl;
wherein $R^{10}$ is selected from haloalkoxy, haloalkyl and haloalkylthio;
wherein $R^{11}$ is selected from hydrido, halo and haloalkyl;
wherein $R^{12}$ is selected from hydrido and haloalkyl;
wherein $R^{13}$ is selected from hydrido, halo and haloalkyl.

**5.** Compound of Claim 4 of formula "C"

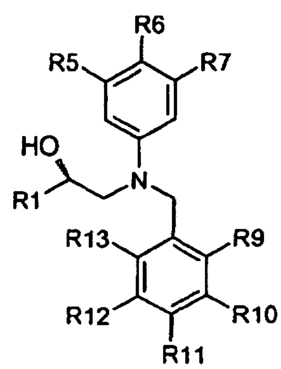

Formula "C"

wherein $R^1$ is selected from trifluoromethyl and chloromethyl;
wherein $R^5$ and $R^6$ are selected from hydrido and methoxy;
wherein $R^7$ is selected from phenyloxy, benzyloxy, 5,6,7,8-tetrahydronaphth-2-yloxy, isopropoxy, cyclopentoxy, bromo, cyclohexylmethoxy and methoxy;
wherein said phenyloxy and benzyloxy groups in $R^7$ may be substituted at one or more substitutable positions with one or more radicals selected from chloro, ethyl, trifluoromethoxy, bromo, fluoro, methyl, isopropyl, trifluoromethyl, isopropoxy and tert-butyl;

wherein $R^9$ is selected from hydrido, fluoro and trifluoromethyl;

wherein $R^{10}$ is selected from 1,1,2,2-tetrafluoroethoxy, trifluoromethoxy, pentafluoroethyl, trifluoromethyl and trifluoromethylthio;

wherein $R^{11}$ is selected from hydrido, trifluoromethyl and fluoro;

wherein $R^{12}$ is selected from hydrido and trifluoromethyl;

wherein $R^{13}$ is selected from hydrido, fluoro and trifluoromethyl.

**6.** Compound of Claim 5, or a pharmaceutically acceptable salt thereof, wherein said compound is selected from the group consisting of:

(2R)-3-[[3-(3-trifluoromethoxyphenoxy)phenyl][[3-( 1,1,2,2-tetrafluoroethoxy)-phenyl]methyl]amino]-1,1,1-trifluoro-2-propanol;

(2R)-3-[[3-(3-isopropylphenoxy)phenyl][[3-(1,1,2,2-tetrafluoroethoxy)phenyl]-methyl]amino]-1,1,1-trifluoro-2-propanol;

(2R)-3-[[3-(4-methylphenoxy)phenyl][[3-(1,1,2,2-tetrafluoroethoxy)phenyl]-methyl]amino]-1,1,1-trifluoro-2-propanol;

(2R)-3-[[3-(2-bromo-5-fluorophenoxy)phenyl][[3-(1,1,2,2-tetrafluoroethoxy)phenyl]-methyl]amino]-1,1,1-trifluoro-2-propanol;

(2R)-3-[[3-(4-chloro-3-ethylphenoxy)phenyl][[3-(1,1,2,2-tetrafluoroethoxy)phenyl]-methyl]amino]-1,1,1-trifluoro-2-propanol;

(2R)-3-[[3-(3-ethylphenoxy)phenyl][[3-(1,1,2,2-tetrafluoroethoxy) phenyl]-methyl]amino]-1,1,1-trifluoro-2-propanol;

(2R)-3-[[3-(phenoxy)phenyl][[3-(1,1,2,2-tetrafluoroethoxy)phenyl]methyl]amino]-1,1,1-trifluoro-2-propanol;

(2R)-3-[[[3-(1,1,2,2-tetrafluoroethoxy)phenyl]methyl][3-[[3-(trifluoromethoxy)-phenyl]methoxy]phenyl]amino]-1,1,1-trifluoro-2-propanol;

(2R)-3-[[[3-(1,1,2,2-tetrafluoroethoxy)phenyl]methyl][3-[[3-(trifluoromethyl)-phenyl]methoxy]phenyl]amino]-1,1,1-trifluoro-2-propanol;

(2R)-3-[[[3-(1,1,2,2-tetrafluoroethoxy)phenyl]methyl][3-[[3,5-difluorophenyl]-methoxy]phenyl]amino]-1,1,1-trifluoro-2-propanol;

(2R)-3-[[3-isopropoxyphenyl][[3-(1,1,2,2-tetrafluoroethoxy)-phenyl]methyl]amino]-1,1,1-trifluoro-2-propanol;

(2R)-3-[[3-(4-chloro-3-ethylphenoxy)phenyl][[3-(pentafluoroethyl)phenyl]methyl]-amino]-1,1,1-trifluoro-2-propanol;

(2R)-3-[[3-(4-chloro-3-ethylphenoxy)phenyl][[2-fluoro-5-(trifluoromethyl)-phenyl]methyl]amino]-1,1,1-trifluoro-2-propanol;

(2R)-3-[[3-(4-chloro-3-ethylphenoxy)phenyl][[2-fluoro-4-(trifluoromethyl)-phenyl]methyl]amino]-1,1,1-trifluoro-2-propanol;

(2R)-3-[[3-(4-chloro-3-ethylphenoxy)phenyl][[ 3-(trifluoromethoxy)phenyl]methyl]amino]-1,1,1-trifluoro-2-propanol.

(2R)-3-[[3-(3-(trifluoromethoxy)phenoxy)phenyl][[ 3-(trifluromethoxy)phenyl]methyl]amino]-1,1,1-trifluoro-2-propanol.

(2R)-3-[[3-(4-methylphenoxy)phenyl][[ 3-(trifluromethoxy)phenyl]methyl]amino]-1,1,1-trifluoro-2-propanol.

7. Compound of Claim 6, or a pharmaceutically acceptable salt thereof, wherein said compound is (2R)-3-[[3-(4-chloro-3-ethylphenoxy)phenyl][[3-(1,1,2,2-tetrafluoroethoxy)phenyl]-methyl]amino]-1,1,1-trifluoro-2-propanol.

8. Compound of Claim 6, or a pharmaceutically acceptable salt thereof, wherein said compound is (2R)-3-[(3-phenoxyphenyl)[[3-(1,1,2,2-tetrafluoroethoxy)phenyl]methyl]amino]- 1,1,1-trifluoro-2-propanol.

9. Compound of Claim 6, or a pharmaceutically acceptable salt thereof, wherein said compound is (2R)-3-[[3-(4-methylphenoxy)phenyl][[3-(1,1,2,2-tetrafluoroethoxy)phenyl]-methyl]amino]-1,1,1-trifluoro-2-propanol.

10. Compound of Claim 6, or a pharmaceutically acceptable salt thereof, wherein said compound is (2R)-3-[[3-(3-isopropylphenoxy)phenyl][[3-(1,1,2,2-tetrafluoroethoxy)phenyl]-methyl]amino]-1,1,1-trifluoro-2-propanol.

11. Compound of Claim 6, or a pharmaceutically acceptable salt thereof, wherein said compound is (2R)-3-[[3-(3-ethylphenoxy)phenyl][[3-(1;1,2,2-tetrafluoroethoxy)phenyl]-methyl]amino]-1,1,1-trifluoro-2-propanol.

12. Compound of Claim 6, or a pharmaceutically acceptable salt thereof, wherein said compound is (2R)-3-[[3-(3-trifluoromethoxylphenoxy)phenyl][[3-(1,1,2,2-tetrafluoroethoxy)phenyl]-methyl]amino]-1,1,1-trifluoro-2-propanol.

13. Compound of Claim 6, or a pharmaceutically acceptable salt thereof, wherein said compound is (2R)-3-[[3-(4-chloro-3-ethylphenoxy)phenyl][[3-(trifluoromethoxy)phenyl]-methyl]amino]-1,1,1-trifluoro-2-propanol.

14. Compound of Claim 6, or a pharmaceutically acceptable salt thereof, wherein said compound is (2*R*)-3-[[[3-(1,1,2,2-tetrafluoroethoxy)phenyl]methyl][3-[[3-(trifluoromethyl)phenyl]methoxy]phenyl]amino]-1,1,1-trifluoro-2-propanol.

15. Compound of Claim 6, or a pharmaceutically acceptable salt thereof, wherein said compound is (2*R*)-3-[[[3-(1,1,2,2-tetrafluoroethoxy)phenyl]methyl][3-[[3-(trifluoromethoxy)phenyl]methoxy]phenyl]amino]-1,1,1-trifluoro-2-propanol.

16. A pharmaceutical composition comprising a therapeutically effective amount of a compound of Claim 1 or a pharmaceutically acceptable salt thereof, together with a pharmaceutically acceptable carrier.

17. The use of compound of Claim 1 or a pharmaceutically acceptable salt thereof for the manufacture of a medicament for treating or preventing a CETP-mediated disorder in a subject.

18. The use of Claim 17 wherein the treating of a CETP-mediated disorder is coronary artery disease.

19. The use of Claim 17 wherein the preventing of a CETP-mediated disorder is coronary artery disease.

20. The use of Claim 17 wherein the preventing of a CETP-mediated disorder is preventing cerebral vascular accident (CVA).

21. The use of Claim 17 wherein the treating of a CETP-mediated disorder is treating dyslipidemia.

22. The use of Claim 17 wherein the preventing of a CETP-mediated disorder is preventing dyslipidemia.

## Patentansprüche

1. Verbindung der Formel M:

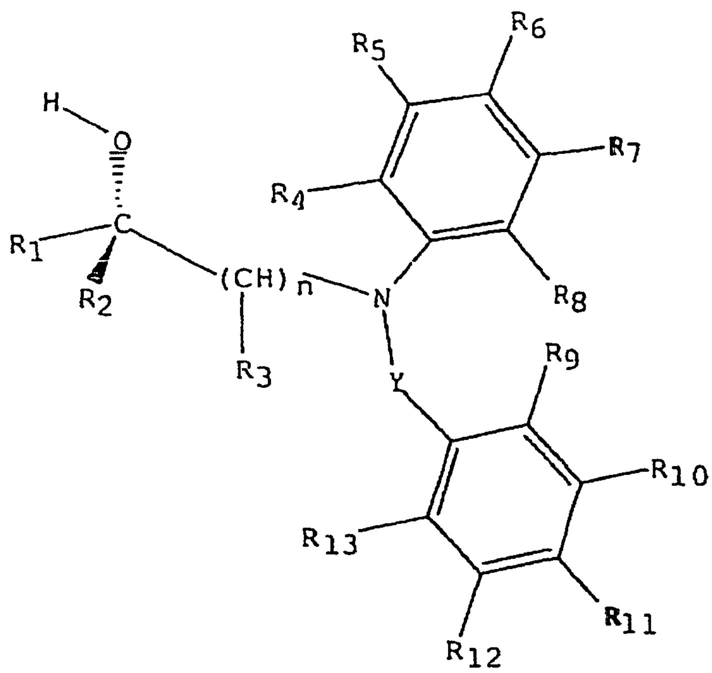

(M)

oder ein pharmazeutisch annehmbares Salz davon, worin
n aus den ganzen Zahlen 1, 2, 3 und 4 ausgewählt ist;
Y für $-(CH_2)_q-$ steht, wobei q den Wert 1 oder 2 hat;
$R_1$ für Halogenalkyl steht;
$R_2$ für Hydrido steht;
$R_3$ für Hydrido steht,
$R_4$, $R_8$, $R_9$ und $R_{13}$ unabhängig voneinander aus der Gruppe, bestehend aus Hydrido, Halogen, Halogenalkyl und Alkyl, ausgewählt sind,
$R_5$, $R_6$, $R_7$, $R_{10}$, $R_{11}$ und $R_{12}$ unabhängig voneinander aus der Gruppe, bestehend aus Hydrido, Carboxy, Heteroaralkylthio, Heteroaralkoxy, Cycloalkylamino, Acylalkyl, Acylalkoxy, Aroylalkoxy, Heterocyclyloxy, Aralkylaryl, Aralkyl, Aralkenyl, Aralkinyl, Heterocyclyl, Perhalogenaralkyl, Aralkylsulfonyl, Aralkylsulfonylalkyl, Aralkylsulfinyl, Aralkylsulfinylalkyl, Halogencycloalkyl, Halogencycloalkenyl, Cycloalkylsulfinyl, Cycloalkylsulfinylalkyl, Cycloalkylsulfonyl, Cycloalkylsulfonylalkyl, Heteroarylamino, N-Heteroarylamino-N-alkylamino, Heteroarylaminoalkyl, Halogenalkylthio, Alkanoyloxy, Alkoxy, Alkoxyalkyl, Heteroaralköxy, Cycloalkoxy, Cycloalkenyloxy, Cycloalkoxyalkyl, Cycloalkylalkoxy, Cycloalkenyloxyalkyl, Halogencycloalkoxy, Halogencycloalkoxyalkyl, Halogencycloalkenyloxy, Halogencycloalkenyloxyalkyl, Hydroxy, Amino, Thio, Nitro, $C_1$-$C_6$-Alkylamino, Alkylthio, Alkylthioalkyl, Arylamino, Aralkylamino, Arylthio, Arylthioalkyl, Heteroaralkoxyalkyl, Alkylsulfinyl, Alkylsulfinylalkyl, Arylsulfinylalkyl, Arylsulfonylalkyl, Heteroarylsulfinylalkyl, Heteroarylsulfonylalkyl, Alkylsulfonyl, Alkylsulfonylalkyl, Halogensulfinylalkyl, Halogenalkylsulfonylalkyl, Alkylsulfonamido, Alkylaminosulfonyl, Amidosulfonyl, Alkylamidosulfonyl, Arylamidosulfonyl, Arylsulfonamido, Alkylarylamidosulfonyl, Arylsulfinyl, Arylsulfonyl, Heteroarylthio, Heteroarylsulfinyl, Heteroarylsulfonyl, Heterocyclylsulfonyl, Heterocyclylthio, Alkanoyl, Alkenoyl, Aroyl, Heteroaroyl, Aralkanoyl, Heteroaralkanoyl, Halogenalkanoyl, Alkyl, Alkenyl, Alkinyl, Alkenyloxy, Alkenyloxyalkyl, Cycloalkyl, Cycloalkylalkanoyl, Cycloalkenyl, $C_3$-$C_7$-Cycloalkylalkyl, $C_3$-$C_7$-Cycloalkenylalkyl, Halogen, Halogenalkyl, Halogenalkenyl, Halogenalkoxy, Hydroxyhalogenalkyl, Hydroxyaralkyl, Hydroxyalkyl, Hydroxyheteroaralkyl, Halogenalkoxyalkyl, Aryl, Heteroaralkinyl, Aryloxy, Aralkoxy, Aryloxyalkyl, gesättigtem Heterocyclyl, teilweise gesättigtem Heterocyclyl, Heteroaryl, Heteroaryloxy, Heteroaryloxyalkyl, Heteroaralkyl, Arylalkenyl, Heteroarylalkenyl, Carboxyalkyl, Carboxyalkoxy, Alkoxycarboxamido, Alkylamidocarbonylamido, Arylamidocarbonylamido, Carboalkoxyalkyl, Carboalkoxyalkenyl, Carboaralkoxy, Carboxamido, Carboxamidoalkyl, Cyano, Carbohalogenalkoxy, Phosphono, Phosphonoalkyl, Diaralkoxyphosphono und Diaralkoxyphosphonoalkyl, ausgewählt sind;
wobei weiterhin, wenn $R_7$ für Aryloxy oder Aralkoxy steht, das genannte Aryloxy oder Aralkoxy an einer oder mehreren substituierbaren Positionen mit einem oder mehreren Resten, ausgewählt aus Amino, Halogen, Nitro,

Alkoxy, Alkyl, Cyano, Cycloalkoxy, Cycloalkyl, Cycloalkylalkoxy, Halogenalkoxy, $C_1$-$C_6$-Alkylamno, Halogenalkyl, Alkanoyl, Halogenalkylthio, Perhalogenaralkyl, Aralkylsulfonyl, Aralkylsulfonylalkyl, Halogencycloalkyl, Halogencycloalkenyl, Halogenalkylthio, Alkanoyloxy, Alkoxy, Alkoxyalkyl, Halogenalkoxyalkyl, Cycloalkoxy, Cycloalkoxyalkyl, Cycloalkylalkoxy, Hydroxy, Thio, Alkylthio, Alkylthioalkyl, Arylamino, Aralkylamino, Arylthio, Arylthioalkyl, Alkylsulfonyl, Alkylsulfonylalkyl, Halogenalkylsulfonylalkyl, Alkylsulfonamido, Alkylaminosulfonyl, Amidosulfonyl, Monoalkylamidosulfonyl, Dialkylamidosulfonyl, Arylsulfonyl, Alkenoyl, Aroyl, Aralkanoyl, Halogenalkanoyl, Alkenyl, Alkinyl, Alkenyloxy, Alkenyloxyalkyl, Cycloalkylalkanoyl, $C_3$-$C_7$-Cycloalkylalkyl, Halogenalkenyl, Hydroxyhalogenalkyl, Hydroxyaralkyl, Hydroxyalkyl, Halogenalkoxyalkyl, Aryl, Aralkyl, Aryloxyl, Aralkoxy, Aryloxyalkyl, Carboxyalkyl, Carboalkoxy, Alkoxycarbonyl, Carboaralkoxy, Carboxamido, Carboxamidoalkyl, Carbohalogenalkoxy, Phosphono, Phosphonoalkyl, Diaralkoxyphosphono und Diaralkoxyphosphonoalkyl, substituiert sein kann,
mit der Maßgabe, dass mindestens eine der Gruppierungen $R_4$, $R_5$, $R_6$, $R_7$ und $R_8$ nicht Hydrido ist,
und mit der weiteren Maßgabe, dass mindestens eine der Gruppierungen $R_9$, $R_{10}$, $R_{11}$, $R_{12}$ und $R_{13}$ nicht Hydrido ist.

**2.** Verbindung nach Anspruch 1 der Formel M:

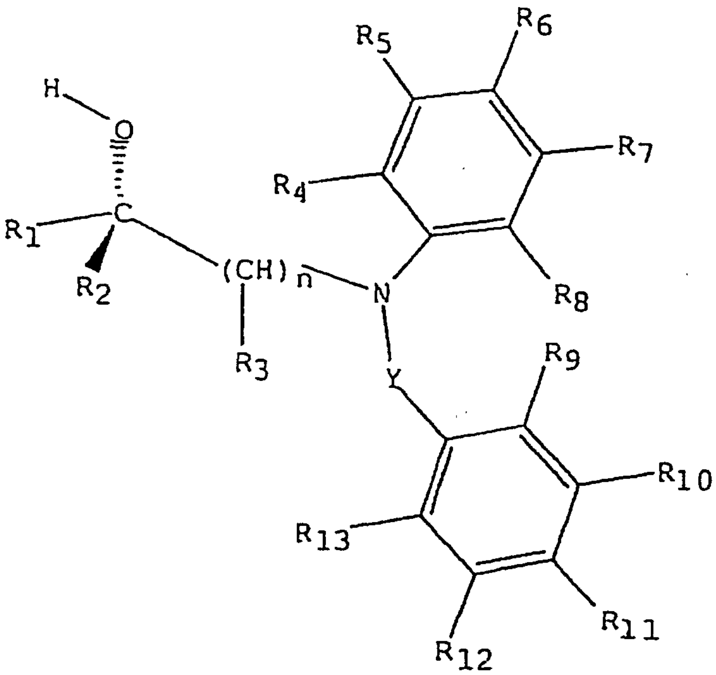

(M)

oder ein pharmazeutisch annehmbares Salz davon, worin
n eine ganze Zahl, ausgewählt aus 1 bis 3, ist;
Y für $-(CH_2)_q-$ steht, wobei q den Wert 1 oder 2 hat;
$R_1$ für Halogenalkyl steht;
$R_2$ für Hydrido steht;
$R_3$ für Hydrido steht,
$R_4$ , $R_8$ , $R_9$ und $R_{13}$ unabhängig voneinander aus der Gruppe, bestehend aus Hydrido, Halogen, Halogenalkyl und Alkyl, ausgewählt sind,
$R_5$, $R_6$, $R_7$, $R_{10}$, $R_{11}$ und $R_{12}$ unabhängig voneinander aus der Gruppe, bestehend aus Hydrido, Heteroaralkoxy, Acylalkyl, Acylalkoxy, Aroylalkoxy, Heterocyclyloxy, Aralkylaryl, Aralkyl, Perhalogenaralkyl, Aralkylsulfonyl, Aralkylsulfonylalkyl, Halogencycloalkyl, Cycloalkylsulfonyl, Cycloalkylsulfonylalkyl, Halogenalkylthio, Alkanoyloxy, Alkoxy, Alkoxyalkyl, Heteroaralkoxy, Cycloalkoxy, Cycloalkoxyalkyl, Cycloalkylalkoxy, Halogencycloalkoxy, Halogencycloalkoxyalkyl, Hydroxy, Amino, Nitro, $C_1$-$C_6$-Alkylamino, Alkylthio, Alkylthioalkyl, Arylamino, Aralkylamino, Arylthio, Arylsulfonylalkyl, Alkylsulfonyl, Alkylsulfonylalkyl, Halogenalkylsulfonylalkyl, Alkylsulfonamido, Alkylaminosulfonyl, Amidosulfonyl, Alkylamidosulfonyl, Arylsulfonyl, Alkanoyl, Aroyl, Aralkanoyl, Heteroaralkanoyl, Halo-

genalkanoyl, Alkyl, Cycloalkyl, Cycloalkylalkanoyl, $C_3$-$C_7$-Cycloalkylalkyl, Halogen, Halogenalkyl, Halogenalkoxy, Hydroxyhalogenalkyl, Hydroxyaralkyl, Hydroxyalkyl, Halogenalkoxyalkyl, Aryl, Aryloxy, Aralkoxy, Aryloxyalkyl, Heteroaryloxy, Heteroaryloxyalkyl, Carboxyalkyl, Carboalkoxy, Alkoxycarboxamido, Carboalkoxyalkyl, Carboaralkoxy, Carboxamido, Carboxamidoalkyl, Cyano, Carbohalogenalkoxy, Phosphono, Phosphonoalkyl, Diaralkoxyphosphono und Diaralkoxyphosphonoalkyl, ausgewählt sind;
wobei weiterhin, wenn $R_7$ für Aryloxy oder Aralkoxy steht, das genannte Aryloxy oder Aralkoxy an einer oder mehreren substituierbaren Positionen mit einem oder mehreren Resten, ausgewählt aus Amino, Halogen, Nitro, Alkoxy, Alkyl, Cyano, Cycloalkoxy, Cycloalkyl, Cycloalkylalkoxy, Halogenalkoxy, $C_1$-$C_6$-Alkylamino, Halogenalkyl, Alkanoyl und Halogenalkylthio, substituiert sein kann,
mit der Maßgabe, dass mindestens eine der Gruppierungen $R_4$, $R_5$, $R_6$, $R_7$ und $R_8$ nicht Hydrido ist,
und mit der weiteren Maßgabe, dass mindestens eine der Gruppierungen $R_9$, $R_{10}$, $R_{11}$, $R_{12}$ und $R_{13}$ nicht Hydrido ist.

**3.** Verbindung nach Anspruch 2 der Formel M:

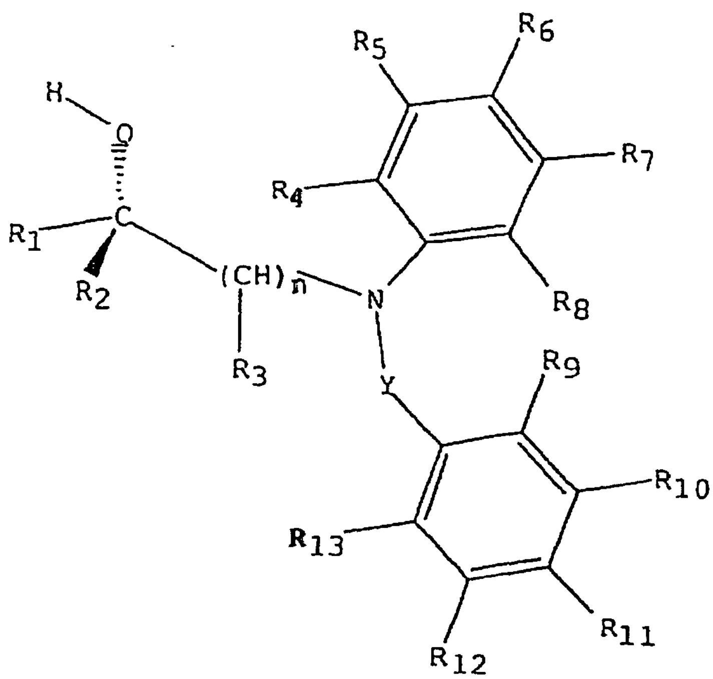

(M)

oder ein pharmazeutisch annehmbares Salz davon, worin
n eine ganze Zahl, ausgewählt aus 1 bis 3, ist;
Y für $-(CH_2)_q-$ steht, wobei q den Wert 1 oder 2 hat;
$R_1$ für Halogenalkyl steht;
$R_2$ für Hydrido steht;
$R_3$ für Hydrido steht,
$R_4$ , $R_8$ , $R_9$ und $R_{13}$ unabhängig voneinander aus der Gruppe, bestehend aus Hydrido, Halogen und Halogenalkyl, ausgewählt sind,
$R_5$, $R_6$, $R_7$, $R_{10}$, $R_{11}$ und $R_{12}$ unabhängig voneinander aus der Gruppe, bestehend aus Hydrido, Heteroaralkoxy, Acylalkyl, Acylalkoxy, Aroylalkoxy, Heterocyclyloxy, Aralkylaryl, Aralkyl, Perhalogenaralkyl, Halogenalkylthio, Alkanoyloxy, Alkoxy, Alkoxyalkyl, Heteroaralkoxy, Cycloalkoxy, Cycloalkoxyalkyl, Cycloalkylalkoxy, Halogencycloalkoxy, Halogencycloalkoxyalkyl, Alkylthio, Alkylthioalkyl, Arylthio, Alkylsulfonyl, Alkylsulfonylalkyl, Halogenalkylsulfonylalkyl, Alkylsulfonamido, Alkylaminosulfonyl, Amidosulfonyl, Alkylamidosulfonyl, Arylsulfonyl, Alkanoyl, Halogenalkanoyl, Alkyl, Cycloalkyl, $C_3$-$C_7$-Cycloalkylalkyl, Halogen, Halogenalkyl, Halogenalkoxy, Halogenalkoxyalkyl, Aryl, Aryloxy, Aralkoxy, Aryloxyalkyl, Heteroaryloxy, Heteroaryloxyalkyl, Carboalkoxy, Carboalkoxyalkyl,

Carboaralkoxy und Carbohalogenalkoxy, ausgewählt sind;
wobei weiterhin, wenn $R_7$ für Aryloxy oder Aralkoxy steht, das genannte Aryloxy oder Aralkoxy an einer oder mehreren substituierbaren Positionen mit einem oder mehreren Resten, ausgewählt aus Amino, Halogen, Nitro, Alkoxy, Alkyl, Cycloalkoxy, Cycloalkyl, Cycloalkylalkoxy, Halogenalkoxy, $C_1$-$C_6$-Alkylamino, Halogenalkyl, Alkanoyl und Halogenthio, substituiert sein kann,

**4.** Verbindung nach Anspruch 3 der Formel "D"

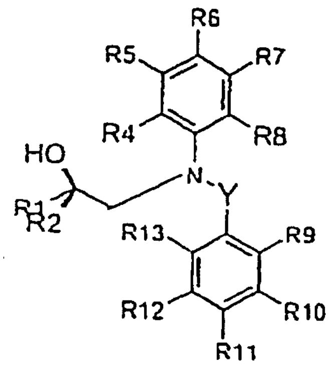

Formel "D"

worin Y für $-CH_2-$ steht;
worin $R^1$ für Halogenalkyl steht;
worin $R^2$ für Hydrido steht;
worin $R^4$ und $R^8$ für Hydrido stehen;
worin $R^5$ und $R^6$ aus Hydrido und Alkoxy ausgewählt sind;
worin $R^7$ aus Aryloxy, Arylalkoxy, 5,6,7,8-Tetrahydronaphth-2-yloxy, Alkoxy, Cycloalkoxy, Cycloalkylalkoxy und Halogen ausgewählt ist;
wobei die genannten Aryloxy- und Arylalkoxy-Gruppen in $R^7$ an einer oder mehreren substituierbaren Positionen mit einem oder mehreren Resten, ausgewählt aus Halogen, Alkyl, Alkoxy, Halogenalkoxy und Halogenalkyl, substituiert sein können;
worin $R^9$ aus Hydrido, Halogen und Halogenalkyl ausgewählt ist;
worin $R^{10}$ aus Halogenalkoxy, Halogenalkyl und Halogenalkylthio ausgewählt ist;
worin $R^{11}$ aus Hydrido, Halogen und Halogenalkyl ausgewählt ist;
worin $R^{12}$ aus Hydrido und Halogenalkyl ausgewählt ist;
worin $R^{13}$ aus Hydrido, Halogen und Halogenalkyl ausgewählt ist.

**5.** Verbindung nach Anspruch 4 der Formel "C"

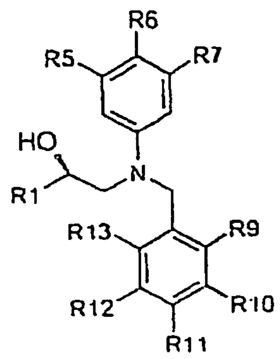

Formel "C"

worin $R^1$ aus Trifluormethyl und Chlormethyl ausgewählt ist;
worin $R^5$ und $R^6$ aus Hydrido und Methoxy ausgewählt sind;
worin $R^7$ aus Phenyloxy, Benzyloxy, 5,6,7,8-Tetrahydronaphth-2-yloxy, Isopropoxy, Cyclopentoxy, Brom, Cyclohexylmethoxy und Methoxy ausgewählt ist;
worin die genannten Phenyloxy- und Benzyloxygruppen in $R^7$ an einer oder mehreren substituierbaren Positionen mit einem oder mehreren Resten, ausgewählt aus Chlor, Ethyl, Trifluormethoxy, Brom, Fluor, Methyl, Isopropyl, Trifluormethyl, Isopropoxy und tert.-Butyl, substituiert sein können;
worin $R^9$ aus Hydrido, Fluor und Trifluormethyl ausgewählt ist,
worin $R^{10}$ aus 1,1,2,2-Tetrafluorethoxy, Trifluormethoxy, Pentafluorethyl, Trifluormethyl und Trifluormethylthio ausgewählt ist;
worin $R^{11}$ aus Hydrido, Trifluormethyl und Fluor ausgewählt ist;
worin $R^{12}$ aus Hydrido und Trifluormethyl ausgewählt ist;
worin $R^{13}$ aus Hydrido, Fluor und Trifluormethyl ausgewählt ist.

**6.** Verbindung nach Anspruch 5 oder ein pharmazeutisch annehmbares Salz davon, wobei die genannte Verbindung aus der Gruppe, bestehend aus:

(2R)-3-[[3-(3-Trifluormethoxyphenoxy)phenyl][[3-(1,1,2,2-tetrafluorethoxy)phenyl]methyl]amino]-1,1,1-trifluor-2-propanol;

(2R)-3-[[3-(3-Isopropylphenoxy)phenyl][[3-(1,1,2,2-tetrafluorethoxy)phenyl]methyl]amino]-1,1,1-trifluor-2-propanol;

(2R)-3-[[3-(4-Methylphenoxy)phenyl][[3-(1,1,2,2-tetrafluorethoxy)phenyl]methyl]amino]-1,1,1-trifluor-2-propanol;

(2R)-3-[[3-(2-Brom-5-fluorphenoxy)phenyl][[3-(1,1,2,2-tetrafluorethoxy)phenyl]methyl]amino]-1,1,1-trifluor-2-propanol;

(2R)-3-[[3-(4-Chlor-3-ethylphenoxy)phenyl][[3-(1,1,2,2-tetrafluorethoxy)phenyl]methyl]amino]-1,1,1-trifluor-2-propanol;

(2R)-3-[[3-(3-Ethylphenoxy)phenyl][[3-(1,1,2,2-tetrafluorethoxy)phenyl]methyl]amino]-1,1,1-trifluor-2-propanol;

(2R)-3-[[3-(Phenoxy)phenyl][[3-(1,1,2,2-tetrafluorethoxy)-phenyl]methyl]amino]-1,1,1-trifluor-2-propanol;

(2R)-3-[[[3-(1,1,2,2-Tetrafluorethoxy)phenyl]methyl][3-[[3-(3-trifluormethoxy)phenyl]methoxy]phenyl]amino]-1,1,1-trifluor-2-propanol;

(2R)-3-[[[3-(1,1,2,2-Tetrafluorethoxy)phenyl]methyl][3-[[3-(trifluormethyl)phenyl]methoxy]phenyl]amino]-1,1,1-trifluor-2-propanol;

(2R)-3-[[[3-(1,1,2,2-Tetrafluorethoxy)phenyl]methyl][3-[[3,5-difluorphenyl]methoxy]phenyl]amino]-1,1,1-trifluor-2-propanol;

(2R)-3-[[3-Isopropoxyphenyl][[3-(1,1,2,2-tetrafluorethoxy)phenyl]methyl]amino]-1,1,1-trifluor-2-propanol;

(2R)-3-[(3-(4-Chlor-3-ethylphenoxy)phenyl][[3-(pentafluorethyl)phenyl]methyl]amino]-1,1,1-trifluor-2-propanol;

(2R)-3-[[3-(4-Chlor-3-ethylphenoxy)phenyl][[2-fluor-5-(trifluormethyl)phenyl]methyl]amino]-1,1,1-trifluor-2-propanol;

(2R)-3-[[3-(4-Chlor-3-ethylphenoxy)phenyl][[2-fluor-4-(trifluormethyl)phenyl]methyl]amino]-1,1,1-trifluor-2-propanol;

(2R)-3-[[3-(4-Chlor-3-ethylphenoxy)phenyl][[3-(trifluormethoxy)phenyl]methyl]amino]-1,1,1-trifluor-2-propanol;

(2R)-3-[[3-(3-(Trifluormethoxy)phenoxy)phenyl][[3-(trifluormethoxy)phenyl]methyl]amino]-1,1,1-trifluor-2-propanol und

(2R)-3-[[3-(4-Methylphenoxy)phenyl][[3-(trifluormethoxy)-phenyl]methyl]amino]-1,1,1-trifluor-2-propanol.

**7.** Verbindung nach Anspruch 6 oder ein pharmazeutisch annehmbares Salz davon, wobei die genannte Verbindung (2R)-3-[[3-(4-Chlor-3-ethylphenoxy)phenyl][[3-(1,1,2,2-tetrafluorethoxy)phenyl]methyl]amino]-1,1,1-trifluor-2-propanol ist.

**8.** Verbindung nach Anspruch 6 oder ein pharmazeutisch annehmbares Salz davon, wobei die genannte Verbindung (2R)-3-[(3-Phenoxyphenyl)[[3-(1,1,2,2-tetrafluorethoxy)-phenyl]methyl]amino]-1,1,1-trifluor-2-propanol ist.

**9.** Verbindung nach Anspruch 6 oder ein pharmazeutisch annehmbares Salz davon, wobei die genannte Verbindung (2R)-3-[[3-(4-Methylphenoxy)phenyl][[3-(1,1,2,2-tetrafluorethoxy)phenyl]methyl]amino]-1,1,1-trifluor-2-propanol ist.

**10.** Verbindung nach Anspruch 6 oder ein pharmazeutisch annehmbares Salz davon, wobei die genannte Verbindung (2R)-3-[[3-(3-Isopropylphenoxy)phenyl][[3-(1,1,2,2-tetrafluorethoxy)phenyl]methyl]amino]-1,1,1-trifluor-2-propanol ist.

**11.** Verbindung nach Anspruch 6 oder ein pharmazeutisch annehmbares Salz davon, wobei die genannte Verbindung (2R)-3-[[3-(3-Ethylphenoxy)phenyl][[3-(1,1,2,2-tetrafluorethoxy)phenyl]methyl]amino]-1,1,1-trifluor-2-propanol ist.

**12.** Verbindung nach Anspruch 6 oder ein pharmazeutisch annehmbares Salz davon, wobei die genannte Verbindung (2R)-3-[[3-(3-Trifluormethoxyphenoxy)phenyl][[3-(1,1,2,2-tetrafluorethoxy)phenyl]methyl]amino]-1,1,1-trifluor-2-propanol ist.

**13.** Verbindung nach Anspruch 6 oder ein pharmazeutisch annehmbares Salz davon, wobei die genannte Verbindung (2R)-3-[[3-(4-Chlor-3-ethylphenoxy)phenyl][[3-(trifluormethoxy)phenyl]methyl]amino]-1,1,1-trifluor-2-propanol ist.

**14.** Verbindung nach Anspruch 6 oder ein pharmazeutisch annehmbares Salz davon, wobei die genannte Verbindung (2R)-3-[[[3-(1,1,2,2-tetrafluorethoxy)phenyl]methyl][3-[[3-(trifluormethyl)phenyl]methoxy]phenyl]amino]-1,1,1-trifluor-2-propanol ist.

**15.** Verbindung nach Anspruch 6 oder ein pharmazeutisch annehmbares Salz davon, wobei die genannte Verbindung (2R)-3-[[[3-(1,1,2,2-Tetrafluorethoxy)phenyl]methyl][3-[[3-(trifluormethoxy)phenyl]methoxy]phenyl]amino]-1,1,1-trifluor-2-propanol ist.

**16.** Pharmazeutisches Präparat, umfassend eine therapeutisch wirksame Menge einer Verbindung nach Anspruch 1 oder eines pharmazeutisch annehmbaren Salzes davon zusammen mit einem pharmazeutisch annehmbaren Träger.

**17.** Verwendung der Verbindung nach Anspruch 1 oder eines pharmazeutisch annehmbaren Salzes davon für die Herstellung eines Medikaments zur Behandlung oder Prophylaxe einer durch CETP vermittelten Störung in einem Menschen.

**18.** Verwendung nach Anspruch 17, wobei die Behandlung der durch CETP vermittelten Störung eine Erkrankung der Coronararterien ist.

**19.** Verwendung nach Anspruch 17, wobei die Prophylaxe der durch CETP vermittelten Störung eine Erkrankung der Coronararterien ist.

**20.** Verwendung nach Anspruch 17, wobei die Prophylaxe einer durch CETP vermittelten Störung die Prophylaxe eines Schlaganfalls (CVA; "cerebral vascular accident") ist.

**21.** Verwendung nach Anspruch 17, wobei die Behandlung der durch CETP vermittelten Störung die Behandlung einer

Dyslipidämie ist.

**22.** Verwendung nach Anspruch 17, wobei die Prophylaxe der durch CETP vermittelten Störung die Behandlung einer Dyslipidämie ist.

## Revendications

**1.** Composé de formule M :

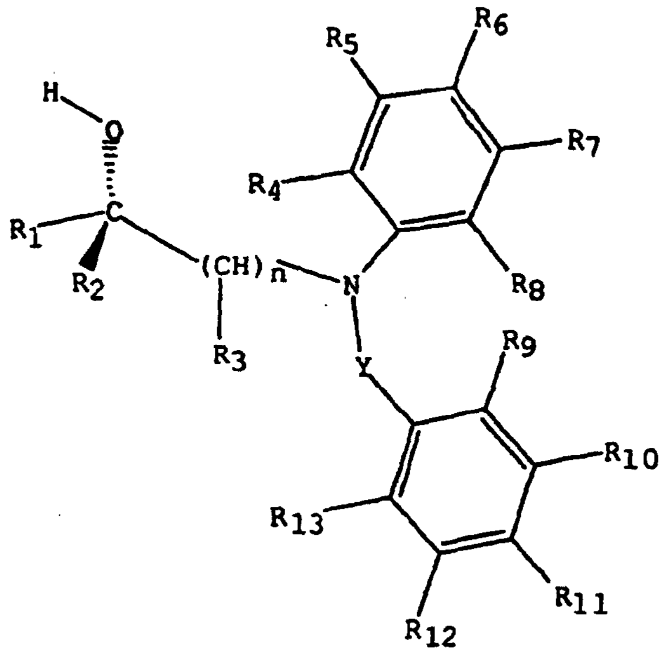

(M).

ou un sel pharmaceutiquement acceptable de celui-ci, dans laquelle ;
n est choisi parmi les nombres entiers 1, 2, 3 et 4 ;
Y est -$(CH_2)_q$- dans lequel q vaut 1 ou 2 ;
$R_1$ est un groupe halogénoalkyle ;
$R_2$ est un atome d'hydrogène ;
$R_3$ est un atome d'hydrogène ;
$R_4$, $R_8$, $R_9$ et $R_{13}$ sont indépendamment choisis dans le groupe constitué des groupes hydrogène, halogène, halogénoalkyle et alkyle ;
$R_5$, $R_6$, $R_7$, $R_{10}$, $R_{11}$ et $R_{12}$ sont indépendamment choisis dans le groupe constitué des groupes hydrogène, carboxy, hétéroaralkylthio, hétéroaralcoxy, cycloalkylamino, acylalkyle, acylalcoxy, aroylalcoxy, hétérocyclyloxy, aralkylaryle, aralkyle, aralcényle, aralcynyle, hétérocyclyle, perhalogénoaralkyle, aralkylsulfonyle, aralkylsulfonylalkyle, aralkylsulfinyle, aralkylsulfinylalkyle, halogénocycloalkyle, halogénocycloalcényle, cycloalkylsulfinyle, cycloalkylsulfinylalkyle, cycloalkylsulfonyle, cycloalkylsufonylalkyle, hétéroarylamino, N-hétéroarylamino-N-alkylamino, hétéroarylaminoalkyle, halogénoalkylthio, alcanoyloxy, alcoxy, alcoxyalkyle, hétéroaralcoxy, cycloalcoxy, cycloalcényloxy, cycloalcoxyalkyle, cycloalkylalcoxy, cycloalcényloxyalkyle, halogénocycloalcoxy, halogénocycloalcoxyalkyle, halogénocycloalcényloxy, halogénocycloalcényloxyalkyle, hydroxy, amino, thio, nitro, (alkyle en $C_1$-$C_6$) amino, alkylthio, alkylthioalkyle, arylamino, aralkylamino, arylthio, arylthioalkyle, hétéroaralcoxyalkyle, alkylsulfinyle, alkylsulfinylalkyle, arylsulfinylalkyle, arylsulfonylalkyle, hétéroarylsulfinylalkyle, hétéroarylsulfonylalkyle, alkylsulfonyle, alkylsulfonylalkyle, halogénosulfinylalkyle, halogénoalkylsulfonylalkyle, alkylsulfonamido, alkylaminosulfonyle, amidosulfonyle, alkylamidosulfonyle, arylamidosulfonyle, arylsulfonamido, alkylarylamidosulfonyle, arylsulfinyle, arylsulfonyle, hétéroarylthio, hétéroarylsulfinyle, hétéroarylsulfonyle, hétérocyclylsulfonyle, hétérocyclylthio, alcanoyle, alcénoyle, aroyle, hétéroaroyle, aralcanoyle, hétéroaralcanoyle, halogénoalcanoyle, alkyle, alcényle, alcynylé, alcényloxy, alcényloxyalkyle, cycloalkyle, cycloalkylalcanoyle, cycloalcényle, (cycloalkyle en $C_3$-$C_7$) alkyle, (cycloalcényle en $C_3$-$C_7$)alkyle, halogène, halogénoalkyle, halogénoalcényle, halogénoalcoxy, hydroxyhalogénoalkyle, hydroxyaralkyle, hydroxyalkyle, hydroxyhétéroaralkyle, halogénoalcoxyalkyle, aryle, hétéroaralcynyle, aryloxy, aralcoxy, aryloxyalkyle, hétérocyclyle saturé, hétérocyclyle partiellement saturé, hétéroaryle, hétéroaryloxy, hétéroaryloxyalkyle, hétéroaralkyle, arylalcényle, hétéroarylalcényle, carboxyalkyle, carboalcoxy, alcoxycarboxamido, alkylamidocarbonylamido, arylamidocarbonylamido, carboalcoxyalkyle, carboalcoxyalcényle, carboaralcoxy, carboxamido, carboxamidoalkyle, cyano, carbohalogénoalcoxy, phosphono, phosphonoalkyle, diaralcoxyphosphono et diaralcoxyphosphonoalkyle ;

en outre, dans laquelle lorsque $R_7$ est un groupe aryloxy ou aralcoxy, ledit groupe aryloxy ou aralcoxy peut être substitué en une ou plusieurs positions substituables par un ou plusieurs radicaux choisis parmi les groupes amino, halogène, nitro, alcoxy, alkyle, cyano, cycloalcoxy, cycloalkyle, cycloalkylalcoxy, halogénoalcoxy, (alkyle en $C_1$-$C_6$)amino, halogénoalkyle, alcanoyle, halogénoalkylthio, perhalogénoaralkyle, aralkylsulforiyle, aralkylsulfonylalkyle, halogénocycloalkyle, halogénocycloalcényle, halogénoalkylthio, alcanoyloxy, alcoxy, alcoxyalkyle, halogénoalcoxyalkyle, cycloalcoxy, cycloalcoxyalkyle, cycloalkylalcoxy, hydroxy, thio, alkylthio, alkylthioalkyle, arylamino, aralkylamino, arylthio, arylthioalkyle, alkylsulfonyle, alkylsulfonylalkyle, halogénoalkylsulfonylalkyle, alkylsulfonamido, alkylaminosulfonyle, amidosulfonyle, monoalkylamidosulfonyle, dialkylamidosulfonyle, arylsulfonyle, alcénoyle, aroyle, aralcanoyle, halogénoalcanoyle, alcényle, alcynyle, alcényloxy, alcényloxyalkyle, cycloalkylalcanoyle, (cycloalkyle en $C_3$-$C_7$) alkyle, halogénoalcényle, hydroxyhalogénoalkyle, hydroxyaralkyle, hydroxyalkyle, halogénoalcoxyalkyle, aryle, aralkyle, aryloxy, aralcoxy, aryloxyalkyle, carboxyalkyle, carboalcoxy, alcoxycarbonyle, carboaralcoxy, carboxamide, carboxamidoalkyle, carbohalogénoalcoxy, phosphono, phosphonoalkyle, diaralcoxyphosphono etdiaralcoxyphosphonoalkyle,

à condition qu'au moins un des groupes $R_4$, $R_5$, $R_6$, $R_7$ et $R_8$ ne soit pas un atome d'hydrogène,

et à une autre condition qu'au moins un des groupes $R_9$, $R_{10}$, $R_{11}$, $R_{12}$ et $R_{13}$ ne soit pas un atome d'hydrogène.

**2.** Composé selon la revendication 1 de formule M :

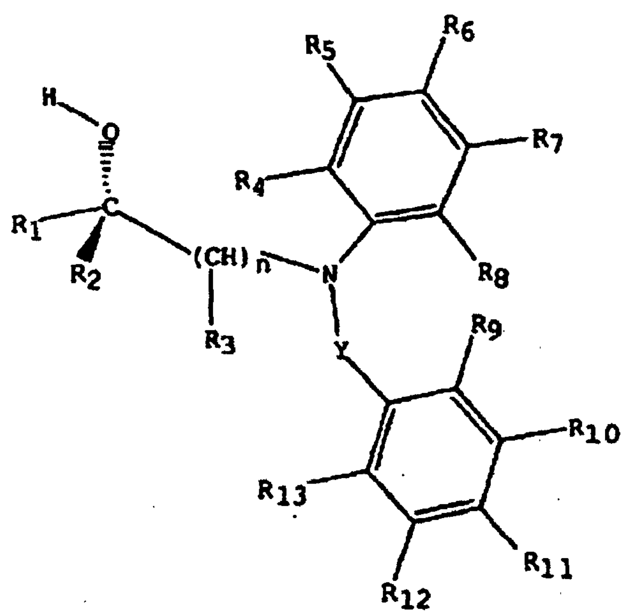

**(M)**

ou un sel pharmaceutiquement acceptable de celui-ci, dans laquelle ;
n est un nombre entier valant de 1, 2 à 3 ;
Y est $-(CH_2)_q-$ dans lequel q vaut 1 ou 2 ;
$R_1$ est un groupe halogénoalkyle ;
$R_2$ est un atome d'hydrogène ;
$R_3$ est un atome d'hydrogène ;

$R_4$, $R_8$, $R_9$ et $R_{13}$ sont indépendamment choisis dans le groupe constitué des groupes hydrogène, halogène et halogénoalkyle ;

$R_5$, $R_6$, $R_7$, $R_{10}$, $R_{11}$ et $R_{12}$ sont indépendamment choisis dans le groupe constitué des groupes hydrogène, hétéroaralcoxy, acylalkyle, acylalcoxy, aroylalcoxy, hétérocyclyloxy, aralkylaryle, aralkyle, perhalogénoaralkyle, aralkylsulfonyle, aralkylsulfonylalkyle, halogénocycloalkyle, cycloalkylsulfonyle, cycloalkylsufonylalkyle, halogénoalkylthio, alcanoyloxy, alcoxy, alcoxyalkyle, hétéroaralcoxy, cycloalcoxy, cycloalcoxyalkyle, cycloalkylalcoxy, halogénocycloalcoxy, halogénocycloalcoxyalkyle, hydroxy, amino, nitro, (alkyle en $C_1$-$C_6$)amino, alkylthio, alkylthioalkyle, arylamino, aralkylamino, arylthio, arylsulfonylalkyle, alkylsulfonyle, alkylsulfonylalkyle, halogénoalkylsulfonylalkyle, alkylsulfonamido, alkylaminosulfonyle, amidosulfonyle, alkylamidosulfonyle, arylsulfonyle, alcanoyle, aroyle, aralcanoyle, hétéroaralcanoyle, halogénoalcanoyle, alkyle, cycloalkyle, cycloalkylalcanoyle, (cycloalkyle en $C_3$-$C_7$)alkyle, halogène, halogénoalkyle, halogénoalcoxy, hydroxyhalogénoalkyle, hydroxyaralkyle, hydroxyalkyle, halogénoalcoxyalkyle, aryle, aryloxy, aralcoxy, aryloxyalkyle, hétéroaryloxy, hétéroaryloxyalkyle, carboxyalkyle, carboalcoxy, alcoxycarboxamido, carboalcoxyalkyle, carboaralcoxy, carboxamido, carboxamidoalkyle, cyano, carbohalogénoalcoxy, phosphono, phosphonoalkyle, diaralcoxyphosphono et diaralcoxyphosphonoalkyle ;

en outre, dans laquelle lorsque $R_7$ est un groupe aryloxy ou aralcoxy, ledit groupe aryloxy ou aralcoxy peut être substitué en une ou plusieurs positions substituables par un ou plusieurs radicaux choisis parmi les groupes amino, halogène, nitro, alcoxy, alkyle, cyano, cycloalcoxy, cycloalkyle, cycloalkylalcoxy, halogénoalcoxy, (alkyle en $C_1$-$C_6$)amino, halogénoalkyle, alcanoyle et halogénoalkylthio ;

à condition qu'au moins un des groupes $R_4$, $R_5$, $R_6$, $R_7$ et $R_8$ ne soit pas un atome d'hydrogène,

et à une autre condition qu'au moins un des groupes $R_9$, $R_{10}$, $R_{11}$, $R_{12}$ et $R_{13}$ ne soit pas un atome d'hydrogène.

**3.** Composé selon la revendication 2 de formule M :

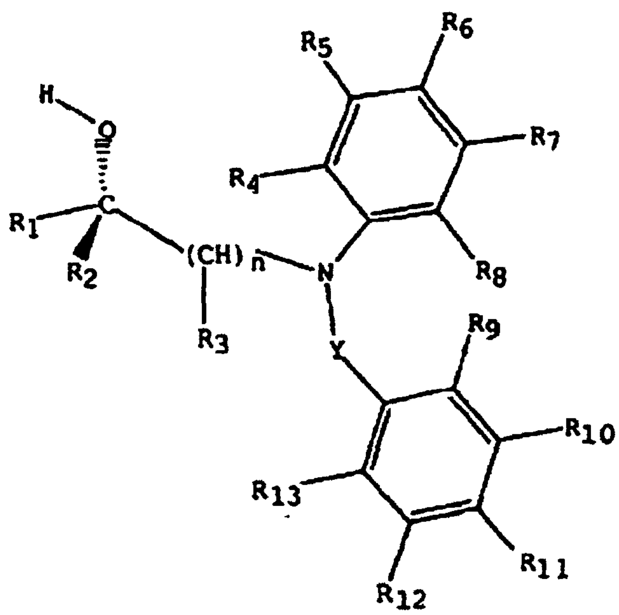

(M)

ou un sel pharmaceutiquement acceptable de celui-ci, dans laquelle ;

n est un nombre entier valant de 1, 2 à 3 ;

Y est $-(CH_2)_q-$ dans lequel q vaut 1 ou 2 ;

$R_1$ est un groupe halogénoalkyle ;

$R_2$ est un atome d'hydrogène ;

$R_3$ est un atome d'hydrogène ;

$R_4$, $R_8$, $R_9$ et $R_{13}$ sont indépendamment choisis dans le groupe constitué des groupes hydrogène, halogène et halogénoalkyle ;

$R_5$, $R_6$, $R_7$, $R_{10}$, $R_{11}$ et $R_{12}$ sont indépendamment choisis dans le groupe constitué des groupes hydrogène, hétéroaralcoxy, acylalkyle, acylalcoxy, aroylalcoxy, hétérocyclyloxy, aralkylaryle, aralkyle, perhalogénoaralkyle,

halogénoalkylthio, alcanoyloxy, alcoxy, alcoxyalkyle, hétéroaralcoxy, cycloalcoxy, cycloalcoxyalkyle, cycloalkylalcoxy, halogénocycloalcoxy, halogénocycloalcoxyalkyle, alkylthio, alkylthioalkyle, arylthio, alkylsulfonyle, alkylsulfonylalkyle, halogénoalkylsulfonylalkyle, alkylsulfonamido, alkylaminosulfonyle, amidosulfonyle, alkylamidosulfonyle, arylsulfonyle, alcanoyle, halogénoalcanoyle, alkyle, cycloalkyle, (cycloalkyle en $C_3$-$C_7$)alkyle, halogène, halogénoalkyle, halogénoalcoxy, halogénoalcoxyalkyle, aryle, aryloxy, aralcoxy, aryloxyalkyle, hétéroaryloxy, hétéroaryloxyalkyle, carboalcoxy, carboalcoxyalkyle, carboaralcoxy et carbohalogénoalcoxy ;

en outre, dans laquelle lorsque $R_7$ est un groupe aryloxy ou aralcoxy, ledit groupe aryloxy ou aralcoxy peut être substitué en une ou plusieurs positions substituables par un ou plusieurs radicaux choisis parmi les groupes amino, halogène, nitro, alcoxy, alkyle, cycloalcoxy, cycloalkyle, cycloalkylalcoxy, halogénoalcoxy, (alkyle en $C_1$-$C_6$) amino, halogénoalkyle, alcanoyle et halogénoalkylthio.

**4.** Composé selon la revendication 3 de formule « D »

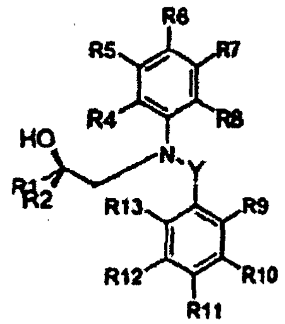

Formule « D »

dans laquelle Y est $-CH_2-$ ;
dans laquelle $R^1$ est un groupe halogénoalkyle ;
dans laquelle $R^2$ est un atome d'hydrogène ;
dans laquelle $R^4$ et $R^8$ sont des atomes d'hydrogène ;
dans laquelle $R^5$ et $R^6$ sont choisis parmi les groupes hydrogène et alcoxy ;
dans laquelle $R^7$ est choisi parmi les groupes aryloxy, arylalcoxy, 5,6,7,8-tétrahydronapht-2-yloxy, alcoxy, cycloalcoxy, cycloalkylalcoxy et halogène ;
dans laquelle lesdits groupes aryloxy et arylalcoxy de $R^7$ peuvent être substitués en une ou plusieurs positions substituables par un ou plusieurs radicaux choisis parmi les groupes halogène, alkyle, alcoxy, halogénoalcoxy et halogénoalkyle ;
dans laquelle $R^9$ est choisi parmi les groupes hydrogène, halogène et halogénoalkyle ;
dans laquelle $R^{10}$ est choisi parmi les groupes halogénoalcoxy, halogénoalkyle et halogénoalkylthio ;
dans laquelle $R^{11}$ est choisi parmi les groupes hydrogène, halogène et halogénoalkyle ;
dans laquelle $R^{12}$ est choisi parmi les groupes hydrogène et halogénoalkyle ;
dans laquelle $R^{13}$ est choisi parmi les groupes hydrogène, halogène et halogénoalkyle.

**5.** Composé selon la revendication 4 de formule « C »

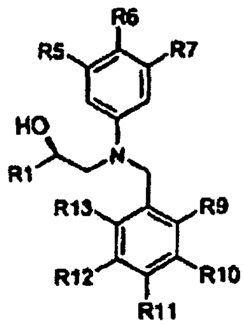

Formule « C »

dans laquelle $R^1$ est choisi parmi les groupes trifluorométhyle et chlorométhyle ;
dans laquelle $R^5$ et $R^6$ sont choisis parmi les groupes hydrogène et méthoxy ;
dans laquelle $R^7$ est choisi parmi les groupes phényloxy, benzyloxy, 5,6,7,8-tétrahydronapht-2-yloxy, isopropoxy, cyclopentoxy, brome, cyclohexylméthyle et méthoxy ;
dans laquelle lesdits groupes phényloxy et benzyloxy de $R^7$ peuvent être substitués en une ou plusieurs positions substituables par un ou plusieurs radicaux choisis parmi les groupes chlore, éthyle, trifluorométhoxy, brome, fluor, méthyle, isopropyle, trifluorométhyle, isopropoxy et tert-butyle ;
dans laquelle $R^9$ est choisi parmi les groupes hydrogène, fluor et trifluorométhyle ;
dans laquelle $R^{10}$ est choisi parmi les groupes 1,1,2,2-tétrafluoroéthoxy, trifluorométhoxy, pentafluoroéthyle, trifluorométhyle et trifluorométhylthio ;
dans laquelle $R^{11}$ est choisi parmi les groupes hydrogène, trifluorométhyle et fluor ;
dans laquelle $R^{12}$ est choisi parmi les groupes hydrogène et trifluorométhyle ;
dans laquelle $R^{13}$ est choisi parmi les groupes hydrogène, fluor et trifluorométhyle.

**6.** Composé selon la revendication 5, ou un sel pharmaceutiquement acceptable de celui-ci, dans lequel ledit composé est choisi dans le groupe constitué de :

(2R)-3-[[3-(3-trifluorométhoxyphénoxy)phényl][[3-(1,1,2,2-tétrafluoroéthoxy)-phényl]méthyl]amino]-1,1,1-trifluoro-2-propanol ;
(2R)-3-[[3-(3-isopropylphénoxy)phényl][[3-(1,1,2,2-tétrafluoroéthoxy)-phényl]méthyl]amino]-1,1,1-trifluoro-2-propanol ;
(2R)-3-[[3-(4-méthylphénoxy)phényl][[3-(1,1,2,2-tétrafluoroéthoxy)-phényl]méthyl]amino]-1,1,1-trifluoro-2-propanol ;
(2R)-3-[[3-(2-bromo-5-fluorophénoxy)phényl][[3-(1,1,2,2-tétrafluoroéthoxy)-phényl]mêthyl]amino]-1,1,1-trifluoro-2-propanol ;
(2R)-3-[[3-(4-chloro-3-éthylphénoxy)phényl][[3-(1,1,2,2-tétrafluoroéthoxy)-phényl]méthyl]amino]-1,1,1-trifluoro-2-propanol ;
(2R)-3-[[3-(3-éthylphénoxy)phényl][[3-(1,1,2,2-tétrafluoroéthoxy)-phényl]méthyl]amino]-1,1,1-trifluoro-2-propanol ;
(2R)-3-[[3-(phénoxy)phényl][[3-(1,1,2,2-tétrafluoroéthoxy)-phényl]méthyl]amino]-1,1,1-trifluoro-2-propanol ;
(2R)-3-[[[3-(1,1,2,2-tétrafluoroéthoxy)phényl]méthyl][3-[[3-(trifluorométhoxy)-phényl]méthoxy]phényl]amino]-1,1,1-trifluoro-2-propanol ;
(2R)-3-[[[3-(1,1,2,2-tétrafluoroéthoxy)phényl]méthyl][3-[[3-(trifluorométhyl)-phényl]méthoxy]phényl]amino]-1,1,1-trifluoro-2-propanol ;
(2R)-3-[[[3-(1,1,2,2-tétrafluoroéthoxy)phényl]méthyl][3-[[3,5-difluorophényl]méthoxy]phényl]amino]-1,1,1-trifluoro-2-propanol ;
(2R)-3-[[3-isopropoxyphényl][[3-(1,1,2,2-tétrafluoroéthoxy)-phényl]méthyl]amino]-1,1,1-trifluoro-2-propanol ;
(2R)-3-[[3-(4-chloro-3-éthylphénoxy)phényl][[3-(pentafluoroéthyl)-phényl]méthyl]amino]-1,1,1-trifluoro-2-propanol ;

(2R)-3-[[3-(4-chloro-3-éthylphénoxy)phényl][[2-fluoro-5-(trifluorométhyl)-phényl]méthyl]amino]-1,1,1-trifluoro-2-propanol ;
(2R)-3-[[3-(4-chloro-3-éthylphénoxy)phényl][[2-fluoro-4-(trifluorométhyl)-phényl]méthyl]amino]-1,1,1-trifluoro-2-propanol ;
(2R)-3-[[3-(4-chloro-3-éthylphénoxy)phényl][[3-(trifluorométhoxy)-phényl]méthyl]amino]-1,1,1-trifluoro-2-propanol ;
(2R)-3-[[3-(3-(trifluorométhoxy)phénoxy)phényl][[3-(trifluorométhoxy)-phényl]méthyl]amino]-1,1,1-trifluoro-2-propanol ;
(2R)-3-[[3-(4-méthylphénoxy)phényl][[3-(trifluorométhoxy)-phényl]méthyl]amino]-1,1,1-trifluoro-2-propanol.

**7.** Composé selon la revendication 6, ou un sel pharmaceutiquement acceptable de celui-ci, dans lequel ledit composé est le (2R)-3-[[3-(4-chloro-3-éthylphénoxy)phényl][[3-(1,1,2,2-tétrafluoroéthoxy)-phényl]méthyl]amino]-1,1,1-trifluoro-2-propanol.

**8.** Composé selon la revendication 6, ou un sel pharmaceutiquement acceptable de celui-ci, dans lequel ledit composé est le (2R)-3-[[(3-phénoxyphényl][[3-(1,1,2,2-tétrafluoroéthoxy)-phényl]méthyl]amino]-1,1,1-trifluoro-2-propanol.

**9.** Composé selon la revendication 6, ou un sel pharmaceutiquement acceptable de celui-ci, dans lequel ledit composé est le (2R) -3-[[3-(4-méthylphénoxy)phényl][ [3-(1,1,2,2-tétrafluoroéthoxy)-phényl]méthyl]amino]-1,1,1-trifluoro-2-propanol.

**10.** Composé selon la revendication 6, ou un sel pharmaceutiquement acceptable de celui-ci, dans lequel ledit composé est le (2R)-3-[[3-(3-isopropylphénoxy)phényl][[3-(1,1,2,2-tétrafluoroéthoxy)-phényl]méthyl]amino]-1,1,1-trifluoro-2-propanol.

**11.** Composé selon la revendication 6, ou un sel pharmaceutiquement acceptable de celui-ci, dans lequel ledit composé est le (2R)-3-[[3-(3-éthylphénoxy)phényl][[3-(1,1,2,2-tétrafluoroéthoxy)-phényl]méthyl]amino]-1,1,1-trifluoro-2-propanol.

**12.** Composé selon la revendication 6, ou un sel pharmaceutiquement acceptable de celui-ci, dans lequel ledit composé est le (2R)-3-[[3-(3-trifluorométhoxyphénoxy)-phényl][[3-(1,1,2,2-tétrafluoroéthoxy)-phényl]méthyl]amino]-1,1,1-trifluoro-2-propanol.

**13.** Composé selon la revendication 6, ou un sel pharmaceutiquement acceptable de celui-ci, dans lequel ledit composé est le (2R)-3-[[3-(4-chloro-3-éthylphénoxy)phényl][[3-(trifluorométhoxy)-phényl]méthyl]amino]-1,1,1-trifluoro-2-propanol.

**14.** Composé selon la revendication 6, ou un sel pharmaceutiquement acceptable de celui-ci, dans lequel ledit composé est le (2R)-3-[[[3-(1,1,2,2-tétrafluoroéthoxy)phényl]-méthyl][3-[[3-(trifluorométhyl)-phényl]méthoxy]phényl]amino]-1,1,1-trifluoro-2-propanol.

**15.** Composé selon 1a revendication 6, ou un sel pharmaceutiquement acceptable de celui-ci, dans lequel ledit composé est le (2R)-3-[[[3-(1,1,2,2-tétrafluoroéthoxy)phényl]-méthyl][3-[[3-(trifluorométhoxy)-phényl]méthoxy]phényl]amino]-1,1,1-trifluoro-2-propanol.

**16.** Composition pharmaceutique comprenant une quantité thérapeutiquement efficace d'un composé selon la revendication 1 ou d'un sel pharmaceutiquement acceptable de celui-ci, avec un vecteur pharmaceutiquement acceptable.

**17.** Utilisation d'un composé selon la revendication 1 ou d'un sel pharmaceutiquement acceptable de celui-ci pour la fabrication d'un médicament destiné au traitement ou à la prévention d'un trouble médié par la CETP chez un sujet.

**18.** Utilisation selon la revendication 17, dans laquelle le traitement d'un trouble médié par la CETP est le traitement d'une maladie coronarienne.

**19.** Utilisation selon la revendication 17, dans laquelle la prévention d'un trouble médié par la CETP est la prévention d'une maladie coronarienne.

**20.** Utilisation selon la revendication 17, dans laquelle la prévention d'un trouble médié par la CETP est la prévention d'un accident vasculaire cérébral (AVC).

**21.** Utilisation selon la revendication 17, dans laquelle le traitement d'un trouble médié par la CETP est le traitement d'une dyslipidémie.

**22.** Utilisation selon la revendication 17, dans laquelle la prévention d'un trouble médié par la CETP est la prévention d'une dyslipidémie.